# EUROPÄISCHE PATENTSCHRIFT

(11) **EP 3 107 920 B1**
(45) Veröffentlichungstag und Bekanntmachung des Hinweises auf die Patenterteilung: **18.10.2017**
(21) Anmeldenummer: 15706404.9
(22) Anmeldetag: 17.02.2015
(51) Int. Cl.: C07D 519/00, A61K 31/519, A61P 9/00

(54) **3-(PYRIMIDIN-2-YL)IMIDAZO[1,2-A]PYRIDINE**
3-(PYRIMIDINE-2-YL)IMIDAZO[1,2-A]PYRIDINES
3-(PYRIMIDIN-2-YL)IMIDAZO[1,2-A]PYRIDINES

(30) Priorität: 19.02.2014 EP 14155731
(43) Veröffentlichungstag der Anmeldung: 28.12.2016
(73) Patentinhaber: Bayer Pharma Aktiengesellschaft, 13353 Berlin (DE)
(72) Erfinder: VAKALOPOULOS, Alexandros, 40721 Hilden (DE); GROMOV, Alexey, 40699 Erkrath (DE); FOLLMANN, Markus, 50859 Köln (DE); BROCKSCHNIEDER, Damian, 42781 Haan (DE); STASCH, Johannes-Peter, 00046 Grottaferrata (IT); MARQUARDT, Tobias, 42115 Wuppertal (DE); TERSTEEGEN, Adrian, 42111 Wuppertal (DE); WUNDER, Frank, 42117 Wuppertal (DE); REDLICH, Gorden, 44799 Bochum (DE); LANG, Dieter, 42553 Velbert (DE); LI, Volkhart Min-Jian, 42553 Velbert (DE)
(74) Vertreter: BIP Patents
(86) Internationale Anmeldenummer: PCT/EP2015/053252
(87) Internationale Veröffentlichungsnummer: WO 2015/124544

(56) Entgegenhaltungen:
- WO-A1-2012/165399
- WO-A1-2013/030288
- WO-A1-2013/104703

## Beschreibung

Die vorliegende Anmeldung betrifft neue 3-(Pyrimidin-2-yl)imidazo[1,2-a]pyridine, Verfahren zu ihrer Herstellung, ihre Verwendung allein oder in Kombinationen zur Behandlung und/oder Prophylaxe von Krankheiten sowie ihre Verwendung zur Herstellung von Arzneimitteln zur Behandlung und/oder Prophylaxe von Krankheiten, insbesondere zur Behandlung und/oder Prophylaxe von kardiovaskulären Erkrankungen.

Eines der wichtigsten zellulären Übertragungssysteme in Säugerzellen ist das cyclische Guanosinmonophosphat (cGMP). Zusammen mit Stickstoffmonoxid (NO), das aus dem Endothel freigesetzt wird und hormonelle und mechanische Signale überträgt, bildet es das NO/cGMP-System. Die Guanylatcyclasen katalysieren die Biosynthese von cGMP aus Guanosintriphosphat (GTP). Die bisher bekannten Vertreter dieser Familie lassen sich sowohl nach strukturellen Merkmalen als auch nach der Art der Liganden in zwei Gruppen aufteilen: Die partikulären, durch natriuretische Peptide stimulierbaren Guanylatcyclasen und die löslichen, durch NO stimulierbaren Guanylatcyclasen. Die löslichen Guanylatcyclasen bestehen aus zwei Untereinheiten und enthalten höchstwahrscheinlich ein Häm pro Heterodimer, das ein Teil des regulatorischen Zentrums ist. Dieses hat eine zentrale Bedeutung für den Aktivierungsmechanismus. NO kann an das Eisenatom des Häms binden und so die Aktivität des Enzyms deutlich erhöhen. Hämfreie Präparationen lassen sich hingegen nicht durch NO stimulieren. Auch Kohlenmonoxid (CO) ist in der Lage, an das Eisen-Zentralatom des Häms zu binden, wobei die Stimulierung durch CO deutlich geringer ist als die durch NO.

Durch die Bildung von cGMP und der daraus resultierenden Regulation von Phosphodiesterasen (PDE), Ionenkanälen und Proteinkinasen spielt die Guanylatcyclase eine entscheidende Rolle bei unterschiedlichen physiologischen Prozessen, insbesondere bei der Relaxation und Proliferation glatter Muskelzellen, der Plättchenaggregation und -adhäsion, der neuronalen Signalübertragung sowie bei Erkrankungen, welche auf einer Störung der vorstehend genannten Vorgänge beruhen. Unter pathophysiologischen Bedingungen kann das NO/cGMP-System supprimiert sein, was zum Beispiel zu Bluthochdruck, einer Plättchenaktivierung, einer vermehrten Zellproliferation, endothelialer Dysfunktion, Atherosklerose, Angina pectoris, Herzinsuffizienz, Myokardinfarkt, Thrombosen, Schlaganfall und sexueller Dysfunktion führen kann.

Eine auf die Beeinflussung des cGMP-Signalweges in Organismen abzielende NO-unabhängige Behandlungsmöglichkeit für derartige Erkrankungen ist aufgrund der zu erwartenden hohen Effizienz und geringen Nebenwirkungen ein vielversprechender Ansatz.

Zur therapeutischen Stimulation der löslichen Guanylatcyclase wurden bisher ausschließlich Verbindungen wie organische Nitrate verwendet, deren Wirkung auf NO beruht. Dieses wird durch Biokonversion gebildet und aktiviert die lösliche Guanylatcyclase durch Angriff am Eisen-Zentralatom des Häms. Neben den Nebenwirkungen gehört die Toleranzentwicklung zu den entscheidenden Nachteilen dieser Behandlungsweise.

Vor einigen Jahren wurden einige Substanzen beschrieben, die die lösliche Guanylatcyclase direkt, d.h. ohne vorherige Freisetzung von NO stimulieren, wie beispielsweise 3-(5'-Hydroxymethyl-2'-furyl)-1-benzylindazol [YC-1; Wu et al., Blood 84 (1994), 4226; Mülsch et al., Brit. J. Pharmacol. 120 (1997), 681]. Zu den neueren Stimulatoren der löslichen Guanylatzyklase gehören u.a. BAY 41-2272, BAY 41-8543 und Riociguat (BAY 63-2521) (siehe z.B. Stasch J.-P. et al., Nat. Rev. Drug Disc. 2006; 5: 755-768; Stasch J.-P. et al., ChemMedChem 2009; 4: 853-865. Stasch J.-P. et al., Circulation 2011; 123: 2263-2273). Interessanterweise zeigen einige dieser sGC Stimulatoren, wie beispielsweise YC-1 oder BAY 41-2272 zusätzlich zur direkten Guanylatzyklasestimulation auch eine PDE 5-inhibibitorische Wirkung. Um den cGMP-pathway zu maximieren, ist es pharmakologisch wünschenswert, die Synthese von cGMP zu stimulieren und gleichzeitig den Abbau über PDE 5 zu inhibieren. Dieses duale Prinzip ist pharmakologisch besonders vorteilhaft (s. z.B. Oudout et al., Eur. Urol. 2011, 60, 1020-1026; Albersen et al., J Sex Med. 2013; 10, 1268-1277).

Das duale Prinzip wird im Sinne der vorliegenden Erfindung erfüllt, wenn die erfindungsgemäßen Verbindungen eine Wirkung an rekombinanter Guanylatcyclase-Reporterzellinien gemäß der Untersuchung unter B-2 als minimal effective concentration (MEC) von ≤ 3 µM zeigen und eine Inhibition der humanen Phosphodiesterase 5 (PDE 5) gemäß der Untersuchung unter B-3 als IC₅₀ < 100 nM zeigen.

Phosphodiesterase-5 (PDE 5) ist der Name für eines der Enzyme, die die Phosphorsäureesterbindung in cGMP spalten, wobei 5'-Guanosinmonophosphat (5'-GMP) entsteht. Beim Menschen kommt die Phosphodiesterase-5 z.B. in der glatten Muskulatur des Penisschwellkörpers (Corpus cavernosum penis) und der Lungenarterien vor. Blockierung des cGMP-Abbaus durch Hemmung von PDE 5 (mit beispielsweise Sildenafil, Vardenafil oder Tadalafil) führt zu vermehrten Signalen der Entspannungs-Signalwege und speziell zu erhöhter Blutzufuhr in den Penisschwellkörper und Druckerniedrigung in den Blutgefäßen der Lunge. Sie werden zur Behandlung der erektilen Dysfunktion und der pulmonalen arteriellen Hypertonie eingesetzt. Neben PDE 5 gibt es weitere, ausschließlich cGMP spaltende Phosphodiesterasen (Stasch J.-P. et al. Circulation 2011; 123, 2263-2273).

Als Stimulatoren der löslichen Guanylatcyclase werden in WO 03/095451 Carbamat-substitutierte 3-Pyrimidinyl-Pyrazolopyridine offenbart. WO 2010/065275 und WO 2011/149921 offenbaren substituierte Pyrrolo- und Dihydropyridopyrimidine als sGC Aktivatoren. Als sGC Stimulatoren werden in WO 2012/004259 annellierte Aminopyrimidine und in WO 2012/004258, WO 2012/143510, WO 2012/152629 und WO 2013/030288 annellierte Pyrimidine und Triazine beschrieben. Unter anderem in EP 0 266 890-A1, WO 89/03833-A1, JP 01258674-A [vgl. Chem. Abstr. 112:178986], WO 96/34866-A1, EP 1 277 754-A1, WO 2006/015737-A1, WO 2008/008539-A2, WO 2008/082490-A2, WO 2008/134553-A1, WO 2010/030538-A2, WO 2011/113606-A1 und WO 2012/165399-A1 sind verschiedene Imidazo[1,2-a]pyridin-Derivate beschrieben, die zur Behandlung von Erkrankungen verwendet werden können.

Aufgabe der vorliegenden Erfindung war die Bereitstellung neuer Substanzen, die als Stimulatoren der löslichen Guanylatcyclase sowie als Stimulatoren der löslichen Guanylatcyclase und Phosphodiesterase-5-Inhibitoren (duales Prinzip) wirken und ein gleiches oder verbessertes therapeutisches Profil gegenüber den aus dem Stand der Technik bekannten Verbindungen aufweisen, wie beispielsweise hinsichtlich ihrer *in-vivo* Eigenschaften, wie beispielsweise ihres pharmakokinetischen und pharmakodynamischen Verhaltens und/oder ihres Metabolismus-Profils und/oder ihrer Dosis-Wirkungsbeziehung.

Gegenstand der vorliegenden Erfindung sind Verbindungen der allgemeinen Formel (I) 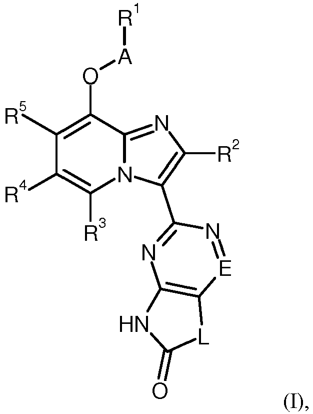 in welcher
- A: für CH₂, CD₂ oder CH(CH₃) steht,
- R¹: für (C₄-C₆)-Alkyl, (C₃-C₇)-Cycloalkyl oder Phenyl steht,
wobei (C₄-C₆)-Alkyl mit 1 oder 2 Substituenten unabhängig voneinander ausgewählt aus der Gruppe Fluor und Trifluormethyl substituiert sein kann,
wobei (C₃-C₇)-Cycloalkyl mit 1 bis 4 Substituenten unabhängig voneinander ausgewählt aus der Gruppe Fluor, Trifluormethyl und (C₁-C₄)-Alkyl substituiert sein kann,
und
wobei Phenyl mit 1 bis 4 Substituenten unabhängig voneinander ausgewählt aus der Gruppe Halogen, Cyano, Monofluormethyl, Difluormethyl, Trifluormethyl, (C₁-C₄)-Alkyl, (C₃-C₆)-Cycloalkyl, (C₁-C₄)-Alkoxy, Difluormethoxy und Trifluormethoxy substituiert sein kann,
- R²: für Wasserstoff, (C₁-C₄)-Alkyl, (C₁-C₄)-Alkoxy-(C₁-C₄)-Alkyl, Cyclopropyl, Monofluormethyl, Difluormethyl oder Trifluormethyl steht,
- R³: für Wasserstoff steht,
- R⁴: für Wasserstoff, Halogen, Cyano, Monofluormethyl, Difluormethyl, Trifluormethyl, (C₁-C₄)-Alkyl, Ethinyl, (C₃-C₇)-Cycloalkyl oder (C₁-C₄)-Alkoxy steht,
- R⁵: für Wasserstoff steht,
- E: für Stickstoff oder CR⁶ steht,
wobei
R⁶ für Wasserstoff, Deuterium, Halogen, Cyano, Difluormethyl, Trifluormethyl, (C₁-C₆)-Alkyl, (C₂-C₆)-Alkinyl, Cyclopropyl, Cyclobutyl, Hydroxy, -OR⁷, -NR⁸R⁹, Hydroxycarbonyl, (C₁-C₄)-Alkoxycarbonyl, -C(=O)-NR¹⁰R¹¹, 5- oder 6-gliedriges Heteroaryl steht,
worin (C₁-C₆)-Alkyl mit 1 bis 3 Substituenten unabhängig voneinander ausgewählt aus der Gruppe Fluor, Difluormethyl, Trifluormethyl, (C₁-C₄)-Alkoxy, Hydroxy, Amino, -N(C=O)R¹², (C₁-C₄)-Alkylsulfonylamino, (C₃-C₆)-Cycloalkylsulfonylamino, Cyclopropyl und Cyclobutyl substituiert sein kann,
worin R¹² für (C₃-C₇)-Cycloalkyl oder (C₁-C₄)-Alkyl steht,
worin (C₁-C₄)-Alkyl mit Trifluormethyl oder Difluormethyl substituiert sein kann, worin (C₂-C₆)-Alkinyl mit 1 oder 2 Substituenten unabhängig voneinander ausgewählt aus der Gruppe Difluormethyl, Trifluormethyl, Hydroxy, Amino, Cyclopropyl und Cyclobutyl substituiert sein kann,
worin 5- oder 6-gliedriges Heteroaryl mit 1 bis 3 Substituenten unabhängig voneinander ausgewählt aus der Gruppe Fluor, Chlor, (C₁-C₄)-Alkyl, Hydroxy, Amino und Cyclopropyl substituiert sein kann,
worin R⁷ für (C₁-C₆)-Alkyl oder -gliedriges Heteroaryl steht,
worin (C₁-C₆)-Alkyl mit Trifluormethyl, (C₁-C₄)-Alkoxy, Hydroxy, Cyclopropyl oder Cyclobutyl substituiert sein kann,
worin R⁸ für Wasserstoff, (C₁-C₆)-Alkyl oder (C₃-C₇)-Cycloalkyl steht,
worin (C₃-C₇)-Cycloalkyl mit 1 bis 3 Substituenten unabhängig voneinander ausgewählt aus der Gruppe (C₁-C₄)-Alkyl, Hydroxy, Amino, Fluor, Trifluormethyl und Difluormethyl substituiert sein kann,
und
worin (C₁-C₆)-Alkyl mit 1 bis 4 Substituenten unabhängig voneinander ausgewählt aus der Gruppe Fluor, (C₁-C₄)-Alkyl, (C₃-C₇)-Cycloalkyl, (C₁-C₄)-Alkoxy, Hydroxy, Amino, Trifluormethyl, Difluormethyl, Monofluormethyl, 5- bis 7-gliedriges Aza-Heterocyclyl und Phenyl substituiert sein kann,
worin Phenyl mit 1 bis 3 Substituenten unabhängig voneinander ausgewählt aus der Gruppe Halogen, Cyano, (C₁-C₄)-Alkyl und (C₁-C₄)-Alkoxy substituiert sein kann, worin 5- bis 7-gliedriges Aza-Heterocyclyl mit 1 bis 4 Substituenten Fluor substituiert sein kann,
   und
worin (C₃-C₇)-Cycloalkyl mit 1 bis 3 Substituenten unabhängig voneinander ausgewählt aus der Gruppe Halogen, (C₁-C₄)-Alkyl und Hydroxy substituiert sein kann,

worin R⁹ für Wasserstoff oder (C₁-C₆)-Alkyl steht,
oder
R⁸ und R⁹ zusammen mit dem Stickstoffatom, an das sie gebunden sind, einen 3- bis 8-gliedrigen Heterocyclus bilden,
worin der 3- bis 8-gliedrige Heterocyclus mit 1 oder 2 Substituenten unabhängig voneinander ausgewählt aus der Gruppe Fluor, (C₁-C₄)-Alkyl, Hydroxycarbonyl, (C₁-C₄)-Alkoxycarbonyl, Hydroxy und Amino substituiert sein kann,
worin (C₁-C₄)-Alkyl mit Hydroxycarbonyl, (C₁-C₄)-Alkoxycarbonyl, Hydroxy oder Amino substituiert sein kann,

worin R¹⁰ für Wasserstoff, (C₁-C₆)-Alkyl oder (C₃-C₇)-Cycloalkyl steht,
worin (C₃-C₇)-Cycloalkyl mit 1 bis 3 Substituenten unabhängig voneinander ausgewählt aus der Gruppe (C₁-C₆)-Alkyl, Hydroxy, Trifluormethyl und Difluormethyl substituiert sein kann,
und
worin (C₁-C₆)-Alkyl mit mit 1 bis 4 Substituenten unabhängig voneinander ausgewählt aus der Gruppe Fluor, (C₃-C₇)-Cycloalkyl (C₁-C₄)-Alkoxy, Hydroxy, Amino, Trifluormethyl und Difluormethyl substituiert sein kann,
worin R¹¹ für Wasserstoff oder (C₁-C₄)-Alkyl steht,
oder
R¹⁰ und R¹¹ zusammen mit dem Stickstoffatom, an das sie gebunden sind, einen 3- bis 7-gliedrigen Heterocyclus bilden,
worin der 3- bis 7-gliedrige Heterocyclus mit 1 bis 3 Substituenten unabhängig voneinander ausgewählt aus der Gruppe Fluor, (C₁-C₄)-Alkyl, Hydroxy und Amino substituiert sein kann,
worin (C₁-C₄)-Alkyl mit Hydroxy substituiert sein kann,

L für eine Gruppe #¹-CR^{13A}R^{13B}-(CR^{14A}R^{14B})ₘ-#² steht,
wobei
#¹ für die Anknüpfstelle an die Carbonylgruppe steht,
#² für die Anknüpfstelle an den Pyrimidin- bzw. Triazinring steht,
m für eine Zahl 0, 1 oder 2 steht"
R^{13A} für Wasserstoff, Trifluormethyl oder (C₁-C₄)-Alkyl steht,
R^{13B} für Wasserstoff, Difluormethyl, Trifluormethyl, (C₁-C₄)-Alkyl oder (C₃-C₇)-Cycloalkyl steht,
worin (C₁-C₆)-Alkyl mit 1 bis 3 Substituenten unabhängig voneinander ausgewählt aus der Gruppe Fluor, Cyano, Trifluormethyl, (C₃-C₇)-Cycloalkyl, Difluormethoxy und Trifluormethoxy substituiert sein kann,
oder
R^{13A} und R^{13B} zusammen mit dem Kohlenstoffatom, an das sie gebunden sind, einen 3-bis 6-gliedrigen Carbocyclus bilden,
R^{14A} für Wasserstoff, Fluor, (C₁-C₄)-Alkyl oder Hydroxy steht,
R^{14B} für Wasserstoff, Fluor, (C₁-C₄)-Alkyl oder Trifluormethyl steht, sowie ihre *N*-Oxide, Salze, Solvate, Salze der *N*-Oxide und Solvate der *N*-Oxide und Salze.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Ameisensäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Die erfindungsgemäßen Verbindungen können in Abhängigkeit von ihrer Struktur in unterschiedlichen stereoisomeren Formen existieren, d.h. in Gestalt von Konfigurationsisomeren oder gegebenenfalls auch als Konformationsisomere (Enantiomere und/oder Diastereomere, einschließlich solcher bei Atropisomeren). Die vorliegende Erfindung umfasst deshalb die Enantiomere und Diastereomere und ihre jeweiligen Mischungen. Aus solchen Mischungen von Enantiomeren und/ oder Diastereomeren lassen sich die stereoisomer einheitlichen Bestandteile in bekannter Weise isolieren; vorzugsweise werden hierfür chromatographische Verfahren verwendet, insbesondere die HPLC-Chromatographie an achiraler bzw. chiraler Phase.

Sofern die erfindungsgemäßen Verbindungen in tautomeren Formen vorkommen können, umfasst die vorliegende Erfindung sämtliche tautomere Formen.

Die vorliegende Erfindung umfasst auch alle geeigneten isotopischen Varianten der erfindungsgemäßen Verbindungen. Unter einer isotopischen Variante einer erfindungsgemäßen Verbindung wird hierbei eine Verbindung verstanden, in welcher mindestens ein Atom innerhalb der erfindungsgemäßen Verbindung gegen ein anderes Atom der gleichen Ordnungszahl, jedoch mit einer anderen Atommasse als der gewöhnlich oder überwiegend in der Natur vorkommenden Atommasse ausgetauscht ist. Beispiele für Isotope, die in eine erfindungsgemäße Verbindung inkorporiert werden können, sind solche von Wasserstoff, Kohlenstoff, Stickstoff, Sauerstoff, Phosphor, Schwefel, Fluor, Chlor, Brom und Iod, wie ²H (Deuterium), ³H (Tritium), ¹³C, ¹⁴C, ¹⁵N, ¹⁷O, ¹⁸O, ³²P, ³³P, ³³S, ³⁴S, ³⁵S, ³⁶S, ¹⁸F, ³⁶Cl, ⁸²Br, ¹²³I, ¹²⁴I, ¹²⁹I und ¹³¹I. Bestimmte isotopische Varianten einer erfindungsgemäßen Verbindung, wie insbesondere solche, bei denen ein oder mehrere radioaktive Isotope inkorporiert sind, können von Nutzen sein beispielsweise für die Untersuchung des Wirkmechanismus oder der Wirkstoff-Verteilung im Körper; aufgrund der vergleichsweise leichten Herstell- und Detektierbarkeit sind hierfür insbesondere mit ³H- oder ¹⁴C-Isotopen markierte Verbindungen geeignet. Darüber hinaus kann der Einbau von Isotopen, wie beispielsweise von Deuterium, zu bestimmten therapeutischen Vorteilen als Folge einer größeren metabolischen Stabilität der Verbindung führen, wie beispielsweise eine Verlängerung der Halbwertszeit im Körper oder eine Reduktion der erforderlichen Wirkdosis; solche Modifikationen der erfindungsgemäßen Verbindungen können daher gegebenenfalls auch eine bevorzugte Ausführungsform der vorliegenden Erfindung darstellen. Isotopische Varianten der erfindungsgemäßen Verbindungen können nach den dem Fachmann bekannten Verfahren hergestellt werden, so beispielsweise nach den weiter unten beschriebenen Methoden und den bei den Ausführungsbeispielen wiedergegebenen Vorschriften, indem entsprechende isotopische Modifikationen der jeweiligen Reagentien und/oder Ausgangsverbindungen eingesetzt werden.

Außerdem umfasst die vorliegende Erfindung auch Prodrugs der erfindungsgemäßen Verbindungen. Der Begriff "Prodrugs" bezeichnet hierbei Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Im Rahmen der vorliegenden Erfindung haben die Substituenten, soweit nicht anders spezifiziert, die folgende Bedeutung:
Alkyl steht im Rahmen der Erfindung für einen linearen oder verzweigten Alkylrest mit 1 bis 6 Kohlenstoffatomen. Beispielhaft und vorzugsweise seien genannt: Methyl, Ethyl, n-Propyl, Isopropyl, n-Butyl, iso-Butyl, 1-Methylpropyl, tert.-Butyl, n-Pentyl, 1-Methylbutyl, 2-Methylbutyl, 3-Methylbutyl, iso-Pentyl, n-Hexyl, 1-Methylpentyl, 1-Ethylbutyl, 2-Methylpentyl, 2-Ethylbutyl, 3-Methylpentyl, 4-Methylpentyl.
Cycloalkyl steht in Rahmen der Erfindung für einen monocyclischen, gesättigten Alkylrest mit 3 bis 7 Kohlenstoffatomen. Beispielhaft und vorzugsweise seien genannt: Cyclopropyl, Cyclobutyl, Cyclopentyl, Cyclohexyl und Cycloheptyl.
5- bis 7-gliedriges Aza-Heterocyclyl steht im Rahmen der Erfindung für einen monocyclischen, gesättigten Heterocyclus mit insgesamt 5 bis 7 Ringatomen, der ein Stickstoffatom enthält und darüberhinaus ein weiteres Ring-Heteroatom aus der Reihe N, O, S, SO oder SO₂ enthalten kann und über ein Ring-Stickstoffatom verknüpft ist. Beispielhaft seien genannt: Pyrrolidinyl, Pyrazolidinyl, Piperidinyl, Piperazinyl, Morpholinyl, Thiomorpholinyl, 1,1-Dioxothiomorpholinyl, Hexahydroazepinyl und Hexahydro-1,4-diazepinyl.
Alkoxy steht im Rahmen der Erfindung für einen linearen oder verzweigten Alkoxyrest mit 1 bis 4 Kohlenstoffatomen. Beispielhaft und vorzugsweise seien genannt: Methoxy, Ethoxy, n-Propoxy, Isopropoxy, 1-Methylpropoxy, n-Butoxy, iso-Butoxy und tert.-Butoxy.
(C₁-C₄)-Alkylsulfonylamino steht im Rahmen der Erfindung für eine Amino-Gruppe mit einem linearen oder verzweigten Alkylsulfonyl-Substituenten, der 1 bis 4 Kohlenstoffatome in der Alkylkette aufweist und über die Sulfonylgruppe mit dem N-Atom verknüpft ist. Beispielhaft und vorzugsweise seien genannt: Methylsulfonylamino, Ethylsulfonylamino, Propylsulfonylamino, n-Butylsulfonylamino, iso-Butylsulfonylamino und tert.-Butylsulfonylamino.
(C₃-C₆)-Cycloalkylsulfonylamino steht im Rahmen der Erfindung für eine Amino-Gruppe mit einem Cycloalkylsulfonyl-Substituenten, der 3 bis 6 Kohlenstoffatome in dem Cycloalkylring aufweist und über die Sulfonylgruppe mit dem N-Atom verknüpft ist. Beispielhaft und vorzugsweise seien genannt: Cyclopropylsulfonylamino, Cyclobutylsulfonylamino, Cyclopentylsulfonylamino, Cyclohexylsulfonylamino.
Heterocyclyl bzw. Heterocyclus steht im Rahmen der Erfindung für einen monocyclischen, oder bicyclischen, gesättigten oder teilweise ungesättigten Heterocyclus mit insgesamt 3 bis 7 Ringatomen, der ein bis drei Ring-Heteroatome aus der Reihe N, O und/oder S enthält und über ein ein Ring-Stickstoffatom verknüpft ist. Beispielhaft seien genannt: Azetidinyl, Oxetanyl, Pyrrolidinyl, Pyrazolidinyl, Tetrahydrofuranyl, Thiolanyl, Piperidinyl, Piperazinyl, Tetrahydropyranyl, Morpholinyl, Thiomorpholinyl, Azepanyl, Diazepanyl, Dihydropyrrolyl, Tetrahydropyridinyl, Dihydrooxazinyl, Dihydropyrazinyl oder 3-Azabicyclo[3.1.0]hex-3-yl. Bevorzugt ist ein gesättigter 5- oder 6-gliedriger Heterocyclus mit ein oder zwei Ring-Heteroatomen aus der Reihe N, O und/oder S. Beispielhaft seien genannt: Azetidinyl, Oxetanyl, Pyrrolidinyl, Pyrazolidinyl, Tetrahydrofuranyl, Piperidinyl, Piperazinyl, Tetrahydropyranyl, Morpholinyl und Thiomorpholinyl Bevorzugt ist ein gesättigter Bicyclus mit ein oder zwei Ring-Heteroatomen aus der Reihe N, O und/oder S. Beispielhaft sei genannt: 3-Azabicyclo[3.1.0]hex-3-yl.
Heteroaryl steht im Rahmen der Erfindung für einen mono- oder gegebenenfalls bicyclischen aromatischen Heterocyclus (Heteroaromaten) mit insgesamt 5 bis 10 Ringatomen, der bis zu drei gleiche oder verschiedene Ring-Heteroatome aus der Reihe N, O und/oder S enthält und über ein Ring-Kohlenstoffatom oder gegebenenfalls über ein Ring-Stickstoffatom verknüpft ist. Beispielhaft seien genannt: Furyl, Pyrrolyl, Thienyl, Pyrazolyl, Imidazolyl, Thiazolyl, Oxazolyl, Isoxazolyl, Isothiazolyl, Triazolyl, Oxadiazolyl, Thiadiazolyl, Tetrazolyl, Pyridyl, Pyrimidinyl, Pyridazinyl, Pyrazinyl, Triazinyl, Benzofuranyl, Benzothienyl, Benzimidazolyl, Benzoxazolyl, Benzothiazolyl, Benzotriazolyl, Indolyl, Indazolyl, Chinolinyl, Isochinolinyl, Naphthyridinyl, Chinazolinyl, Chinoxalinyl, Phthalazinyl, Pyrrolo[2,3-b]pyridin, Pyrazolo[1,5-a]pyridin, Pyrazolo[3,4-b]pyridinyl. Bevorzugt seien genannt: Pyrazolyl, Imidazolyl, Isoxazolyl, Pyridyl, Indolyl, Indazolyl, Chinolinyl, Isochinolinyl, Naphthyridinyl, Chinazolinyl, Chinoxalinyl, Phthalazinyl, Pyrrolo[2,3-b]pyridin, Pyrazolo[1,5-a]pyridin, Pyrazolo[3,4-b]pyridinyl.
Halogen schließt im Rahmen der Erfindung Fluor, Chlor, Brom und Iod ein. Bevorzugt sind Chlor oder Fluor.
Eine Oxo-Gruppe steht im Rahmen der Erfindung für ein Sauerstoffatom, das über eine Doppelbindung an ein Kohlenstoff- oder Schwefelatom gebunden ist.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit ein, zwei oder drei gleichen oder verschiedenen Substituenten ist bevorzugt.

Bevorzugt im Rahmen der vorliegenden Erfindung sind Verbindungen der Formel (I), in welcher
- A: für CH₂ steht,
- R¹: für Phenyl steht,
wobei Phenyl mit 1 bis 4 Substituenten unabhängig voneinander ausgewählt aus der Gruppe Fluor, Chlor, Cyano und Methyl substituiert sein kann,
- R²: für Wasserstoff, Methyl, Ethyl oder Cyclopropyl steht,
- R³: für Wasserstoff steht,
- R⁴: für Wasserstoff, Fluor, Chlor, Methyl oder Ethyl steht,
- R⁵: für Wasserstoff steht,
- E: für Stickstoff oder CR⁶ steht,
wobei
R⁶ für Wasserstoff, Chlor, Iod, Cyano, (C₁-C₆)-Alkyl, (C₂-C₆)-Alkinyl, Cyclopropyl, Hydroxy, -OR⁷, -NR⁸R⁹, Hydroxycarbonyl, (C₁-C₄)-Alkoxycarbonyl, -C(=O)-NR¹⁰R¹¹ oder 5-gliedriges Heteroaryl steht,
worin (C₁-C₆)-Alkyl mit 1 bis 3 Substituenten unabhängig voneinander ausgewählt aus der Gruppe Fluor, Difluormethyl, Trifluormethyl, Methoxy, Ethoxy, Hydroxy, Amino, -N(C=O)R¹², Methylsulfonylamino, Cyclopropyl und Cyclobutyl substituiert sein kann,
worin R¹² für Cyclopropyl, Cyclobutyl, Methyl oder Ethyl steht,
worin (C₂-C₆)-Alkinyl mit Cyclopropyl oder Cyclobutyl substituiert sein kann,
worin 5-gliedriges Heteroaryl mit Chlor, Methyl, Ethyl oder Hydroxy substituiert sein kann,
worin R⁷ für (C₁-C₄)-Alkyl oder Pyrazolyl steht,
worin (C₁-C₄)-Alkyl mit Trifluormethyl, Methoxy, Hydroxy oder Cyclopropyl substituiert sein kann,
worin R⁸ für Wasserstoff, (C₁-C₄)-Alkyl oder (C₃-C₆)-Cycloalkyl steht,
worin (C₃-C₆)-Cycloalkyl mit 1 bis 4 Substituenten unabhängig voneinander ausgewählt aus der Gruppe Methyl, Ethyl oder Hydroxy substituiert sein kann,
und
worin (C₁-C₄)-Alkyl mit 1 bis 4 Substituenten unabhängig voneinander ausgewählt aus der Gruppe Fluor, (C₁-C₄)-Alkyl, (C₃-C₅)-Cycloalkyl, Pyrrolidinyl, Piperidinyl, Methoxy, Ethoxy, Hydroxy, Amino, Trifluormethyl, Difluormethyl, Monofluormethyl und Phenyl substituiert sein kann,
worin Phenyl mit 1 bis 3 Substituenten unabhängig voneinander ausgewählt aus der Gruppe Fluor, Chlor, Cyano und Methoxy substituiert sein kann,
worin Pyrrolidinyl und Piperidinyl zweimal mit Fluor, substituiert sein kann,
und
worin (C₃-C₇)-Cycloalkyl mit Hydroxy substituiert sein kann,

worin R⁹ für Wasserstoff oder Methyl steht,
oder
R⁸ und R⁹ zusammen mit dem Stickstoffatom, an das sie gebunden sind, einen 4- bis 7-gliedrigen Heterocyclus bilden,
worin der 4- bis 7-gliedrige Heterocyclus mit 1 oder 2 Substituenten unabhängig voneinander ausgewählt aus der Gruppe (C₁-C₄)-Alkyl, Hydroxycarbonyl, Hydroxy und Amino substituiert sein kann,
worin (C₁-C₄)-Alkyl mit Hydroxycarbonyl, Hydroxy oder Amino substituiert sein kann,

worin R¹⁰ für Wasserstoff, (C₁-C₄)-Alkyl oder (C₃-C₆)-Cycloalkyl steht,
worin (C₃-C₆)-Cycloalkyl mit 1 bis 3 Substituenten unabhängig voneinander ausgewählt aus der Gruppe Methyl, Ethyl oder Hydroxy substituiert sein kann,
und
worin (C₁-C₄)-Alkyl mit mit 1 oder 2 Substituenten unabhängig voneinander ausgewählt aus der Gruppe Fluor, Hydroxy, Amino, Trifluormethyl und Difluormethyl substituiert sein kann,
worin R¹¹ für Wasserstoff oder Methyl steht,
oder
R¹⁰ und R¹¹ zusammen mit dem Stickstoffatom, an das sie gebunden sind, einen 4- bis 6-gliedrigen Heterocyclus bilden,
worin der 4- bis 6-gliedrige Heterocyclus mit 1 bis 3 Substituenten unabhängig voneinander ausgewählt aus der Gruppe Fluor, Methyl, Ethyl, Hydroxy und Amino substituiert sein kann,
worin Methyl und Ethyl mit Hydroxy substituiert sein können,
- L: für eine Gruppe #¹-CR^{13A}R^{13B}-(CR^{14A}R^{14B})ₘ-#² steht,
wobei
#¹ für die Anknüpfstelle an die Carbonylgruppe steht,
#² für die Anknüpfstelle an den Pyrimidin- bzw. Triazinring steht,
m für eine Zahl 0 steht,
R^{13A} für Wasserstoff oder Methyl steht,
R^{13B} für Wasserstoff, Difluormethyl, Trifluormethyl oder Methyl steht,
oder
R^{13A} und R^{13B} zusammen mit dem Kohlenstoffatom, an das sie gebunden sind, einen Cyclopropylring bilden,
sowie ihre *N*-Oxide, Salze, Solvate, Salze der *N*-Oxide und Solvate der *N*-Oxide und Salze.

Besonders bevorzugt im Rahmen der vorliegenden Erfindung sind Verbindungen der Formel (I), in welcher
- A: für CH₂ steht,
- R¹: für eine Phenyl-Gruppe der Formel 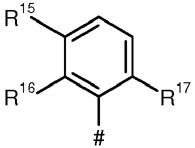 steht, wobei
# für die Anknüpfstelle an A steht,
und
R¹⁵ für Wasserstoff oder Fluor steht,
R¹⁶ und R¹⁷ für Fluor stehen,
- R²: für Methyl steht,
- R³: für Wasserstoff steht,
- R⁴: für Wasserstoff, Chlor oder Methyl steht,
- R⁵: für Wasserstoff steht,
- E: für Stickstoff oder CR⁶ steht,
wobei
R⁶ für Wasserstoff, Chlor, Ethinyl, -OR⁷, -NR⁸R⁹, -C(=O)-NR¹⁰R¹¹, 1H-pyrazol-1-yl oder 1,3-thiazol-5-yl steht,
worin Ethinyl mit Cyclopropyl substituiert ist,
worin 1H-pyrazol-1-yl und 1,3-thiazol-5-yl mit Methyl, Ethyl oder Hydroxy substituiert sein können,
worin R⁷ für Methyl, Ethyl oder 1H-pyrazol-4-yl steht, worin Methyl mit Cyclopropyl substituiert sein kann,
worin Ethyl mit Trifluormethyl, Methoxy oder Hydroxy substituiert sein kann,
worin R⁸ für Wasserstoff, Ethyl, Propyl oder (C₄-C₆)-Cycloalkyl steht,
worin (C₄-C₆)-Cycloalkyl mit 1 oder 2 Substituenten Methyl oder Hydroxy substituiert sein kann,
und
worin Ethyl und Propyl mit 1 bis 3 Substituenten unabhängig voneinander ausgewählt aus der Gruppe Fluor, Methyl, Ethyl, Propyl, Cyclopropyl, Methoxy, Hydroxy, Amino, Trifluormethyl, Difluormethyl, Monofluormethyl und Phenyl substituiert sein können,
worin Phenyl mit 1 oder 2 Substituenten unabhängig voneinander ausgewählt aus der Gruppe Fluor, Chlor und Methoxy substituiert sein kann,
und
worin (C₄-C₇)-Cycloalkyl mit Hydroxy substituiert sein kann,

worin R⁹ für Wasserstoff steht,
oder
R⁸ und R⁹ zusammen mit dem Stickstoffatom, an das sie gebunden sind, einen Piperidinyl-, Pyrrolidinyl- oder 3-Azabicyclo[3.1.0]hex-3-yl-Ring bilden,
worin der Piperidinyl- und Pyrrolidinyl-Ring mit Methyl substituiert sein kann,
worin Methyl mit Hydroxycarbonyl oder Hydroxy substituiert sein kann,
und
worin der 3-Azabicyclo[3.1.0]hex-3-yl-Ring mit Amino substituiert sein kann,
worin R¹⁰ für Wasserstoff, Methyl, Ethyl, n-Propyl oder Cyclopropyl steht,
worin Methyl, Ethyl, n-Propyl mit 1 oder 2 Substituenten unabhängig voneinander ausgewählt aus der Gruppe Fluor, Amino und Trifluormethyl substituiert sein können,
worin R¹¹ für Wasserstoff steht,
oder
R¹⁰ und R¹¹ zusammen mit dem Stickstoffatom, an das sie gebunden sind, einen Pyrrolidinyl-, Piperidinyl- oder Piperazinyl-Ring bilden,
worin der Pyrrolidinyl-Ring mit 1 bis 3 Substituenten unabhängig voneinander ausgewählt aus der Gruppe Fluor, Methyl, Hydroxy und Amino substituiert sein kann,
worin Methyl mit Hydroxy substituiert sein kann,
worin der Piperazinyl-Ring am Stickstoffatom mit Methyl substituiert sein kann,
- L: für eine Gruppe #¹-CR^{13A}R^{13B}-#² steht,
wobei
#¹ für die Anknüpfstelle an die Carbonylgruppe steht,
#² für die Anknüpfstelle an den Pyrimidin- bzw. Triazinring steht,
R^{13A} für Methyl steht,
R^{13B} für Trifluormethyl oder Methyl steht,
sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I), in welcher
- R¹: für eine Phenyl-Gruppe der Formel 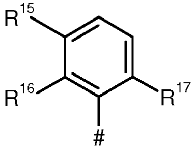 steht, wobei
# für die Anknüpfstelle an A steht,
und
R¹⁵ für Wasserstoff oder Fluor steht,
R¹⁶ und R¹⁷ für Fluor stehen,
sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I), in welcher
- R¹: für eine Phenyl-Gruppe der Formel 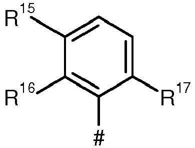 steht, wobei
# für die Anknüpfstelle an A steht,
und
R¹⁵ für Wasserstoff steht,
R¹⁶ und R¹⁷ für Fluor stehen,
sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I), in welcher
- R¹: für eine Phenyl-Gruppe der Formel 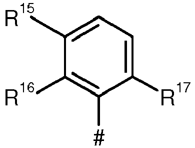 steht, wobei
# für die Anknüpfstelle an A steht,
und
R¹⁵ für Fluor steht,
R¹⁶ und R¹⁷ für Fluor stehen, sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I), in welcher
- R²: für Methyl steht,
sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I), in welcher
- E: für Stickstoff steht,
sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I), in welcher
- E: für CR⁶ steht,
wobei
R⁶ für Wasserstoff, Chlor, Ethinyl, -OR⁷, -NR⁸R⁹, -C(=O)-NR¹⁰R¹¹, 1H-pyrazol-1-yl oder 1,3-thiazol-5-yl steht,
worin Ethinyl mit Cyclopropyl substituiert ist,
worin 1H-pyrazol-1-yl und 1,3-thiazol-5-yl mit Methyl, Ethyl oder Hydroxy substituiert sein können,
worin R⁷ für Methyl, Ethyl oder 1H-pyrazol-4-yl steht,
worin Methyl mit Cyclopropyl substituiert sein kann,
worin Ethyl mit Trifluormethyl, Methoxy oder Hydroxy substituiert sein kann,
worin R⁸ für Wasserstoff, Ethyl, Propyl oder (C₄-C₆)-Cycloalkyl steht,
worin (C₄-C₆)-Cycloalkyl mit 1 oder 2 Substituenten Methyl oder Hydroxy substituiert sein kann,
und
worin Ethyl und Propyl mit 1 bis 3 Substituenten unabhängig voneinander ausgewählt aus der Gruppe Fluor, Methyl, Ethyl, Cyclopropyl, Propyl, Methoxy, Hydroxy, Amino, Trifluormethyl, Difluormethyl, Monofluormethyl und Phenyl substituiert sein können,
worin Phenyl mit 1 oder 2 Substituenten unabhängig voneinander ausgewählt aus der Gruppe Fluor, Chlor und Methoxy substituiert sein kann,
und
worin (C₄-C₇)-Cycloalkyl mit Hydroxy substituiert sein kann,

worin R⁹ für Wasserstoff steht,
oder
R⁸ und R⁹ zusammen mit dem Stickstoffatom, an das sie gebunden sind, einen Piperidinyl-, Pyrrolidinyl- oder 3-Azabicyclo[3.1.0]hex-3-yl-Ring bilden,
worin der Piperidinyl- und Pyrrolidinyl-Ring mit Methyl substituiert sein kann,
worin Methyl mit Hydroxycarbonyl oder Hydroxy substituiert sein kann,
und
worin der 3-Azabicyclo[3.1.0]hex-3-yl-Ring mit Amino substituiert sein kann,
worin R¹⁰ für Wasserstoff, Methyl, Ethyl, n-Propyl oder Cyclopropyl steht,
worin Methyl, Ethyl, n-Propyl mit 1 oder 2 Substituenten unabhängig voneinander ausgewählt aus der Gruppe Fluor, Amino und Trifluormethyl substituiert sein können,
worin R¹¹ für Wasserstoff steht,
oder
R¹⁰ und R¹¹ zusammen mit dem Stickstoffatom, an das sie gebunden sind, einen Pyrrolidinyl-, Piperidinyl- oder Piperazinyl-Ring bilden,
worin der Pyrrolidinyl-Ring mit 1 bis 3 Substituenten unabhängig voneinander ausgewählt aus der Gruppe Fluor, Methyl, Hydroxy und Amino substituiert sein kann,
worin Methyl mit Hydroxy substituiert sein kann,
worin der Piperazinyl-Ring am Stickstoffatom mit Methyl substituiert sein kann,
sowie ihre *N*-Oxide, Salze, Solvate, Salze der *N*-Oxide und Solvate der *N*-Oxide und Salze.

Besonders bevorzugt sind im Rahmen der vorliegenden Erfindung auch Verbindungen der Formel (I), in
welcher
- E: für CR⁶ steht,
wobei
R⁶ für -NR⁸R⁹ oder -C(=O)-NR¹⁰R¹¹ steht,
worin R⁸ für Wasserstoff, Ethyl, Propyl oder (C₄-C₆)-Cycloalkyl steht,
worin (C₄-C₆)-Cycloalkyl mit 1 oder 2 Substituenten Methyl oder Hydroxy substituiert sein kann,
und
worin Ethyl und Propyl mit 1 bis 3 Substituenten unabhängig voneinander ausgewählt aus der Gruppe Fluor, Methyl, Ethyl, Cyclopropyl, Propyl, Methoxy, Hydroxy, Amino, Trifluormethyl,
Difluormethyl, Monofluormethyl und Phenyl substituiert sein können,
worin Phenyl mit 1 oder 2 Substituenten unabhängig voneinander ausgewählt aus der Gruppe Fluor, Chlor und Methoxy substituiert sein kann,
und
worin (C₄-C₇)-Cycloalkyl mit Hydroxy substituiert sein kann,

worin R⁹ für Wasserstoff steht,
oder
R⁸ und R⁹ zusammen mit dem Stickstoffatom, an das sie gebunden sind, einen Piperidinyl-, Pyrrolidinyl- oder 3-Azabicyclo[3.1.0]hex-3-yl-Ring bilden,
worin der Piperidinyl- und Pyrrolidinyl-Ring mit Methyl substituiert sein kann,
worin Methyl mit Hydroxycarbonyl oder Hydroxy substituiert sein kann,
und
worin der 3-Azabicyclo[3.1.0]hex-3-yl-Ring mit Amino substituiert sein kann,
worin R¹⁰ für Wasserstoff, Methyl, Ethyl, n-Propyl oder Cyclopropyl steht,
worin Methyl, Ethyl, n-Propyl mit 1 oder 2 Substituenten unabhängig voneinander ausgewählt aus der Gruppe Fluor, Amino und Trifluormethyl substituiert sein können,
worin R¹¹ für Wasserstoff steht,
oder
R¹⁰ und R¹¹ zusammen mit dem Stickstoffatom, an das sie gebunden sind, einen Pyrrolidinyl-, Piperidinyl- oder Piperazinyl-Ring bilden,
worin der Pyrrolidinyl-Ring mit 1 bis 3 Substituenten unabhängig voneinander ausgewählt aus der Gruppe Fluor, Methyl, Hydroxy und Amino substituiert sein kann,
worin Methyl mit Hydroxy substituiert sein kann,
worin der Piperazinyl-Ring am Stickstoffatom mit Methyl substituiert sein kann,
sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I), in welcher
- L: für eine Gruppe #¹-CR^{13A}R^{13B}-#² steht,
wobei
#¹ für die Anknüpfstelle an die Carbonylgruppe steht,
#² für die Anknüpfstelle an den Pyrimidin- bzw. Triazinring steht,
R^{13A} für Methyl steht,
R^{13B} für Trifluormethyl oder Methyl steht,
sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I), in welcher
- R⁴: für Wasserstoff, Chlor oder Methyl steht,
sowie ihre *N*-Oxide, Salze, Solvate, Salze der *N*-Oxide und Solvate der *N*-Oxide und Salze.

Besonders bevorzugt sind im Rahmen der vorliegenden Erfindung auch Verbindungen der Formel (1), in welcher
- R⁴: für Chlor steht,
sowie ihre *N*-Oxide, Salze, Solvate, Salze der *N*-Oxide und Solvate der *N*-Oxide und Salze.

Die in den jeweiligen Kombinationen bzw. bevorzugten Kombinationen von Resten im Einzelnen angegebenen Reste-Definitionen werden unabhängig von den jeweiligen angegebenen Kombinationen der Reste beliebig auch durch Reste-Definitionen anderer Kombinationen ersetzt.

Besonders bevorzugt sind Kombinationen von zwei oder mehreren der oben genannten Vorzugsbereiche.

Weiterer Gegenstand der Erfindung ist ein Verfahren zur Herstellung der erfindungsgemäßen Verbindungen der Formel (I) dadurch gekennzeichnet, dass man
eine Verbindung der Formel (II) 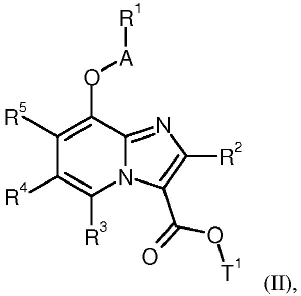 in welcher A, R¹, R², R³, R⁴, R⁵ jeweils die oben angegebenen Bedeutungen haben und
- T¹: für (C₁-C₄)-Alkyl oder Benzyl steht,
in einem inerten Lösungsmittel in Gegenwart einer geeigneten Base oder Säure zu einer Carbonsäure der Formel (III) 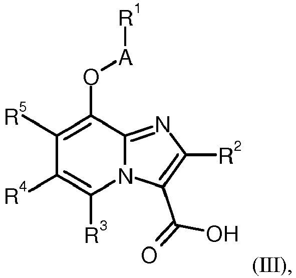 in welcher A, R¹, R², R³, R⁴ und R⁵ jeweils die oben angegebenen Bedeutungen haben, umsetzt, und diese in der Folge in einem inerten Lösungsmittel unter Amidkupplungsbedingungen mit einem Ammoniumsalz in eine Verbindung der Formel (IV) 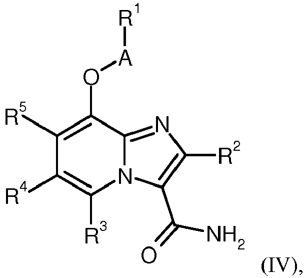 in welcher A, R¹, R², R³, R⁴ und R⁵ jeweils die oben angegebenen Bedeutungen haben, überführt, und diese anschließend in einem inerten Lösungsmittel mit Trifluoressigsäureanhydrid zu einer Verbindung der Formel (V) 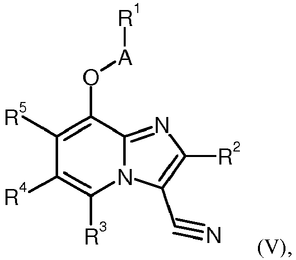 in welcher A, R¹, R², R³, R⁴ und R⁵ jeweils die oben angegebenen Bedeutungen haben, umsetzt, diese in Anwesenheit eines Alkylaluminium-Reagenzes in einem inerten Lösungsmittel in ein Amidin der Formel (VI) 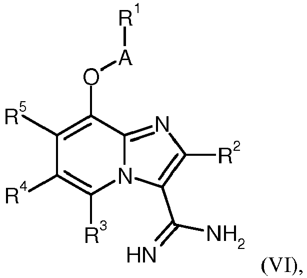 in welcher A, R¹, R², R³, R⁴ und R⁵ jeweils die oben angegebenen Bedeutungen haben, überführt,
oder
eine Verbindung der Formel (V) in einem geeignten Lösungsmittel in Gegenwart einer geeigneten Base mit Hydroxylamin zunächst in eine Verbindung der Formel (VIa) 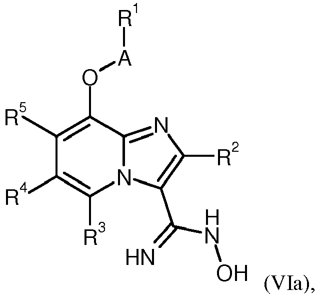 in welcher A, R¹, R², R³, R⁴ und R⁵ jeweils die oben angegebenen Bedeutungen haben, überführt, diese dann durch Hydrogenolyse in Gegenwart eines Palladiumkatalysators, wie beispielsweise Palladium auf Aktivkohle, in einem inerten Lösungsmittel, wie beispielsweise Ethanol oder Essigsäureethylester, in ein Amidin der Formel (VI) überführt,
diese in einem inerten Lösungsmittel in Gegenwart einer geeigneten Base mit einer Verbindung der Formel (VII) 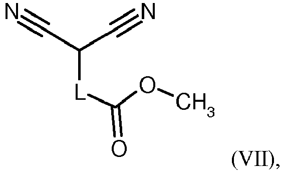 zu einer Verbindung der Formel (VIII) 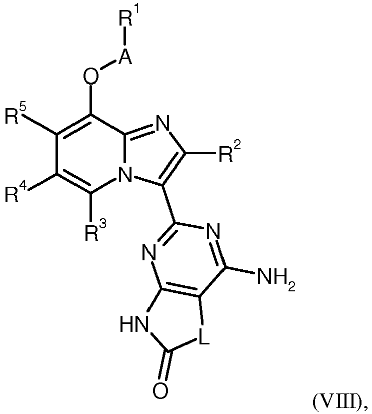 in welcher A, R¹, R², R³, R⁴, R⁵ und L jeweils die oben angegebenen Bedeutungen haben, umgsetzt, die Aminogruppe in einem inerten Lösungsmittel mit Isopentylnitrit und einem Halogen-Äquivalent in eine Verbindung der Formel (IX) 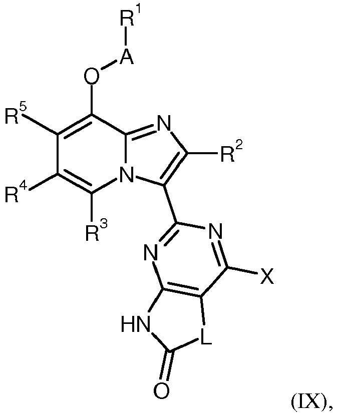 in welcher A, R¹, R², R³, R⁴, R⁵ und L jeweils die oben angegebenen Bedeutungen haben und
- X: für Chlor, Brom oder Iod steht
überführt, und diese
[A] im Anschluss in einem inerten Lösungsmittel gegebenenfalls in Gegenwart einer geeigneten Base mit einer Verbindung der Formel (X) 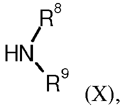 in welcher R⁸ und R⁹ die oben angegebene Bedeutungen haben,
   zu einer Verbindung der Formel (I-A) 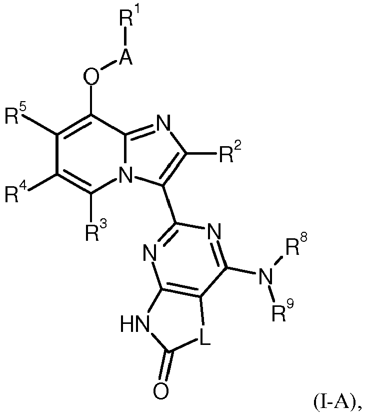 in welcher A, R¹, R², R³, R⁴, R⁵, R⁸, R⁹ und L jeweils die oben angegebenen Bedeutungen haben umsetzt,
   oder
[B] das Iodid der Formel (IX) in einem inerten Lösungsmittel gegebenenfalls in Gegenwart einer geeigneten Base und Kupfer(I)iodid mit einer Verbindung der Formel (XI)

   HO-R⁷ (XI),

   in welcher R⁷ die oben angegebene Bedeutung hat,
   zu einer Verbindung der Formel (I-B) 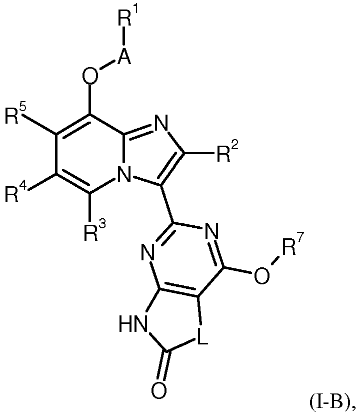 in welcher A, R¹, R², R³, R⁴, R⁵, R⁷ und L jeweils die oben angegebenen Bedeutungen haben umsetzt,
   oder
[C] das Iodid der Formel (IX) in einem inerten Lösungsmittel gegebenenfalls in Gegenwart einer geeigneten Base mit Kupfer(I)cyanid zu einer Verbindung der Formel (I-C) 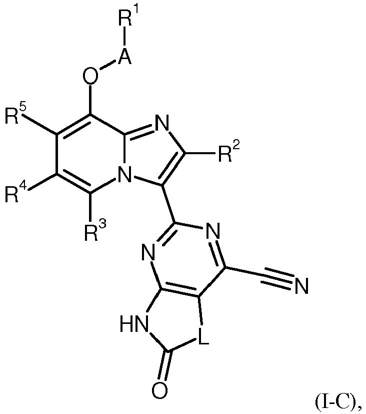 in welcher A, R¹, R², R³, R⁴, R⁵ und L jeweils die oben angegebenen Bedeutungen haben umsetzt, und diese in einem inerten Lösungsmittel mit einer geeigneten wässrigen Base in eine Verbindung der Formel (I-D) 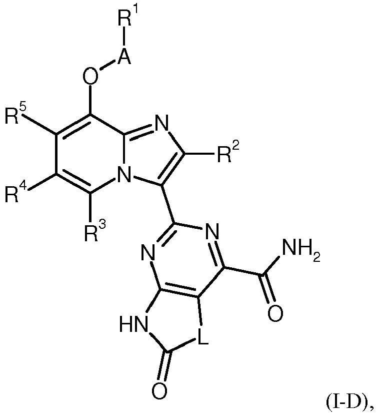 in welcher A, R¹, R², R³, R⁴, R⁵ und L jeweils die oben angegebenen Bedeutungen haben überführt, diese im Anschluß in einem inerten Lösungsmittel mit einer geeigneten wässrigen Säure in eine Säure der Formel (I-E) 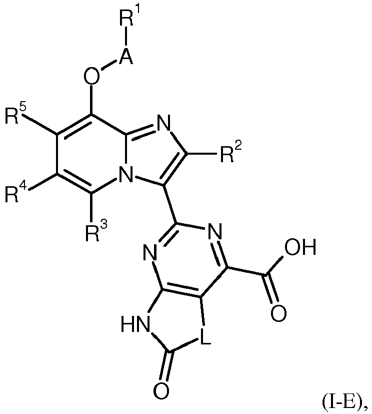 in welcher A, R¹, R², R³, R⁴, R⁵ und L jeweils die oben angegebenen Bedeutungen haben überführt, und diese in der Folge in einem inerten Lösungsmittel unter Amidkupplungsbedingungen mit einem Amin der Formel (XII) 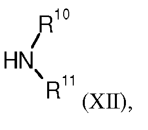 in welcher R¹⁰ und R¹¹ jeweils die oben angegebenen Bedeutungen haben, in eine Verbindung der Formel (I-F) 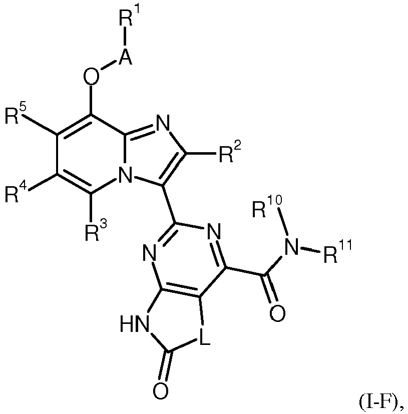 in welcher A, R¹, R², R³, R⁴, R⁵, R¹⁰, R¹¹ und L jeweils die oben angegebenen Bedeutungen haben,
   überführt,
   und gegebenenfalls die resultierenden Verbindungen der Formel (I) gegebenenfalls mit den entsprechenden (i) Lösungsmitteln und/oder (ii) Säuren oder Basen in ihre Solvate, Salze und/oder Solvate der Salze überführt.

Die Verbindungen der Formel (I-A), (I-B), (I-C), (I-D), (I-E) und (I-F) bilden eine Teilmenge der erfindungsgemäßen Verbindungen der Formel (I).

Die beschriebenen Herstellverfahren können durch die folgenden Syntheseschemata (Schema 1 und 2) beispielhaft verdeutlicht werden: 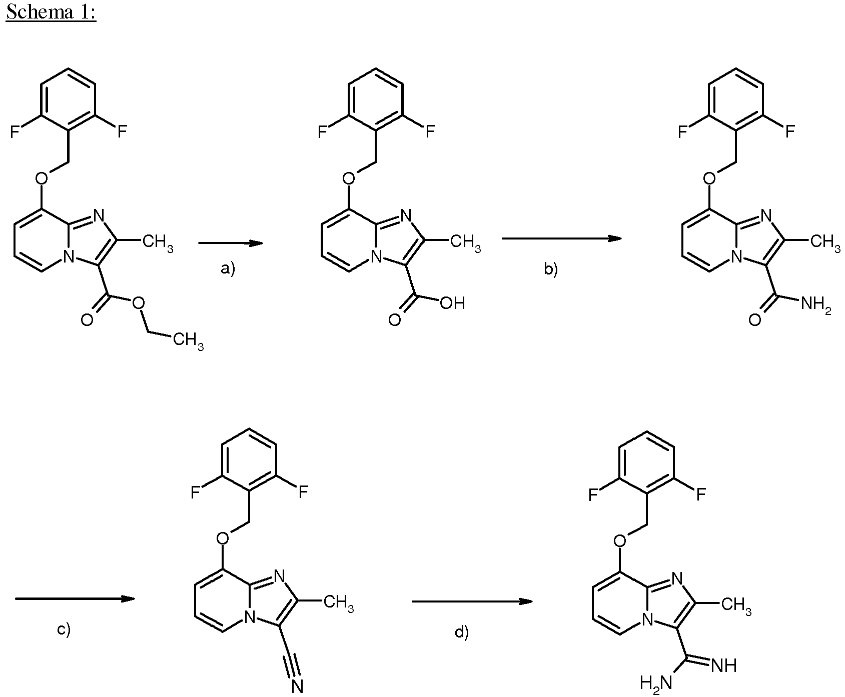 [a): Lithiumhydroxid, THF/Methanol/ H₂O, RT; b): EDCI, HOBT, NHaCl, *N,N-*Diisopropylethylamin, CH₂Cl₂, RT; c): Trifluoressigsäureanhydrid, Pyridin, THF, RT; d): NH₄Cl, Trimethylaluminium, Toluol, Rückfluss]. 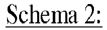 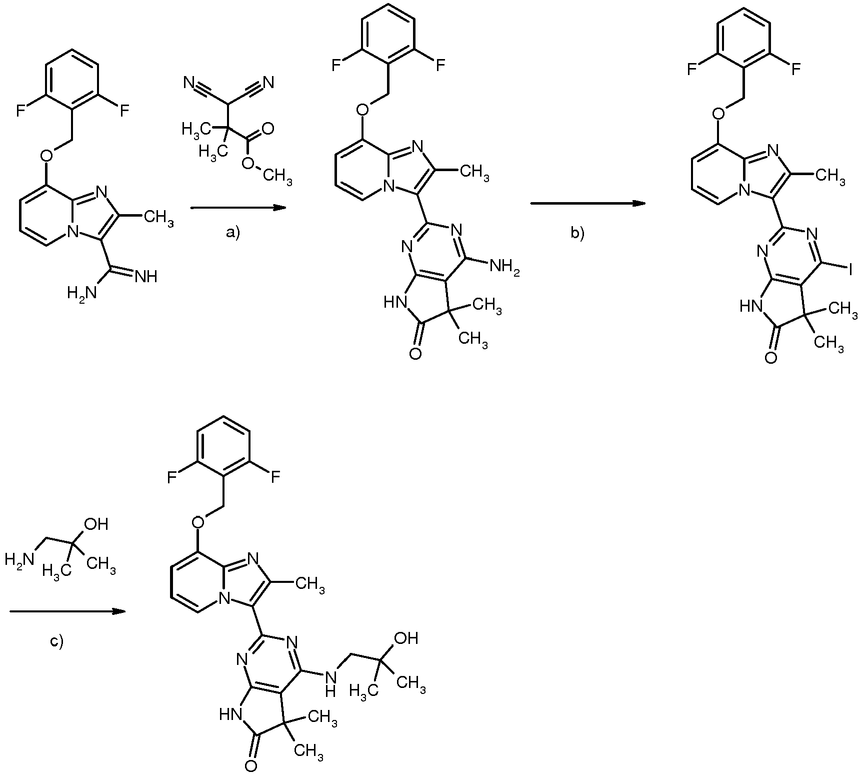 [a): Kalium-tert.-butylat, tert.-Butanol, Rückfluss; b): CH₂I₂, Isopentylnitrit, 1,4-Dioxan, 85°C; c): NMP, 150°C]. 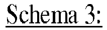 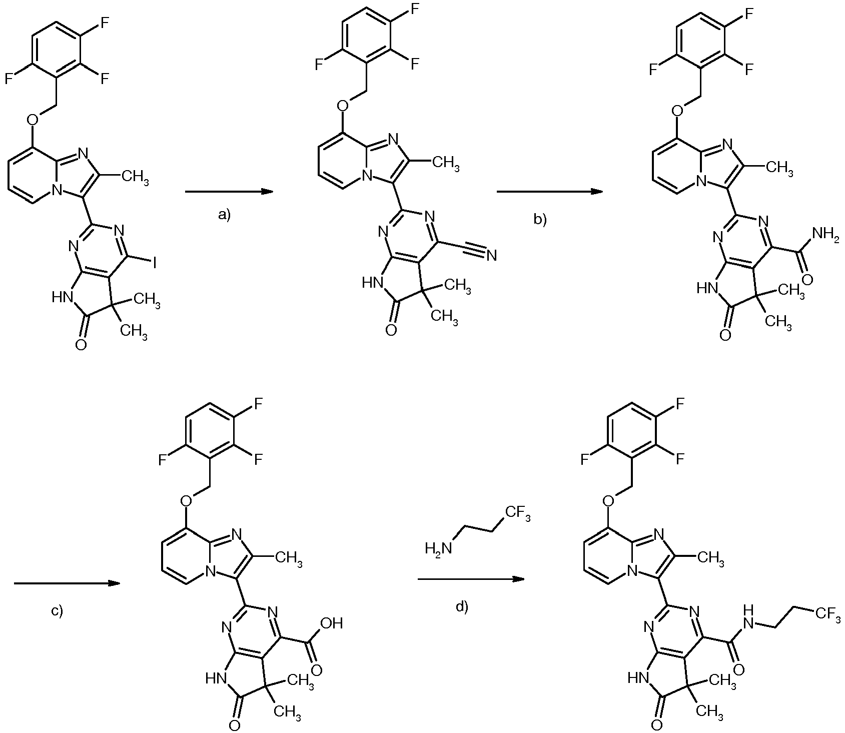 [a): CuCN, DMSO, 150°C; b): NaOH, 1,4-Dioxan, 80-90°C; dann RT, HCl; c): HCl, 80°C; d): T3P (Propanphosphonsäureanhydrid), N,N-Diisopropylethylamin, DMF, RT]. 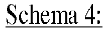 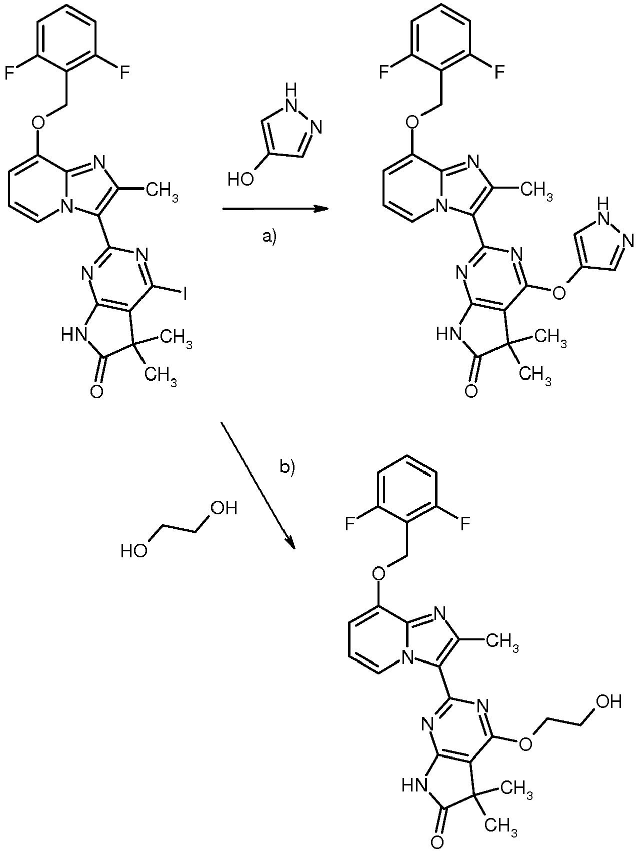 [a): Cu₂O, Cs₂CO₃, 2-Hydroxybenzaldehydoxim, Acetonitril, 160°C; Mikrowelle; b): CuI, 3,4,7,8-Tetramethyl-1,10-phenanthrolin, Cs₂CO₃, Toluol, 140°C]. 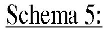 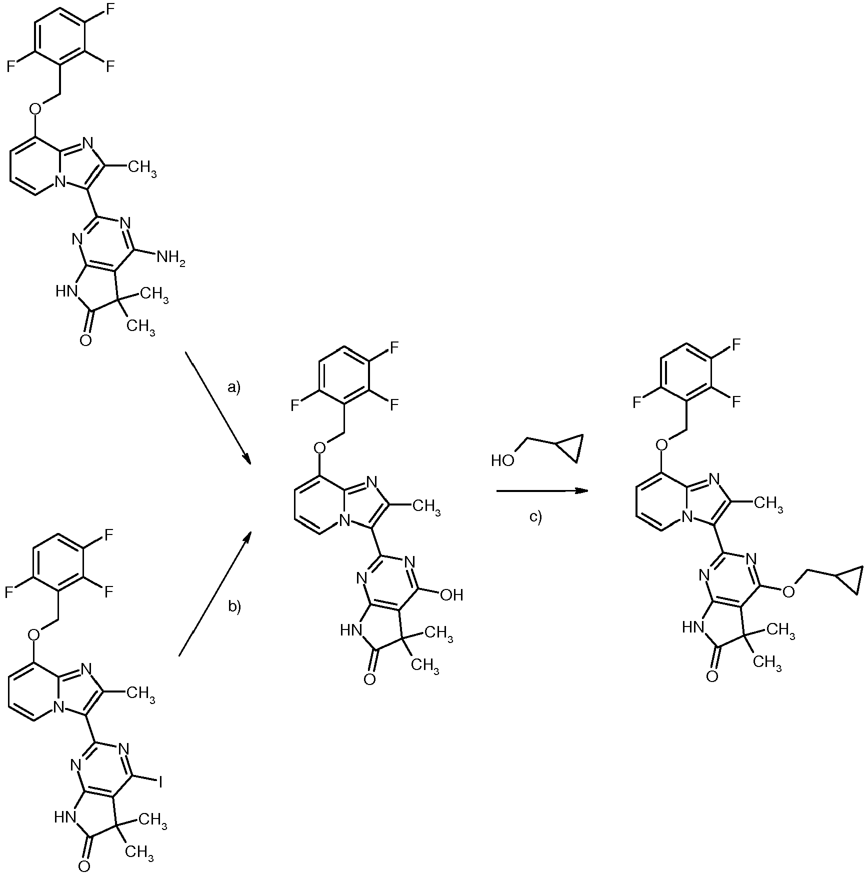 [a): Natriumnitrit, TFA/Wasser, 0°C; b): CH₂I₂, Isopentylnitrit, 1,4-Dioxan/Wasser, 85°C; c): PPh₃, DIAD (Diisopropylazodicarboxylat), THF, RT].

Das Herstellverfahren für die Triazin-substituierten Ausführungsbeispiele kann durch das folgende Syntheseschemata (Schema 3) beispielhaft verdeutlicht werden: 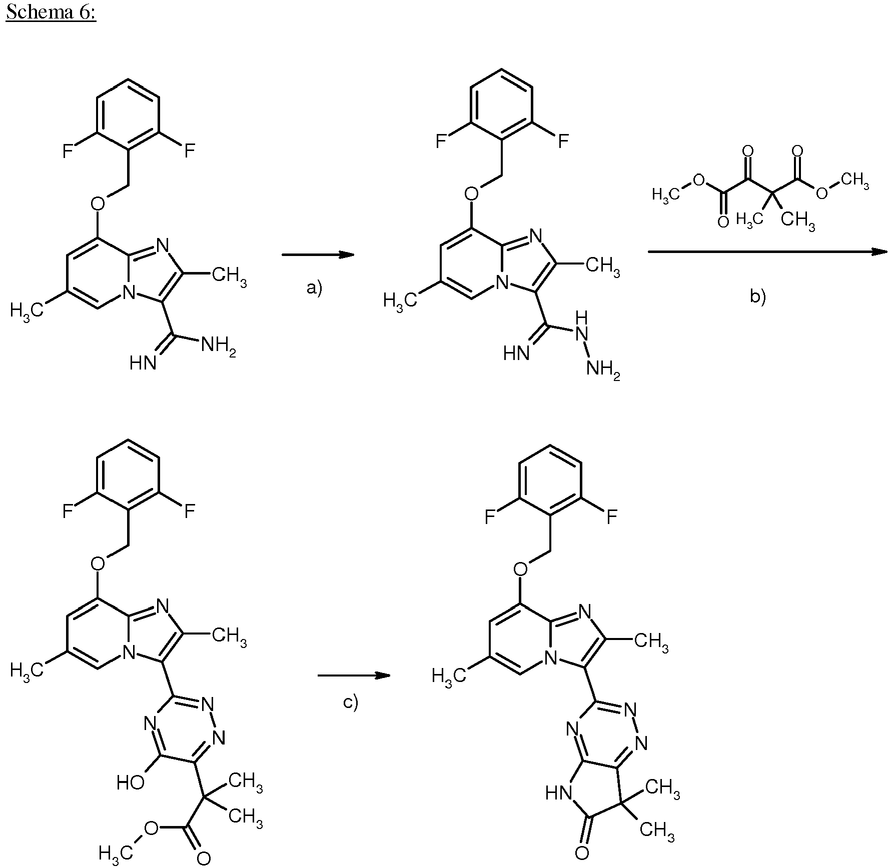 [a): Hydrazinhydrat, NEt₃, EtOH b): EtOH c): (i) POCl₃; (ii) konz. NH₃, Acetonitril].

Die Verbindungen der Formeln (VII), (X), (XI) und (XII) sind kommerziell erhältlich, literaturbekannt oder können in Analogie zu literaturbekannten Verfahren hergestellt werden.

Inerte Lösungsmittel für die Verfahrensschritte (III) → (IV) und (I-E) + (XII) → (I-F) sind beispielsweise Ether wie Diethylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, Halogenkohlenwasserstoffe wie Dichlormethan, Trichlormethan, Tetrachlormethan, 1,2-Dichlorethan, Trichlorethylen oder Chlorbenzol, oder andere Lösungsmittel wie Aceton, Essigsäureethylester, Acetonitril, Pyridin, Dimethylsulfoxid, *N*,*N*-Dimethylformamid, *N*,*N*'-Dimethylpropylenharnstoff (DMPU) oder *N*-Methylpyrrolidon (NMP). Ebenso ist es möglich, Gemische der genannten Lösungsmittel zu verwenden. Bevorzugt sind Dichlormethan, Tetrahydrofuran, Dimethylformamid oder Gemische dieser Lösungsmittel.

Als Kondensationsmittel für die Amidbildung in den Verfahrensschritte (III) → (IV) und (I-E) + (XII) → (I-F) eignen sich beispielsweise Carbodiimide wie *N*,*N*'-Diethyl-, *N*,*N*'-Dipropyl-, *N,N'-*Diisopropyl-, *N*,*N*'-Dicyclohexylcarbodiimid (DCC) oder *N*-(3-Dimethylaminopropyl)-*N*'-ethyl-carbodiimid-Hydrochlorid (EDC), Phosgen-Derivate wie *N*,*N*'-Carbonyldiimidazol (CDI), 1,2-Oxazoliumverbindungen wie 2-Ethyl-5-phenyl-1,2-oxazolium-3-sulfat oder 2-*tert*.-Butyl-5-methyl-isoxazolium-perchlorat, Acylaminoverbindungen wie 2-Ethoxy-1-ethoxycarbonyl-1,2-dihydro-chinolin, oder Isobutylchlorformiat, Propanphosphonsäureanhydrid (T3P), 1-Chlor-*N*,*N*,2-trimethylprop1-en-1-amin, Cyanophosphonsäurediethylester, Bis-(2-oxo-3-oxazolidinyl)-phosphorylchlorid, Benzotriazol-1-yloxy-tris(dimethylamino)phosphonium-hexafluorophosphat, Benzotriazol-1-yloxy-tris(pyrrolidino)phosphonium-hexafluorophosphat (PyBOP), *O*-(Benzotriazol-1-yl)-*N*,*N*,*N*',*N*'-tetramethyluronium-tetrafluoroborat (TBTU), *O*-(Benzotriazol-1-yl)-*N*,*N*,*N*;*N*'-tetramethyluronium-hexafluorophosphat (HBTU), 2-(2-Oxo-1-(2*H*)-pyridyl)-1,1,3,3-tetramethyluronium-tetrafluoroborat (TPTU), *O-*(7-Azabenzotriazol-1-yl)-*N*,*N*,*N*',*N*'-tetramethyl-uronium-hexafluorophosphat (HATU) oder *O*-(1*H*-6-Chlorbenzotriazol-1-yl)-1,1,3,3-tetramethyl-uronium-tetrafluoroborat (TCTU), gegebenenfalls in Kombination mit weiteren Hilfsstoffen wie 1-Hydroxybenzotriazol (HOBt) oder *N*-Hydroxysuccinimid (HOSu), sowie als Basen Alkalicarbonate, z.B. Natrium- oder Kaliumcarbonat oder -hydrogencarbonat, oder organische Basen wie Trialkylamine, z.B. Triethylamin, *N*-Methylmorpholin, N-Methylpiperidin oder *N*,*N*-Diisopropylethylamin. Bevorzugt wird TBTU in Verbindung mit N-Methylmorpholin, HATU in Verbindung mit *N*,*N*-Diisopropylethylamin oder 1-Chlor-*N*,*N*,2-trimethylprop-1-en-1amin verwendet.

Die Kondensationen (III) → (IV) und (I-E) + (XII) → (I-F) wird im Allgemeinen in einem Temperaturbereich von -20°C bis +100°C, bevorzugt bei 0°C bis +60°C durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Alternativ kann die Carbonsäure der Formel (III) und (I-E) auch zunächst in das entsprechende Carbonsäurechlorid überführt werden und dieses dann direkt oder in einer separaten Umsetzung mit einem Amin der Formel (IV) zu den erfindungsgemäßen Verbindungen umgesetzt werden. Die Bildung von Carbonsäurechloriden aus Carbonsäuren erfolgt nach den dem Fachmann bekannten Methoden, beispielsweise durch Behandlung mit Thionylchlorid, Sulfurylchlorid oder Oxalylchlorid in Gegenwart einer geeigneten Base, beispielsweise in Gegenwart von Pyridin, sowie optional unter Zusatz von Dimethylformamid, optional in einem geeigneten inerten Lösemittel.

Die Hydrolyse der Ester-Gruppe T¹ der Verbindungen der Formel (II) erfolgt nach üblichen Methoden, indem man die Ester in inerten Lösungsmitteln mit Säuren oder Basen behandelt, wobei bei letzterem die zunächst entstehenden Salze durch Behandeln mit Säure in die freien Carbonsäuren überführt werden. Im Falle der tert.-Butylester erfolgt die Esterspaltung bevorzugt mit Säuren. Im Falle der Benzylester erfolgt die Esterspaltung bevorzugt hydrogenolytisch mit Palladium auf Aktivkohle oder Raney-Nickel.

Als inerte Lösungsmittel eignen sich für diese Reaktion Wasser oder die für eine Esterspaltung üblichen organischen Lösungsmittel. Hierzu gehören bevorzugt Alkohole wie Methanol, Ethanol, n-Propanol, Isopropanol, n-Butanol oder tert.-Butanol, oder Ether wie Diethylether, Tetrahydrofuran, Dioxan oder Glykoldimethylether, oder andere Lösungsmittel wie Aceton, Dichlormethan, Dimethylformamid oder Dimethylsulfoxid. Ebenso ist es möglich, Gemische der genannten Lösungsmittel einzusetzen. Im Falle einer basischen Ester-Hydrolyse werden bevorzugt Gemische von Wasser mit Dioxan, Tetrahydrofuran, Methanol und/oder Ethanol eingesetzt.

Als Basen für die Ester-Hydrolyse sind die üblichen anorganischen Basen geeignet. Hierzu gehören bevorzugt Alkali- oder Erdalkalihydroxide wie beispielsweise Natrium-, Lithium-, Kalium- oder Bariumhydroxid, oder Alkali- oder Erdalkalicarbonate wie Natrium-, Kalium- oder Calciumcarbonat. Besonders bevorzugt sind Natrium- oder Lithiumhydroxid.

Als Säuren eignen sich für die Esterspaltung im Allgemeinen Schwefelsäure, Chlorwasserstoff/ Salzsäure, Bromwasserstoff/Bromwasserstoffsäure, Phosphorsäure, Essigsäure, Trifluoressigsäure, Toluolsulfonsäure, Methansulfonsäure oder Trifluormethansulfonsäure oder deren Gemische gegebenenfalls unter Zusatz von Wasser. Bevorzugt sind Chlorwasserstoff oder Trifluoressigsäure im Falle der tert.-Butylester und Salzsäure im Falle der Methylester.

Die Esterspaltung erfolgt im Allgemeinen in einem Temperaturbereich von 0°C bis +100°C, bevorzugt bei +0°C bis +50°C.

Die genannten Umsetzungen können bei normalem, erhöhtem oder bei erniedrigtem Druck durchgeführt werden (z.B. von 0.5 bis 5 bar). Im Allgemeinen arbeitet man jeweils bei Normaldruck.

Inerte Lösungsmittel für den Verfahrensschritt (IV) → (V) sind beispielsweise Ether wie Diethylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen oder andere Lösungsmittel wie Aceton, Essigsäureethylester, Acetonitril, Pyridin, Dimethylsulfoxid, *N,N-*Dimethylformamid, *N*,*N*'-Dimethylpropylenharnstoff (DMPU) oder *N*-Methylpyrrolidon (NMP). Ebenso ist es möglich, Gemische der genannten Lösungsmittel zu verwenden. Bevorzugt ist Tetrahydrofuran.

Die Reaktion (IV) → (V) erfolgt im Allgemeinen in einem Temperaturbereich von +20°C bis +100°C, bevorzugt im Bereich von +20°C bis +50°C, gegebenenfalls in einer Mikrowelle. Die Umsetzung kann bei normalem, erhöhtem oder erniedrigtem Druck durchgeführt werden (z.B. im Bereich von 0.5 bis 5 bar). Im Allgemeinen arbeitet man bei Normaldruck.

Inerte Lösungsmittel für den Verfahrensschritt (V) → (VI) sind Kohlenwasserstoffe wie Benzol, Toluol, Xylol, Hexan, Cyclohexan oder Erdölfraktionen oder andere Lösungsmittel wie Acetonitril, Pyridin, Dimethylsulfoxid, *N*,*N*-Dimethylformamid, *N*,*N*'-Dimethylpropylenharnstoff (DMPU) oder *N*-Methylpyrrolidon (NMP). Ebenso ist es möglich, Gemische der genannten Lösungsmittel zu verwenden. Bevorzugt ist Toluol.

Die Reaktion (V) → (VI) erfolgt im Allgemeinen in einem Temperaturbereich von +20°C bis +150°C, bevorzugt im Bereich von +80°C bis +130°C, gegebenenfalls in einer Mikrowelle. Die Umsetzung kann bei normalem, erhöhtem oder erniedrigtem Druck durchgeführt werden (z.B. im Bereich von 0.5 bis 5 bar). Im Allgemeinen arbeitet man bei Normaldruck.

Inerte Lösungsmittel für den Verfahrensschritt (VI) + (VII) → (VIII) sind beispielsweise Alkohole wie Methanol, Ethanol, n-Propanol, Isopropanol, n-Butanol oder tert.-Butanol, Ether wie Diethylether, Dioxan, Dimethoxyethan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid (DMF), Dimethylsulfoxid (DMSO), *N*,*N*'-Dimethylpropylenharnstoff (DMPU), *N*-Methylpyrrolidon (NMP), Pyridin, Acetonitril, Sulfolan oder auch Wasser. Ebenso ist es möglich, Gemische der genannten Lösungsmittel einzusetzen. Bevorzugt ist tert.-Butanol.

Geeignete Basen für den Verfahrensschritt (VI) + (VII) → (VIII) sind Alkalihydroxide wie beispielsweise Lithium-, Natrium- oder Kaliumhydroxid, Alkalicarbonate wie Lithium-, Natrium-, Kalium- oder Cäsiumcarbonat, Alkalihydrogencarbonate wie Natrium- oder Kaliumhydrogencarbonat, Alkalialkoholate wie Natrium- oder Kaliummethanolat, Natrium- oder Kaliumethanolat oder Kalium-tert.-butylat, oder organische Amine wie Triethylamin, Diisopropylethylamin, Pyridin, 1,8-Diazabicyclo[5.4.0]undec-7-en (DBU) oder 1,5-Diazabicyclo[4.3.0]non-5-en (DBN). Bevorzugt ist Kalium-tert.-butylat.

Die Reaktion (VI) + (VII) → (VIII) wird im Allgemeinen in einem Temperaturbereich von +20°C bis +150°C, bevorzugt bei +75°C bis +100°C, gegebenenfalls in einer Mikrowelle, durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Der Verfahrensschritt (VIII) → (IX) erfolgt mit oder ohne Lösungsmittel. Als Lösungsmittel eignen sich alle organischen Lösungsmittel, die unter den Reaktionsbedingungen inert sind. Bevorzugte Lösungsmittel sind Acetonitril, 1,4-Dioxan und Dimethoxyethan.

Die Reaktion (VIII) → (IX) erfolgt im Allgemeinen in einem Temperaturbereich von +20°C bis +100°C, bevorzugt im Bereich von +50°C bis +100°C, gegebenenfalls in einer Mikrowelle. Die Umsetzung kann bei normalem, erhöhtem oder erniedrigtem Druck durchgeführt werden (z.B. im Bereich von 0.5 bis 5 bar). Im Allgemeinen arbeitet man bei Normaldruck.

Als Halogen-Quelle bei der Umsetzung (VIII) → (IX) eignen sich beispielsweise Diiodmethan, eine Mischung aus Cäsiumiodid, Iod und Kupfer-(I)-iodid, Kupfer-(II)-bromid, Kupfer-(11)-chlorid oder Kaliumiodid.

Inerte Lösungsmittel für die Verfahrensschritte (IX) + (X) → (I-A) bzw. (IX) + (XI) → (I-B) bzw. (IX) → (I-C) sind beispielsweise Ether wie Diethylether, Dioxan, Dimethoxyethan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid (DMF), Dimethylsulfoxid (DMSO), *N*,*N*'-Dimethylpropylenharnstoff (DMPU), *N-*Methylpyrrolidon (NMP), Pyridin, Acetonitril oder Sulfolan. Ebenso ist es möglich, Gemische der genannten Lösungsmittel einzusetzen. Bevorzugt ist DMSO und NMP.

Die Reaktionen (IX) + (X) → (I-A) bzw. (IX) + (XI) → (I-B) bzw. (IX) → (I-C) erfolgen im Allgemeinen in einem Temperaturbereich von +20°C bis +180°C, bevorzugt im Bereich von +120°C bis +180°C, gegebenenfalls in einer Mikrowelle. Die Umsetzung kann bei normalem, erhöhtem oder erniedrigtem Druck durchgeführt werden (z.B. im Bereich von 0.5 bis 5 bar). Im Allgemeinen arbeitet man bei Normaldruck.

Die Reaktion (IX) + (XI) → (I-B) erfolgt in Gegenwart eines geeigneten Kupfer-Katalysators wie beispielsweise Kupfer-(I)-iodid, unter Zusatz von 3,4,7,8-Tetramethyl-1,10-Phenanthrolin, sowie einer geeigneten Base wie beispielsweise Erdalkalicarbonate wie Lithium-, Natrium-, Kalium-, Calcium- oder Cäsiumcarbonat, bevorzugt Cäsiumcarbonat.

Geeignete Basen für den Verfahrensschritt (I-C) → (I-D) sind Natiumhydroxid/ Natronlauge, Kaliumhydroxid, Lithiumhydroxid, Bariumhydroxid oder deren Gemische gegebenenfalls unter Zusatz von Wasser. Bevorzugt wird Natronlauge verwendet.

Geeignete Lösungsmittel für die Reaktion (IC) → (I-D) sind Wasser, THF, 1,4-Dioxan, DMF oder DMSO. Ebenso ist es möglich, Gemische der genannten Lösungsmittel einzusetzen.

Die Umsetzung (I-C) → (I-D) wird im Allgemeinen in einem Temperaturbereich von +20°C bis +100°C, bevorzugt bei 65°C bis +100°C durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Geeignete Säuren für den Verfahrensschritt (I-D) → (I-E) sind Chlorwasserstoff/ Salzsäure, Bromwasserstoff/Bromwasserstoffsäure, Schwefelsäure, Essigsäure oder deren Gemische gegebenenfalls unter Zusatz von Wasser. Bevorzugt wird Salzsäure verwendet.

Geeignete Lösungsmittel für die Reaktion (I-D) → (I-E) sind Wasser, THF, 1,4-Dioxan, DMF oder DMSO. Ebenso ist es möglich, Gemische der genannten Lösungsmittel einzusetzen.

Die Umsetzung (I-D) → (I-E) wird im Allgemeinen in einem Temperaturbereich von +20°C bis +100°C, bevorzugt bei 75°C bis +100°C durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Die Verbindungen der Formel (II) sind literaturbekannt oder können hergestellt werden, indem eine Verbindung der Formel (XIII) 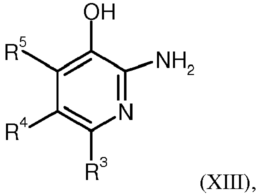 in welcher R³, R⁴ und R⁵ die oben angegebene Bedeutungen haben,
in einem inerten Lösungsmittel in Gegenwart einer geeigneten Base mit einer Verbindung der Formel (XIV) 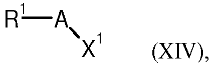 in welcher R¹ die oben angegebene Bedeutung hat und
- X¹: für Chlor, Brom, Iod, O-Triflat oder O-Mesylat steht,
zu einer Verbindung der Formel (XV) 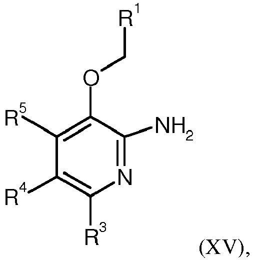 in welcher R¹, R³, R⁴ und R⁵ jeweils die oben angegebenen Bedeutungen haben,
umgesetzt wird, und diese anschliessend in einem inerten Lösungsmittel mit einer Verbindung der Formel (XVI) 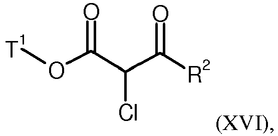 in welcher R² und T¹ jeweils die oben angegebenen Bedeutungen haben,
umgesetzt wird.

Das beschriebene Verfahren wird durch das nachfolgende Schema (Schema 3) beispielhaft verdeutlicht: 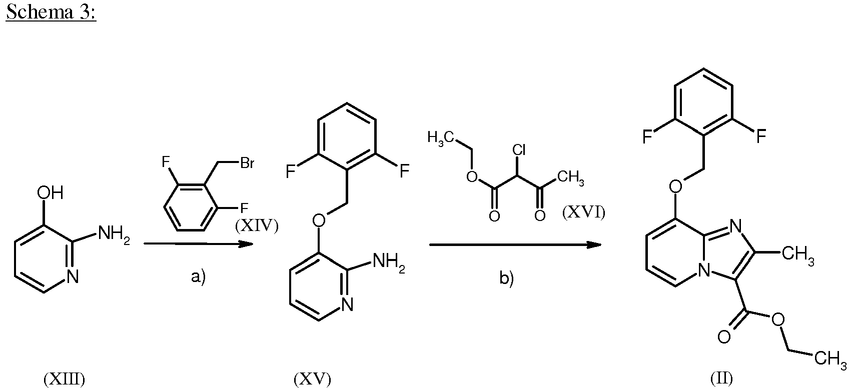 [a): i) NaOMe, MeOH, RT; ii) DMSO, RT; b): EtOH, Molsieb, 80°C].

Die gezeigte Synthesesequenz kann dahingehend modifiziert werden, dass die jeweiligen Reaktionsschritte in einer veränderten Reihenfolge durchlaufen werden. Ein Beispiel für eine solche modifizierte Synthesesequenz ist in Schema 4 gezeigt. 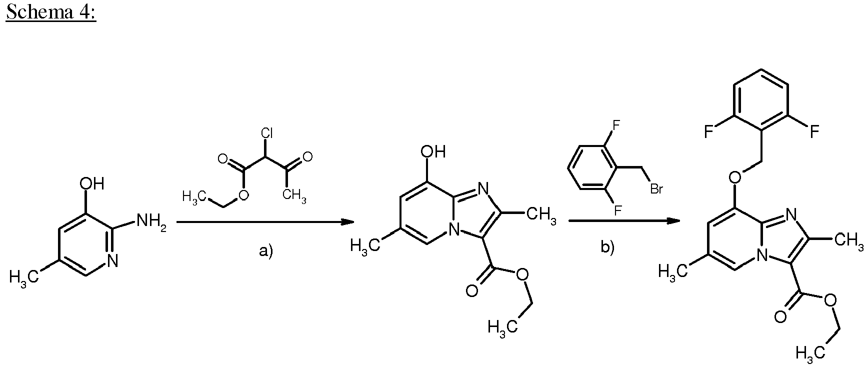 [a): EtOH, Molsieb, 80°C; b): i) Cs₂CO₃, DMF, 50°C].

Inerte Lösungsmittel für den Verfahrensschritt (XIII) + (XIV) → (XV) sind beispielsweise Halogenkohlenwasserstoffe wie Dichlormethan, Trichlormethan, Tetrachlormethan, Trichlorethylen oder Chlorbenzol, Ether wie Diethylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, oder andere Lösungsmittel wie Aceton, Methylethylketon, Essigsäureethylester, Acetonitril, *N*,*N*-Dimethylformamid, Dimethylsulfoxid, *N*,*N*'-Dimethylpropylenharnstoff (DMPU), *N*-Methylpyrrolidon (NMP) oder Pyridin. Ebenso ist es möglich, Gemische der genannten Lösungsmittel einzusetzen. Bevorzugt wird Dimethylformamid oder Dimethylsulfoxid verwendet.

Als Basen für den Verfahrensschritt (XIII) + (XIV) → (XV) eignen sich die üblichen anorganischen oder organischen Basen. Hierzu gehören bevorzugt Alkalihydroxide wie beispielsweise Lithium-, Natrium- oder Kaliumhydroxid, Alkali- oder Erdalkalicarbonate wie Lithium-, Natrium-, Kalium-, Calcium- oder Cäsiumcarbonat gegebenenfalls unter Zusatz eines Alkaliiodids wie beispielsweise Natriumiodid oder Kaliumiodid, Alkali-Alkoholate wie Natrium- oder Kaliummethanolat, Natrium- oder Kaliumethanolat oder Natrium- oder Kalium-tert.-butylat, Alkalihydride wie Natrium- oder Kaliumhydrid, Amide wie Natriumamid, Lithium- oder Kaliumbis(trimethylsilyl)amid oder Lithiumdiisopropylamid, oder organische Amine wie Triethylamin, *N*-Methylmorpholin, *N*-Methylpiperidin, *N*,*N*-Diisopropylethylamin, Pyridin, 1,5-Diazabicyclo[4.3.0]non-5-en (DBN), 1,8-Diazabicyclo[5.4.0]undec-7-en (DBU) oder 1,4-Diazabicyclo[2.2.2]octan (DABCO^{®}). Bevorzugt wird Kaliumcarbonat, Cäsiumcarbonat oder Natriummethanolat verwendet.

Die Reaktion erfolgt im Allgemeinen in einem Temperaturbereich von 0°C bis +120°C, bevorzugt bei +20°C bis +80°C, gegebenenfalls in einer Mikrowelle. Die Umsetzung kann bei normalem, erhöhtem oder bei erniedrigtem Druck durchgeführt werden (z.B. von 0.5 bis 5 bar).

Inerte Lösungsmittel für den Ringschluss zum Imidazo[1,2-a]pyridin-Grundgerüst (XV) + (XVI) → (II) sind die üblichen organischen Lösungsmittel. Hierzu gehören bevorzugt Alkohole wie Methanol, Ethanol, n-Propanol, Isopropanol, n-Butanol oder tert.-Butanol, oder Ether wie Diethylether, Tetrahydrofuran, Dioxan oder Glykoldimethylether, oder andere Lösungsmittel wie Aceton, Dichlormethan, Dimethylformamid oder Dimethylsulfoxid. Ebenso ist es möglich, Gemische der genannten Lösungsmittel einzusetzen. Bevorzugt wird Ethanol verwendet.

Der Ringschluss erfolgt im Allgemeinen in einem Temperaturbereich von +50°C bis +150°C, bevorzugt bei +50°C bis +100°C, gegebenenfalls in einer Mikrowelle.

Der Ringschluss (XV) + (XVI) → (II) erfolgt optional in Gegenwart wasserziehender Reaktionszusätze, beispielsweise in Gegenwart von Molekularsieb (4Å Porengröße). Die Umsetzung (XV) + (XVI) → (II) erfolgt unter Verwendung eines Überschusses des Reagenzes der Formel (XVI), beispielsweise mit 1 bis 20 Äquivalenten des Reagenzes (XVI), wobei die Zugabe dieses Reagenzes einmalig oder in mehreren Portionen erfolgen kann.

Weitere erfindungsgemäße Verbindungen können gegebenenfalls auch hergestellt werden durch Umwandlungen von funktionellen Gruppen einzelner Substituenten, insbesondere den unter R⁶ aufgeführten, ausgehend von den nach obigen Verfahren erhaltenen Verbindungen der Formel (I). Diese Umwandlungen werden nach üblichen, dem Fachmann bekannten Methoden durchgeführt und umfassen beispielsweise Reaktionen wie nukleophile und elektrophile Substitutionen, Oxidationen, Reduktionen, Hydrierungen, Übergangsmetall-katalysierte Kupplungsreaktionen, Eliminierungen, Alkylierung, Aminierung, Veresterung, Esterspaltung, Veretherung, Etherspaltung, Bildung von Carbonamiden, sowie Einführung und Entfernung temporärer Schutzgruppen.

Die erfindungsgemäßen Verbindungen wirken als potente Stimulatoren der löslichen Guanylatcyclase und/oder Inhibitoren von Phosphodiesterase-5, besitzen wertvolle pharmakologische Eigenschaften und können zur Vorbeugung und Behandlung von Erkrankungen bei Menschen und Tieren verwendet werden. Die erfindungsgemäßen Verbindungen eröffnen eine weitere Behandlungsalternative und stellen somit eine Bereicherung der Pharmazie dar.

Die erfindungsgemäßen Verbindungen bewirken eine Gefäßrelaxation und eine Hemmung der Thrombozytenaggregation und führen zu einer Blutdrucksenkung sowie zu einer Steigerung des koronaren Blutflusses. Diese Wirkungen sind über eine direkte Stimulation der löslichen Guanylatcyclase und einen intrazellulären cGMP-Anstieg vermittelt. Außerdem verstärken die erfindungsgemäßen Verbindungen die Wirkung von Substanzen, die den cGMP-Spiegel steigern, wie beispielsweise EDRF (endothelium-derived relaxing factor), NO-Donatoren, Protoporphyrin IX, Arachidonsäure oder Phenylhydrazin-Derivate.

Die erfindungsgemäßen Verbindungen eignen sich zur Behandlung und/oder Prophylaxe von kardio- und cerebrovaskulären, pulmonalen, thromboembolischen und fibrotischen Erkrankungen.

Die erfindungsgemäßen Verbindungen können daher in Arzneimitteln zur Behandlung und/oder Prophylaxe von kardiovaskulären Erkrankungen wie beispielsweise Bluthochdruck (Hypertonie), resistente Hypertonie, akute und chronische Herzinsuffizienz, koronare Herzerkrankung, stabile und instabile Angina pectoris, periphere und kardiale Gefäßerkrankungen, Arrhythmien, Rhythmusstörungen der Vorhöfe und der Kammern sowie Überleitungsstörungen wie beispielsweise atrio-ventrikuläre Blockaden Grad I-III (AB-Block I-III), supraventrikuläre Tachyarrhythmie, Vorhofflimmern, Vorhoffflattern, Kammerflimmern, Kammerflattern, ventrikuläre Tachyarrhytmie, Torsade de pointes-Tachykardie, Extrasystolen des Vorhoffs und des Ventrikels, AV-junktionale Extrasystolen, Sick-Sinus Syndrom, Synkopen, AV-Knoten-Reentrytachykardie, Wolff-Parkinson-White-Syndrom, von akutem Koronarsyndrom (ACS), autoimmune Herzerkrankungen (Perikarditis, Endokarditis, Valvolitis, Aortitis, Kardiomyopathien), Schock wie kardiogenem Schock, septischem Schock und anaphylaktischem Schock, Aneurysmen, Boxerkardiomyopathie (premature ventricular contraction (PVC)), zur Behandlung und/oder Prophylaxe von thromboembolischen Erkrankungen und Ischämien wie myokardiale Ischämie, Myokardinfarkt, Hirnschlag, Herzhypertrophie, transistorischen und ischämischen Attacken, Präeklampsie, entzündliche kardiovaskuläre Erkrankungen, Spasmen der Koronararterien und peripherer Arterien, Ödembildung wie beispielsweise pulmonales Ödem, Hirnödem, renales Ödem oder Herzinsuffizienz-bedingtes Ödem, peripheren Durchblutungsstörungen, Reperfusionsschäden, arterielle und venöse Thrombosen, Mikroalbuminurie, Herzmuskelschwäche, endotheliale Dysfunktion, zur Verhinderung von Restenosen wie nach Thrombolysetherapien, percutan-transluminalen Angioplastien (PTA), transluminalen Koronarangioplastien (PTCA), Herztransplantationen und Bypass-Operationen, sowie mikro- und makrovaskuläre Schädigungen (Vasculitis), erhöhte Spiegel von Fibrinogen und von LDL geringer Dichte sowie erhöhte Konzentrationen von Plasminogenaktivator-Inhibitor 1 (PAI-1), sowie zur Behandlung und/oder Prophylaxe von erektiler Dysfunktion und weiblicher sexueller Dysfunktion eingesetzt werden.

Im Sinne der vorliegenden Erfindung umfasst der Begriff Herzinsuffizienz sowohl akute als auch chronische Erscheinungsformen der Herzinsuffizienz, wie auch spezifischere oder verwandte Krankheitsformen wie akut dekompensierte Herzinsuffizienz, Rechtsherzinsuffizienz, Linksherzinsuffizienz, Globalinsuffizienz, ischämische Kardiomyopathie, dilatative Kardiomyopathie, hypertrophe Kardiomyopathie, idiopathische Kardiomyopathie, angeborene Herzfehler, Herzinsuffizienz bei Herzklappenfehlern, Mitralklappenstenose, Mitralklappeninsuffizienz, Aortenklappenstenose, Aortenklappeninsuffizienz, Trikuspidalstenose, Trikuspidalinsuffizienz, Pulmonalklappenstenose, Pulmonalklappeninsuffizienz, kombinierte Herzklappenfehler, Herzmuskelentzündung (Myokarditis), chronische Myokarditis, akute Myokarditis, virale Myokarditis, diabetische Herzinsuffizienz, alkoholtoxische Kardiomyopathie, kardiale Speichererkrankungen, diastolische Herzinsuffizienz sowie systolische Herzinsuffizienz und akute Phasen der Verschlechterung einer bestehenden chronischen Herzinsuffizienz (worsening heart failure).

Darüber hinaus können die erfindungsgemäßen Verbindungen auch zur Behandlung und/oder Prophylaxe von Arteriosklerose, Lipidstoffwechselstörungen, Hypolipoproteinämien, Dyslipidämien, Hypertriglyceridämien, Hyperlipidämien, Hypercholesterolämien, Abetelipoproteinämie, Sitosterolämie, Xanthomatose, Tangier Krankheit, Fettsucht (Adipositas), Fettleibigkeit (Obesitas) und von kombinierten Hyperlipidämien sowie des Metabolischen Syndroms eingesetzt werden.

Außerdem können die erfindungsgemäßen Verbindungen zur Behandlung und/oder Prophylaxe von primärem und sekundärem Raynaud-Phänomen, von Mikrozirkulationsstörungen, Claudicatio, peripheren und autonomen Neuropathien, diabetischen Mikroangiopathien, diabetischer Retinopathie, diabetischen Geschwüren an den Extremitäten, Gangren, CREST-Syndrom, Erythematose, Onychomykose, rheumatischen Erkrankungen sowie zur Förderung der Wundheilung verwendet werden. Die erfindungsgemäßen Verbindungen eignen sich auch zur Behandlung der Muskeldystrophie, wie der Muskeldystrophie Becker-Kiener (BMD) und Muskeldystrophie Duchenne (DMD).

Weiterhin eignen sich die erfindungsgemäßen Verbindungen zur Behandlung urologischer Erkrankungen wie beispielsweise benignes Prostata-Syndrom (BPS), benigne Prostata-Hyperplasie (BPH), benigne Prostata Vergrösserung (BPE), Blasenentleerungsstörung (BOO), untere Harnwegssyndrome (LUTS, einschließlich Felines Urologisches Syndrom (FUS)), Erkrankungen des Urogenital-Systems einschliesslich neurogene überaktive Blase (OAB) und (IC), Inkontinenz (UI) wie beispielsweise Misch-, Drang-, Stress-, oder Überlauf-Inkontinenz (MUI, UUI, SUI, OUI), Beckenschmerzen, benigne und maligne Erkrankungen der Organe des männlichen und weiblichen Urogenital-Systems.

Weiterhin eignen sich die erfindungsgemäßen Verbindungen zur Behandlung und/oder Prophylaxe von Nierenerkrankungen, insbesondere von aktuer und chronischer Niereninsuffizienz, sowie von akutem und chronischem Nierenversagen. Im Sinne der vorliegenden Erfindung umfasst der Begriff Niereninsuffizienz sowohl akute als auch chronische Erscheinungsformen der Niereninsuffizienz, wie auch zugrundeliegende oder verwandte Nierenerkrankungen wie renale Hypoperfusion, intradialytische Hypotonie, obstruktive Uropathie, Glomerulopathien, Glomerulonephritis, akute Glomerulonephritis, Glomerulosklerose, tubulointerstitielle Erkrankungen, nephropathische Erkrankungen wie primäre und angeborene Nierenerkrankung, Nierenentzündung, immunologische Nierenerkrankungen wie Nierentransplantatabstoßung, Immunkomplex-induzierte Nierenerkrankungen, durch toxische Substanzen induzierte Nephropathie, Kontrastmittelinduzierte Nephropathie, diabetische und nicht-diabetische Nephropathie, Pyelonephritis, Nierenzysten, Nephrosklerose, hypertensive Nephrosklerose und nephrotisches Syndrom, welche diagnostisch beispielsweise durch abnorm verminderte Kreatinin- und/oder Wasser-Ausscheidung, abnorm erhöhte Blutkonzentrationen von Harnstoff, Stickstoff, Kalium und/oder Kreatinin, veränderte Aktivität von Nierenenzymen wie z.B. Glutamylsynthetase, veränderte Urinosmolarität oder Urinmenge, erhöhte Mikroalbuminurie, Makroalbuminurie, Läsionen an Glomerula und Arteriolen, tubuläre Dilatation, Hyperphosphatämie und/oder die Notwendigkeit zur Dialyse charakterisiert werden können. Die vorliegende Erfindung umfasst auch die Verwendung der erfindungsgemäßen Verbindungen zur Behandlung und/oder Prophylaxe von Folgeerscheinungen einer Niereninsuffizienz, wie beispielsweise Lungenödem, Herzinsuffizienz, Urämie, Anämie, Elektrolytstörungen (z.B. Hyperkalämie, Hyponaträmie) und Störungen im Knochen- und Kohlenhydrat-Metabolismus.

Weiterhin eignen sich die erfindungsgemäßen Verbindungen auch zur Behandlung und/oder Prophylaxe von asthmatischen Erkrankungen, pulmonaler arterieller Hypertonie (PAH) und anderen Formen der pulmonalen Hypertonie (PH), umfassend mit Linksherzerkrankung, HIV, Sichelzellanämie, Thromboembolien (CTEPH), Sarkoidose, COPD oder Lungenfibrose assoziierte pulmonale Hypertonie, der chronisch-obstruktive Lungenerkrankung (COPD), des akuten Atemwegssyndrom (ARDS), der akuten Lungenschädigung (ALI), der alpha-1-Antitrypsin-Defizienz (AATD), der Lungenfibrose, des Lungenemphysem (z.B. durch Zigarettenrauch induziertes Lungenemphysem) und der zystischen Fibrose (CF). Außerdem können die genannten Verbindungen als Bronchodilatatoren eingesetzt werden.

Die in der vorliegenden Erfindung beschriebenen Verbindungen stellen auch Wirkstoffe zur Bekämpfung von Krankheiten im Zentralnervensystem dar, die durch Störungen des NO/cGMP-Systems gekennzeichnet sind. Insbesondere sind sie geeignet zur Verbesserung der Wahrnehmung, Konzentrationsleistung, Lernleistung oder Gedächtnisleistung nach kognitiven Störungen, wie sie insbesondere bei Situationen/Krankheiten/Syndromen auftreten wie "Mild cognitive impairment", altersassoziierten Lern- und Gedächtnisstörungen, altersassoziierten Gedächtnisverlusten, vaskulärer Demenz, Schädel-Hirn-Trauma, Schlaganfall, Demenz, die nach Schlaganfällen auftritt ("post stroke dementia"), post-traumatischem Schädel-Hirn-Trauma, allgemeinen Konzentrationsstörungen, Konzentrationsstörungen bei Kindern mit Lern- und Gedächtnisproblemen, Alzheimer'scher Krankheit, Demenz mit Lewy-Körperchen, Demenz mit Degeneration der Frontallappen einschliesslich des Pick's-Syndroms, Parkinson'scher Krankheit, progressiver nuclear palsy, Demenz mit corticobasaler Degeneration, Amyolateralsklerose (ALS), Huntington'scher Krankheit, Demyelinisation, Multipler Sklerose, Thalamischer Degeneration, Creutzfeld-Jacob-Demenz, HIV-Demenz, Schizophrenie mit Demenz oder Korsakoff-Psychose. Sie eignen sich auch zur Behandlung und/oder Prophylaxe von Erkrankungen des Zentralnervensystems wie Angst-, Spannungs- und Depressionszuständen, zentral-nervös bedingten Sexualdysfunktionen und Schlafstörungen sowie zur Regulierung krankhafter Störungen der Nahrungs-, Genuss- und Suchtmittelaufnahme.

Weiterhin eignen sich die erfindungsgemäßen Verbindungen auch zur Regulation der cerebralen Durchblutung und stellen wirkungsvolle Mittel zur Bekämpfung von Migräne dar. Auch eignen sie sich zur Prophylaxe und Bekämpfung der Folgen cerebraler Infarktgeschehen (Apoplexia cerebri) wie Schlaganfall, cerebraler Ischämien und des Schädel-Hirn-Traumas. Ebenso können die erfindungsgemäßen Verbindungen zur Bekämpfung von Schmerzzuständen und Tinnitus eingesetzt werden.

Zudem besitzen die erfindungsgemäßen Verbindungen antünflammatorische Wirkung und können daher als entzündungshemmende Mittel zur Behandlung und/oder Prophylaxe von Sepsis (SIRS), multiplem Organversagen (MODS, MOF), entzündlichen Erkrankungen der Niere, chronischen Darmentzündungen (IBD, Crohn's Disease, UC), Pankreatitis, Peritonitis, rheumatoiden Erkrankungen, entzündlichen Hauterkrankungen sowie entzündlichen Augenerkrankungen eingesetzt werden.

Desweiteren können die erfindungsgemäßen Verbindungen ebenfalls zur Behandlung und/ oder Prophylaxe von Autoimmunerkrankungen eingesetzt werden.

Weiterhin sind die erfindungsgemäßen Verbindungen zur Behandlung und/oder Prophylaxe fibrotischer Erkrankungen der inneren Organe, wie beispielsweise der Lunge, des Herzens, der Niere, des Knochenmarks und insbesondere der Leber, sowie dermatologischer Fibrosen und fibrotischer Erkrankungen des Auges, geeignet. Im Sinne der vorliegenden Erfindungen umfasst der Begriff fibrotischer Erkrankungen insbesondere die folgenden Begriffe Leberfibrose, Leberzirrhose, Lungenfibrose, Endomyocardfibrose, Nephropathie, Glomerulonephritis, interstitielle Nierenfibrose, fibrotische Schäden in Folge von Diabetes, Knochenmarksfibrose und ähnliche fibrotische Erkrankungen, Sklerodermie, digitale Ulzerationen, Morphaea, Keloide, hypertrophe Narbenbildung (auch nach chirurgischen Eingriffen), Naevi, diabetische Retinopathie, proliferative Vitroretinopathie und Erkrankungen des Bindegewebes (z.B. Sarkoidose).

Weiterhin eignen sich die erfindungsgemäßen Verbindungen zur Bekämpfung postoperativer Narbenbildung, z.B. in Folge von Glaukom-Operationen.

Die erfindungsgemäßen Verbindungen können ebenfalls kosmetisch bei alternder und verhornender Haut eingesetzt werden.

Außerdem sind die erfindungsgemäßen Verbindungen zur Behandlung und/ oder Prophylaxe von Hepatitis, Neoplasma, Osteoporose, Glaukom und Gastroparese geeignet.

Weiterer Gegenstand der vorliegenden Erfindung ist die Verbindung der Formel (I) zur Verwendung bei der Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

Die erfindungsgemäßen Verbindungen können allein oder bei Bedarf in Kombination mit anderen Wirkstoffen eingesetzt werden. Weiterer Gegenstand der vorliegenden Erfindung sind Arzneimittel, enthaltend mindestens eine der erfindungsgemäßen Verbindungen und einen oder mehrere weitere Wirkstoffe, insbesondere zur Behandlung und/oder Prophylaxe der zuvor genannten Erkrankungen. Als geeignete Kombinationswirkstoffe seien beispielhaft und vorzugsweise genannt:
- organische Nitrate und NO-Donatoren, wie beispielsweise Natriumnitroprussid, Nitroglycerin, Isosorbidmononitrat, Isosorbiddinitrat, Molsidomin oder SIN-1, sowie inhalatives NO;
- Verbindungen, die den Abbau von cyclischem Guanosinmonophosphat (cGMP) inhibieren, wie beispielsweise Inhibitoren der Phosphodiesterasen (PDE) 1, 2 und/oder 5, insbesondere PDE 5-Inhibitoren wie Sildenafil, Vardenafil und Tadalafil;
- antithrombotisch wirkende Mittel, beispielhaft und vorzugsweise aus der Gruppe der Thrombozytenaggregationshemmer, der Antikoagulantien oder der profibrinolytischen Substanzen;
- den Blutdruck senkende Wirkstoffe, beispielhaft und vorzugsweise aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika; und/oder
- den Fettstoffwechsel verändernde Wirkstoffe, beispielhaft und vorzugsweise aus der Gruppe der Thyroidrezeptor-Agonisten, Cholesterinsynthese-Inhibitoren wie beispielhaft und vorzugsweise HMG-CoA-Reduktase- oder Squalensynthese-Inhibitoren, der ACAT-Inhibitoren, CETP-Inhibitoren, MTP-Inhibitoren, PPAR-alpha-, PPAR-gamma- und/oder PPAR-delta-Agonisten, Cholesterin-Absorptionshemmer, Lipase-Inhibitoren, polymeren Gallensäureadsorber, Gallensäure-Reabsorptionshemmer und Lipoprotein(a)-Antagonisten.

Unter antithrombotisch wirkenden Mittel werden vorzugsweise Verbindungen aus der Gruppe der Thrombozytenaggregationshemmer, der Antikoagulantien oder der profibrinolytischen Substanzen verstanden.

Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Ximelagatran, Dabigatran, Melagatran, Bivalirudin oder Clexane, verabreicht.

Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibitor, wie beispielhaft und vorzugsweise Rivaroxaban, DU-176b, Apixaban, Otamixaban, Fidexaban, Razaxaban, Fondaparinux, Idraparinux, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitamin K-Antagonisten, wie beispielhaft und vorzugsweise Coumarin, verabreicht.

Unter den Blutdruck senkenden Mitteln werden vorzugsweise Verbindungen aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika verstanden.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem alpha-1-Rezeptoren-Blocker, wie beispielhaft und vorzugsweise Prazosin, verabreicht.

Bei einer bevorzugten Ausführungsform der Erfindung werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem Angiotensin AII-Antagonisten, wie beispielhaft und vorzugsweise Losartan, Candesartan, Valsartan, Telmisartan oder Embursatan, verabreicht.

Bei einer bevorzugten Ausführungsform der Erfindung werden die erfindungsgemäßen Verbindungen in Kombination mit einem ACE-Hemmer, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem Endothelin-Antagonisten, wie beispielhaft und vorzugsweise Bosentan, Darusentan, Ambrisentan oder Sitaxsentan, verabreicht.

Bei einer bevorzugten Ausführungsform der Erfindung werden die erfindungsgemäßen Verbindungen in Kombination mit einem Renin-Inhibitor, wie beispielhaft und vorzugsweise Aliskiren, SPP-600 oder SPP-800, verabreicht.

Bei einer bevorzugten Ausführungsform der Erfindung werden die erfindungsgemäßen Verbindungen in Kombination mit einem Mineralocorticoid-Rezeptor-Antagonisten, wie beispielhaft und vorzugsweise Spironolacton oder Eplerenon, verabreicht.

Bei einer bevorzugten Ausführungsform der Erfindung werden die erfindungsgemäßen Verbindungen in Kombination mit einem Schleifendiuretikum, wie beispielsweise Furosemid, Torasemid, Bumetanid und Piretanid, mit kaliumsparenden Diuretika wie beispielsweise Amilorid und Triamteren, mit Aldosteronantagonisten, wie beispielsweise Spironolacton, Kaliumcanrenoat und Eplerenon sowie Thiaziddiuretika, wie beispielsweise Hydrochlorothiazid, Chlorthalidon, Xipamid, und Indapamid, verabreicht.

Unter den Fettstoffwechsel verändernden Mitteln werden vorzugsweise Verbindungen aus der Gruppe der CETP-Inhibitoren, Thyroidrezeptor-Agonisten, Cholesterinsynthese-Inhibitoren wie HMG-CoA-Reduktase- oder Squalensynthese-Inhibitoren, der ACAT-Inhibitoren, MTP-Inhibitoren, PPAR-alpha-, PPAR-gamma- und/oder PPAR-delta-Agonisten, Cholesterin-Absorptionshemmer, polymeren Gallensäureadsorber, Gallensäure-Reabsorptionshemmer, Lipase-Inhibitoren sowie der Lipoprotein(a)-Antagonisten verstanden.

Bei einer bevorzugten Ausführungsform der Erfindung werden die erfindungsgemäßen Verbindungen in Kombination mit einem CETP-Inhibitor, wie beispielhaft und vorzugsweise Dalcetrapib, BAY 60-5521, Anacetrapib oder CETP-vaccine (CETi-1), verabreicht.

Bei einer bevorzugten Ausführungsform der Erfindung werden die erfindungsgemäßen Verbindungen in Kombination mit einem Thyroidrezeptor-Agonisten, wie beispielhaft und vorzugsweise D-Thyroxin, 3,5,3'-Triiodothyronin (T3), CGS 23425 oder Axitirome (CGS 26214), verabreicht.

Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem PPAR-gamma-Agonisten, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-delta-Agonisten, wie beispielhaft und vorzugsweise GW 501516 oder BAY 68-5042, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Lipoprotein(a)-Antagonisten, wie beispielhaft und vorzugsweise Gemcabene calcium (CI-1027) oder Nicotinsäure, verabreicht.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.001 bis 2 mg/kg, vorzugsweise etwa 0.001 bis 1 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen und Akronyme:

- aq.: wässrige Lösung
- abs.: absolut
- ber.: berechnet
- br.: Verbreitertes Signal (NMR Kupplungsmuster)
- CAS-Nr.: Chemical Abstracts Service Nummer
- δ: Verschiebung im NMR Spektrum (Angabe in ppm)
- d: Dublett (NMR-Kupplungsmuster)
- DC: Dünnschichtchromatographie
- DCI: direkte chemische Ionisation (bei MS)
- DMAP: 4-*N*,*N*-Dimethylaminopyridin
- DMF: Dimethylformamid
- DMSO: Dimethylsulfoxid
- EDCI: *N*-[3-(Dimethylamino)propyl]-N'-ethylcarbodiimid
- d. Th.: der Theorie (bei Ausbeute)
- eq.: Äquivalent(e)
- ESI: Elektrospray-Ionisation (bei MS)
- Et: Ethyl
- gef.: gefunden
- h: Stunde(n)
- HATU: N-[(Dimethylamino)(3H-[1,2,3]triazolo[4,5-b]-pyridin-3-yloxy)methylen]-N-methylmethanaminiumhexafluorophosphat
- HOBT: 1H-Benzotriazol-1-ol
- HPLC: Hochdruck-, Hochleistungsflüssigchromatographie
- HRMS: hochaufgelöste Massenspektrometrie
- konz.: konzentriert
- LC-MS: Flüssigchromatographie-gekoppelte Massenspektrometrie
- LiHMDS: Lithiumhexamethyldisilazid
- m: Multiplett
- Me: Methyl
- min: Minute(n)
- MS: Massenspektrometrie
- NMP: 1-Methyl-2-pyrrolidon
- NMR: Kernresonanzspektrometrie
- Pd₂dba₃: Tris-(dibenzylidenaceton)-dipalladium
- Ph: Phenyl
- q: Quartett (NMR Kupplungsmuster)
- quint.: Quintett (NMR Kupplungsmuster)
- R_{F}: Retentionsfaktor (bei Dünnschichtchromatographie)
- RT: Raumtemperatur
- Rₜ: Retentionszeit (bei HPLC)
- s: Singulett (NMR Kupplungsmuster)
- SFC: Supercritical Fluid Chromatography = Superkritische Flüssigkeitschromatographie
- t: Triplett (NMR Kupplungsmuster)
- THF: Tetrahydrofuran
- TBTU: (Benzotriazol-1-yloxy)bisdimethylaminomethyliumfluoroborat
- UV: Ultraviolett-Spektrometrie
- v/v: Volumen zu Volumen-Verhältnis (einer Lösung)
- Xantphos: 4,5-Bis(diphenylphosphino)-9,9-dimethylxanthene
- XPHOS: Dicyclohexyl-(2',4',6'-triisopropylbiphenyl-2-yl)-phosphin

### LC/MS- und HPLC-Methoden:

### Methode 1 (LC-MS):

Instrument: Waters ACQUITY SQD UPLC System; Säule: Waters Acquity UPLC HSS T3 1,8µ 50 x 1mm; Eluent A: 1 l Wasser + 0.25 ml 99%ige Ameisensäure, Eluent B: 1 l Acetonitril + 0.25 ml 99%ige Ameisensäure; Gradient: 0.0 min 90% A → 1.2 min 5% A → 2.0 min 5% A; Ofen: 50°C; Fluss: 0.40 ml/min; UV-Detektion: 210 - 400 nm.

### Methode 2 (LC-MS):

Instrument: Micromass Quattro Premier mit Waters UPLC Acquity; Säule: Thermo Hypersil GOLD 1.9 µ 50 mm x 1 mm; Eluent A: 1 l Wasser + 0.5 ml 50%-ige Ameisensäure, Eluent B: 1 l Acetonitril + 0.5 ml 50%-ige Ameisensäure; Gradient: 0.0 min 90% A → 0.1 min 90% A → 1.5 min 10% A → 2.2 min 10% A; Fluss: 0.33 ml/min; Ofen: 50°C; UV-Detektion: 210 nm.

### Methode 3 (LC-MS):

Gerätetyp MS: Waters Micromass Quattro Micro; Gerätetyp HPLC: Agilent 1100 Serie; Säule: Thermo Hypersil GOLD 3 µ 20 mm x 4 mm; Eluent A: 1 l Wasser + 0.5 ml 50%-ige Ameisensäure, Eluent B: 1 l Acetonitril + 0.5 ml 50%-ige Ameisensäure; Gradient: 0.0 min 100% A → 3.0 min 10% A → 4.0 min 10% A → 4.01 min 100% A (Fluss 2.5 ml/min) → 5.00 min 100% A; Ofen: 50°C; Fluss: 2 ml/min; UV-Detektion: 210 nm.

### Methode 4 (LC-MS):

Instrument MS: Waters SQD; Instrument HPLC: Waters UPLC; Säule: Zorbax SB-Aq (Agilent), 50 mm x 2.1 mm, 1.8 µm; Eluent A: Wasser + 0.025% Ameisensäure, Eluent B: Acetonitril (ULC) + 0.025% Ameisensäure; Gradient: 0.0 min 98% A - 0.9 min 25% A - 1.0 min 5% A - 1.4 min 5% A - 1.41 min 98% A - 1.5 min 98% A; Ofen: 40°C; Fluss: 0.600 ml/min; UV-Detektion: DAD; 210 nm.

### Methode 5 (LC-MS):

Instrument MS: Waters ZQ 2000; Instrument HPLC: Agilent 1100, 2-Säulen-Schaltung, Autosampler: HTC PAL; Säule: YMC-ODS-AQ, 50 mm x 4.6 mm, 3.0 µm; Eluent A: Wasser + 0.1% Ameisensäure, Eluent B: Acetonitril + 0.1% Ameisensäure; Gradient: 0.0 min 100% A - 0.2 min 95% A - 1.8 min 25% A - 1.9 min 10% A - 2.0 min 5% A - 3.2 min 5% A - 3.21 min 100% A - 3.35 min 100% A; Ofen: 40°C; Fluss: 3.0 ml/min; UV-Detektion: 210 nm.

### Methode 6 (präparative HPLC):

Säule: Macherey-Nagel VP 50/21 Nucleosil 100-5 C18 Nautilus. Flußrate: 25 ml/min. Gradient: A = Acetonitril, B= Wasser +0.1% Ameisensäure, 0 min 10% A ; 2.00 min 10% A ; 6.00 min 90% A ; 7.00 min 90% A ; 7.10 min 10% A ; 8 min 10% A; UV-Detektion: 220 nm

### Methode 7 (präparative HPLC):

Säule: Phenomenex Gemini C18; 110A, AXIA, 5 µm, 21.2 X 50 mm 5micron; Gradient: A = Wasser + 0.1 % konz. Ammoniak , B = Acetonitril, 0 min = 10% B, 2 min = 10% B, 6 min = 90% B, 7 min = 90% B, 7.1 min = 10% B, 8 min = 10% B, Flußrate 25 ml/min, UV-Detektion 220 nm.

### Methode 8 (präparative HPLC):

Säule: Axia Gemini 5 µ C18 110 A, 50 x 21,5 mm, P/NO: 00B-4435-P0-AX, S/NO: 35997-2, Gradient: A=Wasser + 0.1 % konz. wäss. Ammoniak , B = Acetonitril, 0 min = 30 % B, 2 min = 30% B, 6 min = 100% B, 7 min = 100% B, 7,1 Min = 30% B, 8 Min=30% B, Flußrate 25 ml/min, UV-Detektion 220 nm.

### Methode 9 (präparative HPLC):

Säule: Macherey-Nagel VP 50/21 Nucleosil 100-5 C18 Nautilus. Flussrate: 25 ml/min. Gradient: A = Wasser + 0.1 % Ameisensäure, B = Methanol, 0 min = 30 % B, 2 min = 30% B, 6 min = 100% B, 7 min = 100% B, 7.1 min = 30% B, 8 min = 30% B, Flussrate 25 ml/min, UV-Detektion 220 nm.

### Methode 10 (präparative HPLC):

Säule: Macherey-Nagel VP 50/21 Nucleosil 100-5 C18 Nautilus. Flussrate: 25 ml/min. Gradient: A = Wasser + 0.1 % konz. aq. Ammoniak, B = Methanol, 0 min = 30 % B, 2 min = 30% B, 6 min = 100% B, 7 min = 100% B, 7.1 min = 30% B, 8 min = 30% B, Flussrate 25 ml/min, UV-Detektion 220 nm.

### Methode 11 (präparative HPLC):

Instrument MS: Waters, Instrument HPLC: Waters (Säule Waters X-Bridge C18, 18 mm x 50 mm, 5 µm, Eluent A: Wasser + 0.05% Triethylamin, Eluent B: Acetonitril (ULC) + 0.05% Triethylamin, Gradient: 0.0 min 95%A - 0.15 min 95%A - 8.0 min 5%A - 9.0 min 5%A; Fluss: 40 ml/min; UV-Detektion: DAD; 210 - 400 nm).
bzw.:

Instrument MS: Waters, Instrument HPLC: Waters (Säule Phenomenex Luna 5µ C18(2) 100A, AXIA Tech. 50 x 21.2 mm, Eluent A: Wasser + 0.05% Ameisensäuren, Eluent B: Acetonitril (ULC) + 0.05% Ameisensäure, Gradient: 0.0 min 95%A - 0.15 min 95%A - 8.0 min 5%A - 9.0 min 5%A; Fluss: 40 ml/min; UV-Detektion: DAD; 210 - 400 nm).

### Methode 12 (LC-MS):

Instrument MS: Waters SQD; Instrument HPLC: Waters UPLC; Saeule: Zorbax SB-Aq (Agilent), 50 mm x 2.1 mm, 1.8 µm; Eluent A: Wasser + 0.025% Ameisensaeure, Eluent B: Acetonitril (ULC) + 0.025% Ameisensaeure; Gradient: 0.0 min 98%A - 0.9 min 25%A - 1.0 min 5%A - 1.4 min 5%A - 1.41 min 98%A - 1.5 min 98%A; Ofen: 40°C; Fluss: 0.600 ml/min; UV-Detektion: DAD; 210 nm.

### Methode 13 (DCI-MS

Gerät: DSQ II; Thermo Fisher-Scientific; DCI mit NH₃, Fluss: 1.1 ml/min; Quellentemeperatur: 200°C; Ionisierungsenergie 70 eV; DCI-Heizfaden bis 800°C aufheizen; Mass-Range 80-900.

### Methode 14 (GC-MS):

Instrument: Micromass GCT, GC6890; Säule: Restek RTX-35, 15 m x 200 µm x 0.33 µm; konstanter Fluss mit Helium: 0.88 ml/min; Ofen: 70°C; Inlet: 250°C; Gradient: 70°C, 30°C/min → 310°C (3 min halten).

### Methode 15 (MS):

Gerät: Waters ZQ; Ionisierungsart: ESI (+); Laufmittel; Acetonitril/Wasser.

### Methode 16 (LCMS):

Instrument: Waters ACQUITY SQD UPLC System; Säule: Waters Acquity UPLC HSS T3 1.8 µ 30 x 2 mm; Eluent A: 1 l Wasser + 0.25 ml 99%ige Ameisensäure, Eluent B: 1 l Acetonitril + 0.25 ml 99%ige Ameisensäure; Gradient: 0.0 min 90% A → 1.2 min 5% A → 2.0 min 5% A Ofen: 50°C; Fluss: 0.60 ml/min; UV-Detektion: 208 - 400 nm.

### Methode 17 (LC-MS):

Instrument: Micromass Quattro Premier mit Waters UPLC Acquity; Säule: Thermo Hypersil GOLD 1.9 µ 50 x 1 mm; Eluent A: 1 l Wasser + 0.5 ml 50%ige Ameisensäure, Eluent B: 1 l Acetonitril + 0.5 ml 50%ige Ameisensäure; Gradient: 0.0 min 97% A → 0.5 min 97% A → 3.2 min 5% A → 4.0 min 5% A Ofen: 50°C; Fluss: 0.3 ml/min; UV-Detektion: 210 nm.

### Methode 18 (präparative HPLC):

Chromatorex C18 10µ 250x20mm Gradient: A = Wasser + 0,5% Ameisensäure, B= Acetonitril, 0min = 5% B, 3min = 5% B vorspülen ohne Substanz, dann Injektion, 5 min = 5% B, 25min = 30% B, 38min = 30% B, 38,1min = 95% B, 43min= 95% B, 43,01min= 5% B, 48,0min= 5% B Flussrate 20 ml/min, Wellenlänge 210 nm.

### Methode 19 (präparative HPLC):

Chromatorex C18 10µ 250x20mm Gradient: A = Wasser + 0,5% Ameisensäure, B= Acetonitril, 0min = 5% B, 3min = 5% B vorspülen ohne Substanz, dann Injektion, 5 min = 5% B, 25min = 50% B, 38min = 50% B, 38,1min = 95% B, 43min= 95% B, 43,01min= 5% B, 48,0min= 5% B Flussrate 20 ml/min, Wellenlänge 210 nm.

### Methode 20 (präparative HPLC):

XBridge Prep. C18 5µ 50x19mm Gradient: A = Wasser + 0.5% Ammoniumhydroxid, B= Acetonitril, 0min = 5% B, 3min = 5% B vorspülen ohne Substanz, dann Injektion, 5 min = 5% B, 25 min = 50% B, 38 min = 50% B, 38.1 min = 95% B, 43 min= 95% B, 43.01min= 5% B, 48.0 min= 5% B Flussrate 15 ml/min, Wellenlänge 210 nm

### Methode 21 (präparative HPLC):

Chromatorex 10µ 250x20mm Gradient: A = Wasser, B = Acetonitril, 0min = 5 % B, 3min = 5% B vorspülen ohne Substanz, dann Injektion, 5min = 5% B, 25min = 95% B, 38min = 95% B, 38,1min = 5% B, 40min=5% B, Flussrate 20 ml/min, Wellenlänge 210 nm.

### Methode 22 (LC-MS):

Instrument MS: Waters (Micromass) QM; Instrument HPLC: Agilent 1100 Serie; Säule: Agilent ZORBAX Extend-C18 3.0x50mm 3.5-Micron; Eluent A: 1 l Wasser + 0.01 mol Ammoniumcarbonat, Eluent B: 1 l Acetonitril; Gradient: 0.0 min 98% A → 0.2min 98% A → 3.0 min 5% A→ 4.5 min 5% A; Ofen: 40°C; Fluss: 1.75 ml/min; UV-Detektion: 210 nm.

### Methode 23 (LC-MS):

Instrument: Agilent MS Quad 6150; HPLC: Agilent 1290; Säule: Waters Acquity UPLC HSS T3 1.8 µ 50 x 2.1 mm; Eluent A: 1 l Wasser + 0.25 ml 99%ige Ameisensäure, Eluent B: 1 l Acetonitril + 0.25 ml 99%ige Ameisensäure; Gradient: 0.0 min 90% A → 0.3 min 90% A → 1.7 min 5% A → 3.0 min 5% A Ofen: 50°C; Fluss: 1,20 ml/min; UV-Detektion: 205 - 305 nm.

### Methode 24 (LC-MS):

Gerätetyp MS: Waters Synapt G2S; Gerätetyp UPLC: Waters Acquity I-CLASS; Säule: Waters, HSST3, 2.1 x 50 mm, C18 1.8 µm; Eluent A: 1 l Wasser + 0.01% Ameisensäure; Eluent B: 1 l Acetonitril + 0.01% Ameisensäure; Gradient: 0.0 min 10% B → 0.3 min 10% B → 1.7 min 95% B → 2.5 min 95% B; Ofen: 50°C; Fluss: 1.20 ml/min; UV-Detektion: 210 nm.

### Methode 25 (FIA/MS, ES):

Instrument: Waters ZQ 2000; Elektrospray-Ionisierung; Eluent A: 1 l Wasser + 0.25 ml 99%ige Ameisensäure , Eluent B: 1 l Acetonitril + 0.25 ml 99%ige Ameisensäure; 25% A, 75% B; Fluss: 0.25 ml/min

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

Die in den folgenden Paragraphen angegebenen Multiplizitäten von Protonensignalen in ¹H-NMR-Spektren geben die jeweils beobachtete Signalform wieder und berücksichtigen keine Signalphänomene höherer Ordnung. Alle Angaben in ¹H-NMR-Spektren geben die Chemischen Verschiebungen δ in ppm an.

Bei Aufreinigungen von erfindungsgemäßen Verbindungen per präparativer HPLC nach den oben beschriebenen Methoden, in denen die Elutionsmittel Zusatzstoffe wie beispielsweise Trifluoressigsäure, Ameisensäure oder Ammoniak enthalten, können die erfindungsgemäßen Verbindungen in Salz-Form, beispielsweise als Trifluoracetat, Formiat oder Ammonium-Salz anfallen, sofern die erfindungsgemäßen Verbindungen eine ausreichend basische bzw. saure Funktionalität enthalten. Ein solches Salz kann durch verschiedene dem Fachmann bekannte Methoden in die entsprechende freie Base bzw. Säure überführt werden.

Die im Folgenden in Synthese-Intermediaten und Ausführungsbeispielen der Erfindung beschriebenen Imidazopyridine können unter sauren Bedingungen stets Salze, auch unterstöchiometrisch, vorliegen, ohne dass diese im ¹H-NMR erkenntlich sind und ohne besondere Angabe und Kennzeichnung dieser in den jeweiligen IUPAC-Namen und Strukturformeln.

Wenn bei den im Folgenden beschriebenen Synthese-Intermediaten und Ausführungsbeispielen der Erfindung eine Verbindung in der Form eines Salzes der korrespondierenden Base bzw. Säure aufgeführt ist, so ist die exakte stöchiometrische Zusammensetzung eines solchen Salzes, wie es nach dem jeweiligen Herstell- und/oder Reinigungsverfahren erhalten wurde, in der Regel nicht bekannt. Sofern nicht genauer spezifiziert, sind daher Namens- und Strukturformel-Zusätze wie beispielsweise "Hydrochlorid", "Trifluoracetat", "Natrium-Salz" bzw. "x HCl", "x CF₃COOH", "x Na⁺" bei solchen Salzen nicht stöchiometrisch zu verstehen, sondern haben allein deskriptiven Charakter bezüglich der enthaltenen salzbildenden Komponenten.

Sinngemäß gleiches gilt für den Fall, dass Synthese-Intermediate oder Ausführungsbeispiele oder Salze hiervon nach den beschriebenen Herstell- und/oder Reinigungsverfahren in Form von Solvaten, wie beispielsweise Hydraten, erhalten wurden, deren stöchiometrische Zusammensetzung (sofern definierter Art) nicht bekannt ist.

### Ausgangsverbindungen und Intermediate:

### Beispiel 1A

### 3-[(2,6-Difluorbenzyl)oxy]pyridin-2-amin

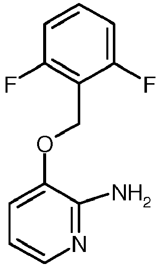

51 g Natriummethanolat (953 mmol, 1.05 Äquivalente) wurden in 1000 ml Methanol bei RT vorgelegt, mit 100 g 2-Amino-3-hydroxypyridin (908 mmol, 1 Äquivalent) versetzt und 15 min bei RT weitergerührt. Die Reaktionsmischung wurde am Vakuum eingeengt, der Rückstand in 2500 ml DMSO aufgenommen und mit 197 g 2,6-Difluorbenzylbromid (953 mmol, 1.05 Äquivalente) versetzt. Nach 4 h bei RT wurde das Reaktionsgemisch auf 20 L Wasser gegossen, für 15 min nachgerührt und der Feststoff abfiltriert. Der Feststoff wurde mit 1 L Wasser sowie 100 ml Iso-Propanol und 500 ml Petrolether nachgewaschen und im Hochvakuum getrocknet. Es wurden 171 g der Titelverbindung (78% d. Th) erhalten.
¹H-NMR (400 MHz, DMSO-d₆): δ = 5.10 (s, 2 H); 5.52 (br. s, 2 H), 6.52 (dd, 1 H); 7.16 - 7.21 (m, 3 H); 7.49 - 7.56 (m, 2 H).

### Beispiel 2A

### Ethyl-8-[(2,6-difluorbenzyl)oxy]-2-methylimidazo[1,2-a]pyridin-3-carboxylat

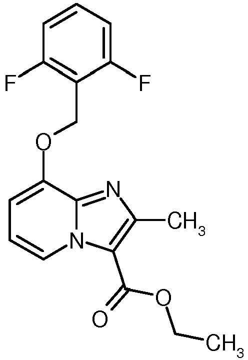

170 g 3-[(2,6-Difluorbenzyl)oxy]pyridin-2-amin (Beispiel 1A; 719 mmol, 1 Äquivalent) wurden in 3800 ml Ethanol vorgelegt, mit 151 g gepulvertem Molekularsieb 3Å und 623 g Ethyl-2-chloracetoacetat (3.6 mol, 5 Äquivalente) versetzt. Die Reaktionsmischung wurde für 24 h zum Rückfluss erhitzt, anschließend über Kieselgel abfiltriert und am Vakuum aufkonzentriert. Es wurde 48 h bei RT belassen und der entstandene Feststoff filtriert. Dann wurde dreimal mit wenig Iso-Propanol gerührt und anschließend abfiltriert und mit Diethylether gewaschen. Es wurden 60.8 g (23% d. Th.) der Titelverbindung erhalten. Die vereinigten Filtrate der Filtrationsschritte wurden eingeengt und der Rückstand an Kieselgel mit Cyclohexan/Diethylether als Eluent chromatographiert. Man erhielt weitere 46.5 g (18% d. Th.; Gesamtausbeute: 41% d. Th.) der Titelverbindung.
LC-MS (Methode 1): Rₜ = 1.01 min
MS (ESpos): m/z = 347 (M+H)⁺
¹H-NMR (400 MHz, DMSO-d₆): δ = 1.36 (t, 3 H); 2.54 (s, 3 H; verdeckt durch DMSO-Signal); 4.36 (q, 2 H); 5.33 (s, 2 H); 7.11 (t, 1 H); 7.18 - 7.27 (m, 3 H); 7.59 (quint, 1 H); 8.88 (d, 1 H).

### Beispiel 3A

### 8-[(2,6-Difluorbenzyl)oxy]-2-methylimidazo[1,2-a]pyridin-3-carbonsäure

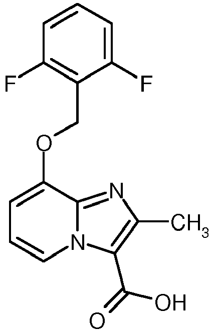

107 g Ethyl-8-[(2,6-difluorbenzyl)oxy]-2-methylimidazo[1,2-a]pyridin-3-carboxylat (Beispiel 2A; 300 mmol, 1 Äquivalent) wurde in 2.8 L THF/Methanol (1:1) gelöst, mit 1.5 L 1 N wässriger Lithiumhydroxid-Lösung (1.5 mol, 5 Äquivalente) versetzt und 16 h bei RT gerührt. Die organischen Lösemittel wurden am Vakuum entfernt und die resultierende wässrige Lösung im Eisbad mit 1 N wässriger Salzsäure auf pH 3-4 eingestellt. Der resultierende Feststoff wurde abfiltriert, mit Wasser und Iso-Propanol nachgewaschen und im Vakuum getrocknet. Es wurden 92 g (95% d. Th.) der Titelverbindung erhalten.
LC-MS (Methode 1): Rₜ = 0.62 min
MS (ESpos): m/z = 319.1 (M+H)⁺
¹H-NMR (400 MHz, DMSO-d₆): δ = 2.55 (s, 3 H; überlagert durch DMSO-Signal); 5.32 (s, 2 H); 7.01 (t, 1 H); 7.09 (d, 1 H); 7.23 (t, 2 H); 7.59 (quint, 1 H); 9.01 (d, 1 H).

### Beispiel 4A

### 5-Chlor-2-nitropyridin-3-ol

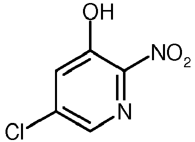

30 g 5-Chlorpyridin-3-ol (232 mmol, 1 Äquivalent) wurden unter Eiskühlung in 228 ml konzentrierter Schwefelsäure gelöst und bei 0 °C langsam mit 24 ml konzentrierter Salpetersäure versetzt. Die Reaktion wurde auf RT erwärmt, über Nacht gerührt und anschließend in ein Eis/Wasser-Gemisch eingerührt und für 30 min nachgerührt. Der Feststoff wurde abfiltriert, mit kaltem Wasser nachgewaschen und an der Luft getrocknet. Es wurden 33 g (82% d. Th.) der Titelverbindung erhalten und ohne weitere Aufreinigung in die Folgereaktion eingesetzt.
LC-MS (Methode 1): Rₜ = 0.60 min
MS (ESneg): m/z = 172.9/174.9 (M-H)⁻
¹H-NMR (400 MHz, DMSO-d₆): δ = 7.71 (d, 1 H); 8.10 (d, 1 H); 12.14 (br. 1 H).

### Beispiel 5A

### 5-Chlor-3-[(2,6-difluorbenzyl)oxy]-2-nitropyridin

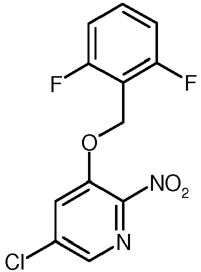

33 g 5-Chlor-2-nitropyridin-3-ol (Beispiel 4A; 189 mmol, 1 Äquivalent) und 61.6 g Cäsiumcarbonat (189 mmol, 1 Äquivalent) wurden in 528 ml DMF vorgelegt, mit 40.4 g 2,6-Difluorbenzylbromid (189 mmol, 1 Äquivalent) versetzt und bei RT über Nacht gerührt. Das Reaktionsgemisch wurde in Wasser/1N wässrige Salzsäure eingerührt. Der Feststoff wurde abfiltriert, mit Wasser nachgewaschen und an der Luft getrocknet. Es wurden 54.9 g (97% d. Th.) der Titelverbindung erhalten.
¹H-NMR (400 MHz, DMSO-d₆): δ = 5.46 (s, 2 H); 7.22 (t, 2 H); 7.58 (q, 1 H); 8.28 (d, 1 H); 8.47 (d, 1 H).

### Beispiel 6A

### 5-Chlor-3-[(2,6-difluorbenzyl)oxy]pyridin-2-amin

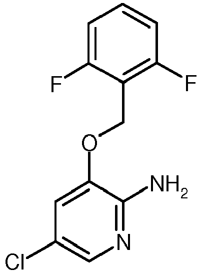

59.7 g 5-Chlor-3-[(2,6-difluorbenzyl)oxy]-2-nitropyridin (Beispiel 5A; 199 mmol, 1 Äquivalent) wurden in 600 ml Ethanol vorgelegt, mit 34.4 g Eisenpulver (616 mmol, 3.1 Äquivalente) versetzt und zum Rückfluss erhitzt. Es wurden langsam 152 ml konzentrierte Salzsäure zugetropft und weitere 30 min am Rückfluss gekocht. Das Reaktionsgemisch wurde abgekühlt und in ein Eis-Wassergemisch eingerührt. Das resultierende Gemisch wurde mit Natriumacetat auf pH 5 eingestellt. Der Feststoff wurde abfiltriert, mit Wasser nachgewaschen und an der Luft und anschließend im Vakuum bei 50°C getrocknet. Es wurden 52.7 g (98% d. Th.) der Titelverbindung erhalten.
LC-MS (Methode 1): Rₜ = 0.93 min
MS (ESpos): m/z = 271.1/273.1 (M+H)⁺
¹H-NMR (400 MHz, DMSO-d₆): δ = 5.14 (s, 2 H); 5.82 (br. s, 2 H); 7.20 (t, 2 H); 7.35 (d, 1 H); 7.55 (q, 1 H); 7.56 (d, 1 H).

### Beispiel 7A

### Ethyl-6-chlor-8-[(2,6-difluorbenzyl)oxy]-2-methylimidazo[1,2-a]pyridin-3-carboxylat

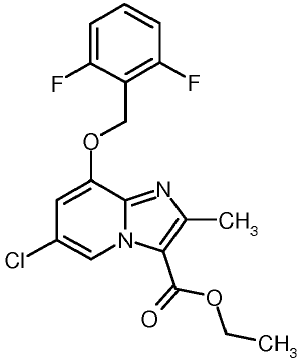

40 g 5-Chlor-3-[(2,6-difluorbenzyl)oxy]pyridin-2-amin (Beispiel 6A; 147.8 mmol; 1 Äquivalent) wurden in 800 ml Ethanol vorgelegt, mit 30 g gepulvertem Molekularsieb 3Å und 128 g Ethyl-2-chloracetoacetat (739 mmol, 5 Äquivalente) versetzt und über Nacht zum Rückfluß erhitzt. Das Reaktionsgemisch wurde eingeengt, der Rückstand in Essigsäureethylester aufgenommen und filtriert. Die Essigsäureethylester-Phase wurde mit Wasser gewaschen, getrocknet, filtriert und eingeengt. Es wurden 44 g (78% d. Th.) der Titelverbindung erhalten.
LC-MS (Methode 1): Rₜ = 1.27 min
MS (ESpos): m/z = 381.2/383.2 (M+H)⁺
¹H-NMR (400 MHz, DMSO-d₆): δ = 1.36 (t, 3 H); 2.54 (s, 3 H; verdeckt durch DMSO-Signal); 4.37 (q, 2 H); 5.36 (s, 2 H); 7.26 (t, 2 H); 7.38 (d, 1 H); 7.62 (q, 1 H); 8.92 (d, 1 H).

### Beispiel 8A

### 6-Chlor-8-[(2,6-difluorbenzyl)oxy]-2-methylimidazo[1,2-a]pyridin-3-carbonsäure

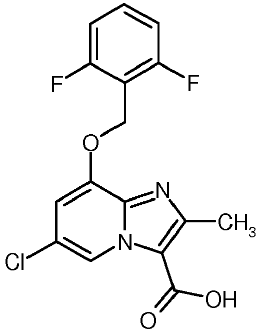

44 g Ethyl-6-chlor-8-[(2,6-difluorbenzyl)oxy]-2-methylimidazo[1,2-a]pyridin-3-carboxylat (Beispiel 7A; 115 mmol, 1 Äquivalent) wurden in 550 ml THF und 700 ml Methanol gelöst, mit 13.8 g Lithiumhydroxid (gelöst in 150 ml Wasser; 577 mmol, 5 Äquivalente) versetzt und über Nacht bei RT gerührt. Es wurde mit 1 N wässriger Salzsäure versetzt und im Vakuum eingeengt. Der erhaltene Feststoff wurde abfiltriert und mit Wasser nachgewaschen. Es wurden 34 g der Titelverbindung (84% d. Th.) erhalten.
LC-MS (Methode 2): Rₜ = 1.03 min
MS (ESpos): m/z = 353.0/355.0 (M+H)⁺
¹H-NMR (400 MHz, DMSO-d₆): δ = 2.54 (s, 3 H; überlagert von DMSO-Signal); 5.36 (s, 2 H); 7.26 (t, 2 H); 7.34 (d, 1 H); 7.61 (q, 1 H); 8.99 (d, 1 H); 13.36 (br. s, 1 H).

### Beispiel 9A

### 6-Chlor-8-[(2,6-difluorbenzyl)oxy]-2-methylimidazo[1,2-a]pyridin-3-carboxamid

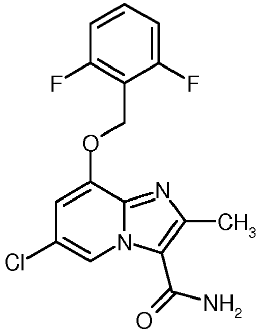

7.0 g (19.85 mmol) 6-Chlor-8-[(2,6-difluorbenzyl)oxy]-2-methylimidazo[1,2-a]pyridin-3-carbonsäure aus Beispiel 8A wurden in 379 ml Dichlormethan vorgelegt, mit 5.71 g (29.77 mmol) 1-(3-Dimethylaminopropyl)-3-ethylcarbodiimidhydrochlorid und 4.02 g (29.77 mmol) 1-Hydroxy-1H-benzotriazol-Hydrat versetzt und 10 min bei Raumtemperatur gerührt. Anschließend wurden 5.31 g (99.23 mmol) Ammoniumchlorid und 24.20 ml (138.9 mmol) *N*,*N*-Diisopropylethylamin zugegeben und über Nacht bei Raumtemperatur gerührt. Das Reaktionsgemisch wurde mit Wasser versetzt, der enthaltene Feststoff wurde abfiltriert, anschließend mit Wasser 30 min bei 50°C verrührt, erneut abfiltriert und mit Wasser gewaschen. Es wurden 6.54 g (93% d. Th.) der Titelverbindung erhalten.
LC-MS (Methode 1): Rₜ = 0.86 min
MS (ESpos): m/z = 352 (M+H)⁺
¹H-NMR (400 MHz, DMSO-d₆): δ = 2.53 (s, 3H), 5.34 (s, 2 H); 7.22 (d, 1 H); 7.25 (t, 2 H); 7.38 (br. s, 2 H), 7.55-7.66 (m, 1 H); 8.90 (d, 1 H).

### Beispiel 10A

### 6-Chlor-8-[(2,6-difluorbenzyl)oxy]-2-methylimidazo[1,2-a]pyridin-3-carbonitril

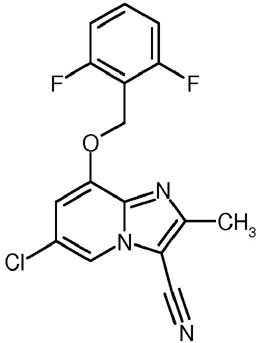

6.43 g (18.29 mmol) 6-Chlor-8-[(2,6-difluorbenzyl)oxy]-2-methylimidazo[1,2-a]pyridin-3-carboxamid aus Beispiel 9A wurden in 82 ml THF vorgelegt und mit 3.79 ml (46.82 mmol) Pyridin versetzt. Dann wurden bei RT 6.61 ml (46.82 mmol) Trifluoressigsäureanhydrid zugetropft und die Reaktionsmischung 1 h bei RT gerührt. Anschließend wurde das Gemisch auf Wasser gegeben und dreimal mit Essigsäureethylester extrahiert. Die vereinigten organischen Phasen wurden einmal mit gesättigter, wässriger Natriumhydrogencarbonat-Lösung, einmal mit 1 N wässriger Salzsäure und einmal mit gesättigter wässriger Natriumchlorid-Lösung gewaschen, über Natriumsulfat getrocknet und einrotiert. Der Rückstand wurde über Nacht im Vakuum getrocknet. Es wurden 6.09 g (90% d. Th.; Reinheit 90%) der Titelverbindung erhalten.
LC-MS (Methode 1): Rₜ = 1.11 min
MS (ESpos): m/z = 334 (M+H)⁺
¹H-NMR (400 MHz, DMSO-d₆): δ = 2.43 (s, 3 H), 5.37 (s, 2 H), 7.25 (t, 2 H), 7.37 - 7.39 (m, 1 H), 7.56 - 7.66 (m, 1 H), 8.46 - 8.50 (m, 1 H).

### Beispiel 11A

### 6-Chlor-8-[(2,6-difluorbenzyl)oxy]-2-methylimidazo[1,2-a]pyridin-3-carboximidamid

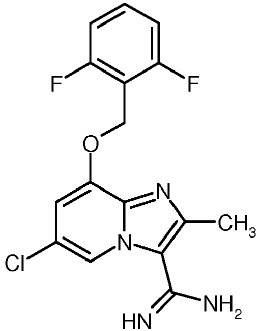

1.70 g (4.59 mmol; Reinheit 90%) 6-Chlor-8-[(2,6-difluorbenzyl)oxy]-2-methylimidazo[1,2-a]pyridin-3-carbonitril aus Beispiel 10A wurden in Analogie zur Vorschrift unter Beispiel 22A umgesetzt. Es wurden 1.79 g (62 % d. Th.; Reinheit ca. 56%) der Titelverbindung erhalten.
LC-MS (Methode 1): Rₜ = 0.60 min
MS (ESpos): m/z = 351 (M+H)⁺

### Beispiel 12A

### 5-Brom-3-[(2,6-difluorbenzyl)oxy]pyridin-2-amin

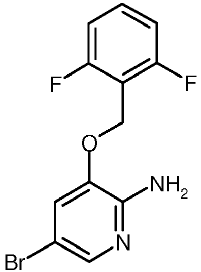

32.6 g 3-[(2,6-Difluorbenzyl)oxy]pyridin-2-amin (Beispiel 1A; 138 mmol, 1 Äquivalent) wurden in 552 ml 10%iger wässriger Schwefelsäure suspendiert und auf 0°C gekühlt. 8.5 ml Brom (165 mmol, 1.2 Äquivalente) wurde in 85 ml Essigsäure gelöst und dann innerhalb von 90 min zur eisgekühlten, Reaktionslösung getropft. Nach erfolgter Zugabe wurde 90 min bei 0°C nachgerührt, anschließend mit 600 ml Essigsäureethylester verdünnt und die wässrige Phase abgetrennt. Die wässrige Phase wurde mit Essigsäureethylester extrahiert. Die organischen Phasen wurden vereinigt, mit gesättigter, wässriger Natriumhydrogencarbonat-Lösung gewaschen, getrocknet und eingeengt. Der Rückstand wurde in Dichlormethan gelöst und an Kieselgel chromatographiert (Petrolether/Essigsäureethylester Gradient als Eluent). Es wurden 24 g (55% d. Th.) der Titelverbindung erhalten.
LC-MS (Methode 1): Rₜ = 0.96 min
MS (ESpos): m/z = 315.1/317.1 (M+H)⁺
¹H-NMR (400 MHz, DMSO-d₆): δ = 5.14 (s, 2 H); 5.83 (br. s, 2 H); 7.20 (t, 2 H); 7.42 (d, 1 H); 7.54 (q, 1 H); 7.62 (d, 1 H).

### Beispiel 13A

### Ethyl-6-brom-8-[(2,6-difluorbenzyl)oxy]-2-methylimidazo[1,2-a]pyridin-3-carboxylat

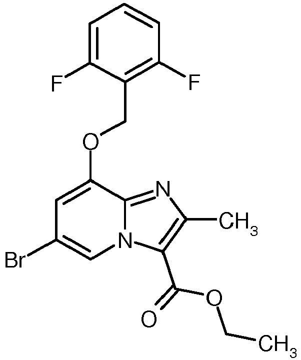

24 g 5-Brom-3-[(2,6-difluorbenzyl)oxy]pyridin-2-amin (Beispiel 12A; 76.2 mmol; 1 Äquivalent) in 400 ml Ethanol wurden mit 16 g gepulvertem Molekularsieb 3Å und 52.7 ml Ethyl-2-chloracetoacetat (380.8 mmol; 5 Äquivalente) versetzt und über Nacht zum Rückfluß erhitzt. Es wurden weitere 8 g Molekularsieb zugegeben und für weitere 24 h zum Rückfluß erhitzt. Das Reaktionsgemisch wurde im Vakuum eingeengt, der Rückstand in Dichlormethan aufgenommen und an Kieselgel chromatographiert (Dichlormethan/Methanol 20:1 als Eluent). Die produkthaltigen Fraktionen wurden eingeengt, der Rückstand mit 100 ml Diethylether 30 min verrührt. Dann wurde abfiltriert, mit wenig Diethylether gewaschen und getrocknet. Es wurden 15 g (45% d. Th.) der Titelverbindung erhalten.
LC-MS (Methode 2): Rₜ = 1.43 min
MS (ESpos): m/z = 414.9/416.8 (M+H)⁺
¹H-NMR (400 MHz, DMSO-d₆): δ = 1.36 (t, 3 H); 2.54 (s, 3 H; verdeckt durch DMSO-Signal); 4.37 (q, 2 H); 5.36 (s, 2 H); 7.25 (t, 2 H); 7.42 (d, 1 H); 7.61 (q, 1 H); 9.00 (d, 1 H).

### Beispiel 14A

### Ethyl-8-[(2,6-difluorbenzyl)oxy]-2,6-dimethylimidazo[1,2-a]pyridin-3-carboxylat

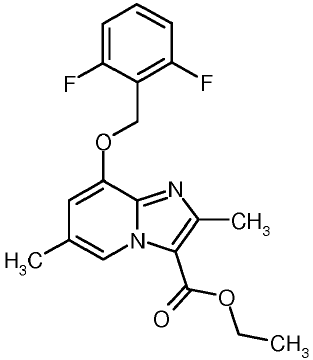

### Methode 1:

600 mg Ethyl-6-brom-8-[(2,6-difluorbenzyl)oxy]-2-methylimidazo[1,2-a]pyridin-3-carboxylat (Beispiel 13A; 1.4 mmol, 1 Äquivalent) und 230 mg 1,1'-Bis(diphenylphosphino)ferrocene-palladium(II)dichlorid dichloromethan Komplex (0.282 mmol, 20 mol%) wurden in 25 ml THF gelöst und mit 0.88 ml (1.76 mmol, 1.2 Äquivalente) einer 2 M Lösung von Methylzinkchlorid in THF versetzt. Die Reaktionsmischung wurde in der Mikrowelle für 40 min auf 100°C erhitzt, dann über Celite filtriert und anschließend im Vakuum eingeengt. Der Rückstand wurde chromatographiert (Biotage Isolera Four). Es wurden 225 mg (38% d. Th.) der Titelverbindung erhalten.

### Methode 2:

20.00 g (85.38 mmol) Ethyl-8-hydroxy-2,6-dimethylimidazo[1,2-a]pyridin-3-carboxylat aus Beispiel 19A, 19.44 g (93.91 mmol) 2,6-Difluorbenzylbromid und 61.20 g (187.83 mmol) Cäsiumcarbonat in 1.18 L DMF wurden 5 h bei 60°C gerührt. Dann wurde das Reaktionsgemisch auf 6.4 L 10%ige wässrige Natriumchlorid-Lösung gegossen und anschließend zweimal mit Essigsäureethylester extrahiert. Die vereinigten organischen Phasen wurden mit 854 ml 10%iger wässriger Natriumchlorid-Lösung gewaschen, getrocknet, eingeengt und der Rückstand über Nacht im Hochvakuum bei RT getrocknet. Es wurden 28.2 g (92% d. Th.; Reinheit ca. 90%) der Titelverbindung erhalten.
LC-MS (Methode 1): Rₜ = 1.05 min
MS (ESpos): m/z = 361.1 (M+H)⁺
¹H-NMR (400 MHz, DMSO-d₆): δ = 1.38 (t, 3 H); 2.36 (s, 3 H); 2.52 (s, 3H verdeckt durch DMSO-Signal); 4.35 (q, 2 H); 5.30 (s, 2 H); 7.10 (s, 1 H); 7.23 (t, 2 H); 7.59 (q, 1 H); 8.70 (s, 1 H).

### Beispiel 15A

### 8-[(2,6-Difluorbenzyl)oxy]-2,6-dimethylimidazo[1,2-a]pyridin-3-carbonsäure

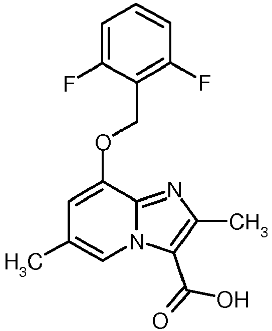

220 mg Ethyl-8-[(2,6-difluorbenzyl)oxy]-2,6-dimethylimidazo[1,2-a]pyridin-3-carboxylat (Beispiel 14A; 0.524 mmol, 1 Äquivalent) wurden in 7 ml THF/Methanol 1:1 gelöst, mit 2.6 ml 1 N wässriger Lithiumhydroxid-Lösung (2.6 mmol, 5 Äquivalente) versetzt und für 16 h bei RT gerührt. Es wurde im Vakuum eingeengt, der Rückstand mit 1N wässriger Salzsäure sauer gestellt und 15 min gerührt. Der Feststoff wurde abfiltriert, mit Wasser nachgewaschen und im Vakuum getrocknet. Es wurden 120 mg der Titelverbindung (60% d. Th.) erhalten.
LC-MS (Methode 1): Rₜ = 0.68 min
MS (ESpos): m/z = 333.1 (M+H)⁺
¹H-NMR (400 MHz, DMSO-d₆): δ = 2.34 (s, 3 H); 2.52 (s, 3H verdeckt durch DMSO-Signal); 5.28 (s, 2 H); 7.09 (s, 1 H); 7.23 (t, 2 H); 7.58 (q, 1 H); 8.76 (s, 1 H); 13.1 (br. s, 1 H).

### Beispiel 16A

### 3-(Benzyloxy)-5-brompyridin-2-amin

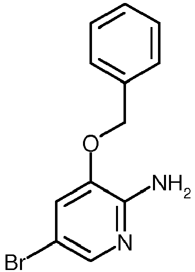

Die Zielverbindung ist literaturbekannt und beschrieben:
1) Palmer, A.M. et al. J Med. Chem. 2007, 50, 6240-6264.
2) ALTANA WO2005/58325
3) ALTANA WO2005/90358
4) Cui, J.T. et al. J Med. Chem. 2011, 54, 6342-6363

### Weitere Herstellungsmethode:

200 g (1 mol) 2-Amino-3-benzyloxypyridin wurden in 4 l Dichlormethan vorgelegt und bei 0°C innerhalb von 30 min mit einer Lösung aus 62 ml (1.2 mol) Brom in 620 ml Dichlormethan versetzt. Nach beendeter Zugabe wurde die Reaktionslösung 60 min bei 0°C gerührt. Dann wurde das Gemisch mit ca. 4 l gesättigter, wässriger Natriumhydrogencarbonat-Lösung versetzt. Die organische Phase wurde abgetrennt und eingeengt. Der Rückstand wurde mittels Kieselgelsäulechromatographie (Petrolether:Essigsäurethylester 6:4) gereinigt und die Produktfraktionen wurden eingeengt. Man erhielt 214 g (77% d. Th.) der Titelverbindung.
LC-MS (Methode 1): Rₜ = 0.92 min
MS (ESpos): m/z = 279 (M+H)⁺
¹H-NMR (400 MHz, DMSO-d₆): δ = 5.16 (s, 2H), 5.94 - 6.00 (m, 2H), 7.26 - 7.29 (m, 1H), 7.31 - 7.36 (m, 1H), 7.37 - 7.43 (m, 2H), 7.47-7.52 (m, 2H), 7.57 - 7.59 (m, 1H).

### Beispiel 17A

### Ethyl-8-(benzyloxy)-6-brom-2-methylimidazo[1,2-a]pyridin-3-carboxylat

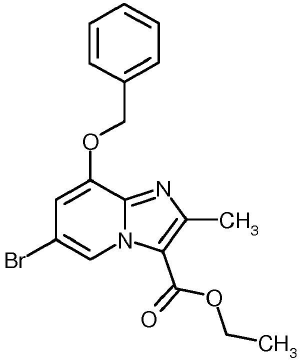

Unter Argon wurden 200 g (0.72 mol) 3-(Benzyloxy)-5-brompyridin-2-amin aus Beispiel 16A, 590 g (3.58 mol) Ethyl-2-chloracetoacetat und 436 g 3A Molekularsieb in 6 l Ethanol suspendiert und 72 h bei Rückfluß gerührt. Die Reaktionsmischung wurde über Kieselgel abfiltriert und eingeengt. Der Rückstand wurde mittels Kieselgelchromatographie (Petrolether:Essigsäureethylester 9:1, anschließend 6:4) gereinigt und die Produktfraktionen wurden eingeengt. Man erhielt 221 g (79% d. Th.) der Zielverbindung.
LC-MS (Methode 16): Rₜ = 1.31 min
MS (ESpos): m/z = 389 (M+H)⁺
¹H-NMR (400 MHz, DMSO-d₆): δ = 1.36 (t, 3 H), 2.58 (s, 3 H), 4.32 - 4.41 (m, 2 H), 5.33 (s, 2 H), 7.28 - 7.32 (m, 1 H), 7.36 - 7.47 (m, 3 H), 7.49 - 7.54 (m, 2 H), 8.98 (d, 1 H).

### Beispiel 18A

### Ethyl-8-(benzyloxy)-2,6-dimethylimidazo[1,2-a]pyridin-3-carboxylat

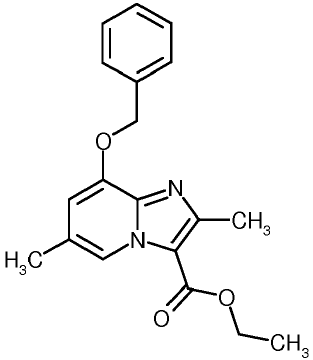

105 g (270 mmol) Ethyl-8-(benzyloxy)-6-brom-2-methylimidazo[1,2-a]pyridin-3-carboxylat aus Beispiel 17A wurden unter Argon in 4.2 l 1,4-Dioxan suspendiert und nacheinander mit 135.4 g (539 mmol, Reinheit 50%) Trimethylboroxin, 31.2 g (27 mmol) Tetrakis(triphenylphosphin)palladium(0) und 78.3 g (566 mmol) Kaliumcarbonat versetzt und 8 h unter Rückfluss gerührt. Die auf RT abgekühlte Reaktionsmischung wurde über Kieselgel vom Niederschlag abfiltriert und das Filtrat eingeengt. Der Rückstand wurde in Dichlormethan gelöst und mittels Kieselgelchromatographie (Dichlormethan:Essigsäureethylester = 9:1) gereinigt. Man erhielt 74 g (84.6% d. Th.) der Zielverbindung.
LC-MS (Methode 16): Rₜ = 1.06 min
MS (ESpos): m/z = 325 (M+H)⁺
¹H-NMR (400 MHz, DMSO-d₆): δ = 1.35 (t, 3 H), 2.34 (br. s, 3 H), 2.56 (s, 3 H), 4.31 - 4.38 (m, 2 H), 5.28 (br. s, 2 H), 6.99 - 7.01 (m, 1 H), 7.35 - 7.47 (m, 3 H), 7.49 - 7.54 (m, 2 H), 8.68 - 8.70 (m, 1 H).

### Beispiel 19A

### Ethyl-8-hydroxy-2,6-dimethylimidazo[1,2-a]pyridin-3-carboxylat

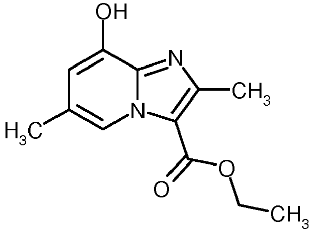

74 g (228 mmol) Ethyl-8-(benzyloxy)-2,6-dimethylimidazo[1,2-a]pyridin-3-carboxylat aus Beispiel wurden in 1254 ml Dichlormethan und 251 ml Ethanol vorgelegt und unter Argon mit 20.1 g 10%igem Palladium auf Aktivkohle (wasserfeucht 50%) versetzt. Das Reaktionsgemisch wurde über Nacht bei RT und Normaldruck hydriert. Die Reaktionsmischung wurde über Kieselgel abfiltriert und eingeengt. Das Rohprodukt wurde mittels Kieselgelchromatographie (Dichlormethan:Methanol = 95:5) gereinigt. Man erhielt 50.4 g (94% d. Th.) der Zielverbindung.
DCI-MS: (Methode 13) (ESpos): m/z = 235.2 (M+H)⁺
¹H-NMR (400 MHz, DMSO-d₆): δ = 1.35 (t, 3 H), 2.27 (s, 3 H), 2.58 (s, 3 H), 4.30 - 4.38 (m, 2 H), 6.65 (d, 1 H), 8.59 (s, 1 H), 10.57 (br. s, 1H).

### Beispiel 20A

### 8-[(2,6-Difluorbenzyl)oxy]-2-methylimidazo[1,2-a]pyridin-3-carboxamid

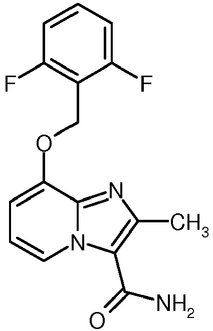

Unter Argon wurden 5.0 g 8-[(2,6-Difluorbenzyl)oxy]-2-methylimidazo[1,2-a]pyridin-3-carbonsäure (Beispiel 3A, 15.71 mmol, 1 Äquivalent) in 300 ml Dichlormethan vorgelegt, bei RT nacheinander mit 4.52 g 1-(3-Dimethylaminopropyl)-3-ethylcarbodiimidhydrochlorid (23.56 mmol, 1.5 Äquivalente) und 3.61 g 1-Hydroxy-1H-benzotriazol Hydrat (HOBT, 23.56 mmol, 1.5 Äquivalente) versetzt und 10 min bei RT gerührt. Dann wurden 4.20 g Ammoniumchlorid (78.55 mmol, 5 Äquivalente) und 19.15 ml *N*,*N*-Diisopropylethylamin (109.96 mmol, 7 Äquivalente) zugegeben und das Gemisch wurde über Nacht bei RT gerührt. Anschließend wurde im Vakuum eingeengt, der Rückstand mit Dichlormethan versetzt, der Feststoff abfiltriert, gut mit Dichlormethan nachgewaschen und über Nacht im Vakuum getrocknet. Es wurden 5.38 g (108% d. Th.) der Titelverbindung erhalten und ohne Reinigung weiter umgesetzt.
LC-MS (Methode 1): Rₜ = 0.65 min
MS (ESpos): m/z = 318.2 (M+H)⁺

### Beispiel 21A

### 8-[(2,6-Difluorbenzyl)oxy]-2-methylimidazo[1,2-a]pyridin-3-carbonitril

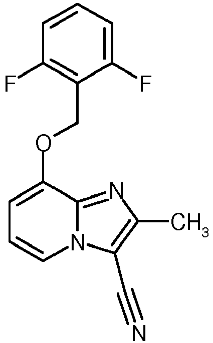

912 mg 8-[(2,6-Difluorbenzyl)oxy]-2-methylimidazo[1,2-a]pyridin-3-carboxamid (Beispiel 20A, 2.87 mmol, 1 Äquivalent) wurden in 13 ml THF vorgelegt und mit 0.6 ml Pyridin (7.36 mmol, 2.56 Äquivalente) versetzt. Dann wurden bei RT 1.04 ml Trifluoressigsäureanhydrid (7.36 mmol, 2.56 Äquivalente) zugetropft und das Gemisch über Nacht bei RT gerührt. Anschließend wurde das Reaktionsgemisch auf Wasser gegeben und dreimal mit Essigsäureethylester extrahiert. Die vereinigten organischen Phasen wurden einmal mit gesättigter, wässriger Natriumhydrogencarbonat-Lösung, einmal mit 1 N wässriger Salzsäure und einmal mit gesättigter wässriger Natriumchlorid-Lösung gewaschen, über Natriumsulfat getrocknet und einrotiert. Der Rückstand wurde über Nacht im Vakuum getrocknet. Es wurden 787 mg (91% d. Th.) der Titelverbindung erhalten.
LC-MS (Methode 1): Rₜ = 0.97 min
MS (ESpos): m/z = 300.1 (M+H)⁺
¹H-NMR (400 MHz, DMSO-d₆): δ = 2.44 (s, 3 H), 5.33 (s, 2 H), 7.10 - 7.16 (m, 1 H), 7.18 - 7.28 (m, 3 H), 7.54 - 7.64 (m, 1 H), 8.22 (d, 1 H).

### Beispiel 22A

### 8-[(2,6-Difluorbenzyl)oxy]-2-methylimidazo[1,2-a]pyridin-3-carboximidamid

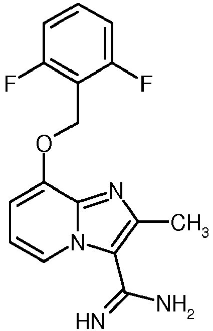

### Lösung A:

135 mg Ammoniumchlorid (2.53 mmol, 2.52 Äquivalente) wurden unter Argon in 3.9 ml Toluol vorgelegt und auf 0°C abgekühlt. Bei dieser Temperatur wurden 1.26 ml 2 M Trimethylaluminium in Toluol (2.53 mmol, 2.52 Äquivalente) zugegeben und die Lösung wurde 2 h bei RT gerührt.

In einem anderen Kolben wurden 300 mg 8-[(2,6-Difluorbenzyl)oxy]-2-methylimidazo[1,2-a]pyridin-3-carbonitril (Beispiel 21A, 1.0 mmol, 1 Äquivalent) in 3.3 ml Toluol vorgelegt, bei RT mit 2 ml der zuvor hergestellten Lösung versetzt und 1 h bei 110°C gerührt. Anschließend wurde nochmals insgesamt zweimal die Lösung A zur Reaktionsmischung hinzugegeben, bis das Edukt komplett umgesetzt war. Dann wurde das Gemisch abgekühlt, bei RT mit Kieselgel und einem 1:1-Gemisch aus Dichlormethan/Methanol versetzt und 30 min bei RT gerührt. Das Kieselgel wurde abfiltriert, mit Methanol nachgewaschen und die Mutterlauge wurde eingeengt. Den Rückstand reinigte man über eine Kieselgelsäule (Eluent: Dichlormethan pur; Dichlormethan:Methanol = 10:2). Es wurden 138 mg (43% d. Th.) der Titelverbindung erhalten.
LC-MS (Methode 1): Rₜ = 0.51 min
MS (ESpos): m/z = 317.1 (M+H)⁺
¹H-NMR (400 MHz, DMSO-d₆): δ = 2.46 (s, 3 H), 5.32 (s, 2 H), 7.04 (t, 1 H), 7.14 (d, 1 H), 7.24 (t, 2 H), 7.53 - 7.66 (m, 1 H), 8.17 (d, 1 H), 9.31 (d, 3 H).

### Beispiel 23A

### 8-[(2,6-Difluorbenzyl)oxy]-N-hydroxy-2-methylimidazo[1,2-a]pyridin-3-carboximidamid

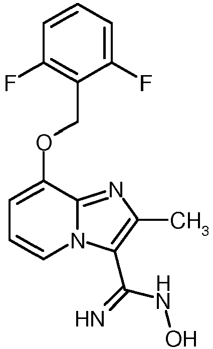

50.0 g (148.9 mmol) 8-[(2,6-Difluorbenzyl)oxy]-2-methylimidazo[1,2-a]pyridin-3-carbonitril aus Beispiel 21A wurden in Ethanol (1.5 1) suspendiert, mit 51.75 g (744.6 mmol) Hydroxylamin-Hydrochlorid und 103 ml (744.6 mmol) Triethylamin versetzt und über Nacht bei RT gerührt. Anschließend wurde bis fast zur Trockene eingeengt, mit Wasser (2.0 1) und Ethanol (100 ml) versetzt und 1 h gerührt. Es bildete sich ein Feststoff, der abfiltriert und mit Wasser gewaschen wurde. Dieser Feststoff wurde über Nacht am Hochvakuum getrocknet. Es wurden 38.5 g (68 % d. Th.; Reinheit 87%) der Titelverbindung erhalten.
LC-MS (Methode 1): Rₜ = 0.56 min
MS (ESpos): m/z = 333.2 (M+H)⁺

### Beispiel 24A

### 8-[(2,6-Difluorbenzyl)oxy]-2-methylimidazo[1,2-a]pyridin-3-carboximidamid Acetat

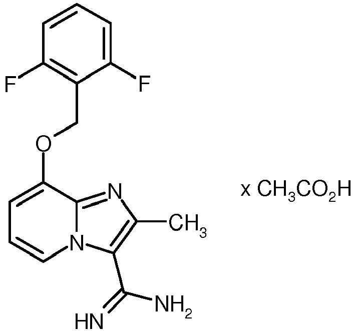

37.5 g (98.4 mmol, 87 % Reinheit) 8-[(2,6-Difluorbenzyl)oxy]-N-hydroxy-2-methylimidazo[1,2-a]pyridin-3-carboximidamid aus Beispiel 23A wurden in Essigsäure (1.01) vorgelegt und mit 11.14 ml (118.08 mmol) Essigsäureanhydrid versetzt. Es wurden 7.5 g Palladium/Kohle (10%ig, feucht) zugegeben und 16 h bei Normaldruck hydriert. Danach wurde über Kieselgur filtriert und mit Ethanol nachgewaschen. Nach Einengen wurde der Rückstand dreimal mit je 500 ml Toluol versetzt und zur Trockene eingeengt. Der Rückstand wurde mit 200 ml Essigsäureethylester verrührt, filtriert und der Rückstand anschließend getrocknet. Es wurden 22.0 g (59% d. Th.) der Titelverbindung erhalten.
LC-MS (Methode 1): Rₜ = 0.51 min
MS (ESpos): m/z = 317.2 (M-CH₃CO₂H+H)⁺
¹H-NMR (400 MHz, DMSO-d₆): δ = 1.82 (s, 3H), 2.46 (s, 3 H), 5.31 (s, 2 H), 6.93 (t, 1 H), 7.01 (d, 1 H), 7.21-7.25 (m, 2 H), 7.55 - 7.63 (m, 1 H), 8.55 (br d, 1 H).

### Beispiel 25A

### 8-[(2,6-Difluorbenzyl)oxy]-2,6-dimethylimidazo[1,2-a]pyridin-3-carboxamid

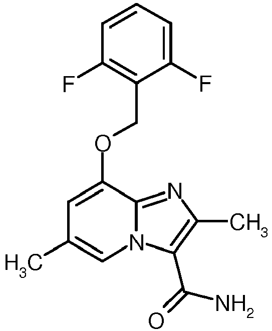

7.0 g (21.07 mmol) 8-[(2,6-Difluorbenzyl)oxy]-2,6-dimethylimidazo[1,2-a]pyridin-3-carbonsäure aus Beispiel 15A wurden in 403 ml Dichlormethan vorgelegt, mit 6.06 g (31.60 mmol) 1-(3-Dimethylaminopropyl)-3-ethylcarbodiimidhydrochlorid und 4.27 g (31.60 mmol) 1-Hydroxy-1H-benzotriazol-Hydrat versetzt und 10 min bei Raumtemperatur gerührt. Anschließend wurden 5.63 g (105.32 mmol) Ammoniumchlorid und 25.68 ml (147.5 mmol) *N*,*N*-Diisopropylethylamin zugegeben und über Nacht bei Raumtemperatur gerührt. Das Reaktionsgemisch wurde mit Wasser versetzt, der enthaltene Feststoff wurde abfiltriert, anschließend mit Wasser 30 min bei 50°C verrührt, erneut abfiltriert und mit Wasser gewaschen. Es wurden 4.59 g (65% d. Th.) der Titelverbindung erhalten. Von den vereinten Filtratfraktionen (Dichlormethan/Wasser) wurden die Phasen getrennt. Die Dichlormethanphase wurde je einmal mit gesättigter, wässriger Natriumhydrogencarbonatlösung und mit gesättigter, wässriger Natriumchloridlösung gewaschen.

Die organische Phase wurde über Natriumsulfat getrocknet, filtriert und eingedampft. Der Rückstand wurde mit wenig Acetonitril verrührt und abfiltriert. Es wurden nochmals 1.29 g (17% d. Th.; Reinheit 93%) der Titelverbindung erhalten.
LC-MS (Methode 1): Rₜ = 0.64 min
MS (ESpos): m/z = 332 (M+H)⁺
¹H-NMR (400 MHz, DMSO-d₆): δ = 2.31 (s, 3H), 2.50 (s, 3 H; verborgen unter DMSO-Signal); 5.28 (s, 2 H); 6.92 (s, 1 H); 7.22 (t, 2 H); 7.35 (br. s, 2 H), 7.53-7.63 (m, 1 H); 8.62 (s, 1 H).

### Beispiel 26A

### 8-[(2,6-Difluorbenzyl)oxy]-2,6-dimethylimidazo[1,2-a]pyridin-3-carbonitril

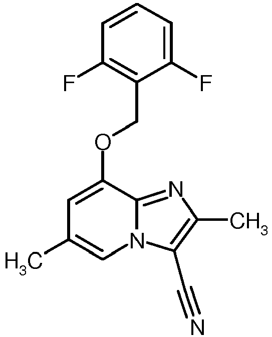

5.70 g (17.20 mmol) 8-[(2,6-Difluorbenzyl)oxy]-2,6-dimethylimidazo[1,2-a]pyridin-3-carboxamid Beispiel 25A wurden in 77 ml THF vorgelegt und mit 3.56 ml (44.0 mmol) Pyridin versetzt. Dann wurden bei RT 6.22 ml (44.0 mmol) Trifluoressigsäureanhydrid zugetropft und die Reaktionsmischung rührte 3 h bei RT. Dann wurde das Gemisch auf Wasser gegeben und dreimal mit Essigsäureethylester extrahiert. Die vereinigten organischen Phasen wurden einmal mit gesättigter, wässriger Natriumhydrogencarbonat-Lösung, einmal mit 1 N wässriger Salzsäure und einmal mit gesättigter wässriger Natriumchlorid-Lösung gewaschen, über Natriumsulfat getrocknet und eingedampft. Der Rückstand wurde über Nacht im Vakuum getrocknet. Es wurden 5.47 g (99% d. Th.) der Titelverbindung erhalten.
LC-MS (Methode 1): Rₜ = 1.12 min
MS (ESpos): m/z = 314 (M+H)⁺
¹H-NMR (400 MHz, DMSO-d₆): δ = 2.37 (s, 3 H), 2.41 (s, 3 H), 5.31 (s, 2 H), 7.12 (s, 1 H), 7.23 (t, 2 H), 7.54 - 7.63 (m, 1 H), 8.09 (s, 1 H).

### Beispiel 27A

### 8-[(2,6-Difluorbenzyl)oxy]-2,6-dimethylimidazo[1,2-a]pyhdin-3-carboximidamid

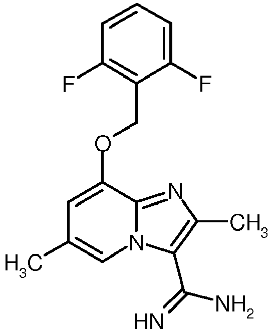

5.47 g (17.46 mmol; Reinheit 98%) 8-[(2,6-Difluorbenzyl)oxy]-2,6-dimethylimidazo[1,2-a]pyridin-3-carbonitril aus Beispiel 26A wurden in Analogie zur Vorschrift unter Beispiel 22A umgesetzt. Es wurden 1.28 g (22 % d. Th.) der Titelverbindung erhalten.
LC-MS (Methode 1): Rₜ = 0.60 min
MS (ESpos): m/z = 331.3 (M+H)⁺
¹H-NMR (400 MHz, DMSO-d₆): δ = 2.35 (s, 3 H), 2.43 (s, 3 H), 5.31 (s, 2 H), 7.06 (s, 1 H), 7.24 (t, 2 H), 7.54 - 7.65 (m, 1 H), 8.02 (s, 1 H), 9.25 (br. s, 3 H).

### Beispiel 28A

### Methyl-3,3-dicyan-2,2-dimethylpropanoat

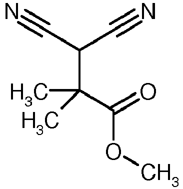

Unter Argon wurden 1.82 g 60%iges Natriumhydrid (45.41 mmol, 1 Äquivalent) in 91 ml THF vorgelegt und bei RT langsam mit 3.0 g Malonsäuredinitril (45.41 mmol, 1 Äquivalent) versetzt.

Dann wurden bei RT 5.9 ml alpha-Bromisobuttersäuremethylester (45.41 mmol, 1 Äquivalent) zugegeben und das Gemisch rührte über Nacht bei RT nach. Es wurden nochmals 5.9 ml alpha-Bromisobuttersäuremethylester (45.41 mmol, 1 Äquivalent) nachgegeben und über Nacht bei 50°C nachgerührt. Dann wurde das Gemisch abgekühlt, mit gesättigter, wässriger Natriumhydrogencarbonat-Lösung versetzt und dreimal mit Essigsäurethylester extrahiert. Die vereinigten organischen Phasen wurden einmal mit gesättigter Natriumchlorid-Lösung gewaschen, über Natriumsulfat getrocknet und einrotiert. Den Rückstand trennte man über eine Kieselgelsäule (Eluent: Cyclohexan pur; Cyclohexan/Essigsäureethylester = 8:2). Es wurden 6.47 g (86% d. Th.) der Titelverbindung erhalten.
¹H-NMR (400 MHz, CDCl₃): δ = 1.53 (s, 6 H), 3.83 (s, 3 H), 4.14 (s, 1 H).

### Beispiel 29A

### rac-3-(3,4-Difluorphenyl)-3-(1,3-dioxo-1,3-dihydro-2H-isoindol-2-yl)propansäure

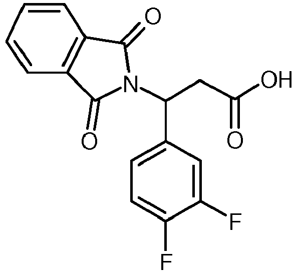

### 1.Stufe:

697 g 3,4-Difluorbenzaldehyd (4.76 mol, 1 Äquivalent) wurden mit 495 g Malonsäure (4.76 mol, 1 Äquivalent) und 733 g Ammoniumacetat (9.52 mol, 2 Äquivalente) in 2788 ml Ethanol 20 h bei Rückfluss unter Argon gerührt. Dann wurde auf RT abgekühlt und über Nacht bei RT gerührt. Der entstandene Feststoff wurde abfiltriert, mit Ethanol und Diethylether gewaschen und im Vakuum getrocknet. Es wurden 590 g (62% d. Th.) *rac*-3-Amino-3-(3,4-difluorphenyl)propansäure erhalten.
*rac*-3-Amino-3-(3,4-difluorphenyl)propansäure:
LC-MS (Methode 1): Rₜ = 0.27 min
MS (ESpos): m/z = 202.0 (M+H)⁺

### 2. Stufe:

0.20 g (0.99 mmol) *rac*-3-Amino-3-(3,4-difluorphenyl)propansäure und 0.15 g (0.99 mmol) Phthalsäureanhydrid wurden in 0.8 ml DMF gelöst und bei 135°C über Nacht zum Rückfluss erhitzt. Die Reaktionslösung wurde auf ca. 9 ml Wasser gegeben. Die resultierende Suspension wurde zweimal mit Essigsäureethylester extrahiert. Die vereinigten organischen Phasen wurden mit Wasser gewaschen, über Natriumsulfat getrocknet, filtriert und eingeengt. Das Rohprodukt wurde mittels präparativer HPLC gereinigt (RP18 Säule, Laufmittel: Acetonitril/Wasser-Gradient unter Zusatz von 0.1% TFA). Es wurden 0.2 g der Titelverbindung (61% d. Th.) erhalten.
LC-MS (Methode 1): Rₜ = 0.97 min
MS (ESpos): m/z = 332 (M+H)⁺
¹H-NMR (400 MHz, DMSO-d₆): δ = 3.24-3.3.33 (m, 1H), 3.44-3.52 (m, 1H), 5.63-5.70 (m, 1H), 7.23-7.28 (m, 1H), 7.36-7.47 (m, 1H), 7.49-7.57 (m, 1H), 7.82-7.90 (m, 4H), 12.51 (br s, 1H).

### Beispiel 30A

### rac-tert.-Butyl-[2-(3,4-difluorphenyl)-2-(1,3-dioxo-1,3-dihydro-2H-isoindol-2-yl)ethyl]carbamat

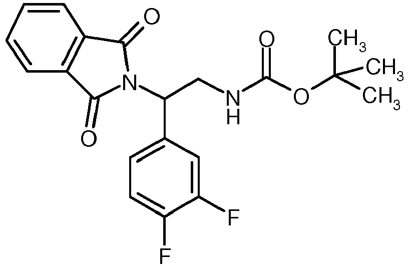

Unter Argon wurde eine Lösung aus 5.0 g *rac*-3-(3,4-Difluorphenyl)-3-(1,3-dioxo-1,3-dihydro-2H-isoindol-2-yl)propansäure (Beispiel 29A, 15.09 mmol) und 3.06 g Triethylamin (30.19 mmol) in 65 ml Toluol vorgelegt, mit 4.36 g Diphenylphosphorazidat (15.85 mmol) versetzt und 3.5 h bei RT gerührt. Anschließend wurden 65 ml tert.-Butanol zugegeben und über Nacht unter Rückfluss gerührt. Nach dem Abkühlen wurde die Reaktionslösung eingeengt und mittels Flashchromatographie gereinigt (Laufmittel: Petrolether/Essigsäureethylester 2:1, isokratisch). Es wurden 3.1 g der Titelverbindung (45% d. Th.) erhalten.
LC-MS (Methode 1): Rₜ = 1.19 min
MS (ESpos): m/z = 403 (M+H)⁺
¹H-NMR (400 MHz, DMSO-d₆): δ = 1.26 (s, 9H), 3.73-3.90 (m, 2H), 5.32-5.39 (m, 1H), 7.20-7.27 (m, 2H), 7.36-7.46 (m, 1H), 7.48-7.56 (m, 1H), 7.81-7.91 (m, 4H).

### Beispiel 31A

### rac-tert.-Butyl-[2-amino-2-(3,4-difluorphenyl)ethyl]carbamat

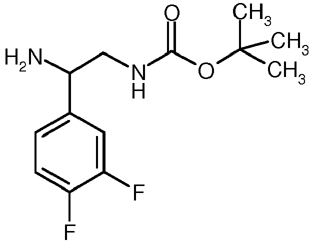

6.13 g *rac*-tert.-Butyl-[2-(3,4-difluorphenyl)-2-(1,3-dioxo-1,3-dihydro-2H-isoindol-2-yl)ethyl-]carbamat (Beispiel 30A, Reinheit ca. 60%, ca. 9.14 mmol) wurde in 13.1 ml 40%iger, wässriger Methylamin-Lösung vorgelegt und über Nacht bei 60°C in einem verschlossenen Gefäß gerührt. Das Reaktionsgemisch wurde eingeengt und der Rückstand mittels Kieselgel-Chromatographie gereinigt (Laufmittel: Dichlormethan: Methanol:Diethylamin 30:1:0.1; 20:1:0.1). Es wurden 1.83 g der Titelverbindung (74% d. Th.) erhalten.
LC-MS (Methode 2): Rₜ = 0.65 min
MS (ESpos): m/z = 273 (M+H)⁺
¹H-NMR (400 MHz, DMSO-d₆): δ = 1.33 (s, 9H), 1.96 (br s, 2H), 2.92-3.10 (m, 2H), 3.81-3.88 (m, 1H), 6.76-6.82 (m, 1H), 7.11-7.17 (m, 1H), 7.27-7.40 (m, 2H).

### Beispiel 32A

### ent- tert.-Butyl-[2-amino-2-(3,4-difluorphenyl)ethyl]carbamat (Enantiomer A)

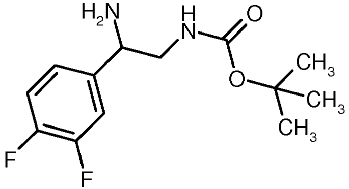

100 mg rac-tert.-Butyl-[2-amino-2-(3,4-difluorphenyl)ethyl]carbamat (Beispiel 31A) wurden an chiraler Phase in die Enantiomere getrennt [Säule: Daicel Chiralpak AY-H, 5 µm, 250 x 20 mm, Eluent: 80% iso-Hexan, 20% Ethanol + 0.2% Diethylamin, Fluß 15 ml/min; 30°C, Detektion: 220 nm].
Ausbeute: 43 mg Enantiomer A (>99% ee)

Rₜ = 4.58 min [Daicel Chiralpak AY-H, 5µm, 250 x 4.6 mm; Eluent: 80% iso-Hexan, 20% Ethanol + 0.2% Diethylamin; Fluss 1.0 ml/min; 30°C; Detektion: 220 nm].

### Beispiel 33A

### ent- tert.-Butyl-[2-amino-2-(3,4-difluorphenyl)ethyl]carbamat (Enantiomer B):

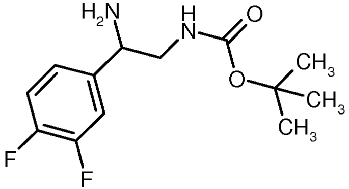

100 mg rac-tert.-Butyl-[2-amino-2-(3,4-difluorphenyl)ethyl]carbamat (Beispiel 31A) wurden an chiraler Phase in die Enantiomere getrennt [Säule: Daicel Chiralpak AY-H, 5 µm, 250 x 20 mm, Eluent: 80% iso-Hexan, 20% Ethanol + 0.2% Diethylamin, Fluss 15 ml/min; 30°C, Detektion: 220 nm].

### Ausbeute: 44 mg Enantiomer B (>99% ee)

Rₜ = 5.61 min [Daicel Chiralpak AY-H, 5µm, 250 x 4.6 mm; Eluent: 80% iso-Hexan, 20% Ethanol + 0.2% Diethylamin; Fluss 1.0 ml/min; 30°C; Detektion: 220 nm].

### Beispiel 34A

### ent-1-(3,4-Difluorphenyl)ethan-1,2-diamin-Dihydrochlorid (Enantiomer A)

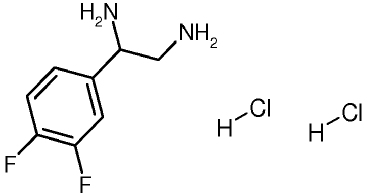

528 mg (1.94 mmol) *ent*-tert.-Butyl-[2-amino-2-(3,4-difluorphenyl)ethyl]carbamat (Enantiomer A) (Beispiel 32A) wurden in 10 ml Diethylether vorgelegt, mit 9.7 ml Salzsäure (2 N in Diethylether) versetzt und für 2 Tage bei Raumtemperatur gerührt. Das Reaktionsgemisch wurde eingedampft und im Hochvakuum getrocknet. Es wurden 475 mg (99% d. Th.) der Titelverbindung erhalten.
LC-MS (Methode 1): Rₜ = 0.17 min
MS (ESpos): m/z = 173 (M-2HCl+H)⁺

### Beispiel 35A

### Methyl-3,3-dicyan-2-(trifluormethyl)acrylat

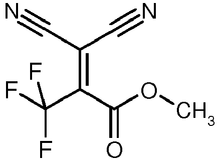

Die Synthese dieser Verbindung ist beschrieben in Journal of Fluorine Chemistry, 1991, vol. 51, # 3 S. 323 - 334.

### Beispiel 36A

### Methyl-2-(dicyanmethyl)-3,3,3-trifluor-2-methylpropanoat (Racemat)

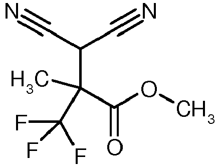

3.00 g (14.698 mmol) Methyl-3,3-dicyan-2-(trifluormethyl)acrylat aus Beispiel 35A wurden in Tetrahydrofuran (30 ml) gelöst und auf 0°C abgekühlt. Anschließend wurden 7.35 ml (22.047 mmol) Methylmagnesiumchlorid (3 M in THF) so zugetropft, dass die Temperatur 5°C nicht überstieg. Nach vollständiger Zugabe wurde 10 min nachgerührt. Der Ansatz wurde dann mit 1N wässriger Salzsäure versetzt und anschließend mit Essigsäureethylester extrahiert. Die Phasen wurden getrennt und die wässrige Phase wurde noch zweimal mit Essigsäureethylester extrahiert. Die vereinigten organischen Phasen wurden mit gesättigter wässriger Natriumchlorid-Lösung gewaschen, über Natriumsulfat getrocknet, filtriert und eingeengt. Das Rohprodukt wurde anschließend über Kieselgel chromatographiert (Laufmittel: Cyclohexan, danach Cyclohexan:Essigsäureethylester 9:1 (v:v). Nach Einengen erhielt man 3.24 g (63% d. Th.) der Titelverbindung.
¹H-NMR (400 MHz, CDCl₃): δ = 1.81 (s, 3H), 3.95 (s, 3H), 4.48 (s, 1H).

### Beispiel 37A

### tert.-Butyl-(1-{[(2-{8-[(2,6-difluorbenzyl)oxy]-2-methylimidazo[1,2-a]pyridin-3-yl}-5,5-dimethyl-6-oxo-6,7-dihydro-5H-pyrrolo[2,3-d]pyrimidin-4-yl)amino]methyl}cyclopropyl)carbamat

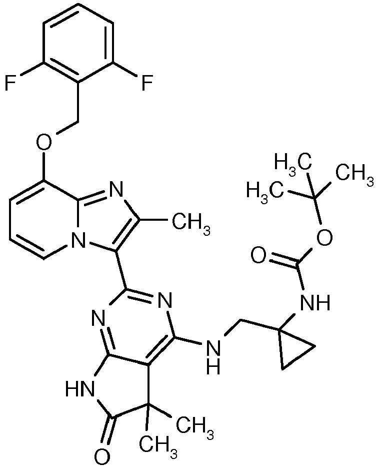

100 mg (171 µmol) 4-Chlor-2-{8-[(2,6-difluorbenzyl)oxy]-2-methylimidazo[1,2-a]pyridin-3-yl}-5,5-dimethyl-5,7-dihydro-6H-pyrrolo[2,3-d]pyrimidin-6-on aus Beispiel 4 wurden in 1.5 ml NMP vorgelegt, mit 128 mg (685 µmol) tert.-Butyl-[1-(aminomethyl)cyclopropyl]carbamat und 0.03 ml (171 µmol) *N*,*N*-Diisopropylethylamin versetzt und 5 h bei 150 °C in der Mikrowelle gerührt. Anschließend wurden nochmals 32 mg (171 µmol) tert.-Butyl-[1-(aminomethyl)-cyclopropyl]-carbamat zugegeben und 2 h bei 150 °C in der Mikrowelle gerührt. Die Reaktionslösung wurde mit Acetonitril/Wasser/TFA verdünnt und mittels präparativer HPLC gereinigt (RP18 Säule, Laufmittel: Acetonitril/Wasser-Gradient unter Zusatz von 0.1% TFA). Es wurden 73 mg (59% d. Th., Reinheit 86%) der Titelverbindung erhalten.
LC-MS (Methode 1): Rₜ = 1.01 min
MS (ESpos): m/z = 620 (M+H)⁺

### Beispiel 38A

### Ethyl-8-(benzyloxy)-2-methylimidazo[1,2-a]pyridin-3-carboxylat

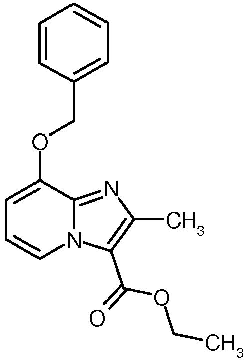

25 g 2-Amino-3-benzyloxypyridin (124.8 mmol, 1 Äquivalent) wurde in 781 ml Ethanol gelöst, mit 102.7 g Ethyl-2-chloracetoacetat (624.2 mmol, 5 Äquivalente) und mit 15 g 4Å Molekularsieb versetzt und 2 d zum Rückfluß erhitzt (Badtemperatur 100°C). Dann wurde eingeengt und am Rotationsverdampfer mit Trockeneiskühlung überschüssiges Ethyl-2-chloracetoacetat abdestilliert. Der Rückstand wurde über Kieselgelchromatographie gereinigt (Laufmittel Cyclohexan: Essigsäureethylester = 9:1, 4:1). Es wurden 20.81 g der Titelverbindung (54% d. Th.) erhalten.
LC-MS (Methode 2): Rₜ = 1.12 min
MS (ESpos): m/z = 311 (M+H)⁺
¹H-NMR (400 MHz, DMSO-d₆): δ = 1.35 (t, 3H), 2.59 (s, 3H), 4.34 (q, 2H), 5.32 (s, 2H), 7.01-7.09 (m, 2H), 7.33-7.48 (m, 3H), 7.52 (d, 2H), 8.81-8.86 (m, 1H).

### Beispiel 39A

### 8-(Benzyloxy)-2-methylimidazo[1,2-a]pyridin-3-carbonsäure

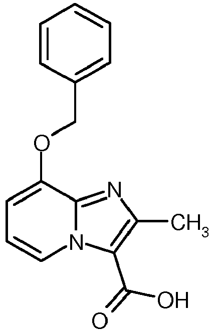

Eine Lösung von 15.7 g Ethyl-8-(benzyloxy)-2-methylimidazo[1,2-a]pyridin-3-carboxylat (50.59 mmol) aus Beispiel 38A in 253 ml 1,4-Dioxan wurde mit 253 ml 2 N wässriger Natronlauge versetzt und 14 h bei RT gerührt. Dann wurde mit 101 ml 6 N wässriger Salzsäure versetzt. Der enstandene Feststoff wurde abfiltriert, mit Wasser und Methyl-tert.-butylether nachgewaschen und dann über Nacht im Vakuum bei 40°C getrocknet. Man erhielt 15.49 g (108% d. Th.) 8-(Benzyloxy)-2-methylimidazo[1,2-a]pyridin-3-carbonsäure.
LC-MS (Methode 2): Rₜ = 0.66 min
MS (ESpos): m/z = 283.0 (M+H)⁺
¹H-NMR (400 MHz, DMSO-d₆): δ = 2.67 (s, 3H), 5.41 (s, 2H), 7.30 (m, 1H), 7.35 - 7.48 (m, 4H), 7.57 (d, 2H), 9.02 (d, 1H).

### Beispiel 40A

### 8-(Benzyloxy)-2-methylimidazo[1,2-a]pyridin-3-carboxamid

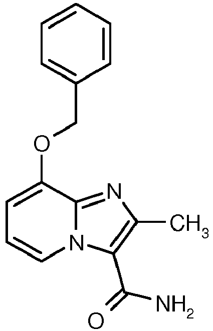

Unter Argon wurden 52.5 g (16.22 mmol, Reinheit 84%) 8-(Benzyloxy)-2-methylimidazo[1,2-a]pyridin-3-carbonsäure (Beispiel 39A) in 2 l Dichlormethan vorgelegt und bei RT nacheinander mit 44.92 g (234.3 mmol) 1-(3-Dimethylaminopropyl)-3-ethylcarbodiimid-hydrochlorid und 35.9 g (234.3 mmol)1-Hydroxy-1H-benzotriazol Hydrat versetzt und 10 min bei RT gerührt. Anschließend wurden 41.78 g (781.1 mmol) Ammoniumchlorid und 190.5 ml (1093.5 mmol) *N,N-*Diisopropylethylamin zugegeben und das Gemisch rührte 3 d bei RT nach. Es wurden jeweils 4.49 g (23.43 mmol) 1-(3-Dimethylaminopropyl)-3-ethylcarbodiimidhydrochlorid, 3.59 g 23.43 (mmol) 1-Hydroxy-1H-benzotriazol-Hydrat, 4.18 g (78.1 mmol) Ammoniumchlorid und 14.13 g (109.4 mmol) *N*,*N*-Diisopropylethylamin zugegeben und nochmals über Nacht bei RT gerührt. Nach beendeter Reaktionszeit wurde das Gemisch mit 2 l Wasser versetzt und der entstandene Niederschlag abfiltiert. Der Feststoff wurde zweimal mit je 500 ml Wasser und Dichlormethan gewaschen und getrocknet. Die organische Phase wurde mit Wasser gewaschen und eingeengt. Der Rückstand wurde mit dem Feststoff vereinigt und mit tert.-Butylmethylether ausgerührt. Es wurde mit tert.-Butylmethylether nachgewaschen und am Hochvakuum getrocknet. Es wurden 40.80 g (93% d. Th.) der Titelverbindung erhalten.
LC-MS (Methode 1): Rₜ = 0.58 min
MS (ESpos): m/z = 282 (M+H)⁺
¹H NMR (400 MHz, DMSO-*d₆*) δ = 2.56 (s, 3 H), 5.28 (s, 2 H), 6.90 (d, 2 H), 7.38 (d, 1 H), 7.43 (t, 2 H), 7.51 (d, 2 H), 8.75 (dd, 1 H).

### Beispiel 41A

### 8-(Benzyloxy)-2-methylimidazo[1,2-a]pyridin-3-carbonitril

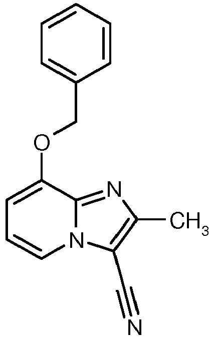

358 g (1.273 mol) 8-(Benzyloxy)-2-methylimidazo[1,2-a]pyridin-3-carboxamid (Beispiel 40A) wurden in 5.65 l THF vorgelegt und mit 263.5 ml (3.258 mol) Pyridin versetzt. Dann wurden bei RT 460.1 ml (3.258 mmol) Trifluoressigsäureanhydrid zugetropft und die Reaktionsmischung wurde über Nacht bei RT gerührt. Nach beendeter Reaktionszeit wurde das Gemisch auf 20 l Essigsäureethylester gegeben und je einmal mit 2.69 l 1 N wässriger Salzsäure, 2.69 l 10%iger wässriger Natriumhydrogencarbonat-Lösung und 6.27 l Wasser gewaschen. Der unlösliche Feststoff wurde abfiltriert und getrocknet.

Dieser Feststoff wurde in 22 l Essigsäureethylester gelöst und nochmal mit 6 l Wasser gewaschen. Die organische Phase wurde getrocknet und eingedampft. Es wurden 140 g (42% d. Th.) der Titelverbindung erhalten.

Die organische Phase der ersten Phasentrennung wurde getrocknet, eingedampft und im Hochvakuum getrocknet. Der Rückstand wurde in 33 l Essigsäureethylester gelöst und mit 20 l 10%ige wässriger Natriumhydrogencarbonat-Lösung 7 h bei RT ausgerührt. Die organische Phase wurde abgetrennt, mit 10%iger wässriger Natriumchlorid-Lösung gewaschen, getrocknet und eingedampft. Der erhaltene Rückstand wurde am Hochvakuum getrocknet. Somit wurden nochmals 153 g (46% d. Th.) der Titelverbindung erhalten.
MS (ESpos): m/z = 264 (M+H)⁺
¹H NMR (500 MHz, DMSO-*d*₆) δ = 2.47 (s, 3 H), 5.32 (s, 2 H), 7.07 - 7.11 (m, 2 H), 7.35 - 7.49 (m, 1 H), 7.43 (t, 2 H), 7.51 (d, 2 H), 8.15 - 8.20 (m, 1 H).

### Beispiel 42A

### 8-Hydroxy-2-methylimidazo[1,2-a]pyridin-3-carbonitril

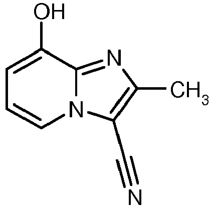

68 g (258 mmol) 8-(Benzyloxy)-2-methylimidazo[1,2-a]pyridin-3-carbonitril (Beispiel 41A) wurden in 1.7 l Dichlormethan und 425 ml Ethanol vorgelegt und unter Argon mit 28 g 10%igem Palladium auf Aktivkohle (wasserfeucht 50%) versetzt. Das Reaktionsgemisch wurde 8 h bei RT und Normaldruck hydriert. Es wurden nochmals 13.6 g 10%iges Palladium auf Aktivkohle (wasserfeucht 50%) hinzugegeben und das Gemisch wurde nochmals 4 h bei RT hydriert. Das während der Reaktion ausgefallende Produkt wurde mit 20 l Dichlormethan/Methanol (7/3) wieder in Lösung gebracht und das Gemisch wurde 45 min bei RT gerührt. Die Reaktionsmischung wurde über Kieselgur abfiltriert und eingeengt. Die anfallenden Kristalle wurden mit tert.-Butylmethylether aufgeschlämmt, abgesaugt und am HV getrocknet. Es wurden 38.5 g (86% d. Th.) der Titelverbindung erhalten.
LC-MS (Methode 1): Rₜ = 0.54 min
MS (ESpos): m/z = 174 (M+H)⁺

### Beispiel 43A

### 2-Methyl-8-[(2,3,6-trifluorbenzyl)oxy]imidazo[1,2-a]pyridin-3-carbonitril

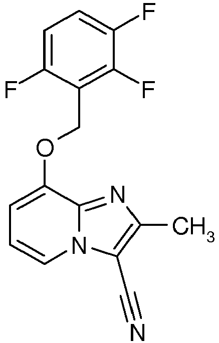

4.90 g (28.28 mmol) 8-Hydroxy-2-methylimidazo[1,2-a]pyridin-3-carbonitril (Beispiel 42A) und 20.27 g Cäsiumcarbonat (62.22 mmol) wurden in 390 ml DMF vorgelegt, mit 7 g (31.11 mmol) 2-(Brommethyl)-1,3,4-trifluorbenzol versetzt und bei 60°C für 30 min gerührt. Das Reaktionsgemisch wurde auf Wasser gegossen und 30 min bei RT gerührt. Der ausgefallene Feststoff wurde abfiltriert, mit Wasser nachgewaschen und am Hochvakuum getrocknet. Es wurden 8.8 g (98% d. Th.) der Titelverbindung erhalten.
LC-MS (Methode 1): Rₜ = 1.01 min
MS (ESpos): m/z = 318 (M+H)⁺
¹H NMR (400 MHz, DMSO-*d₆*) δ = 2.45 (s, 3 H), 5.38 (s, 2 H), 7.11-7.17 (m, 1 H), 7.21 (d, 1 H), 7.25 - 7.34 (m, 1 H), 7.62 - 7.73 (m, 1 H), 8.23 (d, 1 H).

### Beispiel 44A

### N-Hydroxy-2-methyl-8-[(2,3,6-trifluorbenzyl)oxy]imidazo[1,2-a]pyridin-3-carboximidamid

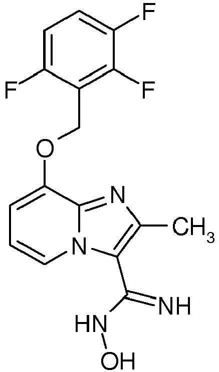

18.20 g (57.36 mmol) 2-Methyl-8-[(2,3,6-trifluorbenzyl)oxy]imidazo[1,2-a]pyridin-3-carbonitril aus Beispiel 43A wurden in 610 ml Ethanol suspendiert und anschließend mit 39.86 g (573.64 mmol) Hydroxylamin-Hydrochlorid versetzt. 79.96 ml (573.64 mmol) Triethylamin wurden zugetropft und es wurde über Nacht bei RT gerührt. Dann wurde am Rotationsverdampfer bei 40-45°C bis auf ein Drittel eingeengt, mit Wasser (250 ml) versetzt und bis ca. 250 ml wieder eingeengt. 0.7 l Wasser wurden zugesetzt. Der entstandene Feststoff wurde abfiltriert und mit Wasser gewaschen. Der Feststoff wurde als eine Suspension in Acetonitril (50 ml) im Vakuum eingeengt und über Nacht am Hochvakuum getrocknet. Es wurden 20.9 g (91% d. Th., Reinheit 87%) der Titelverbindung erhalten.
LC-MS (Methode 1): Rₜ = 0.57 min
MS (ESpos): m/z = 351 (M+H)⁺
¹H NMR (400 MHz, DMSO-*d₆*) δ = 2.40 (s, 3 H), 5.34 (s, 2 H), 5.90 (br. s, 2 H), 6.81 - 6.93 (m, 2 H), 7.24 - 7.33 (m, 1 H), 7.60 - 7.73 (m, 1 H), 8.33 (d, 1 H), 9.79 (s, 1 H).

### Beispiel 45A

### 2-Methyl-8-[(2,3,6-trifluorbenzyl)oxy]imidazo[1,2-a]pyridin-3-carboximidamid

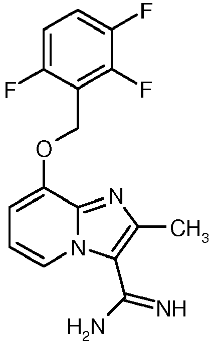

24.18 g (60.74 mmol) N-Hydroxy-2-methyl-8-[(2,3,6-trifluorbenzyl)oxy]imidazo[1,2-a]pyridin-3-carboximidamid aus Beispiel 44A wurden in 167 ml Essigsäure gelöst, mit 6.59 ml (69.85 mmol) Essigsäureanhydrid versetzt und 15 min bei RT gerührt. Anschließend wurden 39.71 g (607.43 mmol) Zinkstaub (< 10 µm) unter starkem Rühren zugegeben und es wurde 30 min bei RT gerührt. Die Reaktionsmischung wurde mit 170 ml Wasser verdünnt, wobei es stark schäumte. Der Feststoff wurde abfiltriert und zweimal mit 50 ml einer Mischung Wasser/Essigsäure (1/1) nachgewaschen. Das Filtrat wurde unter Kühlung in 733 ml 33%-ige wässrige Ammoniaklösung getropft und 30 min bei RT gerührt. Der entstandene Feststoff wurde abfiltriert, mit Wasser nachgewaschen und über Nacht am Hochvakuum getrocknet. Es wurden 22.32 g (73% d. Th., Reinheit 66%) der Titelverbindung erhalten. Das Filtrat wurde dreimal mit Dichlormethan extrahiert. Die vereinigten organischen Phasen wurden mit gesättigter wässriger Natriumchlorid-Lösung gewaschen, über Natriumsulfat getrocknet, filtriert, am Rotationsverdampfer bei 40 - 45°C eingeengt und über Nacht am Hochvakuum getrocknet. Es wurden zusätzlich 1.72 g (7% d. Th; Reinheit: 81%) der Titelverbindung erhalten.
LC-MS (Methode 1): Rₜ = 0.50 min
MS (ESpos): m/z = 335 (M+H)⁺

### Beispiel 46A

### 8-[(2,6-Difluorbenzyl)oxy]-2-methylimidazo[1,2-a]pyridin-3-carboximidohydrazid

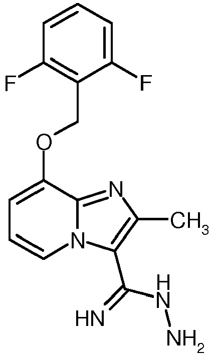

456 mg (1.44 mmol) 8-[(2,6-Difluorbenzyl)oxy]-2-methylimidazo[1,2-a]pyridin-3-carboximidamid aus Beispiel 22A wurden in 12 ml Ethanol vorgelegt und bei 0°C mit 0.80 ml (5.77 mmol) Triethylamin sowie 0.088 ml (1.44 mmol) Hydrazinhydrat (80%ig) versetzt. Das Gemisch wurde erst 10 min bei 0°C und anschließend über Nacht bei RT gerührt. Das Reaktionsgemisch wurde am Rotationsverdampfer bei RT eingeengt. Der Rückstand wurde im Hochvakuum getrocknet. Es wurden 501 mg (105 % d. Th.) der Titelverbindung erhalten. Das Produkt wurde ohne weitere Reinigung in die Folgestufe eingesetzt.
LC-MS (Methode 1): Rₜ = 0.51 min
MS (EIpos): m/z = 332 [M+H]⁺

### Beispiel 47A

### Methyl-2-(3-{8-[(2,6-difluorbenzyl)oxy]-2-methylimidazo[1,2-a]pyridin-3-yl}-5-hydroxy-1,2,4-triazin-6-yl)-2-methylpropanoat

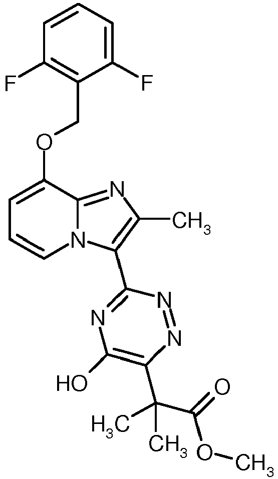

0.291 g (1.55 mmol) Dimethyl-2,2-dimethyl-3-oxobutandioat (beschrieben in C. J. A. Daley et al. J. Am. Chem. Soc. 2002, 124(14), 3680-3691) wurden in 7 ml Ethanol vorgelegt und zum Rückfluss erhitzt. Dazu wurde eine Suspension von 0.341 g (1.03 mmol) 8-[(2,6-Difluorbenzyl)oxy]-2-methylimidazo[1,2-a]pyridin-3-carboximidohydrazid aus Beispiel 46A in 15 ml Ethanol getropft. Es wurde über Nacht unter Rückfluss gerührt. Nach dem Abkühlen wurde das Gemisch eingedampft und der Rückstand mit Diethylether versetzt. Der entstandene Niederschlag wurde abfiltriert, mit Diethylether gewaschen und im Hochvakuum getrocknet. Es wurden 0.407 g (45 % d. Th.; Reinheit ca. 54%) der Titelverbindung (roh) erhalten, die ohne weitere Reinigung in der Folgestufe eingesetzt wurde.
LC-MS (Methode 1): Rₜ = 0.86 min
MS (EIpos): m/z = 470 [M+H]⁺

### Beispiel 48A

### Methyl-2-(5-chlor-3-{8-[(2,6-difluorbenzyl)oxy]-2-methylimidazo[1,2-a]pyridin-3-yl}-1,2,4-triazin-6-yl)-2-methylpropanoat

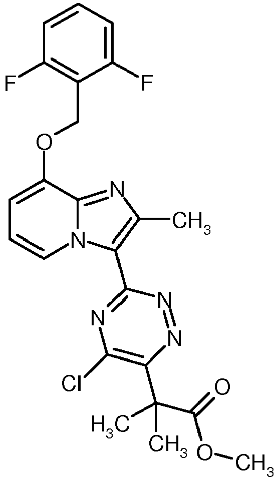

450 mg (0.52 mmol; Reinheit ca. 54%) Methyl-2-(3-{8-[(2,6-difluorbenzyl)oxy]-2-methylimidazo[1,2-a]pyridin-3-yl}-5-hydroxy-1,2,4-triazin-6-yl)-2-methylpropanoat aus Beispiel 47A wurden mit 6.0 ml Phosphorylchlorid versetzt und über Nacht bei RT gerührt. Anschließend wurde das Gemisch 7 h bei 50°C gerührt. Das Reaktionsgemisch wurde ohne weitere Aufreinigung in die Folgereaktion eingesetzt.
LC-MS (Methode 1): Rₜ = 1.20 min
MS (EIpos): m/z = 488 [M+H]⁺.

### Beispiel 49A

### 8-[(2,6-Difluorbenzyl)oxy]-2,6-dimethylimidazo[1,2-a]pyridin-3-carboximidohydrazid

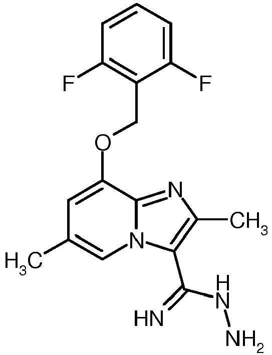

600 mg (1.82 mmol) 8-[(2,6-Difluorbenzyl)oxy]-2,6-dimethylimidazo[1,2-a]pyridin-3-carboximidamid aus Beispiel 27A wurden in 15 ml Ethanol vorgelegt und mit 2.03 ml (14.53 mmol) Triethylamin sowie 0.22 ml (3.63 mmol) Hydrazinhydrat (80%ig) versetzt. Das Gemisch wurde über Nacht bei 50°C gerührt. Das Reaktionsgemisch wurde am Rotationsverdampfer bei RT eingeengt. Der Rückstand wurde im Hochvakuum getrocknet. Es wurden 581 mg (109 % d. Th.) der Titelverbindung erhalten. Das Produkt wurde ohne weitere Reinigung in die Folgestufe eingesetzt.
LC-MS (Methode 1): Rₜ = 0.55 min
MS (EIpos): m/z = 346 [M+H]⁺

### Beispiel 50A

### Methyl-2-(3-{8-[(2,6-difluorbenzyl)oxy]-2,6-dimethylimidazo[1,2-a]pyridin-3-yl}-5-hydroxy-1,2,4-triazin-6-yl)-2-methylpropanoat

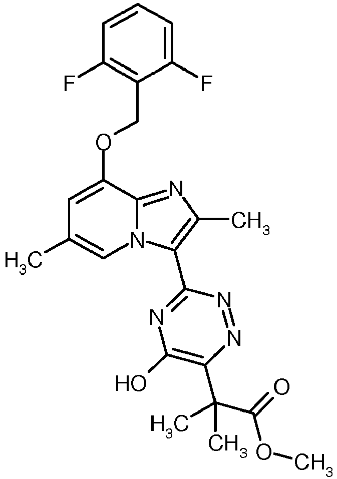

0.514 g (2.73 mmol) Dimethyl-2,2-dimethyl-3-oxobutandioat (beschrieben in C. J. A. Daley et al. J. Am. Chem. Soc. 2002, 124(14), 3680-3691) wurden in 13 ml Ethanol vorgelegt und zum Rückfluss erhitzt. Dazu wurde eine Suspension von 0.629 g (1.82 mmol) 8-[(2,6-Difluorbenzyl)oxy]-2,6-dimethylimidazo[1,2-a]pyridin-3-carboximidohydrazid aus Beispiel 49A in 27 ml Ethanol getropft und es wurde über Nacht unter Rückfluss gerührt. Nach dem Abkühlen wurde das Gemisch eingedampft. Der Rückstand wurde mit Diethylether versetzt, der entstandene Niederschlag 30 min bei RT gerührt, abfiltriert mit Diethylether gewaschen und im Hochvakuum getrocknet. Es wurden 0.713 g (81 % d. Th.) der Titelverbindung erhalten, die ohne weitere Reinigung in der Folgestufe eingesetzt wurden.
LC-MS (Methode 1): Rₜ = 0.89 min
MS (EIpos): m/z = 484 [M+H]⁺

### Beispiel 51A

### Methyl-2-(5-chlor-3-{8-[(2,6-difluorbenzyl)oxy]-2,6-dimethylimidazo[1,2-a]pyridin-3-yl}-1,2,4-triazin-6-yl)-2-methylpropanoat

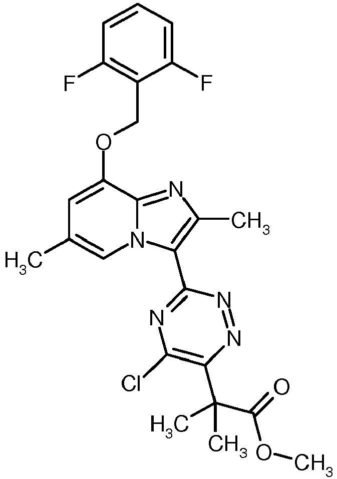

658 mg (1.36 mmol) Methyl-2-(3-{8-[(2,6-difluorbenzyl)oxy]-2,6-dimethylimidazo[1,2-a]pyridin-3-yl}-5-hydroxy-1,2,4-triazin-6-yl)-2-methylpropanoat aus Beispiel 50A wurden mit 8.5 ml Phosphorylchlorid versetzt und über Nacht bei RT gerührt. Das Reaktionsgemisch wurde ohne weitere Aufreinigung in die Folgereaktion eingesetzt.
LC-MS (Methode 1): Rₜ = 1.27 min
MS (EIpos): m/z = 502 [M+H]⁺.

### Beispiel 52A

### 2-Amino-3-fluor-2-(fluormethyl)propanonitril

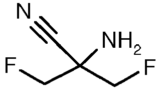

8.75 g (178.6 mmol) Natriumcyanid wurden in 132 ml 2N Ammoniak-Lösung in Methanol vorgelegt. 15.0 g (159.5 mmol) 1,3-Difluoraceton und 9.55 g (178.6 mmol) Ammoniumchlorid wurden bei RT zugegeben. Die Suspension wurde 2 h bei 70°C Ölbadtemperatur gerührt. Die abgekühlte Reaktionsmischung wurde mit 300 ml Diethylether versetzt, 10 min gerührt und vom Feststoff abfiltriert. Das Filtrat wurde in Vakuum eingeengt (50°C, 70 mbar). Es wurden 19.2 g (100% d. Th.) der Zielverbindung erhalten. Das Produkt wurde ohne weitere Aufreinigung weiter umgesetzt.
GC-MS (Methode 14): Rₜ = 1.78 min
MS (ESpos): m/z = 121 (M+H)⁺

### Beispiel 53A

### Benzyl-(2-cyan-1,3-difluorpropan-2-yl)carbamat

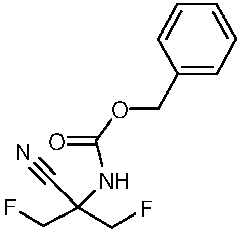

5.0 g (41.6 mmol) 2-Amino-3-fluor-2-(fluormethyl)propanonitril aus Beispiel 52A wurden in 14.5 ml (83.3 mmol) *N,N*-Diisopropylethylamin vorgelegt. 10.65 g (62.5 mmol) Chlorameisensäurebenzylester wurden bei RT langsam zugetropft und es wurde drei Tage bei RT gerührt. Die Reaktionsmischung wurde mit 25 ml Dichlormethan verdünnt und zu einer Lösung von 12.9 g (124.9 mmol) N-(2-Aminoethyl)ethan-1,2-diamin in 225 ml Dichlormethan bei 0°C zugetropft und 10 min gerührt. Anschließend wurden 200 ml gesättigte Ammoniumchlorid-Lösung bei RT zugetropft. Die Phasen wurden getrennt und die wässrige Phase wurde dreimal mit Dichlormethan extrahiert. Die vereinigten organischen Phasen wurden eingeengt. Das Rohprodukt wurde anschließend über Kieselgel chromatographiert (Laufmittel: Cyclohexan:Essigsäureethylester-Gradient). Es wurden 4.40 g (42 % d. Th.) der Titelverbindung erhalten.
GC-MS (Methode 1): Rₜ = 0.92 min
MS (ESpos): m/z = 255 (M+H)⁺

### Beispiel 54A

### Benzyl-tert.-butyl-[3-fluor-2-(fluormethyl)propan-1,2-diyl]biscarbamat

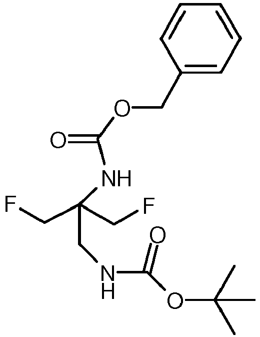

3.28 g (12.90 mmol) Benzyl-(2-cyan-1,3-difluorpropan-2-yl)carbamat aus Beispiel 53A wurden in 52 ml abs. Ethanol bei RT vorgelegt. 2.44 g (64.5 mmol) Natriumborhydrid wurden portionsweise bei RT zugegeben und es wurde 2.5 h bei RT gerührt. Anschließend wurde eine Lösung von 3.66 g (16.77 mmol) Di-tert.-butyldicarbonat in 37 ml abs. Ethanol bei RT langsam zugetropft und danach noch 30 min gerührt. Weitere 281 mg (1.29 mmol) Di-tert-butyldicarbonat wurden zugegeben und es wurde 15 min gerührt. Zur Reaktionsmischung wurden 100 ml gesättigte Ammoniumchlorid-Lösung gegeben und mit ca. 45 ml 1 N wässriger Salzsäure wurde das Gemisch auf ca. pH = 4 eingestellt. Die Reaktionsmischung wurde in Vakuum vom Ethanol befreit und anschließend wurde mit Wasser versetzt. Der entstandene Feststoff wurde abfiltriert, mit Wasser nachgewaschen und am Hochvakuum getrocknet. Man erhielt 3.37 g (73 % d. Th.) der Titelverbindung.
GC-MS (Methode 1): Rₜ = 1.07 min
MS (ESpos): m/z = 359 (M+H)⁺

### Beispiel 55A

### Benzyl-[1-amino-3-fluor-2-(fluormethyl)propan-2-yl]carbamat

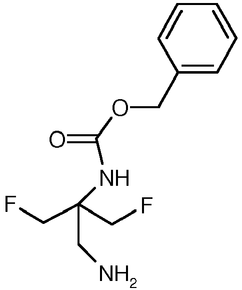

1.00 g (2.79 mmol) Benzyl-tert.-butyl-[3-fluor-2-(fluormethyl)propan-1,2-diyl]biscarbamat aus Beispiel 54A wurden mit 5 ml Dichlormethan und 2 ml Trifluoressigsäure versetzt und 30 min bei RT gerührt. Die Reaktionsmischung wurde am Vakuum eingeengt. Der Rückstand wurde mit gesättigter, wässriger Natriumhydrogencarbonat-Lösung versetzt (pH 8-9) und viermal mit Dichlormethan extrahiert. Die vereinigten organischen Phasen wurden einmal mit gesättigter Natriumchlorid-Lösung gewaschen, über Natriumsulfat getrocknet, bei 25°C eingedampft und am Hochvakuum getrocknet. Es wurden 745 mg der Titelverbindung (103% d. Th) erhalten. Das Produkt wurde ohne weitere Aufreinigung weiter umgesetzt.
GC-MS (Methode 22): Rₜ = 1.98 min
MS (ESpos): m/z = 259 (M+H)⁺

### Beispiel 56A

### 3-Fluor-2-(fluormethyl)propan-1,2-diamin

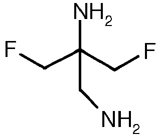

89 mg (0.344 mmol) Benzyl-[1-amino-3-fluor-2-(fluormethyl)propan-2-yl]carbamat aus Beispiel 55A wurden in 0.38 ml 1-Methyl-2-pyrrolidon vorgelegt und unter Argon mit 22 mg 10%igem Palladium auf Aktivkohle versetzt. Das Reaktionsgemisch wurde 4 h bei RT und Normaldruck hydriert. Die Reaktionsmischung wurde durch einen PTFE-Spritzenfilter (0.45 µm) filtriert und der Filter anschließend mit 0.2 ml 1-Methyl-2-pyrrolidon nachgewaschen. Die vereinigten Lösungen wurden direkt in die nächste Reaktion eingesetzt.

### Beispiel 57A

### rac-Benzyl-4-amino-3,3-difluorpyrrolidin-1-carboxylat (Racemat)

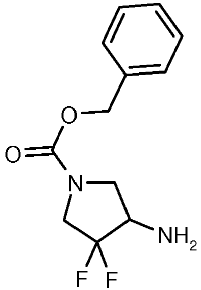

500 mg (1.77 mmol) Benzyl-4-azido-3,3-difluorpyrrolidin-1-carboxylat (WO 2011088045) wurden in 1.25 ml THF und 0.25 ml Wasser vorgelegt. 8.9 ml Trimethylphosphin (1 M Lösung in THF) wurden unter Eisbadkühlung langsam zugetropft und es wurde 15 min bei RT gerührt. Die Reaktionsmischung wurde mit gesättigter, wässriger Natriumhydrogencarbonat-Lösung versetzt und bei 30°C in Vakuum vom THF befreit. Der Rückstand wurde dreimal mit Essigsäurethylester extrahiert. Die vereinigten organischen Phasen wurden einmal mit gesättigter Natriumchlorid-Lösung gewaschen, über Natriumsulfat getrocknet und eingedampft. Den Rückstand trennte man über eine Kieselgelsäule (Eluent: Cyclohexan pur; Cyclohexan/Essigsäureethylester-Gradient). Es wurden 372 mg (82% d. Th.) der Titelverbindung erhalten.
LC-MS (Methode 22): Rₜ = 1.98 min
MS (ESpos): m/z = 257 (M+H)⁺

### Beispiel 58A

### rac-4,4-Difluorpyrrolidin-3-amin (Racemat)

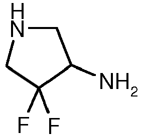

90 mg (0.35 mmol) Benzyl-4-amino-3,3-difluorpyrrolidin-1-carboxylat aus Bespiel 57A wurden in 0.39 ml 1-Methyl-2-pyrrolidon vorgelegt und unter Argon mit 22 mg 10%igem Palladium auf Aktivkohle versetzt. Das Reaktionsgemisch wurde 2 h bei RT und Normaldruck hydriert. Die Reaktionsmischung wurde durch einen PTFE-Spritzenfilter (0.45 µm) filtriert und der Filter anschließend mit 0.2 ml 1-Methyl-2-pyrrolidon nachgewaschen. Die vereinigten Lösungen wurden direkt in die nächste Reaktion eingesetzt.

### Beispiel 59A

### 3-Azabicyclo[3.1.0]hexan-1-amin Dihydrochlorid

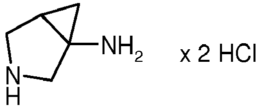

Die Synthese zur Titelverbindung ist beschrieben in: M. Gensini et al., Eur. J. Org. Chem. 2002, 2499-2507.

### Beispiel 60A

### rac-2-1{[-1,1-Difluorpropan-2-yliden]amino}-2-phenylethanol (Racemat)

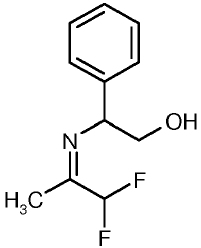

Unter Argon wurden 8.0 g (85.05 mmol) 1,1-Difluoraceton in 90.6 ml Dichlormethan vorgelegt und mit 2 g Magnesiumsulfat und 2 g 4Å Molsieb (Pulver) versetzt. Nach Zugabe von 12.02 g (87.60 mmol) *rac*-2-Amino-2-phenylethanol wurde das Gemisch über Nacht unter Rückfluss gerührt. Es wurde über Celite abfiltriert und mit Dichlormethan gewaschen. Das Filtrat wurde eingedampft und getrocknet. Es erfolgte eine Kieselgelchromatoprahie (Laufmittel: Cyclohexan-Essigsäureethylether-Gradient). Man erhielt 9.6 g (53% d. Th.) der Zielverbindung.
LC-MS (Methode 22): Rₜ = 2.20 min
MS (ESIpos): m/z = 214 (M+H)⁺.

### Beispiel 61A

### 3,3-Difluor-2-[(2-hydroxy-1-phenylethyl)amino]-2-methylpropanonitril (Diastereomere)

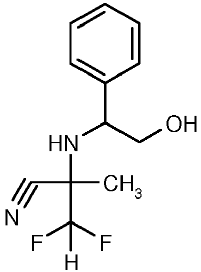

9.6 g (45.02 mmol) *rac*-2-1{[-1,1-Difluorpropan-2-yliden]amino}-2-phenylethanol Beispiel 60A wurden unter Argon in 116 ml Ethanol vorgelegt, mit 14.01 ml (112.55 mmol) Trimetylsilylcyanid versetzt und 16 h unter Rückfluss gerührt. Anschließend wurden nochmals 10 ml (80.39 mmol) Trimetylsilylcyanid zugegeben und es wurde weiter für 16 h unter Rückfluss gerührt. Die Reaktionslösung wurde auf RT abgekühlt, eingedampft, dreimal mit je 100 ml Toluol versetzt und eingedampft. Er erfolgte eine Reinigung mittels präparativer SFC [Ethylpyridin-SFC, 5µm, 125 x 30mm; Eluent: Kohlendioxid/Methanol-Gradient; Fluss: 100 ml/min; Wellenlänge: 220 nm; Temperatur: 40°C]. Man erhielt 6.97 g (64% d. Th.) der Zielverbindung.
LC-MS (Methode 1): Rₜ = 0.86 min und 0.90 min.
MS (ESIneg): m/z = 241 (M+H)⁺.

### Beispiel 62A

### 2-[(3-Amino-1,1-difluor-2-methylpropan-2-yl)amino]-2-phenylethanol Trifluoracetat (Diastereomere)

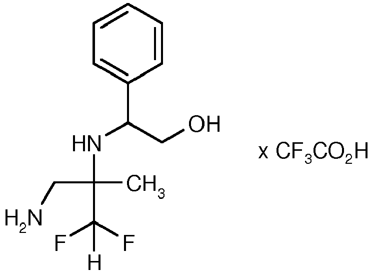

1.0 g (4.16 mmol) 3,3-Difluor-2-[(2-hydroxy-1-phenylethyl)amino]-2-methylpropanonitril (Diastereomere) aus Beispiel 61A wurde in 100 ml tert.-Butylmethylether vorgelegt, auf 0°C abgekühlt, mit 197 mg (5.20 mmol) Lithiumaluminiumhydrid versetzt und 16 h bei RT gerührt. Anschließend wurde auf 0°C abgekühlt, erst mit 416 µl Wasser, dann mit 416 µl 2 N wässriger Natriumhydroxid-Lösung und 832 µl Wasser versetzt. Die entstandene Mischung wurde über Celite filtriert und mit tert.-Butylmethylether und wenig Methanol gewaschen. Das Filtrat wurde eingedampft, der Rückstand mit Acetonitril/Wasser/TFA versetzt und mittels präparativer HPLC gereinigt (RP18 Säule, Laufmittel: Acetonitril/Wasser-Gradient unter Zusatz von 0.1% TFA). Man erhielt 866 mg (58% d. Th.) der Zielverbindungen.
LC-MS (Methode 1): Rₜ = 0.44 min und 0.46 min.
MS (ESIpos): m/z = 245 (M-TFA+H)⁺.

### Beispiel 63A

### rac-3,3-Difluor-2-methylpropan-1,2-diamin (Racemat)

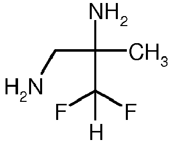

82 mg (0.336 mmol) *rac*-2-[(3-Amino-1,1-difluor-2-methylpropan-2-yl)amino]-2-phenylethanol Beispiel 62A wurden mit gesättigter, wässriger Natriumhydrogencarbonatlösung gewaschen und mit Methyl-tert.-butylether extrahiert. Die organischen Phasen wurden vereinigt, getrocknet und eingeengt. Das freie Amin wurde dann in 0.33 ml 1-Methyl-2-pyrrolidon vorgelegt und unter Argon mit 71 mg 10%igem Palladium auf Aktivkohle versetzt. Das Reaktionsgemisch wurde über Wochenende bei RT und Normaldruck hydriert. Die Reaktionsmischung wurde durch einen PTFE-Spritzenfilter (0.45 µm) filtriert. Der Filter wurde mit 0.2 ml 1-Methyl-2-pyrrolidon nachgewaschen. Die vereinigten Lösungen wurden direkt in die nächste Reaktion eingesetzt.

### Beispiel 64A

### 2-Methyl-4-phenyl-2-(trifluormethyl)-1,3-oxazolidin (Diastereomere)

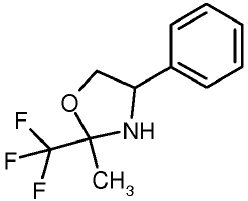

55.13 g (492.0 mmol) 1,1,1-Trifluoraceton in Toluol (1.35 1) wurden mit 45 g (328.0 mmol) *rac*-2-Amino-2-phenylethanol und 8.24 g (32.8 mmol) Pyridinium-p-toluolsulfonat versetzt. Die Reaktionsmischung wurde 16 h unter Rückfluss am Wasserabscheider erhitzt. Das Gemisch wurde auf 0°C abgekühlt, der entstandene Feststoff abfiltriert und im Hochvakuum getrocknet. Man erhielt 68.6 g (77% d. Th., Reinheit 85%) der Zielverbindung.
LC-MS (Methode 1): Rₜ = 0.99 min
MS (ESIpos): m/z = 232 (M+H)⁺.
¹H-NMR (400 MHz, DMSO-d₆): δ = 1.54 (s, 3H), 3.56 (t, 1H), 3.81 (d, 1H), 4.28 (t, 1H), 4.35 - 4.43 (m, 1H), 7.29 - 7.47 (m, 5H).

### Beispiel 65A

### 3,3,3-Trifluor-2-[(2-hydroxy-1-phenylethyl)amino]-2-methylpropanonitril (Diastereomere)

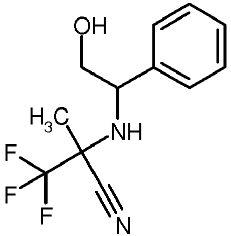

52.8 g (228.3 mmol) 2-Methyl-4-phenyl-2-(trifluormethyl)-1,3-oxazolidin (Diastereomere) Beispiel 64A wurden unter Argon in Dichlormethan (21) vorgelegt und auf 0°C abgekühlt. 42.85 ml (342.5 mmol) Trimetylsilylcyanid und 42.1 ml (342.5 mmol) Bortrifluorid-diethylether-Komplex wurden langsam zugegeben und es wurde 16 h bei RT gerührt. Anschließend wurde die Reaktionslösung in 1.5 1 gesättigte Natriumhydrogencarbonat-Lösung gegossen. Dann wurde mit 400 g Natriumhydrogencarbonat versetzt und die Lösung mit konz. Natronlauge auf pH 10 gestellt. Die wässrige Lösung wurde dreimal mit 500 ml Dichlormethan extrahiert, die vereinigten organischen Phasen über Natriumsulfat getrocknet, abfiltriert und eingeengt. Man erhielt 56.8 g (96% d. Th., 2 Diastereomere) der Zielverbindung.
LC-MS (Methode 1): Rₜ = 0.89 min und 0.93 min.
MS (ESIneg): m/z = 303 (M-H+HCOOH)-.

### Beispiel 66A

### 2-[(3-Amino-1,1,1-trifluor-2-methylpropan-2-yl)amino]-2-phenylethanol (Diastereomere)

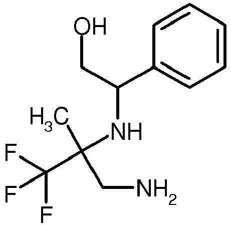

31 g (120.0 mmol) 3,3,3-Trifluor-2-[(2-hydroxy-1-phenylethyl)amino]-2-methylpropanonitril Beispiel 65A wurde in tert.-Butylmethylether (3.1 1) vorgelegt, auf 0°C abgekühlt, mit 18.25 g (480.2 mmol) Lithiumaluminiumhydrid versetzt und die Reaktionsmischung 16 h bei RT gerührt. Anschließend wurde auf 0°C abgekühlt, erst mit 24 ml Wasser gequencht, dann mit 24 ml 15%iger wässriger Kaliumhydroxid-Lösung und 48 ml Wasser versetzt. Die entstandene Mischung wurde über Kieselgel filtriert und mit tert.-Butylmethylether gewaschen. Die organische Phase wurde abgetrennt, über Natriumsulfat getrocknet, filtriert und eingeengt. Man erhielt 29.2 g (83% d. Th., Reinheit 89%) der Zielverbindung.
LC-MS (Methode 1): Rₜ = 0.52 min
MS (ESIpos): m/z = 263 (M+H)⁺.

### Beispiel 67A

### tert.-Butyl-{3,3,3-trifluor-2-[(2-hydroxy-1-phenylethyl)amino]-2-methylpropyl}carbamat (Diastereomere)

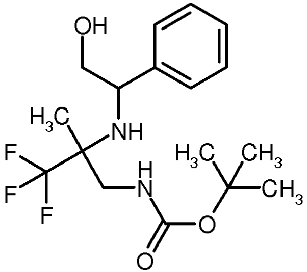

26.2 g (99.9 mmol) 2-[(3-Amino-1,1,1-trifluor-2-methylpropan-2-yl)amino]-2-phenylethanol (Diastereomere) Beispiel 66A in THF (500 ml) wurden mit 29.1 ml (209.8 mmol) Triethylamin und 23.98 g (109.9 mmol) Di-tert.-butyldicarbonat (gelöst in 286 ml THF) versetzt. Die Reaktionsmischung wurde 16 h bei RT gerührt. Anschließend wurde eingeengt und in jeweils 500 ml gesättigter, wässriger Natriumhydrogencarbonat-Lösung und Essigsäureethylester aufgenommen. Die Phasen wurden getrennt, die organische Phase über Natriumsulfat getrocknet, abfiltriert und eingeengt. Man erhielt 39.80 g (110% d. Th.) der Zielverbindung, welche ohne weitere Reinigung in die nächste Stufe eingesetzt wurde.
FIA-MS (Methode 25, ESpos): m/z = 363 (M+H)⁺

### Beispiel 68A

### rac-tert.-Butyl-(2-amino-3,3,3-trifluor-2-methylpropyl)carbamat

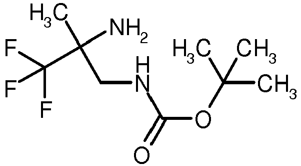

39 g (107.6 mmol) tert.-Butyl-{3,3,3-trifluor-2-[(2-hydroxy-1-phenylethyl)amino]-2-methylpropyl}carbamat Beispiel 67A wurden unter Argon in Ethanol (700 ml) vorgelegt und mit 5.44 g (53.8 mmol) Palladium(II)hydroxid (20% auf Aktivkohle, wasserfeucht ca. 60%ig) versetzt. Die Reaktionsmischung wurde 16 h bei Normaldruck hydriert. Dann wurde über Kieselgel filtriert und eingeengt. Der Rückstand wurde mittels Kieselgelchromatographie (Cyclohexan/Essigsäureethylester-Gradient: 9/1 nach 6/4) gereinigt. Man erhielt 15.8 g (61% d. Th.) der Zielverbindung.
FIA-MS (Methode 25, ESpos): m/z = 243 (M+H)⁺
¹H-NMR (400 MHz, CDCl₃): δ = 1.22 (s, 3H), 1.45 (s, 9H), 3.13 - 3.23 (m, 1H), 3.37 - 3.48 (m, 1H), 4.89 (br. s, 1H).

### Beispiel 69A

### rac-3,3,3-Trifluor-2-methylpropan-1,2-diamindihydrochlorid

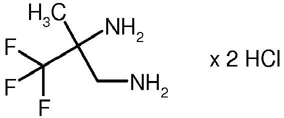

15 g (61.9 mmol) rac-tert.-Butyl-(2-amino-3,3,3-trifluor-2-methylpropyl)carbamat aus Beispiel 68A in Dioxan (188 ml) wurden mit 188 ml 4 M Chlorwasserstoff in Dioxan versetzt. Die Reaktionsmischung wurde 16 h bei RT gerührt, dann eingeengt und unter Argon aufbewahrt. Man erhielt 14.4 g (108% d. Th.) der Zielverbindung, die nicht weiter gereinigt wurde.
FIA-MS (Methode 25, ESpos): m/z = 143 (M-2HCl+H)⁺
¹H-NMR (400 MHz, D₂O): δ = 1.40 (s, 3H), 3.21 - 3.31 (m, 2H).

### Ausführungsbeispiele

### Beispiel 1

### 4-Amino-2-{8-[(2,6-difluorbenzyl)oxy]-2-methylimidazo[1,2-a]pyridin-3-yl}-5,5-dimethyl-5,7-dihydro-6H-pyrrolo[2,3-d]pyrimidin-6-on

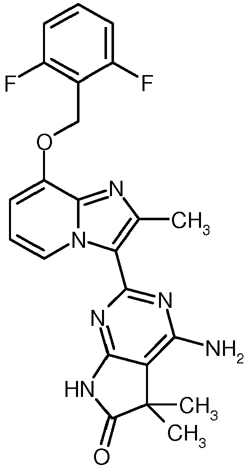

135 mg 8-[(2,6-Difluorbenzyl)oxy]-2-methylimidazo[1,2-a]pyridin-3-carboximidamid (Beispiel 22A, 0.42 mmol, 1 Äquivalent) wurden unter Argon in 8 ml tert.-Butanol vorgelegt, bei RT nacheinander mit 71.8 mg Kalium-tert.-butylat (0.64 mmol, 1.5 Äquivalente) und 85 mg Methyl-3,3-dicyan-2,2-dimethylpropanoat (Beispiel 28A, 0.51 mmol, 1.2 Äquivalente) in 2 ml tert.-Butanol versetzt und über Nacht zum Rückfluss erhitzt. Dann wurde abgekühlt, mit Wasser versetzt und 10 min bei RT gerührt. Der entstandene Feststoff wurde abgesaugt, mit Wasser gewaschen und über Nacht im Vakuum getrocknet. Es wurden 117.6 mg (61% d. Th.) der Titelverbindung erhalten.
LC-MS (Methode 1): Rₜ = 0.73 min
MS (ESpos): m/z = 451 (M+H)⁺
¹H-NMR (400 MHz, DMSO-d₆): δ = 1.35 (s, 6 H), 2.73 (s, 3 H), 5.31 (s, 2 H), 6.72 (br. s, 2 H), 6.92 (t, 1 H), 7.00 (d, 1 H), 7.24 (t, 2 H), 7.53 - 7.65 (m, 1 H), 9.67 (d, 1 H), 10.89 (s, 1 H).

### Beispiel 2

### 2-{8-[(2,6-Difluorbenzyl)oxy]-2-methylimidazo[1,2-a]pyridin-3-yl}-4-iod-5,5-dimethyl-5,7-dihydro-6H-pyrrolo[2,3-d]pyrimidin-6-on

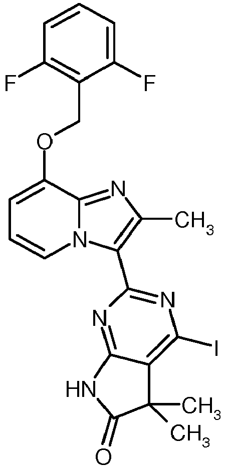

15.0 g (33.30 mmol) 4-Amino-2-{8-[(2,6-difluorbenzyl)oxy]-2-methylimidazo[1,2-a]pyridin-3-yl}-5,5-dimethyl-5,7-dihydro-6H-pyrrolo[2,3-d]pyrimidin-6-on aus Beispiel 1 wurden zusammen mit 53.69 g (200.47 mmol) Diiodmethan und 23.48 g (200.47 mmol) Isopentylnitrit in 268 ml abs. 1,4-Dioxan unter Argon über Nacht bei 85°C gerührt. Es wurden 15.34 g (57.28 mmol) Diiodmethan und 6.70 g (57.28 mmol) Isopentylnitrit hinzugegeben und das Gemisch wurde über Nacht bei 85°C gerührt. Es wurden nochmals 7.67 g (28.64 mmol) Diiodmethan und 3.35 g (28.64 mmol) Isopentylnitrit hinzugegeben und das Gemisch wurde über Nacht bei 85°C gerührt. Die Reaktionslösung wurde eingedampft, der Rückstand in Dichlormethan gelöst und mittels Kieselgelchromatographie gereinigt (Dichlormethan; Dichlormethan/Methanol = 75:1 bis 30:1). Es wurden 9.07 g (48% d. Th.) der Titelverbindung erhalten.
LC-MS (Methode 1): Rₜ = 1.04 min
MS (ESpos): m/z = 562 (M+H)⁺
¹H NMR (400 MHz, DMSO-*d*₆) δ = 1.41 (s, 6 H), 2.71 (s, 3 H), 5.34 (s, 2 H), 5.76 (s, 1 H), 7.06 - 7.14 (m, 2 H), 7.24 (t, 2 H), 7.56 - 7.65 (m, 1 H), 9.41 (d, 1 H), 11.68 (s, 1 H).

### Beispiel 3

### 2-{8-[(2,6-Difluorbenzyl)oxy]-2-methylimidazo[1,2-a]pyridin-3-yl}-4-iod-5,5-dimethyl-5,7-dihydro-6H-pyrrolo[2,3-d]pyrimidin-6-on Trifluoracetat

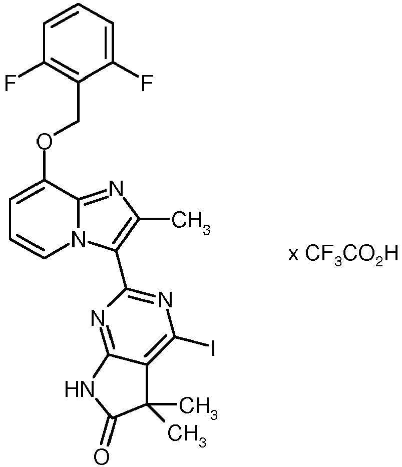

4.0 g (8.88 mmol) 4-Amino-2-{8-[(2,6-difluorbenzyl)oxy]-2-methylimidazo[1,2-a]pyridin-3-yl}-5,5-dimethyl-5,7-dihydro-6H-pyrrolo[2,3-d]pyrimidin-6-on aus Beispiel 1 wurden zusammen mit 11.13 g (41.56 mmol) Diiodmethan und 4.87 g (41.56 mmol) Isopentylnitrit in 73 ml abs. 1,4-Dioxan unter Argon vorgelegt und über Nacht bei 85°C gerührt. Die Reaktionslösung wurde eingedampft, in Acetonitril/Wasser/TFA gelöst und mittels präparativer HPLC gereinigt (RP18 Säule, Laufmittel: Acetonitril/Wasser-Gradient unter Zusatz von 0.1% TFA). Es wurden 2.45 g (38% d. Th; Reinheit ca. 93%) der Titelverbindung erhalten.
LC-MS (Methode 1): Rₜ = 1.05 min
MS (ESpos): m/z = 562 (M-TFA+H)⁺

### Beispiel 4

### 4-Chlor-2-{8-[(2,6-difluorbenzyl)oxy]-2-methylimidazo[1,2-a]pyridin-3-yl}-5,5-dimethyl-5,7-dihydro-6H-pyrrolo[2,3-d]pyrimidin-6-on

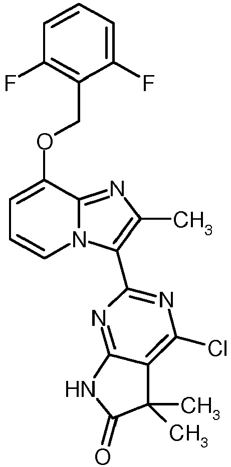

500 mg (1.11 mmol) 4-Amino-2-{8-[(2,6-difluorbenzyl)oxy]-2-methylimidazo[1,2-a]pyridin-3-yl}-5,5-dimethyl-5,7-dihydro-6H-pyrrolo[2,3-d]pyrimidin-6-on aus Beispiel 1 wurden zusammen mit 746 mg (5.55 mmol) Kupfer(II)chlorid und 195 mg (1.67 mmol) Isopentylnitrit in 20 ml abs. Acetonitril unter Argon vorgelegt und 5 h bei 85°C gerührt. Das Gemisch wurde mit 2.22 ml 1 N wässriger Salzsäure versetzt und anschließend dreimal mit Essigsäureethylester extrahiert. Die Phasen wurden getrennt und die wässrige Phase wurde noch zweimal mit Essigsäureethylester extrahiert. Die vereinigten organischen Phasen wurden mit gesättigter wässriger Natriumchlorid-Lösung gewaschen, über Natriumsulfat getrocknet, filtriert und eingeengt. Der Rückstand wurde in Acetonitril/Wasser/TFA gelöst und mittels präparativer HPLC gereinigt (RP18 Säule, Laufmittel: Acetonitril/Wasser-Gradient unter Zusatz von 0.1% TFA). Die Produktfraktionen wurden eingedampft, in Dichlormethan und wenig Methanol gelöst und zweimal mit gesättigter, wässriger Natriumhydrogencarbonatlösung gewaschen. Die vereinigten wässrigen Phasen wurden zweimal mit Dichlormethan reextrahiert. Die vereinigten organischen Phasen wurden über Natriumsulfat getrocknet, filtriert und eingedampft. Es wurden 158 mg (30% d. Th.) der Titelverbindung erhalten.
LC-MS (Methode 1): Rₜ = 1.05 min
MS (ESpos): m/z = 470 (M+H)⁺
¹H NMR (400 MHz, DMSO-*d*₆) δ = 1.45 (s, 6 H), 2.79 (s, 3 H), 5.43 (s, 2 H), 7.26 (t, 2 H), 7.34 - 7.43 (m, 1 H), 7.45 - 7.52 (m, 1 H), 7.56 - 7.68 (m, 1 H), 9.52 (d, 1 H), 11.96 (s, 1 H).

### Beispiel 5

### 2-{8-[(2,6-Difluorbenzyl)oxy]-2-methylimidazo[1,2-a]pyridin-3-yl}-4-hydroxy-5,5-dimethyl-5,7-dihydro-6H-pyrrolo[2,3-d]pyrimidin-6-on Trifluoracetat

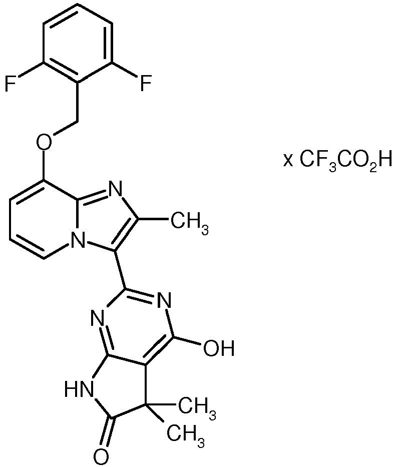

100 mg (0.21 mmol) 4-Amino-2-{8-[(2,6-difluorbenzyl)oxy]-2-methylimidazo[1,2-a]pyridin-3-yl}-5,5-dimethyl-5,7-dihydro-6H-pyrrolo[2,3-d]pyrimidin-6-on aus Beispiel 1 wurden in 0.84 ml TFA gelöst. Bei 0°C wurden 0.09 ml (5.19 mmol) Wasser und 21.5 mg (0.31 mmol) Natriumnitrit hinzugegeben und die Lösung wurde 5 min bei 0°C gerührt. Die Reaktionsmischung wurde in 3.5 ml Wasser gegeben. Der entstandene Niederschlag wurde filtriert, mit Wasser gewaschen und im Hochvakuum getrocknet. Es wurden 121 mg (99% d. Th.) der Titelverbindung erhalten.
LC-MS (Methode 1): Rₜ = 0.75 min
MS (ESpos): m/z = 452 (M-TFA+H)⁺
¹H NMR (400 MHz, DMSO-*d*₆) δ = 1.34 (s, 6 H), 5.43 (br. s, 2 H), 7.18 - 7.68 (m, 5 H), 9.82 (br. s, 1 H), 11.32 (br. s, 1 H), 12.18 (br. s, 1 H) [weiteres Signal unter Lösungsmittelpeaks].

### Beispiel 6

### rac-4-Amino-2-{8-[(2,6-difluorbenzyl)oxy]-2-methylimidazo[1,2-a]pyridin-3-yl}-5-methyl-5-(trifluormethyl)-5,7-dihydro-6H-pyrrolo[2,3-d]pyrimidin-6-on (Racemat)

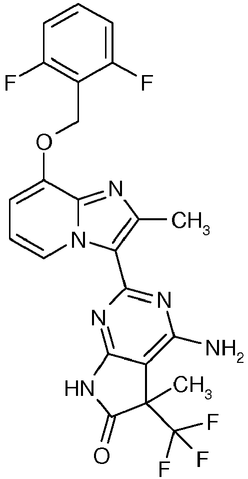

1.39 g (3.69 mmol) 8-[(2,6-Difluorbenzyl)oxy]-2-methylimidazo[1,2-a]pyridin-3-carboximidamid Acetat (Beispiel 24A) wurden unter Argon in 47.5 ml tert.-Butanol vorgelegt und bei RT nacheinander mit 621 mg (5.53 mmol) Kalium-tert.-butylat und 974 mg (4.43 mmol) Methyl-2-(dicyanmethyl)-3,3,3-trifluor-2-methylpropanoat (Beispiel 36A; Racemat) in 16 ml tert.-Butanol versetzt und über Nacht zum Rückfluss erhitzt. Dann wurde abgekühlt und eingeengt. Der Rückstand wurde mit Wasser versetzt und 10 min bei RT gerührt. Der entstandene Niederschlag wurde fitriert, mit Wasser gewaschen und über Nacht im Vakuum getrocknet. Es wurden 1.6 g (77% d. Th; Reinheit: 90%) der Titelverbindung erhalten.
LC-MS (Methode 1): Rₜ = 0.82 min
MS (ESpos): m/z = 505 (M+H)⁺
¹H NMR (500 MHz, DMSO-*d*₆) δ = 1.11 (s, 1 H), 1.72 (s, 3 H), 2.74 (s, 3 H), 5.28 - 5.38 (m, 2 H), 6.82 - 6.99 (m, 3 H), 7.04 (d, 1 H), 7.19 - 7.29 (m, 3 H), 7.53 - 7.64 (m, 1 H), 9.71 (d, 1 H).

### Beispiel 7

### rac-2-{8-[(2,6-difluorbenzyl)oxy]-2-methylimidazo[1,2-a]pyridin-3-yl}-4-iod-5-methyl-5-(trifluormethyl)-5,7-dihydro-6H-pyrrolo[2,3-d]pyrimidin-6-on Trifluoracetat (Racemat)

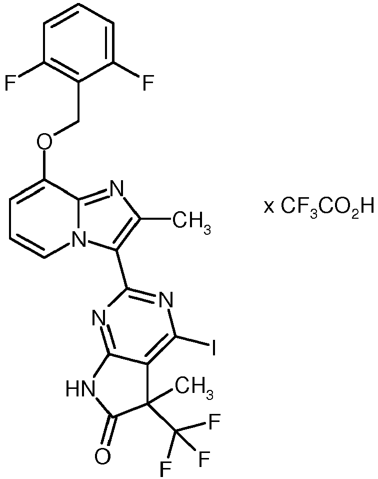

1.50 g (2.68 mmol; Reinheit 90%) *rac*-4-Amino-2-{8-[(2,6-difluorbenzyl)oxy]-2-methylimidazo[1,2-a]pyridin-3-yl}-5-methyl-5-(trifluormethyl)-5,7-dihydro-6H-pyrrolo[2,3-d]pyrimidin-6-on (Racemat) aus Beispiel 6 wurden mit 3.58 g (13.38 mmol) Diiodmethan und 1.57 g (13.38 mmol) Isopentylnitrit in 23.4 ml abs. 1,4-Dioxan unter Argon vorgelegt und über Nacht bei 85°C gerührt. Die Reaktionslösung wurde eingedampft, der Rückstand in Acetonitril/Wasser/TFA gelöst und mittels präparativer HPLC gereinigt (RP18 Säule, Laufmittel: Acetonitril/Wasser-Gradient unter Zusatz von 0.1% TFA). Es wurden 0.65 g (29% d. Th.; Reinheit ca. 86%) der Titelverbindung erhalten.
LC-MS (Methode 1): Rₜ = 1.17 min
MS (ESpos): m/z = 616 (M-TFA+H)⁺

### Beispiel 8

### rac-2-{8-[(2,6-Difluorbenzyl)oxy]-2-methylimidazo[1,2-a]pyridin-3-yl}-4-hydroxy-5-methyl-5-(trifluormethyl)-5,7-dihydro-6H-pyrrolo[2,3-d]pyrimidin-6-on Trifluoracetat

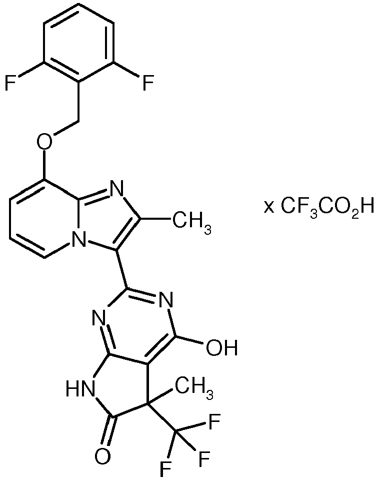

Die Zielverbindung entstand als Nebenprodukt bei der Präparation von Beispiel 7. Man erhielt 135 mg (8% d. Th.) der Titelverbindung.
LC-MS (Methode 1): Rₜ = 0.88 min
MS (ESpos): m/z = 506 (M-TFA+H)⁺

### Beispiel 9

### 4-Amino-5,5-dimethyl-2-{2-methyl-8-[(2,3,6-trifluorbenzyl)oxy]imidazo[1,2-a]pyridin-3-yl}-5,7-dihydro-6H-pyrrolo[2,3-d]pyrimidin-6-on

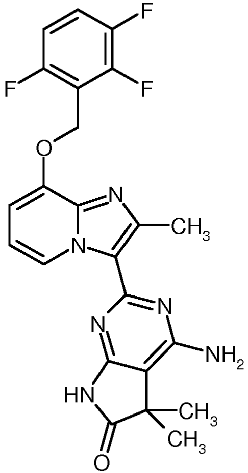

7.31 g (15.52 mmol, Reinheit ca. 71%) 2-Methyl-8-[(2,3,6-trifluorbenzyl)oxy]imidazo[1,2-a]pyridin-3-carboximidamid (Beispiel 45A) wurden unter Argon in 300 ml tert.-Butanol vorgelegt, bei RT nacheinander mit 1.25 ml (21.86 mmol) Essigsäure, 3.68 g (32.79 mmol) Kalium-tert.-butylat und 4.36 g (26.23 mmol) Methyl-3,3-dicyan-2,2-dimethylpropanoat (Beispiel 28A) in 10 ml tert.-Butanol versetzt und über Nacht unter Rückfluss erhitzt. Dann wurde das Gemisch abgekühlt und mit 700 ml Wasser versetzt. Der entstandene Niederschlag wurde filtriert, mit Wasser nachgewaschen und über Nacht im Vakuum getrocknet. Um die restliche Menge von Wasser zu entfernen wurde der Feststoff in Acetonitril mittels Ultraschall 1 h suspendiert, am Rotationsverdampfer eingeengt und über Nacht am Hochvakuum getrocknet. Es wurden 6.20 g (79% d. Th; Reinheit: 92%) der Titelverbindung erhalten.
LC-MS (Methode 1): Rₜ = 0.71 min
MS (ESpos): m/z = 469 (M+H)⁺
¹H NMR (400 MHz, DMSO-*d*₆) δ = 1.35 (s, 6 H), 2.74 (s, 3 H), 5.37 (s, 2 H), 6.73 (s, 2 H), 6.91 (t, 1 H), 7.00 (d, 1 H), 7.24 - 7.35 (m, 1 H), 7.61 - 7.73 (m, 1 H), 9.69 (d, 1 H), 10.89 (s, 1 H).

### Beispiel 10

### 4-Iod-5,5-dimethyl-2-{2-methyl-8-[(2,3,6-trifluorbenzyl)oxy]imidazo[1,2-a]pyridin-3-yl}-5,7-dihydro-6H-pyrrolo[2,3-d]pyrimidin-6-on

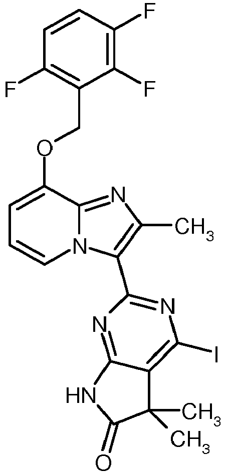

6.00 g (12.81 mmol) 4-Amino-5,5-dimethyl-2-{2-methyl-8-[(2,3,6-trifluorbenzyl)oxy]imidazo[1,2-a]pyridin-3-yl}-5,7-dihydro-6H-pyrrolo[2,3-d]pyrimidin-6-on aus Beispiel 9 wurden mit 17.15 g (64.04 mmol) Diiodmethan, 7.50 g (64.04 mmol) Isopentylnitrit und 10 g aktiviertem 4Å Molekularsieb (Pulver < 50 µm) in 120 ml abs. 1,4-Dioxan unter Argon vorgelegt und 22 h bei 85°C gerührt. Danach wurden 6.86 g (25.62 mmol) Diiodmethan und 3.00 g (25.62 mmol) Isopentylnitrit zugegeben und 18 h bei 85°C gerührt. Das Gemisch wurde abgekühlt und vom Molekularsieb abfiltriert. Der Filterrückstand wurde mit Dioxan nachgewaschen. Die vereinigten Filtrate wurden am Rotationsverdampfer eingeengt und das Rohprodukt mittels Kieselgelchromatographie gereinigt (Dichlormethan; Cyclohexan; Cyclohexan/Essigsäureethylester-Gradient). Man erhielt 4.51 g (56% d. Th., Reinheit 92%) der Titelverbindung.
LC-MS (Methode 1): Rₜ = 1.06 min
MS (ESpos): m/z = 580 (M+H)⁺
¹H NMR (400 MHz, DMSO-*d*₆) δ = 1.42 (s, 6 H), 2.72 (s, 3 H), 5.39 (s, 2 H), 7.05 - 7.15 (m, 2 H), 7.25 - 7.35 (m, 1 H), 7.62 - 7.74 (m, 1 H), 9.42 (d, 1 H), 11.68 (s, 1 H).

### Beispiel 11

### 4-Hydroxy-5,5-dimethyl-2-{2-methyl-8-[(2,3,6-trifluorbenzyl)oxy]imidazo[1,2-a]pyridin-3-yl}-5,7-dihydro-6H-pyrrolo[2,3-d]pyrimidin-6-on

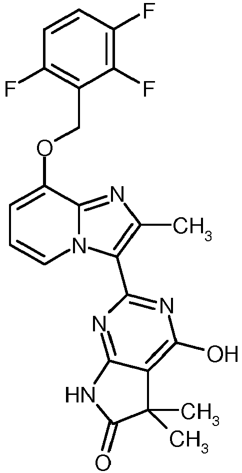

Die Zielverbindung entstand als Nebenprodukt bei der Präparation von Beispiel 10. Man erhielt 693 mg (12% d. Th., Reinheit 94%) der Titelverbindung.
LC-MS (Methode 1): Rₜ = 0.75 min
MS (ESpos): m/z = 470 (M+H)⁺

### Beispiel 12

### 4-[(2-Amino-2-methylpropyl)amino]-2-{8-[(2,6-difluorbenzyl)oxy]-2-methylimidazo[1,2-a]pyridin-3-yl}-5,5-dimethyl-5,7-dihydro-6H-pyrrolo[2,3-d]pyrimidin-6-on

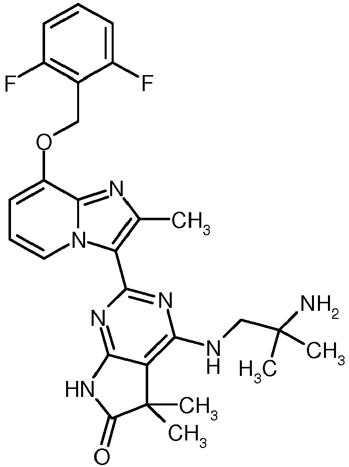

70 mg (0.10 mmol) 2-{8-[(2,6-Difluorbenzyl)oxy]-2-methylimidazo[1,2-a]pyridin-3-yl}-4-iod-5,5-dimethyl-5,7-dihydro-6H-pyrrolo[2,3-d]pyrimidin-6-on Trifluoracetat aus Beispiel 3 wurden in 1.1 ml NMP vorgelegt, mit 46 mg (0.52 mmol) 2-Methylpropan-1,2-diamin versetzt und 2 h bei 150°C in der Mikrowelle gerührt. Anschließend wurden nochmals 46 mg (0.52 mmol) 2-Methylpropan-1,2-diamin hinzugegeben und das Gemisch wurde 1 h bei 150°C in der Mikrowelle gerührt. Die Reaktionslösung wurde mit Acetonitril/Wasser/Ameisensäure gelöst und mittels präparativer HPLC gereinigt (RP18 Säule, Laufmittel: Acetonitril/Wasser-Gradient unter Zusatz von 0.1% Ameisensäure). Die Produktfraktionen wurden eingedampft, in Dichlormethan und wenig Methanol gelöst und zweimal mit gesättigter, wässriger Natriumhydrogencarbonatlösung gewaschen. Die vereinigten wässrigen Phasen wurden zweimal mit Dichlormethan reextrahiert. Die vereinigten organischen Phasen wurden über Natriumsulfat getrocknet, filtriert und eingedampft. Es wurden 38 mg (67% d. Th.) der Titelverbindung erhalten.
LC-MS (Methode 1): Rₜ = 0.66 min
MS (ESpos): m/z = 522 (M+H)⁺
¹H NMR (400 MHz, DMSO-*d*₆) δ = 1.04 (s, 6 H), 1.39 (s, 6 H), 2.73 (s, 3 H), 3.45 (d, 2 H), 5.31 (s, 2 H), 6.32 (t, 1 H), 6.96 (t, 1 H), 7.02 (d, 1 H), 7.23 (t, 2 H), 7.55 - 7.64 (m, 1 H), 9.62 (d, 1 H), 10.98 (br. s, 1 H).

### Beispiel 13

### rac-4-[(2-Amino-2-methylpentyl)amino]-2-{8-[(2,6-difluorbenzyl)oxy]-2-methylimidazo[1,2-a]pyridin-3-yl}-5,5-dimethyl-5,7-dihydro-6H-pyrrolo[2,3-d]pyrimidin-6-on (Racemat)

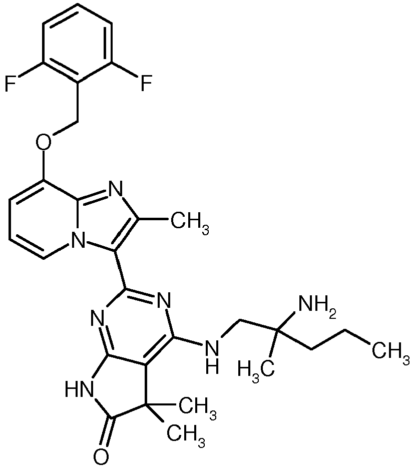

50 mg (0.07 mmol) 2-{8-[(2,6-Difluorbenzyl)oxy]-2-methylimidazo[1,2-a]pyridin-3-yl}-4-iod-5,5-dimethyl-5,7-dihydro-6H-pyrrolo[2,3-d]pyrimidin-6-on Trifluoracetat aus Beispiel 3 wurden in 0.5 ml NMP vorgelegt, mit 66 mg (0.35 mmol) *rac*-2-Methylpentan-1,2-diamin-Dihydrochlorid (Racemat) und 0.15 ml (0.83 mmol) *N,N*-Diisopropylethylamin versetzt und 6 h bei 150°C in der Mikrowelle gerührt. Anschließend wurden nochmals 40 mg (0.21 mmol) 2-Methylpentan-1,2-diamin-Dihydrochlorid und 0.08 ml (0.42 mmol) *N,N*-Diisopropylethylamin hinzugegeben und das Gemisch wurde 1.5 h bei 150°C in der Mikrowelle gerührt. Die Reaktionslösung wurde mit Acetonitril/Wasser/TFA verdünnt und mittels präparativer HPLC gereinigt (RP18 Säule, Laufmittel: Acetonitril/Wasser-Gradient unter Zusatz von 0.1% TFA). Die Produktfraktionen wurden eingedampft und in Dichlormethan und wenig Methanol gelöst und zweimal mit gesättigter, wässriger Natriumhydrogencarbonatlösung gewaschen. Die vereinigten wässrigen Phasen wurden zweimal mit Dichlormethan reextrahiert. Die vereinigten organischen Phasen wurden über Natriumsulfat getrocknet, filtriert und eingedampft. Das Rohprodukt wurde nochmals mittels Dickschichtchromatographie gereinigt (Laufmittel: Dichlormethan:Methanol = 10:1). Es wurden 3 mg (7% d. Th.) der Titelverbindung erhalten.
LC-MS (Methode 1): Rₜ = 0.73 min
MS (ESpos): m/z = 550 (M+H)⁺
¹H NMR (400 MHz, DMSO-*d*₆) δ = 0.79 - 0.87 (m, 3 H), 1.03 (s, 3 H), 1.29 - 1.43 (m, 10 H), 2.73 (s, 3 H), 3.48 - 3.63 (m, 2 H), 5.31 (s, 2 H), 6.19 - 6.25 (m, 1 H), 6.95 (t, 1 H), 7.02 (d, 1 H), 7.23 (t, 2 H), 7.55 - 7.64 (m, 1 H), 9.58 (d, 1 H), 11.00 (br. s, 1 H).

### Beispiel 14

### ent-4-{[2-Amino-2-(3,4-difluorphenyl)ethyl]amino}-2-{8-[(2,6-difluorbenzyl)oxy]-2-methylimidazo[1,2-a]pyridin-3-yl}-5,5-dimethyl-5,7-dihydro-6H-pyrrolo[2,3-d]pyrimidin-6-on

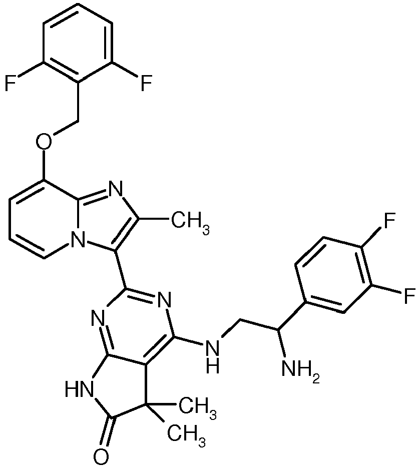

120 mg (0.18 mmol) 2-{8-[(2,6-Difluorbenzyl)oxy]-2-methylimidazo[1,2-a]pyridin-3-yl}-4-iod-5,5-dimethyl-5,7-dihydro-6H-pyrrolo[2,3-d]pyrimidin-6-on Trifluoracetat aus Beispiel 3 wurden in 1.8 ml NMP vorgelegt, mit 174 mg (0.71 mmol) *ent*-1-(3,4-Difluorphenyl)ethan-1,2-diamin-Dihydrochlorid (Enantiomer A) aus Beispiel 34A und 0.27 ml (1.56 mmol) *N,N-*Diisopropylethylamin versetzt und 6 h bei 150°C in der Mikrowelle gerührt. Die Reaktionslösung wurde mit Acetonitril/Wasser/TFA verdünnt und mittels präparativer HPLC gereinigt (RP18 Säule, Laufmittel: Acetonitril/Wasser-Gradient unter Zusatz von 0.1% TFA). Die Produktfraktionen wurden eingedampft, in Dichlormethan und wenig Methanol gelöst und zweimal mit gesättigter, wässriger Natriumhydrogencarbonatlösung gewaschen. Die vereinigten wässrigen Phasen wurden zweimal mit Dichlormethan extrahiert. Die vereinigten organischen Phasen wurden über Natriumsulfat getrocknet, filtriert und eingedampft. Das Rohprodukt wurde nochmals mittels Dickschichtchromatographie gereinigt (Laufmittel: Dichlormethan:Methanol = 20:1). Es wurden 19 mg (17% d. Th.) der Titelverbindung erhalten.
LC-MS (Methode 1): Rₜ = 0.69 min
MS (ESpos): m/z = 606 (M+H)⁺
¹H NMR (400 MHz, DMSO-*d*₆) δ = 1.27 (s, 3 H), 1.33 (s, 3 H), 2.73 (s, 3 H), 3.51 - 3.61 (m, 1 H), 3.63 - 3.72 (m, 1 H), 4.24 (t, 1 H), 5.34 (s, 2 H), 6.58 (d, 1 H), 6.94 (t, 1 H), 7.04 (d, 1 H), 7.09 - 7.16 (m, 1 H), 7.22 - 7.34 (m, 3 H), 7.37 - 7.44 (m, 1 H), 7.56 - 7.66 (m, 1 H), 9.57 (d, 1 H), 10.93 (s, 1 H).

### Beispiel 15

### 2-{8-[(2,6-Difluorbenzyl)oxy]-2-methylimidazo[1,2-a]pyridin-3-yl}-4-[(trans-4-hydroxycyclohexyl)amino]-5,5-dimethyl-5,7-dihydro-6H-pyrrolo[2,3-d]pyrimidin-6-on

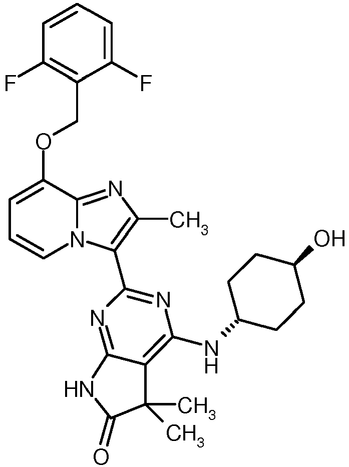

50 mg (0.07 mmol) 2-{8-[(2,6-Difluorbenzyl)oxy]-2-methylimidazo[1,2-a]pyridin-3-yl}-4-iod-5,5-dimethyl-5,7-dihydro-6H-pyrrolo[2,3-d]pyrimidin-6-on Trifluoracetat aus Beispiel 3 wurden in 0.6 ml NMP vorgelegt, mit 26 mg (0.22 mmol) *trans*-4-Aminocyclohexanol und 0.04 ml (0.22 mmol) *N,N*-Diisopropylethylamin versetzt und 3 h bei 150°C in der Mikrowelle gerührt. Die Reaktionslösung wurde mit Acetonitril/Wasser/Ameisensäure verdünnt und mittels präparativer HPLC gereinigt (RP18 Säule, Laufmittel: Acetonitril/Wasser-Gradient unter Zusatz von 0.1% Ameisensäure). Die Produktfraktionen wurden eingedampft, in Dichlormethan und wenig Methanol gelöst und zweimal mit gesättigter, wässriger Natriumhydrogencarbonatlösung gewaschen. Die vereinigten wässrigen Phasen wurden zweimal mit Dichlormethan extrahiert. Die vereinigten organischen Phasen wurden über Natriumsulfat getrocknet, filtriert und eingedampft. Es wurden 5 mg (12% d. Th.) der Titelverbindung erhalten.
LC-MS (Methode 1): Rₜ = 0.82 min
MS (ESpos): m/z = 549 (M+H)⁺
¹H NMR (400 MHz, DMSO-*d*₆) δ = 1.21 - 1.40 (m, 8 H), 1.43 - 1.59 (m, 2 H), 1.85 - 1.95 (m, 4 H), 2.73 (s, 3 H), 3.35 - 3.48 (m, 1 H), 4.05 - 4.15 (m, 1 H), 4.58 (d, 1 H), 5.32 (s, 2 H), 6.16 (d, 1 H), 6.95 (t, 1 H), 7.03 (d, 1 H), 7.25 (t, 2 H), 7.56 - 7.66 (m, 1 H), 9.58 (d, 1 H), 10.92 (s, 1 H).

### Beispiel 16

### 2-{8-[(2,6-Difluorbenzyl)oxy]-2-methylimidazo[1,2-a]pyridin-3-yl}-4-[(2-hydroxy-2-methylpropyl)amino]-5,5-dimethyl-5,7-dihydro-6H-pyrrolo[2,3-d]pyrimidin-6-on

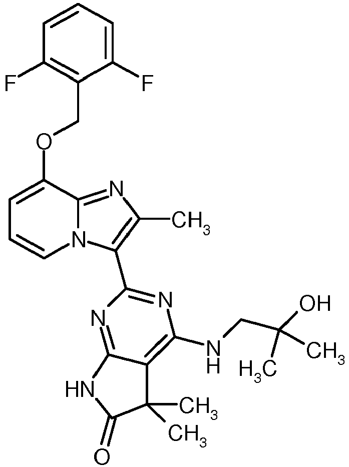

200 mg (0.28 mmol) 2-{8-[(2,6-Difluorbenzyl)oxy]-2-methylimidazo[1,2-a]pyridin-3-yl}-4-iod-5,5-dimethyl-5,7-dihydro-6H-pyrrolo[2,3-d]pyrimidin-6-on Trifluoracetat aus Beispiel 3 wurden in 2.8 ml NMP vorgelegt, mit 123 mg (1.38 mmol) 1-Amino-2-methylpropan-2-ol versetzt und 6 h bei 150°C in der Mikrowelle gerührt. Die Reaktionslösung wurde mit Acetonitril/Wasser/TFA gelöst und mittels präparativer HPLC gereinigt (RP18 Säule, Laufmittel: Acetonitril/Wasser-Gradient unter Zusatz von 0.1% TFA). Die Produktfraktionen wurden eingedampft, in Dichlormethan und wenig Methanol gelöst und zweimal mit gesättigter, wässriger Natriumhydrogencarbonatlösung gewaschen. Die vereinigten wässrigen Phasen wurden zweimal mit Dichlormethan reextrahiert.

Die vereinigten organischen Phasen wurden über Natriumsulfat getrocknet, filtriert und eingedampft. Es wurden 89 mg (60% d. Th) der Titelverbindung erhalten.
LC-MS (Methode 1): Rₜ = 0.81 min
MS (ESpos): m/z = 523 (M+H)⁺
¹H NMR (400 MHz, DMSO-*d*₆) δ = 1.13 (s, 6 H), 1.39 (s, 6 H), 2.74 (s, 3 H), 3.56 (d, 2 H), 4.64 (s, 1 H), 5.31 (s, 2 H), 6.20 (t, 1 H), 6.94 (t, 1 H), 7.01 (d, 1 H), 7.23 (t, 2 H), 7.55 - 7.64 (m, 1 H), 9.60 (d, 1 H), 10.97 (s, 1 H).

In Analogie zu Beispiel 16 wurden die in Tabelle 1 gezeigten Beispielverbindungen hergestellt, indem 2-{8-[(2,6-Difluorbenzyl)oxy]-2-methylimidazo[1,2-a]pyridin-3-yl}-4-iod-5,5-dimethyl-5,7-dihydro-6H-pyrrolo[2,3-d]pyrimidin-6-on aus Beispiel 2 oder das korrespondierende Trifluoracetat-Salz Beispiel 3 mit den entsprechenden, kommerziell erhältlichen oder zuvor beschriebenen Aminen (3 - 10 Äquivalente) unter den beschriebenen Reaktionsbedingungen (Reaktionszeit: 1 - 9 h; Temperatur: 150°C) in der Mikrowelle umgesetzt wurden. Bei Einsatz von Salzen der Amine erfolgte der Zusatz von *N,N*-Diisopropylethylamin (3 - 10 Äquivalente).

### Beispielhafte Aufarbeitung der Reaktionsmischung:

Das Reaktionsgemisch wurde mit Wasser/TFA verdünnt und mittels präparativer HPLC gereinigt (RP18 Säule, Laufmittel: Acetonitril/Wasser-Gradient unter Zusatz von 0.1% TFA oder 0.05% Ameisensäure). Das Rohprodukt wurde zusätzlich oder alternativ mittels Dickschichtchromatographie oder Kieselgelchromatographie gereinigt (Laufmittel: Dichlormethan/Methanol). Die produkthaltigen Fraktionen wurden eingedampft.

Der Rückstand wurde gegebenenfalls in Dichlormethan aufgenommen und mit gesättigter, wässriger Natriumhydrogencarbonatlösung gewaschen. Die wässrige Phase wurde zweimal mit Dichlormethan extrahiert, die vereinigten organischen Phasen über Natriumsulfat getrocknet, filtriert, eingedampft und lyophilisiert.

**Tabelle 1:**

| **Beispiel** | **IUPAC-Name / Struktur (Ausbeute)** | **Analytische Daten** |
|---|---|---|
| **17** | 2-{8-[(2,6-Difluorbenzyl)oxy]-2-methylimidazo[1,2-a]pyridin-3-yl}-4-{[(1-hydroxycyclopropyl)methyl]amino}-5,5-dimethyl-5,7-dihydro-6H-pyrrolo[2,3-d]pyrimidin-6-on | LC-MS (Methode 1): Rₜ = 0.79 min |
| | | MS (ESpos): m/z = 521 (M+H)⁺ |
| | 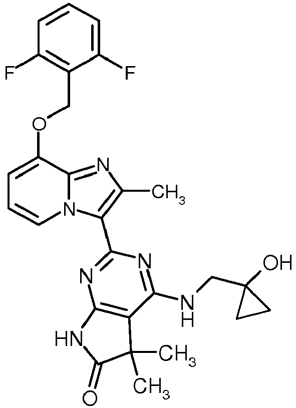 | ¹H NMR (400 MHz, DMSO-*d*₆) =0.50 - 0.59 (m, 4 H), 1.37 (s, 6 H), 2.72 (s, 3 H), 3.76 (d, 2 H), 5.32 (s, 2 H), 5.45 (s, 1 H), 6.44 (t, 1 H), 6.94 (t, 1 H), 7.01 (d, 1 H), 7.24 (t, 2 H), 7.55 - 7.64 (m, 1 H), 9.56 (d, 1 H), 10.95 (s, 1 H). |
| | (6% d. Th.) | |
| **18** | *rac*-2-{8-[(2,6-Difluorbenzyl)oxy]-2-methylimidazo[1,2-a]pyridin-3-yl}-5,5-dimethyl-4-[(3,3,3-trifluor-2-hydroxypropyl)amino]-5,7-dihydro-6H-pyrrolo[2,3-d]pyrimidin-6-on | LC-MS (Methode 1): Rₜ = 0.85 min |
| | | MS (ESpos): m/z = 563 (M+H)⁺ |
| | 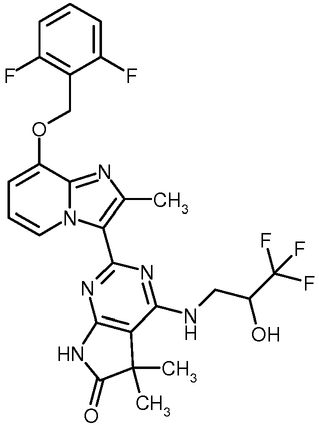 | ¹H NMR (400 MHz, DMSO-*d*₆) δ = 1.36/1.37 (2 x s, 6 H), 2.71 (s, 3 H), 3.55 - 3.66 (m, 1 H), 3.80 - 3.91 (m, 1 H), 4.25 - 4.39 (m, 1 H), 5.32 (s, 2 H), 5.45 (s, 1 H), 6.49 (d, 1 H), 6.79 (t, 1 H), 6.92 (t, 1 H), 7.03 (d, 1 H), 7.24 (t, 2 H), 7.55 - 7.65 (m, 1 H), 9.53 (d, 1 H), 11.00 (s, 1 H). |
| | (11% d. Th.) | |
| **19** | 2-{8-[(2,6-Difluorbenzyl)oxy]-2-methylimidazo[1,2-a]pyridin-3-yl}-5,5-dimethyl-4-[(3,3,3-trifluorpropyl)amino]-5,7-dihydro-6H-pyrrolo[2,3-d]pyrimidin-6-on | LC-MS (Methode 1): Rₜ = 0.94 min |
| | | MS (ESpos): m/z = 547 (M+H)⁺ |
| | 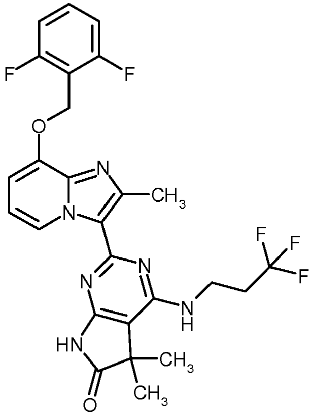 | ¹H NMR (400 MHz, DMSO-*d*₆) δ = 1.36 (s, 6 H), 2.57 - 2.69 (m 2 H), 2.72 (s, 3 H), 3.73 - 3.81 (m, 2 H), 5.33 (s, 2 H), 6.76 (t, 1 H), 6.93 (t, 1 H), 7.02 (d, 1 H), 7.23 (t, 2 H), 7.54 - 7.63 (m, 1 H), 9.54 (d, 1 H), 10.95 (s, 1 H). |
| | (33% d. Th.) | |
| **20** | *rac*-4-{[2-Amino-3-(4-methoxyphenyl)-2-methylpropyl]amino}-2-8-[(2,6-difluorbenzyl)oxy]-2-methylimidazo[1,2-a]pyridin-3-yl}-5,5-dimethyl-5,7-dihydro-6H-pyrrolo[2,3-d]pyrimidin-6-on | LC-MS (Methode 1): Rₜ = 0.76 min |
| | | MS (ESpos): m/z = 628 (M+H)⁺ |
| | 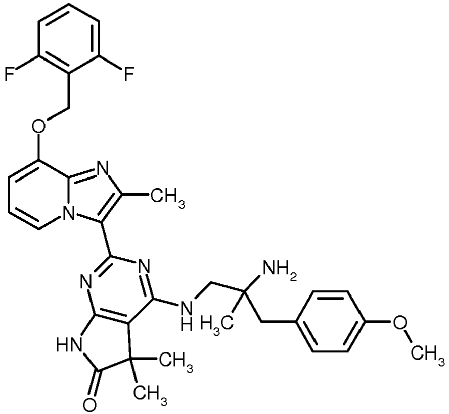 | ¹H NMR (400 MHz, DMSO-*d*₆) δ = 1.25 (s, 3 H), 1.39 (s, 6 H), 2.62 (s, 3 H), 2.68 - 2.79 (m, 2 H), 3.39 - 3.66 (m, 2 H), 3.72 (s, 3 H), 5.31 (s, 2 H), 6.08 - 6.20 (m, 1 H), 6.83 (d, 2 H), 6.92 (t, 1 H), 7.02 (d, 1 H), 7.16 (d, 2 H), 7.23 (t, 2 H), 7.55 - 7.64 (m, 1 H), 9.51 (d, 1 H), 10.98 (br. s, 1 H). |
| | (40% d. Th.; Reinheit 90%) | |
| **21** | *rac*-4-[(2-Amino-3-methoxy-2-methylpropyl)amino]-2-{8-[(2,6-difluorbenzyl)oxy]-2-methylimidazo[1,2-a]pyridin-3-yl}-5,5-dimethyl-5,7-dihydro-6H-pyrrolo [2,3-d]pyrimidin-6-on | LC-MS (Methode 1): Rₜ = 0.67 min |
| | | MS (ESpos): m/z = 552 (M+H)⁺ |
| | 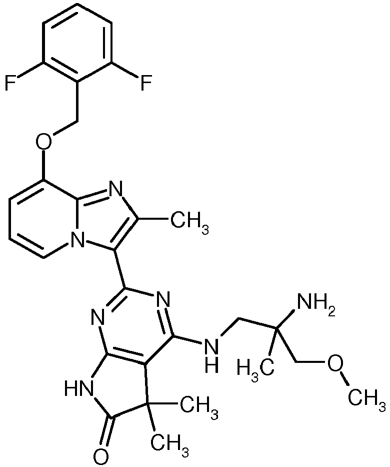 | ¹H NMR (400 MHz, DMSO-*d*₆) δ = 1.02 (s, 3 H), 1.39 (s, 6 H), 1.62 (br. s, 2 H), 2.73 (s, 3 H), 3.19 (s, 2 H), 3.26 (s, 3 H), 3.45 - 3.54 (m, 1 H), 3.56 - 3.63 (m, 1 H), 5.32 (s, 2 H), 6.17 - 6.23 (m, 1 H), 6.94 (t, 1 H), 7.02 (d, 1 H), 7.23 (t, 2 H), 7.56 - 7.65 (m, 1 H), 9.61 (d, 1 H), 10.97 (br. s, 1 H). |
| | (77% d. Th.) | |
| **22** | 2-{8-[(2,6-Difluorbenzyl)oxy]-2-methylimidazo[1,2-a]pyridin-3-yl}-4-{[(2S)-2,3-dihydroxypropyl]amino}-5,5-dimethyl-5,7-dihydro-6H-pyrrolo[2,3-d]pyrimidin-6-on | LC-MS (Methode 1): Rₜ = 0.72 min |
| | | MS (ESpos): m/z = 525 (M+H)⁺ |
| | 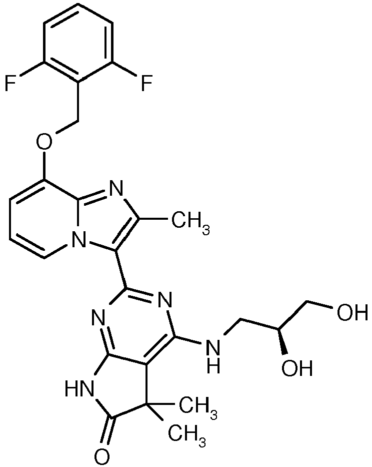 | ¹H NMR MHz, DMSO-*d*₆) δ (400 = 1.36 (s, 6 H), 2.73 (s, 3 H), 3.38 - 3.53 (m, 3 H), 3.57 - 3.69 (m, 1 H), 3.71 - 3.84 (m, 1 H), 4.56 - 4.72 (m, 1 H), 4.77 - 4.88 (m, 1 H), 5.32 (s, 2 H), 6.50 (t, 1 H), 6.93 (t, 1 H), 7.01 (d, 1 H), 7.19 - 7.30 (m, 2 H), 7.55 - 7.66 (m, 1 H), 9.58 (d, 1 H), 10.93 (br. s, 1 H). |
| | (39% d. Th., Reinheit 93%) | |
| **23** | 2-{8-[(2,6-Difluorbenzyl)oxy]-2-methylimidazo[1,2-a]pyridin-3-yl}-4-{[(2R)-2,3-dihydroxypropyl]amino}-5,5-dimethyl-5,7-dihydro-6H-pyrrolo[2,3-d]pyrimidin-6-on | LC-MS (Methode 1): Rₜ = 0.72 min |
| | | MS (ESpos): m/z = 525 (M+H)⁺ |
| | 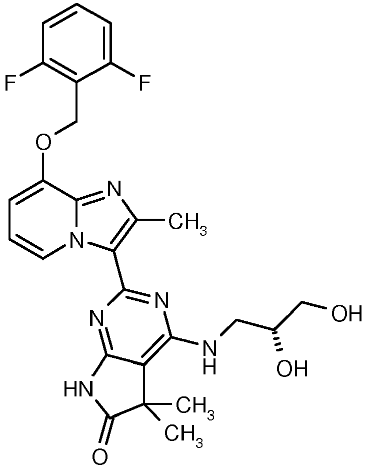 | ¹H NMR (400 MHz, DMSO-*d*₆) δ = 1.36 (s, 6 H), 2.73 (s, 3 H), 3.38 - 3.53 (m, 3 H), 3.57 - 3.69 (m, 1 H), 3.71 - 3.84 (m, 1 H), 4.56 - 4.72 (m, 1 H), 4.77 - 4.88 (m, 1 H), 5.32 (s, 2 H), 6.48 (br. s, 1 H), 6.93 (t, 1 H), 7.01 (d, 1H), 7.18 - 7.30 (m, 2 H), 7.55 - 7.66 (m, 1 H), 9.58 (d, 1 H), 10.93 (br. s, 1 H). |
| | (54% d. Th.) | |
| **24** | 2-{8-[(2,6-Difluorbenzyl)oxy]-2-methylimidazo[1,2-a]pyridin-3-yl}-4-[(2-hydroxyethyl)amino]-5,5-dimethyl-5,7-dihydro-6H-pyrrolo[2,3-d]pyrimidin-6-on Trifluoroacetat | LC-MS (Methode 1): Rₜ = 0.76 min |
| | | MS (ESpos): m/z = 495 (M-TFA+H)⁺ |
| | 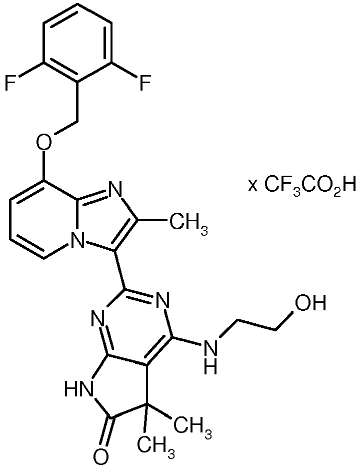 | ¹H NMR (400 MHz, DMSO-*d*₆) δ = 1.37 (s, 6 H), 2.81 (s, 3 H), 3.53 - 3.63 (m, 5 H), 5.45 (s, 2 H), 6.77 (br. s, 1 H), 7.22 - 7.31 (m, 2 H), 7.39 (br. s, 1 H), 7.46 - 7.68 (m, 2 H), 9.73 (d, 1 H), 11.04 (s, 1 H). |
| | (63% d. Th.) | |
| **25** | *rac*-4-[(2-Amino-3,3,3-trifluorpropyl)amino]-2-{8-[(2,6-difluorbenzyl)oxy]-2-methylimidazo[1,2-a]pyridin-3-yl}-5,5-dimethyl-5,7-dihydro-6H-pyrrolo [2,3-d]pyrimidin-6-on | LC-MS (Methode 1): Rₜ = 0.81 min |
| | | MS (ESpos): m/z = 562 (M+H)⁺ |
| | 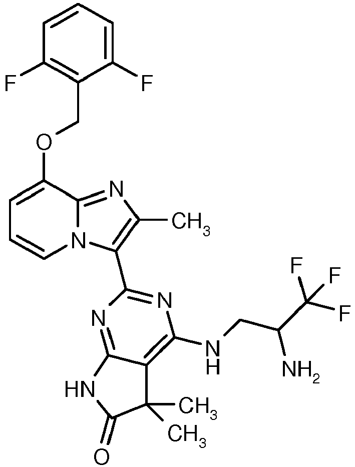 | ¹H NMR (500 MHz, DMSO-*d*₆) δ = 1.37/1.39 (2 x s, 6 H), 2.72 (s, 3 H), 3.39 - 3.49 (m, 1 H), 3.60 - 3.71 (m, 1 H), 3.88 - 3.98 (m, 1 H), 5.33 (s, 2 H), 6.68 (t, 1 H), 6.93 (t, 1 H), 7.02 (d, 1 H), 7.18 - 7.30 (m, 2 H), 7.53 - 7.64 (m, 1 H), 9.54 (d, 1 H), 10.98 (s, 1 H). |
| | (14% d. Th., Reinheit 94%) | |
| **26** | 2-{8-[(2,6-Difluorbenzyl)oxy]-2-methylimidazo[1,2-a]pyridin-3-yl}-4-{[2-(3,3-difluorpyrrolidin-1-yl)ethyl]amino}-5,5-dimethyl-5,7-dihydro-6H-pyrrolo [2,3-d]pyrimidin-6-on | LC-MS (Methode 1): Rₜ = 0.79 min |
| | | MS (ESpos): m/z = 584 (M+H)⁺ |
| | 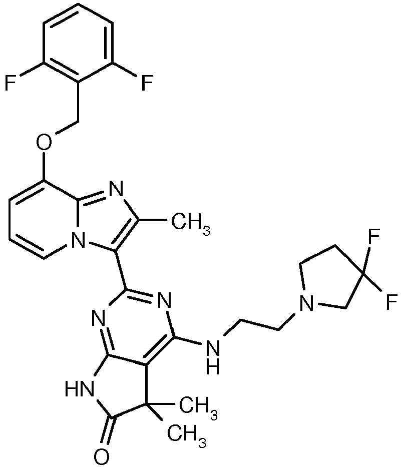 | ¹H NMR (500 MHz, DMSO-*d*₆) δ = 1.35 (s, 6 H), 2.13 - 2.29 (m, 2 H), 2.67 - 2.81 (m, 7 H), 2.95 (t, 2 H), 3.62 (d, 2 H), 5.33 (s, 2 H), 6.59 (t, 1 H), 6.93 (t, 1 H), 7.01 (d, 1 H), 7.24 (t, 2 H), 7.53 - 7.63 (m, 1 H), 9.56 (d, 1 H), 10.91 (s, 1 H). |
| | (15% d. Th.) | |
| **27** | *rac*-4-[(2-Amino-3,3,4,4-tetrafluorbutyl)amino]-2-{8-[(2,6-difluorbenzyl)oxy]-2-methylimidazo[1,2-a]pyridin-3-yl}-5,5-dimethyl-5,7-dihydro-6H-pyrrolo [2,3-d]pyrimidin-6-on | LC-MS (Methode 1): Rₜ = 0.86 min |
| | | MS (ESpos): m/z = 594 (M+H)⁺ |
| | 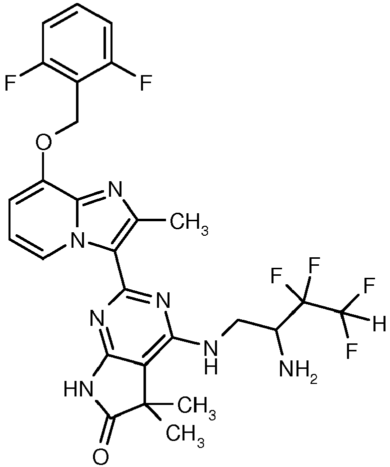 | ¹H NMR (400 MHz, DMSO-*d*₆) δ = 1.37 und 1.39 (2 s, 6 H), 2.71 (s, 3 H), 3.39 - 3.49 (m, 1 H), 3.50 - 3.62 (m, 1 H), 3.94 - 4.04 (m, 1 H), 5.32 (s, 2 H), 6.60 (t, 1 H), 6.91 (t, 1 H), 7.04 (d, 1 H), 7.19 - 7.29 (m, 2 H), 7.55 - 7.65 (m, 1 H), 9.53 (d, 1 H), 10.97 (s, 1 H). |
| | (13% d. Th.) | |
| **28** | 2-{8-[(2,6-difluorbenzyl)oxy]-2-methylimidazo[1,2-a]pyridin-3-yl}-4-[(4-hydroxy-4-methylcyclohexyl)amino]-5,5-dimethyl-5,7-dihydro-6H-pyrrolo[2,3-d]pyrimidin-6-on Formiat (Diastereomer 1) | LC-MS (Methode 1): Rₜ = 0.85 min |
| | | MS (ESpos): m/z = 563 (M-HCO₂H+H)⁺ |
| | 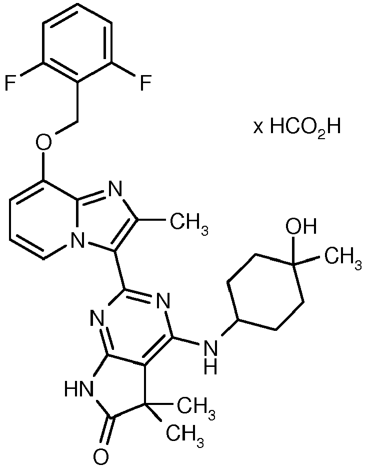 | ¹H NMR (500 MHz, DMSO-*d*₆) δ = 1.20 (s, 3 H), 1.37 (s, 6 H), 1.4 - 1.65 (m, 6 H), 1.78 - 1.86 (m, 2 H), 2.73 (s, 3 H), 4.11 - 4.21 (m, 1 H), 4.34 (s, 1 H), 5.33 (s, 2 H), 6.08 (d, 1 H), 6.95 (t, 1 H), 7.02 (d, 1 H), 7.24 (t, 2 H), 7.53 - 7.63 (m, 1 H), 9.57 (d, 1 H), 10.89 (s, 1 H). |
| | (15% d. Th.) | |
| **29** | 2-{8-[(2,6-difluorbenzyl)oxy]-2-methylimidazo[1,2-a]pyridin-3-yl}-4-[(4-hydroxy-4-methylcyclohexyl)amino]-5,5-dimethyl-5,7-dihydro-6H-pyrrolo[2,3-d]pyrimidin-6-on Formiat (Diastereomer 2) | LC-MS (Methode 1): Rₜ = 0.88 min |
| | | MS (ESpos): m/z = 563 (M-HCO₂H+H)⁺ |
| | 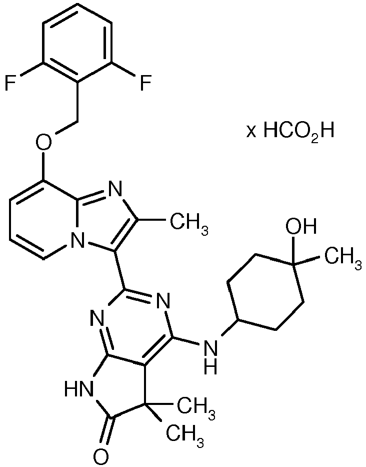 | ¹H NMR (500 MHz, DMSO-*d*₆) δ = 1.16 (s, 3 H), 1.32 - 1.45 (m, 8 H), 1.57 - 1.71 (m, 4 H), 1.80 - 1.93 (m, 2 H), 2.72 (s, 3 H), 3.99 - 4.11 (m, 1 H), 5.33 (s, 2 H), 6.19 (d, 1 H), 6.93 (t, 1 H), 7.00 (d, 1 H), 7.24 (t, 2 H), 7.59 (t, 1 H), 8.16 (s, 1 H), 9.57 (d, 1 H), 10.86 (s, 1 H). |
| | (19% d. Th., Reinheit 92%) | |
| **30** | 2-{8-[(2,6-Difluorbenzyl)oxy]-2-methylimidazo[1,2-a]pyridin-3-yl}-4-[(3-hydroxycyclobutyl)amino]-5,5-dimethyl-5,7-dihydro-6H-pyrrolo[2,3-d]pyrimidin-6-on [cis-/trans-Gemisch] | LC-MS (Methode 1): Rₜ = 0.73 min |
| | | MS (ESpos): m/z = 521 (M+H)⁺ |
| | 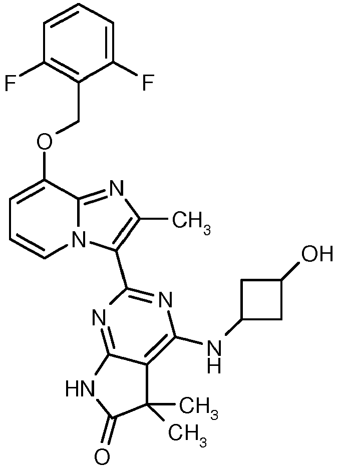 | ¹H NMR (400 MHz, DMSO-*d*₆) δ = 1.38 (s, 6 H), 1.98 - 2.09 und 2.18 - 2.30 (m, 2 H), 2.37 - 2.47 und 2.58 - 2.69 (m, 2 H), 2.71 - 2.75 (m, 3 H), 3.87 - 3.96 und 4.27 - 4.38 (m, 1 H), 4.14 - 4.17 und 4.68 - 4.76 (m, 1 H), 5.02 und 5.09 (d, 1 H), 5.32 (s, 2 H), 6.48 - 6.58 (m, 1 H), 6.91 - 7.06 (m, 2 H), 7.19 - 7.28 (m, 2H), 7.56 - 7.64 (m, 1 H), 9.53 - 9.60 (m, 1 H), 10.89 (s, 1 H). |
| | (8% d. Th.) | |

### Beispiel 31

### 4-[(2-Amino-2-methylpropyl)amino]-5,5-dimethyl-2-{2-methyl-8-[(2,3,6-trifluorbenzyl)oxy]imidazo[1,2-a]pyridin-3-yl}-5,7-dihydro-6H-pyrrolo[2,3-d]pyrimidin-6-on Formiat

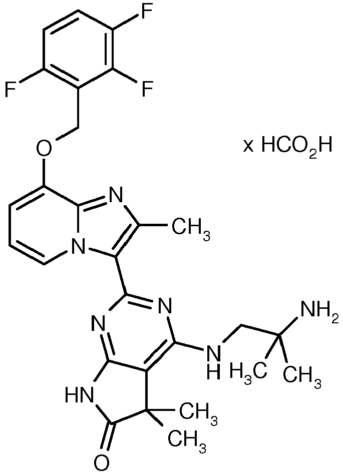

73 mg (0.126 mmol) 4-Iod-5,5-dimethyl-2-{2-methyl-8-[(2,3,6-trifluorbenzyl)oxy]imidazo[1,2-a]pyridin-3-yl}-5,7-dihydro-6H-pyrrolo[2,3-d]pyrimidin-6-on aus Beispiel 10 wurden in 0.47 ml NMP vorgelegt, mit 56 mg (0.63 mmol) 2-Methylpropan-1,2-diamin versetzt und 4.5 h bei 130°C in einem geschlossenen Gefäß gerührt. Die Reaktionsmischung wurde mittels präparativer HPLC gereinigt (RP18 Säule, Laufmittel: Acetonitril/Wasser-Gradient unter Zusatz von 0.05% Ameisensäure). Es wurden 37 mg (51% d. Th.) der Titelverbindung erhalten.
LC-MS (Methode 1): Rₜ = 0.62 min
MS (ESpos): m/z = 541 (M-HCO₂H+H)⁺
¹H NMR (400 MHz, DMSO-*d*₆) δ = 1.17 (s, 6 H), 1.41 (s, 6 H), 2.74 (s, 3 H), 3.61 - 3.69 (m, 2 H), 5.37 (s, 2 H), 6.64 - 6.74 (m, 1 H), 7.94 - 7.06 (m, 2 H), 7.26 - 7.35 (m, 1 H), 7.61 - 7.74 (m, 1 H), 8.32 (br. s, 2 H), 9.56 (d, 1 H), 11.00 (br. s, 1 H).

In Analogie zu Beispiel 31 wurden die in Tabelle 2 gezeigten Beispielverbindungen hergestellt, indem die entsprechenden Iodide aus Beispiel 10, Beispiel 2 oder Beispiel 3 mit den entsprechenden, kommerziell erhältlichen oder zuvor beschriebenen Aminen oder Diaminen (4 - 6 Äquivalente; ggf. als Hydrochlorid-Salze) und gegebenenfalls Zusatz von 1,8-Diazabicyclo(5.4.0)undec-7-en (4 - 12 Äquivalente) unter den beschriebenen Reaktionsbedingungen (Reaktionszeit: 1 - 5 h; Temperatur: 130°C) in einem geschlossenen Gefäß umgesetzt wurden.

### Beispielhafte Aufarbeitung der Reaktionsmischung:

Das Reaktionsgemisch wurde verdünnt und mittels präparativer HPLC gereinigt (RP18 Säule, Laufmittel: Acetonitril/Wasser-Gradient unter Zusatz von 0.1% TFA oder 0.05% Ameisensäure). Das Rohprodukt wurde zusätzlich oder alternativ mittels Kieselgelchromatographie gereinigt (Laufmittel: Dichlormethan/Methanol).

Gegebenenfalls wurden die produkthaltigen Fraktionen eingedampft, der Rückstand in Dichlormethan aufgenommen und mit gesättigter, wässriger Natriumhydrogencarbonatlösung gewaschen. Die wässrige Phase wurde zweimal mit Dichlormethan extrahiert, die vereinigten organischen Phasen über Natriumsulfat getrocknet, filtriert, eingedampft und lyophilisiert.

**Tabelle 2:**

| **Beispiel** | **IUPAC-Name / Struktur (Ausbeute)** | **Analytische Daten** |
|---|---|---|
| **32** | *rac*-rel-4-[(1R,5S)-1-Amino-3-azabicyclo[3.1.0]hex-3-yl]-2-{8-[(2,6-difluorbenzyl)oxy]-2-methylimidazo[1,2-a]pyridin-3-yl}-5,5-dimethyl-5,7-dihydro-6H-pyrrolo[2,3-d]pyrimidin-6-on Formiat | LC-MS (Methode 1): Rₜ = 0.62 min |
| | | MS (ESpos): m/z = 532 (M-HCO₂H+H)⁺ |
| | 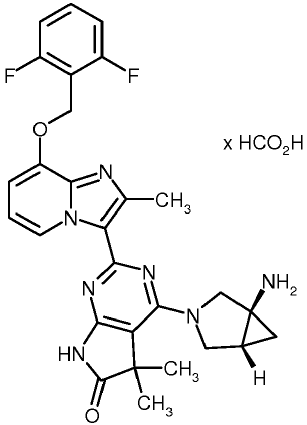 | ¹H NMR (500 MHz, DMSO-*d*₆) δ = 0.48 - 0.58 (m, 1 H), 0.88 - 0.98 (m, 1 H), 1.39 (s, 6 H), 1.47 - 1.59 (m, 1 H), 2.73 (s, 3 H), 3.55 - 3.65 (m, 1 H), 3.73 - 3.87 (m, 2 H), 4.04 - 4.10 (m, 1 H), 5.33 (s, 2 H), 6.97 (t, 1 H), 7.03 (d, 1 H), 7.23 (t, 2 H), 7.55 - 7.65 (m, 1 H), 8.14 (s, 1 H), 9.53 (d, 1 H), 11.09 (br. s, 1 H). |
| | (22% d. Th.) ¹⁾ | |
| **33** | 4-{[2-Amino-3-fluor-2-(fluormethyl)propyl]amino}-2-{8-[(2,6-difluorbenzyl)oxy]-2-methylimidazo[1,2-a]pyridin-3-yl}-5,5-dimethyl-5,7-dihydro-6H-pyrrolo[2,3-d]pyrimidin-6-on | LC-MS (Methode 1): Rₜ = 0.61 min |
| | | MS (ESpos): m/z = 558 (M+H)⁺ |
| | | ¹H NMR (500 MHz, DMSO-*d*₆) δ = 1.40 (s, 6 H), 2.73 (s, 3 H), 3.72 (d, 2 H), 4.21 (d, 1 H), 4.28 - 4.34 (m, 2 H), 4.42 (d, 1 H), 5.33 (s, 2 H), 6.38 (t, 1 H), 6.93 (t, 1 H), 7.03 (d, 1 H), 7.24 (t, 2 H), 7.56 - 7.65 (m, 1 H), 9.58 (d, 1 H), 10.98 (s, 1 H). |
| | 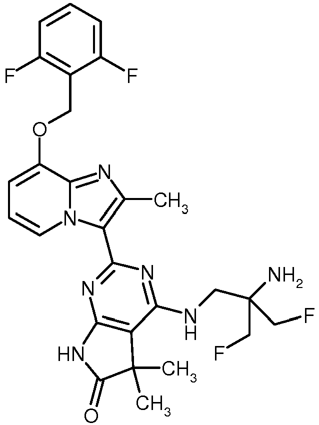 | |
| | (10% d. Th.) | |
| **34** | 4-[(2-Hydroxy-2-methylpropyl)amino]-5,5-dimethyl-2-{2-methyl-8-[(2,3,6-trifluorbenzyl)oxy]imidazo[1,2-a]pyridin-3-yl}-5,7-dihydro-6H-pyrrolo[2,3-d]pyrimidin-6-on | LC-MS (Methode 1): Rₜ = 0.81 min |
| | | MS (ESpos): m/z = 541 (M+H)⁺ |
| | | ¹H NMR (500 MHz, DMSO-*d*₆) δ = 1.14 (s, 6 H), 1.40 (s, 6 H), 2.73 (s, 3 H), 3.57 (d, 2 H), 4.62 (s, 1 H), 5.38 (s, 2 H), 6.18 (t, 1 H), 6.94 (t, 1 H), 7.01 (d, 1 H), 7.25 - 7.33 (m, 1 H), 7.62 - 7.70 (m, 1 H), 9.60 (d, 1 H), 10.94 (s, 1 H). |
| | 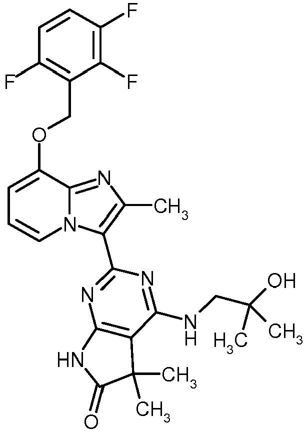 | |
| | (20% d. Th.) | |
| **35** | *rac*-4-[(2-Amino-3-methoxy-2-methylpropyl)amino]-5,5-dimethyl-2-{2-methyl-8-[(2,3,6-trifluorbenzyl)oxy]imidazo[1,2-a]pyridin-3-yl}-5,7-dihydro-6H-pyrrolo[2,3-d]pyrimidin-6-on Formiat | LC-MS (Methode 1): Rₜ = 0.62 min |
| | | MS (ESpos): m/z = 570 (M-HCO₂H+H)⁺ |
| | 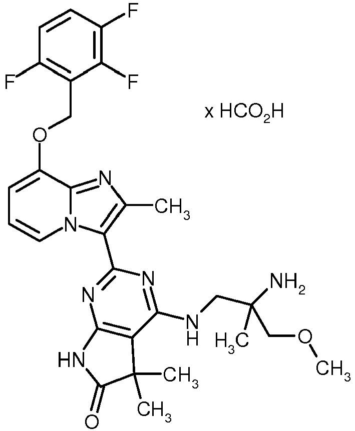 | ¹H NMR (500 MHz, DMSO-*d*₆) δ = 1.08 (s, 3 H), 1.39 (s, 6 H), 2.74 (s, 3 H), 3.20 - 3.26 (m, 5 H), 3.53 - 3.58 (m, 1 H), 3.65 - 3.70 (m, 1 H), 5.38 (s, 2 H), 6.33 (t, 1 H), 6.94 (t, 1 H), 7.02 (d, 1 H), 7.26 - 7.33 (m, 1 H), 7.62 - 7.70 (m, 1 H), 8.25 (s, 1 H), 9.59 (d, 1 H), 10.98 (br. s, 1 H). |
| | (43% d. Th.) | |
| **36** | 2-{8-[(2,6-Difluorbenzyl)oxy]-2-methylimidazo[1,2-a]pyridin-3-yl}-4-[(2S)-2-(hydroxymethyl)pyrrolidin-1-yl]-5,5-dimethyl-5,7-dihydro-6H-pyrrolo[2,3-d]pyrimidin-6-on | LC-MS (Methode 1): Rₜ = 0.81 min |
| | | MS (ESpos): m/z = 535 (M+H)⁺ |
| | | ¹H NMR (500 MHz, DMSO-*d*₆) δ = 1.34 (s, 3 H), 1.48 (s, 3 H), 1.87 - 2.14 (m, 5 H), 2.73 (s, 3 H), 3.37 - 3.43 (m, 1 H), 3.57 - 3.64 (m, 2 H), 3.72 - 3.78 (m, 1 H), 4.57 - 4.62 (m, 1 H), 4.78 (t, 1 H), 5.33 (s, 2 H), 6.93 (t, 1 H), 7.02 (d, 1 H), 7.23 (t, 2 H), 7.57 - 7.65 (m, 1 H), 9.57 (d, 1 H), 11.08 (br. s, 1 H). |
| | 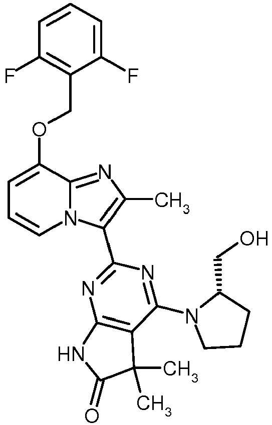 | |
| | (64% d. Th.) ²⁾ | |
| **37** | 2-{8-[(2,6-Difluorbenzyl)oxy]-2-methylimidazo[1,2-a]pyridin-3-yl}-4-[(2R)-2-(hydroxymethyl)pyrrolidin-1-yl]-5,5-dimethyl-5,7-dihydro-6H-pyrrolo[2,3-d]pyrimidin-6-on | LC-MS (Methode 1): Rₜ = 0.82 min |
| | | MS (ESpos): m/z = 535 (M+H)⁺ |
| | | ¹H NMR (500 MHz, DMSO-*d*₆) δ = 1.34 (s, 3 H), 1.48 (s, 3 H), 1.86 - 2.14 (m, 5 H), 2.73 (s, 3 H), 3.37 - 3.43 (m, 1 H), 3.57 - 3.64 (m, 2 H), 3.72 - 3.78 (m, 1 H), 4.57 - 4.62 (m, 1 H), 4.78 (t, 1 H), 5.33 (s, 2 H), 6.93 (t, 1 H), 7.03 (d, 1 H), 7.23 (t, 2 H), 7.57 - 7.65 (m, 1 H), 9.57 (d, 1 H), 11.08 (br. s, 1 H). |
| | 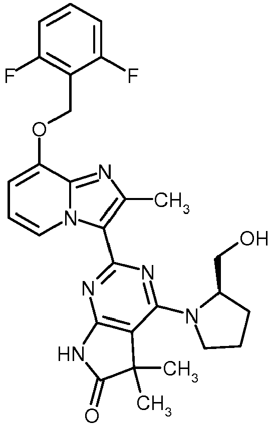 | |
| | (48% d. Th.) ²⁾ | |
| **38** | [1-(5,5-Dimethyl-2-{2-methyl-8-[(2,3,6-trifluorbenzyl)oxy]imidazo[1,2-a]pyridin-3-yl}-6-oxo-6,7-dihydro-5H-pyrrolo[2,3-d]pyrimidin-4-yl)piperidin-4-yl]essigsäure | LC-MS (Methode 1): Rₜ = 0.85 min |
| | | MS (ESpos): m/z = 595 (M+H)⁺ |
| | | ¹H NMR (400 MHz, DMSO-*d*₆) δ = 1.23 - 1.38 (m, 1 H), 1.40 (s, 6 H), 1.51 - 1.63 (m, 1 H), 1.75 - 1.93 (m, 2 H), 1.94 - 2.08 (m, 1 H), 2.19 - 2.27 (m, 2 H), 2.73 (s, 3 H), 2.80 (t, 1 H), 3.04 (t, 1 H), 4.15 - 4.23 (m, 1 H), 4.27 - 4. 35 (m, 1 H), 5.39 (s, 2 H), 6.97 (t, 1 H), 7.02 (d, 1 H), 7.27 - 7.34 (m, 1 H), 7.62 - 7.71 (m, 1 H), 9.52 (d, 1 H), 11.18 (s, 1 H), 12.24 (br. s, 1 H). |
| | 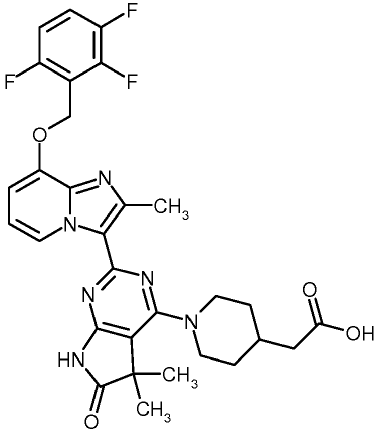 | |
| | (28% d. Th.) | |
| **39** | *rac*-4-[(2-Amino-3,3-difluor-2-methylpropyl)amino]-2-{8-[(2,6-difluorbenzyl)oxy]-2-methylimidazo[1,2-a]pyridin-3-yl}-5,5-dimethyl-5,7-dihydro-6H-pyrrolo[2,3-d]pyrimidin-6-on | LC-MS (Methode 1): Rₜ = 0.64 min |
| | | MS (ESpos): m/z = 558 (M+H)⁺ |
| | | ¹H NMR (400 MHz, DMSO-*d*₆) δ = 1.30 (s, 3 H), 1.41 (s, 6 H), 2.73 (s, 3 H), 3.77 - 3.87 (m, 1 H), 3.99 - 4.09 (m, 1 H), 5.38 (s, 2 H), 6.24 (t, 1 H), 6.54 - 6.63 (m, 1 H), 7.02 - 7.14 (m, 1 H), 7.24 (t, 2 H), 7.56 - 7.66 (m, 1 H), 8.48 (br. s, 2 H), 9.48 (d, 1 H), 11.16 (s, 1 H). |
| | 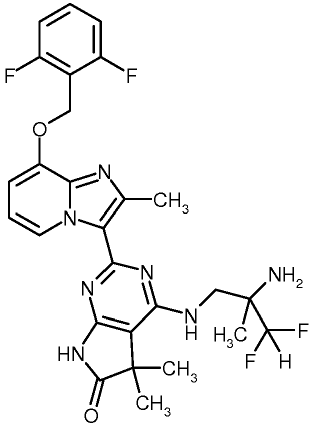 | |
| | (14% d. Th.) | |

| | | |
|---|---|---|
| ¹⁾ Bei der Reaktion wurden *rac*-rel-(1R,5S)-3-Azabicyclo[3.1.0]hexan-1-amin Dihydrochlorid (4 Äquivalente) und 1,8-Diazabicyclo(5.4.0)undec-7-en (8 Äquivalente) eingesetzt. ²⁾ Es erfolgte eine alternative Aufarbeitung: Zur Reaktionsmischung wurde Acetonitril und Wasser gegeben. Der entstandene Niederschlag wurde abfiltriert, mit wenig Acetonitril und Wasser gewaschen und im Hochvakuum getrocknet. | | |

### Beispiel 40

### rac-4-[(2-Amino-3,3,3-trifluor-2-methylpropyl)amino]-2-{8-[(2,6-difluorbenzyl)oxy]-2-methylimidazo[1,2-a]pyridin-3-yl}-5,5-dimethyl-5,7-dihydro-6H-pyrrolo[2,3-d]pyrimidin-6-on

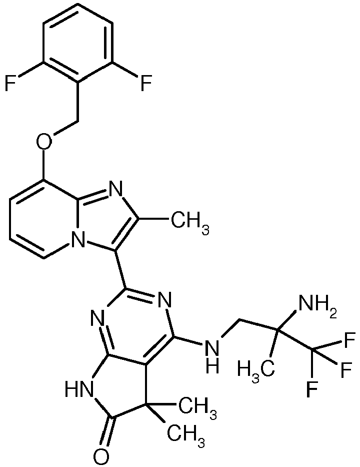

Herstellung des freien Amins: 160 mg (0.90 mmol) *rac*-3,3,3-Trifluor-2-methylpropan-1,2-diamindihydrochlorid aus Beispiel 69A wurden in Dichlormethan/Methanol gelöst und über eine Ionentauschersäule (StratoSpheres TM PL-HCO3 MP), die mit 1 ml Dichlormethan vorgespült wurde, gegeben. Es wurde mit 2 ml Dichlormethan nachgespült und die Lösung (enthält das freie Amin) wurde eingedampft.

70 mg (0.15 mmol) 4-Chlor-2-{8-[(2,6-difluorbenzyl)oxy]-2-methylimidazo[1,2-a]pyridin-3-yl}-5,5-dimethyl-5,7-dihydro-6H-pyrrolo[2,3-d]pyrimidin-6-on aus Beispiel 4 wurden in 1.3 ml NMP vorgelegt, mit *rac-* 3,3,3-trifluor-2-methylpropan-1,2-diamin (ca. 115 mg; 0.82 mmol; gelöst in 0.3 ml NMP) und 0.15 ml (0.88 mmol) *N,N*-Diisopropylethylamin versetzt und 3 h bei 150°C in der Mikrowelle gerührt. Anschließend wurden nochmals 81 mg (0.57 mmol) rac-3,3,3-trifluor-2-methylpropan-1,2-diamin (Herstellung wie oben beschrieben) zugegeben und 2 h bei 150 °C in der Mikrowelle gerührt. Die Reaktionslösung wurde mit Acetonitril/Wasser/TFA verdünnt und mittels präparativer HPLC gereinigt (RP18 Säule, Laufmittel: Acetonitril/Wasser-Gradient unter Zusatz von 0.1% TFA). Die Produktfraktionen wurden eingedampft, in Dichlormethan und wenig Methanol gelöst und zweimal mit gesättigter, wässriger Natriumhydrogencarbonatlösung gewaschen. Die vereinigten wässrigen Phasen wurden zweimal mit Dichlormethan extrahiert. Die vereinigten organischen Phasen wurden über Natriumsulfat getrocknet, filtriert und eingedampft. Es wurden 31 mg (36% d. Th.) der Titelverbindung erhalten.
LC-MS (Methode 1): Rₜ = 0.86 min
MS (ESpos): m/z = 576 (M+H)⁺
¹H NMR (400 MHz, DMSO-*d*₆) δ = 1.15 (s, 3 H), 1.40 (s, 6 H), 2.06 - 2.16 (br. s, 2 H), 2.73 (s, 3 H), 3.66 (dd, 1 H), 3.93 (dd, 1 H), 5.33 (s, 2 H), 6.36 (t, 1 H), 6.94 (t, 1 H), 7.02 (d, 1 H), 7.20 - 7.28 (m, 2 H), 7.54 - 7.64 (m, 1 H), 9.58 (d, 1 H), 11.00 (s, 1 H).

### Beispiel 41

### rac-4-[(2-Amino-2-methylpropyl)amino]-2-{8-[(2,6-difluorbenzyl)oxy]-2-methylimidazo[1,2-a]pyridin-3-yl}-5-methyl-5-(trifluormethyl)-5,7-dihydro-6H-pyrrolo[2,3-d]pyrimidin-6-on

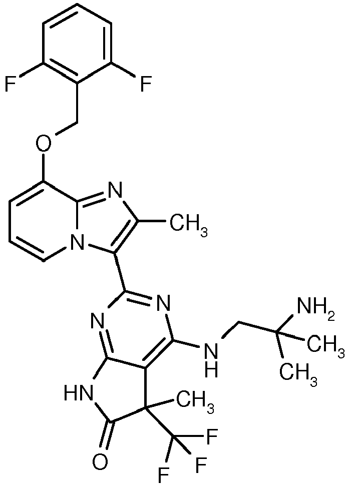

80 mg (0.09 mmol) (Reinheit 86%) *rac*-2-{8-[(2,6-difluorbenzyl)oxy]-2-methylimidazo[1,2-a]pyridin-3-yl}-4-iod-5-methyl-5-(trifluormethyl)-5,7-dihydro-6H-pyrrolo[2,3-d]pyrimidin-6-on Trifluoracetat aus Beispiel 7 wurden in 1 ml NMP vorgelegt, mit 49 µl (0.47 mmol) 1,2-Diamino-2-methylpropan versetzt und 3 h bei 150°C in der Mikrowelle gerührt. Die Reaktionslösung wurde mit Acetonitril/Wasser/TFA gelöst und mittels präparativer HPLC gereinigt (RP18 Säule, Laufmittel: Acetonitril/Wasser-Gradient unter Zusatz von 0.1% TFA). Die Produktfraktionen wurden eingedampft, in Dichlormethan und wenig Methanol gelöst und zweimal mit gesättigter, wässriger Natriumhydrogencarbonatlösung gewaschen. Die vereinigten wässrigen Phasen wurden zweimal mit Dichlormethan extrahiert. Die vereinigten organischen Phasen wurden über Natriumsulfat getrocknet, filtriert und eingedampft. Es wurden 33 mg (60% d. Th.) der Titelverbindung erhalten.
LC-MS (Methode 1): Rₜ = 0.71 min
MS (ESpos): m/z = 576 (M+H)⁺
¹H NMR (400 MHz, DMSO-*d*₆) δ = 1.11 (d, 6 H), 1.75 (s, 3 H), 2.75 (s, 3 H), 3.43 - 3.43 (m, 1 H), 3.57 - 67 (m, 1 H), 5.33 (s, 2 H), 6.36 (br. s, 1 H), 7.02 (t, 1 H), 7.06 (d, 1 H), 7.20 - 7.29 (m, 2 H), 7.55 - 7.65 (m, 1 H), 9.55 (d, 1 H).

### Beispiel 42

### 4-{[(1-Aminocyclopropyl)methyl]amino}-2-{8-[(2,6-difluorbenzyl)oxy]-2-methylimidazo[1,2-a]pyridin-3-yl}-5,5-dimethyl-5,7-dihydro-6H-pyrrolo[2,3-d]pyrimidin-6-on

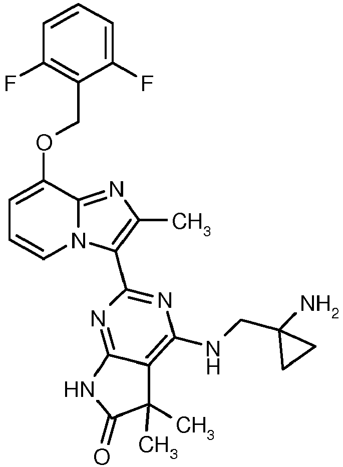

Zu einer Lösung aus 73 mg (0.1 mmol) (Reinheit 86%) tert-Butyl-(1-{[(2-{8-[(2,6-difluorbenzyl)oxy]-2-methylimidazo[1,2-a]pyridin-3-yl}-5,5-dimethyl-6-oxo-6,7-dihydro-5H-pyrrolo[2,3-d]pyrimidin-4-yl)amino]methyl}cyclopropyl)carbamat aus Beispiel 37A in 1 ml Diethylether wurden 2 ml 2 M wässrige Salzsäure in Diethylether gegeben. Es wurde für 16 h bei RT nachgerührt. Dann wurde eingeengt, am Hochvakuum getrocknet, der Rückstand in Acetonitril/Wasser gelöst und mittels präparativer HPLC gereinigt (RP18 Säule, Laufmittel: Acetonitril/Wasser-Gradient unter Zusatz von 0.1% TFA). Die Produktfraktionen wurden eingedampft, in Dichlormethan und wenig Methanol gelöst und zweimal mit gesättigter, wässriger Natriumhydrogencarbonatlösung gewaschen. Die vereinigten wässrigen Phasen wurden zweimal mit Dichlormethan extrahiert. Die vereinigten organischen Phasen wurden über Natriumsulfat getrocknet, filtriert und eingedampft. Es wurden 17 mg (31% d. Th.) der Titelverbindung erhalten.
LC-MS (Methode 1): Rₜ = 0.64 min
MS (ESpos): m/z = 520 (M+H)⁺
¹H NMR (400 MHz, DMSO-*d*₆) δ = 0.37 - 0.43 (m, 2 H), 0.49 - 0.55 (m, 2 H), 1.38 (s, 6 H), 1.91 (br. s, 2 H), 2.72 (s, 3 H), 3.62 (d, 2 H), 5.32 (s, 2 H), 6.48 - 6.56 (m, 1 H), 6.91 - 7.05 (m, 2 H), 7.19 - 7.29 (m, 2 H), 7.55 - 7.64 (m, 1 H), 9.55 (d, 1 H), 10.93 (br. s, 1 H).

### Beispiel 43

### 2-{8-[(2,6-Difluorbenzyl)oxy]-2-methylimidazo[1,2-a]pyhdin-3-yl}-4-(4-hydroxy-1H-pyrazol-1-yl)-5,5-dimethyl-5,7-dihydro-6H-pyrrolo[2,3-d]pyrimidin-6-on

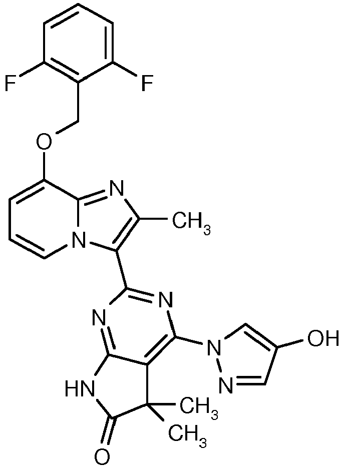

Zu einer Lösung aus 100 mg (0.18 mmol) (Reinheit 86%) 2-{8-[(2,6-Difluorbenzyl)oxy]-2-methylimidazo [1,2-a]pyridin-3-yl}-4-iod-5,5-dimethyl-5,7-dihydro-6H-pyrrolo[2,3-d]pyrimidin-6-on aus Beispiel 2 in 2 ml Acetonitril wurden unter Argon nacheinander 176 mg (2.10 mmol) 1H-Pyrazol-4-ol, 114 mg (0.35 mmol) Cäsiumcarbonat, 5 mg (0.04 mmol) Kupfer(I)oxid und 19 mg (0.14 mmol) 2-Hydroxybenzaldehydoxim gegeben. Das Reaktionsgemisch wurde für 2 h bei 160 °C in der Mikrowelle bestrahlt. Das Gemisch wurde in TFA/Wasser aufgenommen, über einen Milliporefilter filtriert und mittels präparativer HPLC gereinigt (RP18 Säule, Laufmittel: Acetonitril/Wasser-Gradient unter Zusatz von 0.1% TFA). Die produkthaltigen Fraktionen wurden eingedampft, in Dichlormethan und wenig 2 M Ammoniak in Methanol gelöst und mittels Dickschichtchromatographie (Laufmittel: Dichlormethan/Methanol = 10/1) nachgereinigt. Es wurden 5 mg (5% d. Th; Reinheit ca. 95%) der Titelverbindung erhalten.
LC-MS (Methode 1): Rₜ = 0.89 min
MS (ESpos): m/z = 518 (M+H)⁺
¹H NMR (500 MHz, DMSO-*d*₆) δ = 1.56 (s, 6 H), 2.81 (s, 3 H), 5.36 (s, 2 H), 7.05 (t, 1 H), 7.11 (d, 1 H), 7.20 - 7.30 (m, 2 H), 7.54 - 7.64 (m, 1 H), 7.70 (s, 1 H), 8.15 (s, 1 H), 9.38 (br. s, 1 H), 9.48 (d, 1 H), 11.53 - 11.81 (m, 1 H).

### Beispiel 44

### 2-{8-[(2,6-Difluorbenzyl)oxy]-2-methylimidazo[1,2-a]pyridin-3-yl}-5,5-dimethyl-4-(1H-pyrazol-4-yloxy)-5,7-dihydro-6H-pyrrolo[2,3-d]pyrimidin-6-on

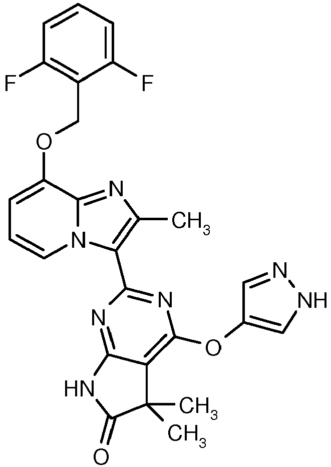

Die Zielverbindung entstand bei der Durchführung der Zielverbindung des Beispiels 43: Zu einer Lösung aus 100 mg (0.18 mmol) (Reinheit 86%) 2-{8-[(2,6-Difluorbenzyl)oxy]-2-methylimidazo[1,2-a]pyridin-3-yl}-4-iod-5,5-dimethyl-5,7-dihydro-6H-pyrrolo[2,3-d]pyrimidin-6-on aus Beispiel 2 in 2 ml Acetonitril wurden unter Argon nacheinander 176 mg (2.10 mmol) 1H-Pyrazol-4-ol, 114 mg (0.35 mmol) Cäsiumcarbonat, 5 mg (0.04 mmol) Kupfer(I)oxid und 19 mg (0.14 mmol) 2-Hydroxybenzaldehydoxim gegeben. Das Reaktionsgemisch wurde für 2 h bei 160 °C in der Mikrowelle bestrahlt. Das Gemisch wurde in TFA/Wasser aufgenommen, über einen Milliporefilter filtriert und mittels präparativer HPLC gereinigt (RP18 Säule, Laufmittel: Acetonitril/Wasser-Gradient unter Zusatz von 0.1% TFA). Die produkthaltigen Fraktionen wurden eingedampft, in Dichlormethan und wenig 2 M Ammoniak in Methanol gelöst und mittels Dickschichtchromatographie (Laufmittel: Dichlormethan/Methanol = 10/1) nachgereinigt. Es wurden 15 mg (16% d. Th.) der Titelverbindung erhalten.
LC-MS (Methode 1): Rₜ = 0.83 min
MS (ESpos): m/z = 518 (M+H)⁺
¹H NMR (500 MHz, DMSO-*d*₆) δ = 1.45 (s, 6 H), 5.31 (s, 2 H), 6.81 (t, 1 H), 7.05 (d, 1 H), 7.23 (t, 2 H), 7.56 - 7.61 (m, 2 H), 7.93 (br. s, 1 H), 9.17 (d, 1 H), 11.48 (s, 1 H), 12.89 (br. s, 1 H).

### Beispiel 45

### ent-4-[(2-Amino-3-methoxy-2-methylpropyl)amino]-2-{8-[(2,6-difluorbenzyl)oxy]-2-methylimidazo[1,2-a]pyridin-3-yl}-5,5-dimethyl-5,7-dihydro-6H-pyrrolo[2,3-d]pyrimidin-6-on (Enantiomer A)

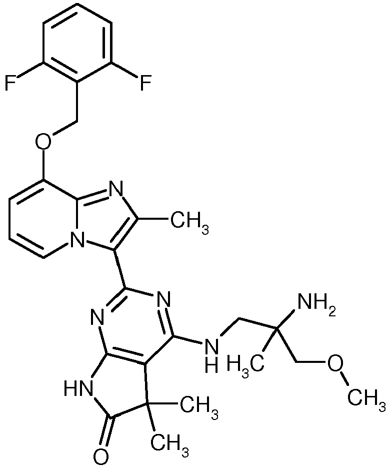

531 mg *rac*-4-[(2-Amino-3-methoxy-2-methylpropyl)amino]-2-{8-[(2,6-difluorbenzyl)oxy]-2-methylimidazo[1,2-a]pyridin-3-yl}-5,5-dimethyl-5,7-dihydro-6H-pyrrolo[2,3-d]pyrimidin-6-on (Beispiel 21) wurden an chiraler Phase in die Enantiomere getrennt [Säule: Daicel Chiralpak OD-H, 5 µm, 250 x 20 mm, Eluent: 50% iso-Hexan, 50% Ethanol + 0.2% Diethylamin, Fluß 15 ml/min; 40°C, Detektion: 220 nm].
Ausbeute: 283 mg Enantiomer A (99% Reinheit, >99% ee)
Rₜ = 4.18 min [Daicel Chiralpak OD-H, 5µm, 250 x 4.6 mm; Eluent: 50% iso-Hexan, 50% Ethanol + 0.2% Diethylamin; Fluss 1.0 ml/min; 40°C; Detektion: 235 nm].

### Beispiel 46

### ent-4-[(2-Amino-3-methoxy-2-methylpropyl)amino]-2-{8-[(2,6-difluorbenzyl)oxy]-2-methylimidazo[1,2-a]pyridin-3-yl}-5,5-dimethyl-5,7-dihydro-6H-pyrrolo[2,3-d]pyrimidin-6-on (Enantiomer B)

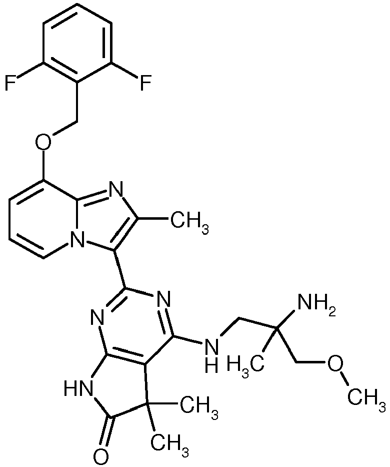

531 mg *rac*-4-[(2-Amino-3-methoxy-2-methylpropyl)amino]-2-{8-[(2,6-difluorbenzyl)oxy]-2-methylimidazo[1,2-a]pyridin-3-yl}-5,5-dimethyl-5,7-dihydro-6H-pyrrolo[2,3-d]pyrimidin-6-on (Beispiel 21) wurden an chiraler Phase in die Enantiomere getrennt [Säule: Daicel Chiralpak OD-H, 5 µm, 250 x 20 mm, Eluent: 50% iso-Hexan, 50% Ethanol + 0.2% Diethylamin, Fluß 15 ml/min; 40°C, Detektion: 220 nm].
Ausbeute: 126 mg Enantiomer B (99% Reinheit, >99% ee)
Rₜ = 7.15 min [Daicel Chiralpak OD-H, 5µm, 250 x 4.6 mm; Eluent: 50% iso-Hexan, 50% Ethanol + 0.2% Diethylamin; Fluss 1.0 ml/min; 40°C; Detektion: 235 nm].

### Beispiel 47

### 2-{8-[(2,6-Difluorbenzyl)oxy]-2-methylimidazo[1,2-a]pyridin-3-yl}-4-(2-hydroxyethoxy)-5,5-dimethyl-5,7-dihydro-6H-pyrrolo[2,3-d]pyrimidin-6-on

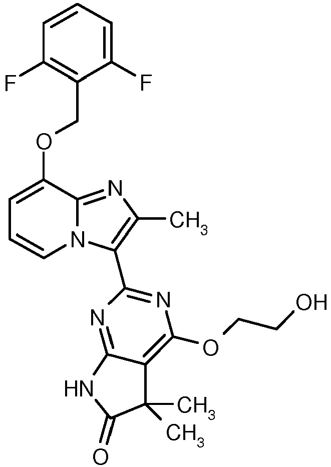

100 mg (0.178 mmol) 2-{8-[(2,6-Difluorbenzyl)oxy]-2-methylimidazo[1,2-a]pyridin-3-yl}-4-iod-5,5-dimethyl-5,7-dihydro-6H-pyrrolo[2,3-d]pyrimidin-6-on aus Beispiel 2 und 221 mg (3.56 mmol) Ethylenglykol wurden vorgelegt und mit 8.4 mg (0.036 mmol) 3,4,7,8-Tetramethyl-1,10-phenanthrolin, 3.4 mg (0.018 mmol) Kupfer(I)iodid und 116 mg (0.356 mmol) Cäsiumcarbonat versetzt. Anschließend wurde das Reaktionsgemisch in 3 ml Toluol suspendiert. Diese Suspension wurde 6 h bei 140°C in der Mikrowelle gerührt. Die Suspension wurde in Dichlormethan/Wasser aufgenommen und ausgeschüttelt. Die organische Phase wurde eingedampft. Der Rückstand wurde in Dichlormethan/wenig Methanol aufgenommen und mittels Dickschichtchromatographie gereinigt (Laufmittel: Dichlormethan/Methanol = 30/1). Es wurden 3.4 mg (4% d. Th., Reinheit 81%) der Zielverbindung erhalten.
LC-MS (Methode 1): Rₜ = 0.78 min
MS (ESpos): m/z = 496 (M+H)⁺

### Beispiel 48

### 2-{8-[(2,6-Difluorbenzyl)oxy]-2-methylimidazo[1,2-a]pyridin-3-yl}-5,5-dimethyl-6-oxo-6,7-dihydro-5H-pyrrolo[2,3-d]pyrimidin-4-carbonitril

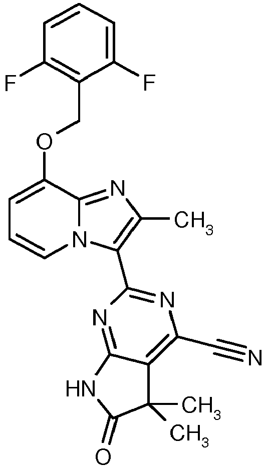

200 mg (0.36 mmol) 2-{8-[(2,6-Difluorbenzyl)oxy]-2-methylimidazo[1,2-a]pyridin-3-yl}-4-iod-5,5-dimethyl-5,7-dihydro-6H-pyrrolo[2,3-d]pyrimidin-6-on aus Beispiel 2 und 35 mg (0.39 mmol) Kupfer(I)-cyanid wurden in 3.8 ml DMSO gelöst und das Gemisch wurde unter Argon für 2 h bei 150 °C gerührt. Das Reaktionsgemisch wurde abgekühlt und vorsichtig mit gesättigter, wässriger Ammoniumchloridlösung/33%iger wässriger Ammoniaklösung (3/1) und Essigsäureethylester versetzt und 30 min bei Raumtemperatur gerührt. Es wurde über Celite abgesaugt, mit Essigsäureethylester nachgewaschen und die beiden Phasen des Filtrates wurden getrennt. Die organische Phase wurde dreimal mit gesättigter wässriger Ammoniumchloridlöung/33%iger wässriger Ammoniaklösung (3/1) und einmal mit gesättigter, wässriger Natriumchloridlösung gewaschen. Anschließend wurde die organische Phase über Natriumsulfat getrocknet, eingeengt und im Hochvakuum getrocknet. Das Rohprodukt wurde mittels präparativer HPLC gereinigt (RP18 Säule, Laufmittel: Methanol/Wasser-Gradient unter Zusatz von 0.1% TFA. Die Produktfraktionen wurden in Dichlormethan gelöst und zweimal mit gesättigter, wässriger Natriumhydrogencarbonatlösung gewaschen. Die vereinigten wässrigen Phasen wurden einmal mit Dichlormethan extrahiert. Die vereinten organischen Phasen wurden über Natriumsulfat getrocknet, filtriert und einrotiert. Es wurden 100 mg (60% d. Th; Reinheit ca. 95%) der Titelverbindung erhalten.
LC-MS (Methode 1): Rₜ = 0.99 min
MS (ESpos): m/z = 461 (M+H)⁺
¹H NMR (400 MHz, DMSO-*d*₆) δ = 1.48 (s, 6 H), 2.73 (s, 3 H), 5.35 (s, 2 H), 7.08 - 7.18 (m, 2 H), 7.19 - 7.28 (m, 2 H), 7.58 - 7.64 (m, 1 H), 9.42 (d, 1 H), 12.08 (br. s, 1 H).

### Beispiel 49

### 4-(Aminomethyl)-2-{8-[(2,6-difluorbenzyl)oxy]-2-methylimidazo[1,2-a]pyridin-3-yl}-5,5-dimethyl-5,7-dihydro-6H-pyrrolo[2,3-d]pyrimidin-6-on

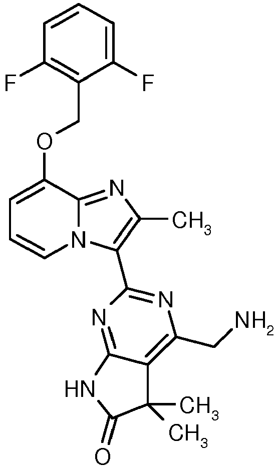

138 mg (0.28 mmol; Reinheit ca. 95%) 2-{8-[(2,6-Difluorbenzyl)oxy]-2-methylimidazo[1,2-a]pyridin-3-yl}-5,5-dimethyl-6-oxo-6,7-dihydro-5H-pyrrolo[2,3-d]pyrimidin-4-carbonitril aus Beispiel 48 wurden in 26 ml Essigsäure gelöst, mit 15 mg Palladium/Kohle (10%ig) versetzt und 3.5 h bei Normaldruck hydriert. Nach Zugabe von weiteren 15 mg Palladium/Kohle (10%ig) wurde nochmals für 45 min bei Normaldruck hydriert. Das Reaktionsgemisch wurde über einen Millipore-Filter filtriert, das Filtrat eingedampft und der Rückstand mittels präparativer HPLC gereinigt (RP18 Säule, Laufmittel: Acetonitril/Wasser-Gradient unter Zusatz von 0.1% TFA). Die Produktfraktionen wurden in Dichlormethan aufgenommen und zweimal mit gesättigter, wässriger Natriumhydrogencarbonatlösung gewaschen. Die vereinigten wässrigen Phasen wurden zweimal mit Dichlormethan extrahiert. Die vereinigten organischen Phasen wurden über Natriumsulfat getrocknet, filtriert und eingedampft. Es wurden 98 mg (74% d. Th.) der Zielverbindung erhalten.
LC-MS (Methode 1): Rₜ = 0.53 min
MS (ESpos): m/z = 465 (M+H)⁺
¹H NMR (500 MHz, DMSO-*d*₆) δ = 1.40 (s, 6 H), 2.76 (s, 3 H), 3.85 (s, 2 H), 5.33 (s, 2 H), 7.00 (t, 1 H), 7.06 (d, 1 H), 7.19 - 7.28 (m, 2 H), 7.57 - 7.64 (m, 1 H), 9.61 (d, 1 H).

### Beispiel 50

### 2-{8-[(2,6-Difluorbenzyl)oxy]-2-methylimidazo[1,2-a]pyridin-3-yl}-5,5-dimethyl-6-oxo-6,7-dihydro-5H-pyrrolo[2,3-d]pyrimidin-4-carboxamid Hydrochlorid

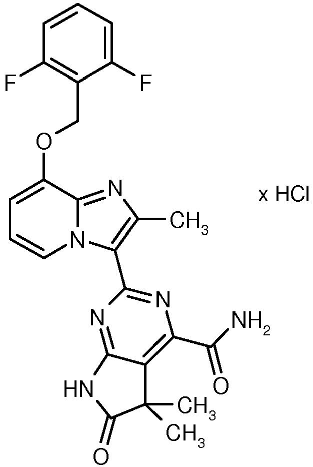

1.50 g (2.9 mmol; Reinheit ca. 90%) 2-{8-[(2,6-Difluorbenzyl)oxy]-2-methylimidazo[1,2-a]pyridin-3-yl}-5,5-dimethyl-6-oxo-6,7-dihydro-5H-pyrrolo[2,3-d]pyrimidin-4-carbonitril aus Beispiel 48 wurden in 13.9 ml 1,4-Dioxan vorgelegt, mit 3.62 ml (7.24 mmol) 2 N wässriger Natronlauge versetzt und 20 h bei 90°C gerührt. Das Reaktionsgemisch wurde mit 17.4 ml 1 N wässriger Salzsäure versetzt und eingedampft. Der Rückstand wurde mit Wasser verrührt, abfiltriert und mit Wasser nachgewaschen. Der abfiltrierte Feststoff wurde im Hochvakuum getrocknet. Es wurden 1.02 g (74% d. Th.) der Zielverbindung erhalten. Das Produkt wurde ohne weitere Aufreinigung weiter umgesetzt.
LC-MS (Methode 17): Rₜ = 1.74 min
MS (ESpos): m/z = 479 (M+H)⁺
¹H NMR (500 MHz, DMSO-*d*₆) δ = 1.49 (s, 6 H), 2.80 (s, 3 H), 5.43 (s, 2 H), 7.19 - 7.32 (m, 3 H), 7.39 (br. s, 1 H), 7.57 - 7.64 (m, 1 H), 8.04 (d, 2 H), 9.51 (d, 1 H), 11.84 (s, 1 H).

### Beispiel 51

### 2-{8-[(2,6-Difluorbenzyl)oxy]-2-methylimidazo[1,2-a]pyridin-3-yl}-5,5-dimethyl-6-oxo-6,7-dihydro-5H-pyrrolo[2,3-d]pyrimidin-4-carbonsäure

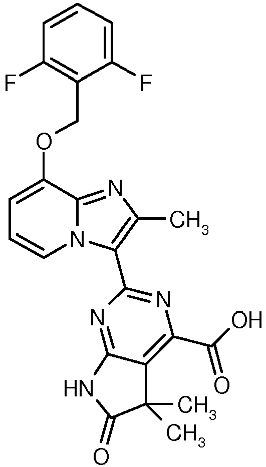

955 mg (1.85 mmol) 2-{8-[(2,6-Difluorbenzyl)oxy]-2-methylimidazo[1,2-a]pyridin-3-yl}-5,5-dimethyl-6-oxo-6,7-dihydro-5H-pyrrolo[2,3-d]pyrimidin-4-carboxamid Hydrochlorid aus Beispiel 50 wurden mit 17.3 ml halbkonzentrierter Salzsäure, 7.6 ml DMSO und 7.64 ml 1,4-Dioxan versetzt und 11 h bei 85°C gerührt. Die Reaktionslösung wurde am Rotationsverdampfer eingeengt. Anschließend wurde mit Wasser verdünnt und 30 min bei Raumtemperatur gerührt. Der enthaltene Feststoff wurde abfiltriert und getrocknet. Man erhielt so 256 mg (26% d. Th.; Reinheit ca. 89%) der Zielverbindung. Das Filtrat wurde dreimal mit Essigsäureethylester extrahiert. Die wässrige Phase wurde zur Hälfte eingedampft. Dabei fiel ein Feststoff aus. Dieser Niederschlag wurde abfiltriert und getrockent. Man erhielt 323 mg (33% d. Th.; Reinheit ca. 91%) der Zielverbindung. Das Filtrat wurde fünfmal mit Dichlormethan extrahiert. Die vereinigten organischen Phasen wurden eingeengt. Der Rückstand (DMSO-haltige Lösung) wurde mit Acetonitril/Wasser verdünnt und mittels präparativer HPLC portionsweise gereinigt (RP18 Säule, Laufmittel: Acetonitril/Wasser-Gradient unter Zusatz von 0.1% TFA). Hierbei wurden 105 mg (11% d. Th.; Reinheit ca. 90%) der Zielverbindung erhalten. In Summe wurden 684 mg (69% d. Th.; Reinheit ca. 90%) der Zielverbindung erhalten.
LC-MS (Methode 1): Rₜ = 0.68 min
MS (ESpos): m/z = 480 (M+H)⁺
¹H NMR (500 MHz, DMSO-*d*₆) δ = 1.49 (s, 6 H), 2.84 (s, 3 H), 5.43 (s, 2 H), 7.20 - 7.29 (m, 2 H), 7.30 - 7.52 (m, 2 H), 7.56 - 7.64 (m, 1 H), 9.81 (d, 1 H), 11.91 (s, 1 H), 14.09 (br. s, 1 H).

### Beispiel 52

### 5,5-Dimethyl-2-{2-methyl-8-[(2,3,6-trifluorbenzyl)oxy]imidazo[1,2-a]pyridin-3-yl}-6-oxo-6,7-dihydro-5H-pyrrolo[2,3-d]pyrimidin-4-carbonitril

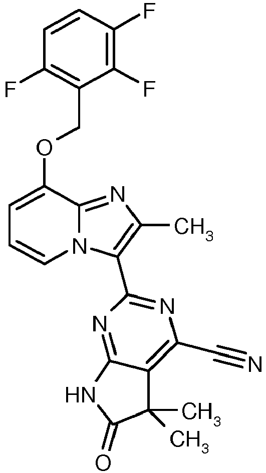

1.0 g (1.73 mmol) 4-Iod-5,5-dimethyl-2-{2-methyl-8-[(2,3,6-trifluorbenzyl)oxy]imidazo[1,2-a]pyridin-3-yl}-5,7-dihydro-6H-pyrrolo[2,3-d]pyrimidin-6-on aus Beispiel 10 und 170 mg (1.90 mmol) Kupfer(I)-cyanid wurden in 19 ml DMSO gelöst und das Gemisch wurde unter Argon für 3 h bei 150°C gerührt. Das Reaktionsgemisch wurde abgekühlt und vorsichtig mit gesättigter, wässriger Ammoniumchloridlösung/33%iger wässriger Ammoniaklösung (3/1) und Essigsäureethylester versetzt und 30 min bei Raumtemperatur gerührt. Es wurde über Celite gerührt, mit Essigsäureethylester nachgewaschen und die beiden Phasen des Filtrates wurden getrennt. Die organische Phase wurde dreimal mit gesättigter, wässriger Ammoniumchloridlöung/33%iger wässriger Ammoniaklösung (3/1) gewaschen. Anschließend wurde die organische Phase über Natriumsulfat getrocknet, einrotiert und im Hochvakuum getrocknet. Der Celite-Filterkuchen und die Natriumsulfatrückstände wurden mit Methanol/Dichlormethan (1/1) ausgerührt, abfiltriert und einrotiert. Der Rückstand wurde mit Wasser nachgewaschen und in Hochvakuum getrocknet. Alle Fraktionen von der Titelverbindung wurden vereinigt (689 mg; 95% d. Th.) und ohne weitere Aufreinigung weiter umgesetzt.
LC-MS (Methode 1): Rₜ = 0.98 min
MS (ESpos): m/z = 479 (M+H)⁺
¹H NMR (400 MHz, DMSO-*d*₆) δ = 12.08 (br. s, 1 H), 9.42 (d, 1 H), 7.62 - 7.74 (m, 1 H), 7.25 - 7.37 (m, 1 H), 7.08 - 7.21 (m, 2H), 5.40 (s, 2 H), 2.74 (s, 3 H), 1.48 (s, 6 H).

### Beispiel 53

### 5,5-Dimethyl-2-{2-methyl-8-[(2,3,6-trifluorbenzyl)oxy]imidazo[1,2-a]pyridin-3-yl}-6-oxo-6,7-dihydro-5H-pyrrolo[2,3-d]pyrimidin-4-carboxamid

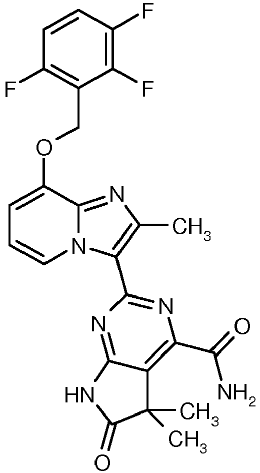

689 mg (1.44 mmol) 5,5-Dimethyl-2-{2-methyl-8-[(2,3,6-trifluorbenzyl)oxy]imidazo[1,2-a]pyridin-3-yl}-6-oxo-6,7-dihydro-5H-pyrrolo[2,3-d]pyrimidin-4-carbonitril aus Beispiel 52 wurden in 7 ml 1,4-Dioxan vorgelegt, mit 1.8 ml (3.58 mmol) 2 N wässriger Natronlauge versetzt und über Nacht bei 80°C gerührt. Das Reaktionsgemisch wurde mit 3.6 ml 1 N wässriger Salzsäure versetzt und eingedampft. Der Rückstand wurde mit Wasser verrührt, abfiltriert und mit Wasser nachgewaschen. Der abfiltrierte Feststoff wurde im Hochvakuum getrocknet. Es wurden 680 mg (95% d. Th.) der Zielverbindung erhalten. Das Produkt wurde ohne weitere Aufreinigung weiter umgesetzt.
LC-MS (Methode 1): Rₜ = 0.78 min
MS (ESpos): m/z = 497 (M+H)⁺
¹H NMR (400 MHz, DMSO-*d*₆) δ = 1.49 (s, 6 H), 2.77 (s, 3 H), 5.41 (s, 2 H), 7.02 - 7.22 (m, 2 H), 7.24 - 7.35 (m, 1 H), 7.61 - 7.72 (m, 1 H), 8.00 (d, 2 H), 9.44 (d, 1 H), 11.77 (s, 1 H).

### Beispiel 54

### 5,5-Dimethyl-2-{2-methyl-8-[(2,3,6-trifluorbenzyl)oxy]imidazo[1,2-a]pyridin-3-yl}-6-oxo-6,7-dihydro-5H-pyrrolo[2,3-d]pyrimidin-4-carbonsäure

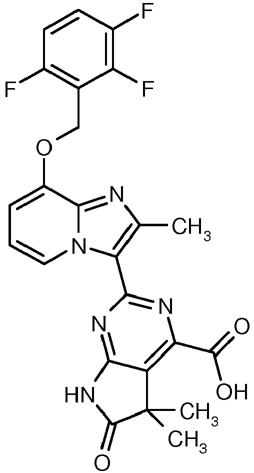

680 mg (1.37 mmol) 5,5-Dimethyl-2-{2-methyl-8-[(2,3,6-trifluorbenzyl)oxy]imidazo[1,2-a]pyridin-3-yl}-6-oxo-6,7-dihydro-5H-pyrrolo[2,3-d]pyrimidin-4-carboxamid aus Beispiel 53 wurden mit 11.9 ml konzentrierter Salzsäure versetzt und 14 h bei 80°C gerührt. Das Reaktionsgemisch wurde mit Wasser verdünnt. Der ausgefallene Feststoff wurde abfiltriert, mit Wasser gewaschen und am Hochvakuum getrocknet. Es wurden 426 mg (54% d. Th., Reinheit ca. 87%) der Zielverbindung erhalten. Das Produkt wurde ohne weitere Aufreinigung weiter umgesetzt.
LC-MS (Methode 23): Rₜ = 0.90 min
MS (ESpos): m/z = 498 (M+H)⁺
¹H NMR (400 MHz, DMSO-*d*₆) δ = 1.47 (s, 6 H), 2.79 (s, 3 H), 5.40 (s, 2 H), 7.03 - 7.21 (m, 2 H), 7.26 - 7.36 (m, 1 H), 7.61 - 7.71 (m, 1 H), 9.74 (d, 1 H), 11.83 (s, 1 H), 13.99 (br. s, 1 H).

### Beispiel 55

### N-Cyclopropyl-2-{8-[(2,6-difluorbenzyl)oxy]-2-methylimidazo[1,2-a]pyridin-3-yl}-5,5-dimethyl-6-oxo-6,7-dihydro-5H-pyrrolo[2,3-d]pyrimidin-4-carboxamid

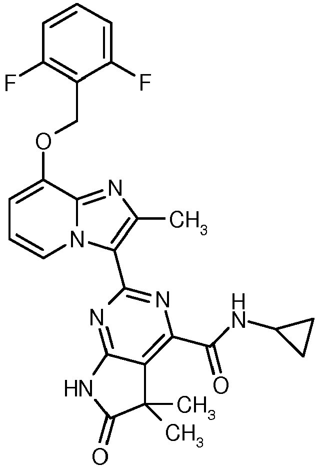

40 mg (0.076 mmol; Reinheit ca. 90%) 2-{8-[(2,6-Difluorbenzyl)oxy]-2-methylimidazo[1,2-a]pyridin-3-yl}-5,5-dimethyl-6-oxo-6,7-dihydro-5H-pyrrolo[2,3-d]pyrimidin-4-carbonsäure aus Beispiel 51, 8.7 mg (0.152 mmol) Cyclopropylamin und 40 µl (0.228 mmol) N,N-Diisopropylethylamin wurden in 1 ml DMF bei RT gelöst und anschließend mit 68 µl (0.114 mmol; 50%ig in Essigsäureethylester) 2,4,6-Tripropyl-1,3,5,2,4,6-trioxatriphosphinan-2,4,6-trioxid (Propanphosphonsäureanhydrid = T3P) versetzt. Das Gemisch wurde 2 h bei RT gerührt. Die Reaktionslösung wurde eingedampft, mit TFA/Wasser/Acetonitril versetzt und mittels präparativer HPLC gereinigt (RP18 Säule, Laufmittel: Acetonitril/Wasser-Gradient unter Zusatz von 0.1% TFA).

Die Produktfraktionen wurden eingedampft, anschließend in Dichlormethan aufgenommen und mit gesättigter, wässriger Natriumhydrogencarbonatlösung zweimal gewaschen. Die vereinigten wässrigen Phasen wurden zweimal mit Dichlormethan reextrahiert. Die vereinigten organischen Phasen wurden über Natriumsulfat getrocknet, filtriert, eingedampft und lyophilisiert. Es wurden 30 mg (75% d. Th.) der Zielverbindung erhalten.
LC-MS (Methode 1): Rₜ = 0.88 min
MS (ESpos): m/z = 519 (M+H)⁺
¹H NMR (500 MHz, DMSO-*d*₆) δ = 0.60 - 0.66 (m, 2 H), 0.76 - 0.82 (m, 2 H), 1.49 (s, 6 H), 2.73 (s, 3 H), 2.89 - 2.97 (m, 1 H), 5.34 (s, 2 H), 7.04 (t, 1 H), 7.09 (d, 1 H), 7.20 - 7.27 (m, 2 H), 7.57 - 7.64 (m, 1 H), 8.55 (d, 1 H), 9.40 (d, 1 H), 11.75 (s, 1 H).

In Analogie zu Beispiel 55 wurden die in Tabelle 3 gezeigten Beispielverbindungen hergestellt, indem die Carbonsäuren aus Beispiel 51 oder Beispiel 54 mit den entsprechenden, kommerziell erhältlichen oder zuvor beschriebenen Aminen (2 - 10 Äquivalente), Propanphosphonsäureanhydrid (1.5 - 4.5 Äquivalente) und *N,N*-Diisopropylethylamin (3 - 5 Äquivalente) unter den beschriebenen Reaktionsbedingungen (Reaktionszeit: 1 - 48 h; Temperatur: RT) umgesetzt wurden.

### Beispielhafte Aufarbeitung der Reaktionsmischung:

Das Reaktionsgemisch wurde mit Wasser, TFA oder Ameisensäure verdünnt und mittels präparativer HPLC gereinigt (RP18 Säule, Laufmittel: Acetonitril/Wasser-Gradient unter Zusatz von 0.1% TFA oder 0.05% Ameisensäure). Das Rohprodukt wurde zusätzlich oder alternativ mittels Dickschichtchromatographie oder Kieselgelchromatographie gereinigt (Laufmittel: Dichlormethan/Methanol). Die produkthaltigen Fraktionen wurden eingedampft.

Der Rückstand wurde ggf. in Dichlormethan aufgenommen und mit gesättigter, wässriger Natriumhydrogencarbonatlösung gewaschen. Die wässrige Phase wurde zweimal mit Dichlormethan extrahiert, die vereinigten organischen Phasen über Natriumsulfat getrocknet, filtriert, eingedampft und lyophilisiert.

**Tabelle 3:**

| **Beispiel** | **IUPAC-Name / Struktur (Ausbeute)** | **Analytische Daten** |
|---|---|---|
| **56** | 2-{8-[(2,6-Difluorbenzyl)oxy]-2-methylimidazo[1,2-a]pyridin-3-yl}-N-(2-hydroxy-2-methylpropyl)-5,5-dimethyl-6-oxo-6,7-dihydro-5H-pyrrolo[2,3-d]pyrimidin-4-carboxamid | LC-MS (Methode 1): Rₜ = 0.83 min |
| | | MS (ESpos): m/z = 551 (M+H)⁺ |
| | 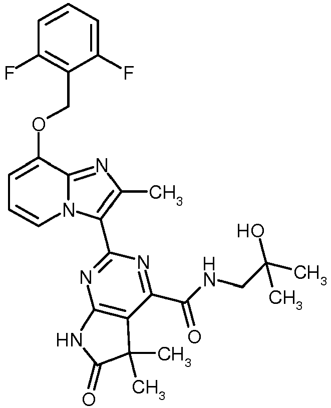 | ¹H NMR (400 MHz, DMSO-*d*₆) δ = 1.18 (s, 6 H), 1.51 (s, 6 H), 2.77 (s, 3 H), 4.77 (s, 1 H), 5.34 (s, 2 H), 7.02 (t, 1 H), 7.11 (d, 1 H), 7.20 - 7.29 (m, 2 H), 7.55 - 7.64 (m, 1 H), 8.51 (t, 1 H), 9.45 (d, 1 H), 11.78 (s, 1 H), [weiteres Signal unter Lösungsmittelpeak verborgen]. |
| | (64% d. Th.) | |
| **57** | *rac*-N-(2-Amino-3,3,3-trifluor-2-methylpropyl)-5,5-dimethyl-2-{2-methyl-8-[(2,3,6-trifluorbenzyl)oxy]imidazo[1,2-a]pyridin-3-yl}-6-oxo-6,7-dihydro-5H-pyrrolo[2,3-d]pyrimidin-4-carboxamid | LC-MS (Methode 1): Rₜ = 0.90 min |
| | | MS (ESpos): m/z = 622 (M+H)⁺ |
| | 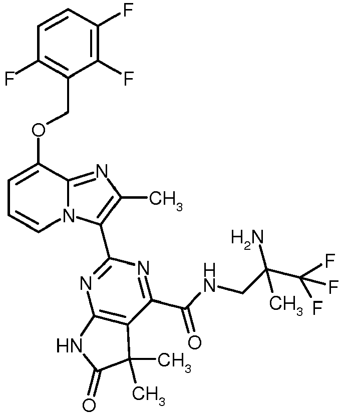 | ¹H NMR (400 MHz, DMSO-*d*₆) δ = 1.19 (s, 3 H), 1.50 (s, 3 H), 1.51 (s, 3 H), 2.78 (s, 3 H), 3.37 - 3.42 (m, 1 H), 3.63 - 3.70 (m, 1 H), 5.40 (s, 2 H), 7.05 (t, 1 H), 7.11 (d, 1 H), 7.26 - 7.33 (m, 1 H),7.63 - 7.71 (m, 1 H), 8.61 - 8.69 (m, 1 H), 9.45 (d, 1 H), 11.80 (s, 1 H) . |
| | (29% d. Th.) | |
| **58** | N-(2-Amino-2-methylpropyl)-5,5-dimethyl-2-{2-methyl-8-[(2,3,6-trifluorbenzyl)oxy]imidazo[1,2-a]pyridin-3-yl}-6-oxo-6,7-dihydro-5H-pyrrolo[2,3-d]pyrimidin-4-carboxamid Trifluoracetat | LC-MS (Methode 1): Rₜ = 0.67 min |
| | | MS (ESpos): m/z = 568 (M-TFA+H)⁺ |
| | 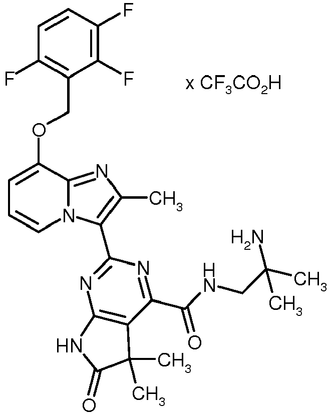 | ¹H NMR (400 MHz, DMSO-*d*₆) δ = 1.31 (s, 6 H), 1.52 (s, 6 H), 2.81 (s, 3 H), 5.44 (s, 2 H), 7.09 (t, 1 H), 7.23 (d, 1 H), 7.63 - 7.73 (m, 1 H), 7.86 (br. s, 3 H), 8.87 - 8.93 (m, 1 H), 9.50 (d, 1 H), 11.88 (s, 1 H) [weiteres Signal unter Lösungsmittelpeak verborgen (3.25 - 3.75 ppm)]. |
| | (27% d. Th.)¹⁾ | |
| **59** | 5,5-Dimethyl-2-{2-methyl-8-[(2,3,6-trifluorbenzyl)oxy]imidazo[1,2-a]pyridin-3-yl}-6-oxo-N-(3,3,3-trifluorpropyl)-6,7-dihydro-5H-pyrrolo[2,3-d]pyrimidin-4-carboxamid | LC-MS (Methode 1): Rₜ = 0.97 min |
| | | MS (ESpos): m/z = 593 (M+H)⁺ |
| | 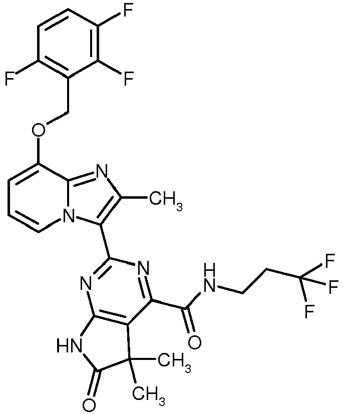 | ¹H NMR (400 MHz, DMSO-*d*₆) δ = 1.49 (s, 6 H), 2.5 - 2.69 (m, 2 H), 2.76 (s, 3 H), 3.58 - 3.65 (m, 2 H), 5.40 (s, 2 H), 7.01 (t, 1 H), 7.10 (d, 1 H), 7.25 - 7.35 (m, 1 H), 7.61 - 7.72 (m, 1 H), 8.74 - 8.82 (m, 1 H), 9.40 (d, 1 H), 11.78 (s, 1H). |
| | (47% d. Th.) | |
| **60** | 5,5-Dimethyl-4-[(4-methylpiperazin-1-yl)carbonyl]-2-{2-methyl-8-[(2,3,6-trifluorbenzyl)oxy]imidazo[1,2-a]pyridin-3-yl}-5,7-dihydro-6H-pyrrolo[2,3-d]pyrimidin-6-on Trifluoracetat | LC-MS (Methode 1): Rₜ = 0.64 min |
| | | MS (ESpos): m/z = 580 (M-TFA+H)⁺ |
| | 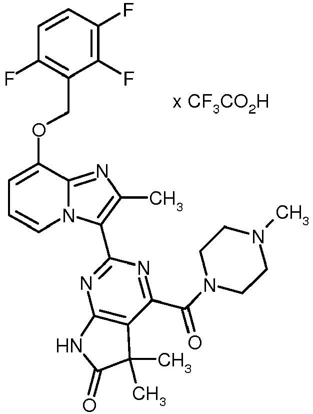 | |
| | (62% d. Th.)¹⁾ | |
| **61** | *rac*-4-[(4-Amino-3,3-difluorpyrrolidin-1-yl)carbonyl]-5,5-dimethyl-2-{2-methyl-8-[(2,3,6-trifluorbenzyl)oxy]imidazo[1,2-a]pyridin-3-yl}-5,7-dihydro-6H-pyrrolo[2,3-d]pyrimidin-6-on Trifluoracetat | LC-MS (Methode 1): Rₜ = 0.71 min |
| | | MS (ESpos): m/z = 602 (M-TFA+H)⁺ |
| | 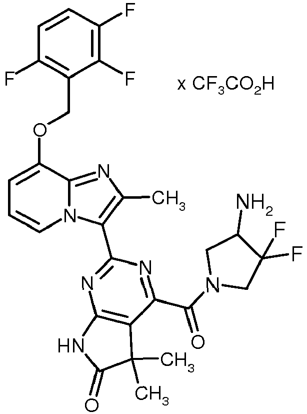 | |
| | (22% d. Th.) | |
| **62** | *rac*-4-[(3-Hydroxypyrrolidin-1-yl)carbonyl]-5,5-dimethyl-2-{2-methyl-8-[(2,3,6-trifluorbenzyl)oxy]imidazo[1,2-a]pyridin-3-yl}-5,7-dihydro-6H-pyrrolo[2,3-d]pyrimidin-6-on | LC-MS (Methode 1): Rₜ = 0.71 min |
| | | MS (ESpos): m/z = 567 (M+H)⁺ |
| | 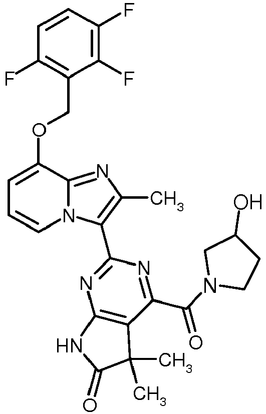 | |
| | (23% d. Th.)¹⁾ | |
| **63** | *rac*-4-{[3-(Hydroxymethyl)pyrrolidin-1-yl]carbonyl}-5,5-dimethyl-2-{2-methyl-8-[(2,3,6-trifluorbenzyl)oxy]imidazo[1,2-a]pyridin-3-yl}-5,7-dihydro-6H-pyrrolo[2,3-d]pyrimidin-6-on | LC-MS (Methode 1): Rₜ = 0.73 min |
| | | MS (ESpos): m/z = 581 (M+H)⁺ |
| | 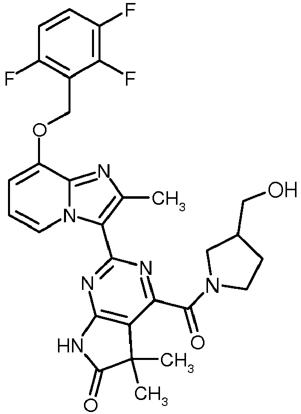 | |
| | (7% d. Th.)¹⁾ | |
| **64** | 5,5-Dimethyl-2-{2-methyl-8-[(2,3,6-trifluorbenzyl)oxy]imidazo[1,2-a]pyridin-3-yl}-4-(piperazin-1-ylcarbonyl)-5,7-dihydro-6H-pyrrolo[2,3-d]pyrimidin-6-on Trifluoracetat | LC-MS (Methode 1): Rₜ = 0.62 min |
| | | MS (ESpos): m/z = 566 (M-TFA+H)⁺ |
| | 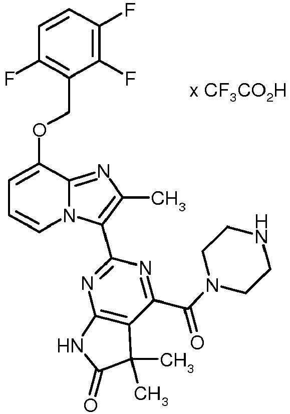 | |
| | (11% d. Th.)¹⁾ | |

| | | |
|---|---|---|
| ¹⁾ Das Produkt wurde anschließend noch einmal mittels präparativer HPLC gereinigt (RP18 Säule, Laufmittel: Acetonitril/Wasser-Gradient unter Zusatz von 1% TFA). | | |

### Beispiel 65

### N-[(2-{8-[(2,6-Difluorbenzyl)oxy]-2-methylimidazo[1,2-a]pyridin-3-yl}-5,5-dimethyl-6-oxo-6,7-dihydro-5H-pyrrolo[2,3-d]pyrimidin-4-yl)methyl]methansulfonamid

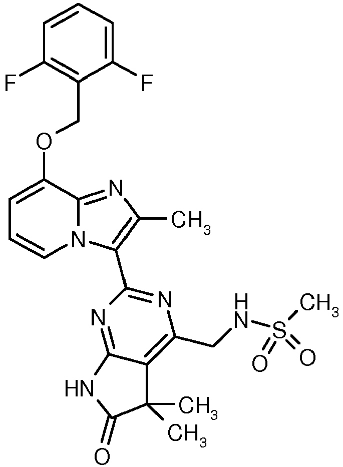

30 mg (0.06 mmol) 4-(Aminomethyl)-2-{8-[(2,6-difluorbenzyl)oxy]-2-methylimidazo[1,2-a]pyridin-3-yl}-5,5-dimethyl-5,7-dihydro-6H-pyrrolo[2,3-d]pyrimidin-6-on aus Beispiel 49 wurden unter Argon in 0.67 ml Dichlormethan/DMF (1/1) vorgelegt. Bei RT wurden 18 mg (0.14 mmol) N,N-Diisopropylethylamin hinzugegeben und 15 min gerührt. Anschließend wurden bei RT 8 mg (0.07 mmol) Methansulfonsäurechlorid hinzugegeben und das Gemisch wurde 1 h bei Raumtemperatur gerührt. Es wurden nochmals 2.3 mg (0.02 mmol) Methansulfonsäurechlorid hinzugegeben und das Gemisch wurde 1 h bei RT gerührt. Die Reaktionslösung wurde eingedampft und der Rückstand wurde in Dichlormethan aufgenommen und mittels Dickschichtchromatographie gereinigt (Laufmittel: Dichlormethan/Methanol = 10/1). Es wurden 26 mg der Zielverbindung (72% d. Th.) erhalten.
LC-MS (Methode 1): Rₜ = 0.95 min
MS (ESpos): m/z = 543 (M+H)⁺
¹H NMR (500 MHz, DMSO-*d*₆) δ = 1.43 (s, 6 H), 2.77 (s, 3 H), 2.98 (s, 3 H), 4.34 (d, 2 H), 5.34 (s, 2 H), 6.97 (t, 1 H), 7.07 (d, 1 H), 7.20 - 7.28 (m, 2 H), 7.56 - 7.64 (m, 1 H), 7.70 - 7.75 (m, 1 H), 9.71 (d, 1 H), 11.56 (s, 1 H).

### Beispiel 66

### N-[(2-{8-[(2,6-Difluorbenzyl)oxy]-2-methylimidazo[1,2-a]pyridin-3-yl}-5,5-dimethyl-6-oxo-6,7-dihydro-5H-pyrrolo[2,3-d]pyrimidin-4-yl)methyl]cyclopropancarboxamid

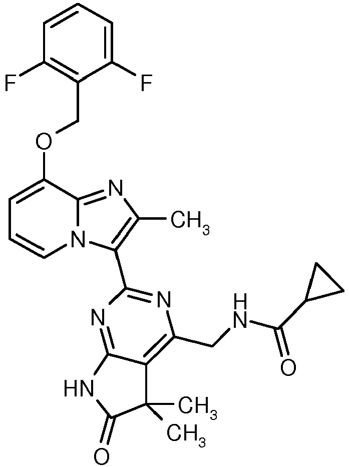

40 mg (0.08 mmol) 4-(Aminomethyl)-2-{8-[(2,6-difluorbenzyl)oxy]-2-methylimidazo[1,2-a]pyridin-3-yl}-5,5-dimethyl-5,7-dihydro-6H-pyrrolo[2,3-d]pyrimidin-6-on aus Beispiel 49 wurden vorgelegt. Bei 0°C wurden unter Argon 38 mg (0.30 mmol) N,N-Diisopropylethylamin und eine Lösung von 10 mg (0.09 mmol) Cyclopropancarbonylchlorid in 3 ml Tetrahydrofuran hinzugegeben. Die Lösung wurde 1 h bei RT gerührt. Die Reaktionslösung wurde eingedampft, der Rückstand in DichlormethanMethanol aufgenommen und mittels Dickschichtchromatographie gereinigt (Laufmittel: Dichlormethan/Methanol = 30/1). Es wurden 34 mg der Zielverbindung (70% d. Th., Reinheit 92%) erhalten.
LC-MS (Methode 1): Rₜ = 0.74 min
MS (ESpos): m/z = 533 (M+H)⁺
¹H NMR (500 MHz, DMSO-*d*₆) δ = 0.68 - 0.73 (m, 4 H), 1.42 (s, 6 H), 1.70 - 1.78 (m, 1 H), 2.77 (s, 3 H), 4.47 (d, 2 H), 5.34 (s, 2 H), 6.98 (br. s, 1 H), 7.10 (br. s, 1 H), 7.20 - 7.28 (m, 2 H), 7.56 - 7.64 (m, 1 H), 8.73 (t, 1 H), 9.66 (d, 1 H), 11.53 (s, 1 H).

### Beispiel 67

### 4-Amino-2-{6-chlor-8-[(2,6-difluorbenzyl)oxy]-2-methylimidazo[1,2-a]pyridin-3-yl}-5,5-dimethyl-5,7-dihydro-6H-pyrrolo[2,3-d]pyrimidin-6-on

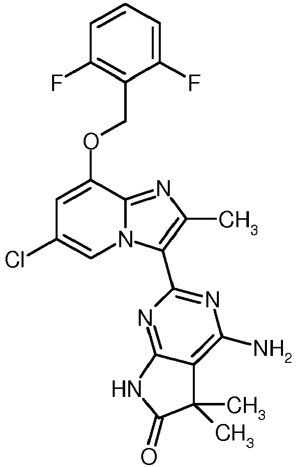

50 mg (0.08 mmol; Reinheit ca. 56%) 6-Chlor-8-[(2,6-difluorbenzyl)oxy]-2-methylimidazo[1,2-a]pyridin-3-carboximidamid aus Beispiel 11A wurden unter Argon in 0.9 ml tert.-Butanol vorgelegt und bei RT nacheinander mit 13.5 mg (0.12 mmol) Kalium-tert.-butylat und 16 mg (0.10 mmol) Methyl-3,3-dicyan-2,2-dimethylpropanoat aus Beispiel 28A versetzt und 3 h zum Rückfluss erhitzt. Das Reaktionsgemisch wurde eingedampft, der Rückstand mit Wasser versetzt und 20 min bei Raumtemperatur gerührt. Der Feststoff wurde abfiltriert, mit Wasser gut gewaschen und getrocknet. Das Rohprodukt wurde in Dichlormethan/Methanol gelöst und mittels Dickschichtchromatographie gereinigt (Laufmittel: Dichlormethan/2M Ammoniak in Methanol 10/1). Es wurden 9 mg (23% d. Th.) der Titelverbindung erhalten.
LC-MS (Methode 1): Rₜ = 0.90 min
MS (ESpos): m/z = 485 (M+H)⁺
¹H-NMR (500 MHz, DMSO-d₆): δ = 1.35 (s, 6 H), 2.73 (s, 3 H), 5.35 (s, 2 H), 6.78 (br. s, 2 H), 7.18 (d, 1 H), 7.22 - 7.29 (m, 2 H), 7.58 - 7.66 (m, 1 H), 9.73 (d, 1 H), 10.95 (s, 1 H).

### Beispiel 68

### 3-{8-[(2,6-Difluorbenzyl)oxy]-2-methylimidazo[1,2-a]pyridin-3-yl}-7,7-dimethyl-5,7-dihydro-6H-pyrrolo[2,3-e][1,2,4]triazin-6-on

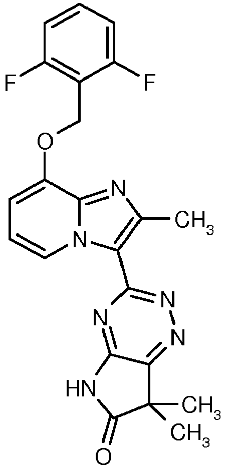

Die Reaktionslösung von Methyl-2-(5-chlor-3-{8-[(2,6-difluorbenzyl)oxy]-2-methylimidazo[1,2-a]pyridin-3-yl}-1,2,4-triazin-6-yl)-2-methylpropanoat aus Beispiel 48A wurde mit 60 ml trockenem Acetonitril verdünnt und langsam bei 0°C zu 60 ml einer 33%igen wässrigen Ammoniaklösung getropft. Das Reaktionsgemisch wurde über Nacht bei Raumtemperatur gerührt und anschließend am Rotationsverdampfer eingeengt. Der Rückstand wurde mit Wasser und Essigsäureethylester versetzt und die beiden Phasen wurden getrennt. Die wässrige Phase wurde zweimal mit Essigsäureethylester extrahiert. Die vereinigten organischen Phasen wurden über Natriumsulfat getrocknet, filtriert und eingedampft. Das Rohprodukt wurde mittels präparativer HPLC gereinigt (RP18 Säule, Laufmittel: Acetonitril/Wasser-Gradient unter Zusatz von 0.05% Ameisensäure). Es wurden 77 mg (34% d. Th. über zwei Stufen) der Zielverbindung erhalten.
LC-MS (Methode 1): Rₜ = 0.79 min
MS (ESpos): m/z = 437 (M+H)⁺
¹H NMR (400 MHz, DMSO-*d*₆) δ = 1.45 (s, 6 H), 2.73 (s, 3 H), 5.34 (s, 2 H), 7.08 (t, 1 H), 7.13 (d, 1 H), 7.20 - 7.30 (m, 2 H), 7.55 - 7.65 (m, 1 H), 9.45 (d, 1 H), 12.12 (s, 1 H).

### Beispiel 69

### 3-{8-[(2,6-Difluorbenzyl)oxy]-2,6-dimethylimidazo[1,2-a]pyridin-3-yl}-7,7-dimethyl-5,7-dihydro-6H-pyrrolo[2,3-e][1,2,4]triazin-6-on

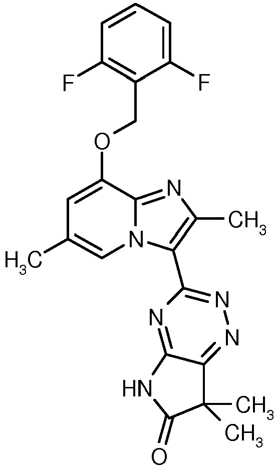

Die Reaktionslösung von Methyl-2-(5-chlor-3-{8-[(2,6-difluorbenzyl)oxy]-2,6-dimethyl-imidazo[1,2-a]pyridin-3-yl}-1,2,4-triazin-6-yl)-2-methylpropanoat aus Beispiel 51A wurde mit 85 ml trockenem Acetonitril verdünnt und langsam bei 0°C zu 85 ml einer 33%igen wässrigen Ammoniaklösung getropft. Das Reaktionsgemisch wurde über Nacht bei Raumtemperatur gerührt und anschließend am Rotationsverdampfer eingeengt. Der Rückstand wurde mit Wasser und Essigsäureethylester versetzt und die beiden Phasen wurden getrennt. Die wässrige Phase wurde zweimal mit Essigsäureethylester extrahiert. Die vereinigten organischen Phasen wurden über Natriumsulfat getrocknet, filtriert und eingedampft. Das Rohprodukt wurde mit Acetonitril versetzt. Der entstandene Niederschlag wurde abfiltriert, mit wenig Acetonitril gewaschen und im Hochvakuum getrocknet. Es wurden 218 mg (35% d. Th. über zwei Stufen) der Zielverbindung erhalten.
LC-MS (Methode 1): Rₜ = 0.88 min
MS (ESpos): m/z = 451 (M+H)⁺
¹H NMR (400 MHz, DMSO-*d*₆) δ = 1.45 (s, 6 H), 2.39 (s, 3 H), 2.70 (s, 3 H), 5.33 (s, 2 H), 7.04 (s, 1 H), 7.20 - 7.39 (m, 2 H), 7.55 - 7.65 (m, 1 H), 9.30 (s, 1 H), 12.08 (s, 1 H).

### Beispiel 70

### 4-(Cyclopropylethinyl)-5,5-dimethyl-2-{2-methyl-8-[(2,3,6-trifluorbenzyl)oxy]imidazo[1,2-a]pyridin-3-yl}-5,7-dihydro-6H-pyrrolo[2,3-d]pyrimidin-6-on

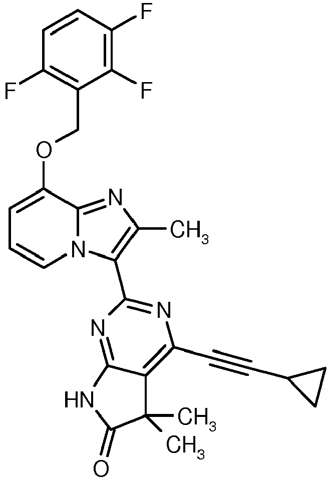

Unter Argonatmosphäre wurden 200 mg (0.345 mmol) 4-Iod-5,5-dimethyl-2-{2-methyl-8-[(2,3,6-trifluorbenzyl)oxy]imidazo[1,2-a]pyridin-3-yl}-5,7-dihydro-6H-pyrrolo[2,3-d]pyrimidin-6-on (Beispiel 10) und 68 mg (1.036 mmol) Ethinylcyclopropan in 5.6 ml abs. THF vorgelegt. Es wurden 105 mg (1.036 mmol) Diisopropylamin, 20 mg (0.104 mmol) Kupfer(I)iodid und 49 mg (0.070 mmol) Dichlorbis(triphenylphosphin)palladium(II) hinzugegeben und das Gemisch wurde 48 h zum Rückfluss erhitzt. Die Reaktionsmischung wurde abgekühlt, über Celite filtriert, mit THF gewaschen, eingeengt und der Rückstand mittels präparativer HPLC (RP-C18, Laufmittel: Acetonitril/Wasser-Gradient unter Zusatz von 0.05% Ameisensäure) gereinigt. Es wurden 83 mg der Zielverbindung erhalten (44% d. Th.).
LC-MS (Methode 1) Rₜ = 0.98 min
MS (ESIpos): m/z = 518 (M+H)⁺
¹H-NMR (400 MHz, DMSO-d₆): δ [ppm] = 0.86 - 0.92 (m, 2 H), 1.03 - 1.09 (m, 2 H), 1.40 (s, 6 H), 1.72 - 1.80 (m, 1 H), 2.73 (s, 3 H), 5.38 (s, 2 H), 7.03 - 7.11 (m, 3 H), 7.26 - 7.34 (m, 1 H), 7.62 - 7.73 (m, 1 H), 9.47 (dd, 1 H), 11.58 (s, 1 H).

### Beispiel 71

### 4-(2-Cyclopropylethyl)-5,5-dimethyl-2-{2-methyl-8-[(2,3,6-trifluorbenzyl)oxy]imidazo[1,2-a]pyridin-3-yl}-5,7-dihydro-6H-pyrrolo[2,3-d]pyrimidin-6-on

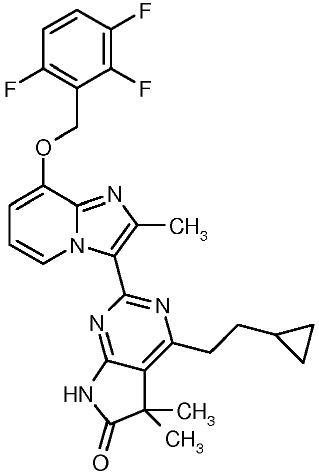

Es wurden 72 mg (0.14 mmol) 4-(Cyclopropylethinyl)-5,5-dimethyl-2-{2-methyl-8-[(2,3,6-trifluorbenzyl)oxy]imidazo[1,2-a]pyridin-3-yl}-5,7-dihydro-6H-pyrrolo[2,3-d]pyrimidin-6-on (Beispiel 70) in 14 ml abs. Methanol gelöst. Die Lösung wurde in einer Durchfluss-Hydrierapparatur (H-Cube der Firma Thales Nano, Budapest, Modell HC-2-SS), bestückt mit einer 10%-igen Palladium/Kohle-Kartusche, bei 10 bar Wasserstoff-Druck hydriert. Die Reaktionsmischung wurde eingeengt und der Rückstand mittels präparativer HPLC (RP-C18, Laufmittel: Acetonitril/Wasser-Gradient unter Zusatz von 0.05% Ameisensäure) gereinigt. Es wurden 15 mg der Zielverbindung erhalten (20% d. Th.).
LC-MS (Methode 1) Rₜ = 1.03 min
MS (ESIpos): m/z = 522 (M+H)⁺
¹H-NMR (400 MHz, DMSO-d₆): δ [ppm] = 0.05-0.12 (m, 2 H), 0.38 - 0.45 (m, 2 H), 0.76 - 0.86 (m, 1 H), 1.42 (s, 6 H), 1.70 - 1.78 (m, 2 H), 2.75 (s, 3 H), 2.82 - 2.89 (m, 2 H), 5.39 (s, 2 H), 6.99 - 7.08 (m, 2 H), 7.25 - 7.33 (m, 1 H), 7.62 - 7.72 (m, 1 H), 9.57 (dd, 1 H), 11.46 (s, 1 H).

### Beispiel 72

### 5,5-Dimethyl-2-{2-methyl-8-[(2,3,6-trifluorbenzyl)oxy]imidazo[1,2-a]pyridin-3-yl}-4-propyl-5,7-dihydro-6H-pyrrolo[2,3-d]pyrimidin-6-on

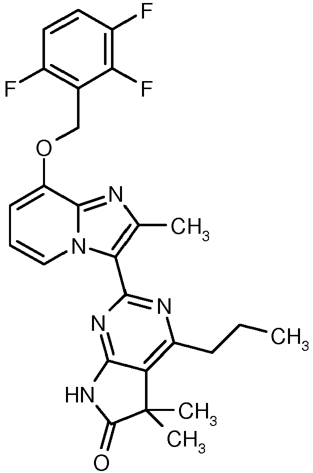

Unter Argon wurden 100 mg (0.173 mmol) 4-Iod-5,5-dimethyl-2-{2-methyl-8-[(2,3,6-trifluorbenzyl)oxy]imidazo[1,2-a]pyridin-3-yl}-5,7-dihydro-6H-pyrrolo[2,3-d]pyrimidin-6-on (Beispiel 10) in 2.2 ml Dioxan vorgelegt. Dann wurden 3.7 mg (0.004 mmol) PdCl₂(dppf) CH₂Cl₂ zugeben und 1.38 ml (0.69 mmol) einer 0.5 M Lösung von n-Propylzinkbromid in Tetrahydrofuran zugetropft. Anschließend wurde 7 h bei 120°C in der Mikrowelle erhitzt. Die Reaktionsmischung wurde abgekühlt, abfiltriert, mit Dioxan gewaschen, eingeengt und der Rückstand mittels präparativer HPLC (RP-C18, Laufmittel: Acetonitril/Wasser-Gradient unter Zusatz von 0.05% Ameisensäure) gereinigt. Die Produktfraktionen wurden nochmals mittels präparativer Dickschichtchromatographie gereinigt (Laufmittel: Dichlormethan/Methanol = 10/1). Es wurden 5.3 mg der Zielverbindung erhalten (5% d. Th.; Reinheit 74%).
LC-MS (Methode 1) Rₜ = 0.97 min
MS (ESIpos): m/z = 496 (M+H)⁺
¹H-NMR (400MHz, DMSO-d₆): δ [ppm] = 1.02 (t, 3 H), 1.42 (s, 6 H), 1.80 - 1.93 (m, 2 H), 2.73 - 2.81 (m, 2 H), 5.38 (s, 2 H), 6.97 - 7.08 (m, 2 H), 7.24 - 7.34 (m, 1 H), 7.60 - 7.72 (m, 1 H), 9.58 (d, 1 H), 11.46 (s, 1 H).

### Beispiel 73

### 5,5-Dimethyl-4-(2-methyl-1,3-thiazol-5-yl)-2-{2-methyl-8-[(2,3,6-trifluorbenzyl)oxy]imidazo[1,2-a]pyridin-3-yl}-5,7-dihydro-6H-pyrrolo[2,3-d]pyrimidin-6-on

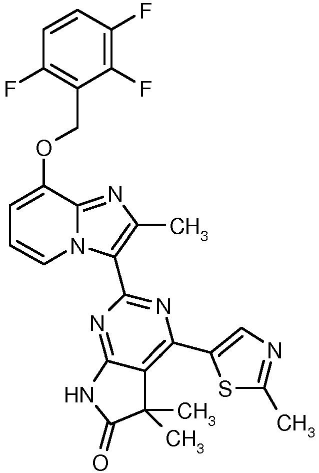

Unter Argonatmosphäre wurden 100 mg (0.173 mmol) 4-Iod-5,5-dimethyl-2-{2-methyl-8-[(2,3,6-trifluorbenzyl)oxy]imidazo[1,2-a]pyridin-3-yl}-5,7-dihydro-6H-pyrrolo[2,3-d]pyrimidin-6-on (Beispiel 10) in 3.8 ml Dioxan suspendiert und mit 117 mg (0.518 mmol) 2-Methyl-5-(4,4,5,5-tetramethyl-1,3,2-dioxaborolan-2-yl)-1,3-thiazol und 1.38 ml (0.69 mmol) 0.5 M wässriger Kaliumcarbonatlösung vesetzt. Nach 10 min wurden 44 mg (0.038 mmol) Tetrakis(triphenylphosphin)palladium(0) zugegeben und 1 h lang bei 140°C in der Mikrowelle gerührt. Die Reaktionsmischung wurde abgekühlt, über Celite filtriert, mit Dioxan gewaschen, eingeengt und der Rückstand mittels präparativer HPLC (RP-C18, Laufmittel: Acetonitril/Wasser-Gradient unter Zusatz von 0.05% Ameisensäure) gereinigt. Man erhielt 7 mg (7% d. Th.) der Zielverbindung.
LC-MS (Methode 1) Rₜ = 0.91 min
MS (ESIpos): m/z = 551 (M+H)⁺
¹H-NMR (400 MHz, DMSO-d₆): δ [ppm] = 1.51 (s, 6 H), 2.76 (s, 3 H), 2.79 (s, 3 H), 5.41 (s, 2 H), 7.04 - 7.14 (m, 2 H), 7.26 - 7.33 (m, 1 H), 7.61 - 7.73 (m, 1 H), 8.30 (s, 1 H), 9.51 (dd, 1 H), 11.77 (s, 1 H).

### Beispiel 74

### 5,5-Dimethyl-2-{2-methyl-8-[(2,3,6-trifluorbenzyl)oxy]imidazo[1,2-a]pyridin-3-yl}-4-(1H-pyrazol-5-yl)-5,7-dihydro-6H-pyrrolo[2,3-d]pyrimidin-6-on

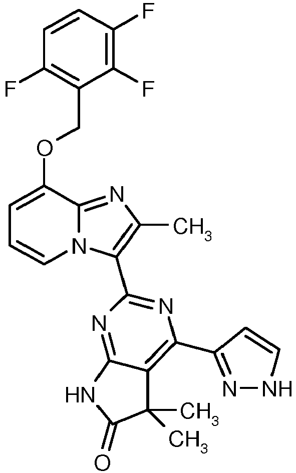

Unter Argonatmosphäre wurden 100 mg (0.173 mmol) 4-Iod-5,5-dimethyl-2-{2-methyl-8-[(2,3,6-trifluorbenzyl)oxy]imidazo[1,2-a]pyridin-3-yl}-5,7-dihydro-6H-pyrrolo[2,3-d]pyrimidin-6-on (Beispiel 10) in 3.8 ml Dioxan suspendiert und mit 100 mg (0.518 mmol) 5-(4,4,5,5-Tetramethyl-1,3,2-dioxaborolan-2-yl)-1H-pyrazol und 1.38 ml (0.69 mmol) 0.5 M wässriger Kaliumcarbonatlösung vesetzt. Nach 10 min wurden 44 mg (0.038 mmol) Tetrakis(triphenylphosphin)palladium(0) zugegeben und 30 min lang bei 140°C in der Mikrowelle gerührt. Die Reaktionsmischung wurde abgekühlt, über Celite filtriert, mit Dioxan gewaschen, eingeengt und der Rückstand mittels präparativer HPLC (RP-C18, Laufmittel: Acetonitril/Wasser-Gradient unter Zusatz von 0.05% Ameisensäure) gereinigt. Man erhielt 41 mg (46% d. Th.) der Zielverbindung.
LC-MS (Methode 1) Rₜ = 0.87 min
MS (ESIpos): m/z = 520 (M+H)⁺
¹H-NMR (400 MHz, DMSO-d₆): δ [ppm] = 1.60 (s, 6 H), 2.82 (s, 3 H), 5.41 (s, 2 H), 7.05 - 7.09 (m, 3 H), 7.33 - 7.27 (m, 1 H), 7.63 - 7.71 (m, 1 H), 7.95 - 7.97 (m, 1 H), 9.61 (dd, 1 H), 11.55 (s, 1 H), 13.48 (s, 1 H).

### Beispiel 75

### 5,5-Dimethyl-2-{2-methyl-8-[(2,3,6-trifluorbenzyl)oxy]imidazo[1,2-a]pyridin-3-yl}-5,7-dihydro-6H-pyrrolo[2,3-d]pyrimidin-6-on Formiat

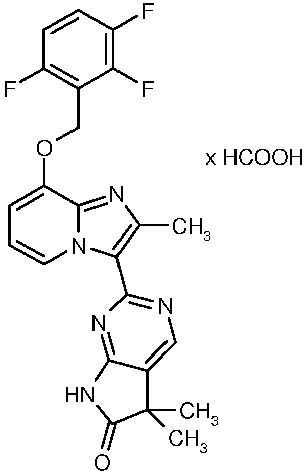

100 mg (0.173 mmol) 4-Iod-5,5-dimethyl-2-{2-methyl-8-[(2,3,6-trifluorbenzyl)oxy]imidazo[1,2-a]pyridin-3-yl}-5,7-dihydro-6H-pyrrolo[2,3-d]pyrimidin-6-on (Beispiel 10) wurden in DMF (4 ml) mit 19.6 mg 10%-igem Palladium auf Kohle 2 Tage bei Normaldruck und RT hydriert. Es wurde über Celite filtriert, mit DMF gewaschen, eingeengt und der Rückstand mittels präparativer HPLC (RP-C18, Laufmittel: Acetonitril/Wasser-Gradient unter Zusatz von 0.05% Ameisensäure) gereinigt. Man erhielt 19 mg (22% d. Th.) der Zielverbindung.
LC-MS (Methode 1): Rₜ = 0.83 min
MS (ESIpos): m/z = 454 [M+-HCOOH+H]⁺
¹H-NMR (400 MHz, DMSO-d₆): δ [ppm] = 1.38 (s, 6 H), 2.74 (s, 3 H), 5.39 (s, 2 H), 6.97 - 7.04 (m, 1 H), 7.04 - 7.10 (m, 1 H), 7.25 - 7.33 (m, 1 H), 8.19 (s, 1 H), 8.60 (s, 1 H), 9.53 (d, 1 H), 11.48 (s, 1 H).

### Beispiel 76

### 4-(Cyclopropylmethoxy)-5,5-dimethyl-2-{2-methyl-8-[(2,3,6-trifluorbenzyl)oxy]imidazo[1,2-a]pyridin-3-yl}-5,7-dihydro-6H-pyrrolo[2,3-d]pyrimidin-6-on

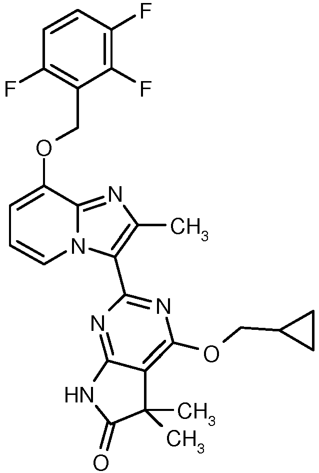

Unter Argon wurden 100 mg (0.213 mmol) 4-Hydroxy-5,5-dimethyl-2-{2-methyl-8-[(2,3,6-trifluorbenzyl)oxy]imidazo[1,2-a]pyridin-3-yl}-5,7-dihydro-6H-pyrrolo[2,3-d]pyrimidin-6-on (Beispiel 11), 17 mg (0.23 mmol) Cyclopropylmethanol und 60 mg (0.23 mmol) Triphenylphosphin in 0.84 ml THF suspendiert, 10 min im Ultraschallbad belassen, anschließend mit 49 mg (0.230 mmol) Diisopropylazodicarboxylat (DIAD) versetzt und über Nacht bei RT gerührt. Weitere 6 mg (0.08 mmol) Cyclopropylmethanol, 20 mg (0.08 mmol) Triphenylphosphin und 14 mg (0.07 mmol) Diisopropylazodicarboxylat wurden zugegeben und es wurde über Nacht bei RT gerührt. Die Reaktionsmischung wurde mittels präparativer HPLC (RP-C18, Laufmittel: Acetonitril/Wasser-Gradient unter Zusatz von 0.05% Ameisensäure) gereinigt. Man erhielt 19 mg (17% d. Th.) der Zielverbindung.
LC-MS (Methode 1) Rₜ = 1.06 min
MS (ESIpos): m/z = 524 (M+H)⁺
¹H-NMR (400 MHz, DMSO-d₆): δ [ppm] = 0.37 - 0.42 (m, 2 H), 0.56 - 0.62 (m, 2 H), 1.27 - 1.39 (m, 1 H), 1.37 (s, 6 H), 2.76 (s, 3 H), 4.36 (d, 2 H), 5.39 (s, 2 H), 6.99 - 7.11 (m, 2 H), 7.26 - 7.34 (m, 1 H), 7.63 - 7.71 (m, 1 H), 9.52 (d, 1 H), 11.29 (s, 1 H).

### Beispiel 77

### 5,5-Dimethyl-2-{2-methyl-8-[(2,3,6-trifluorbenzyl)oxy]imidazo[1,2-a]pyridin-3-yl}-4-(3,3,3-trifluorpropoxy)-5,7-dihydro-6H-pyrrolo[2,3-d]pyrimidin-6-on

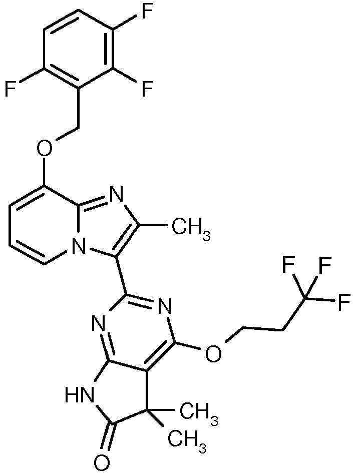

Unter Argon wurden 100 mg (0.213 mmol) 4-Hydroxy-5,5-dimethyl-2-{2-methyl-8-[(2,3,6-trifluorbenzyl)oxy]imidazo[1,2-a]pyridin-3-yl}-5,7-dihydro-6H-pyrrolo[2,3-d]pyrimidin-6-on (Beispiel 11), 26 mg (0.23 mmol) 3,3,3-Trifluorpropan-1-ol und 60 mg (0.23 mmol) Triphenylphosphin in 0.84 ml THF suspendiert, 10 min im Ultraschallbad gemischt, anschließend mit 49 mg (0.230 mmol) Diisopropylazodicarboxylat (DIAD) versetzt und über Nacht bei RT gerührt. Weitere 9 mg (0.08 mmol) 3,3,3-Trifluorpropan-1-ol, 20 mg (0.08 mmol) Triphenylphosphin und 15 mg (0.07 mmol) Diisopropylazodicarboxylat wurden zugegeben und es wurde über Nacht bei RT gerührt. Die Reaktionsmischung wurde mittels präparativer HPLC (RP-C18, Laufmittel: Acetonitril/Wasser-Gradient unter Zusatz von 0.05% Ameisensäure) gereinigt. Man erhielt 48 mg (40% d. Th.) der Zielverbindung.
LC-MS (Methode 1): Rₜ = 1.04 min
MS (ESIpos): m/z = 566 [M+H]⁺
¹H-NMR (400 MHz, DMSO-d₆): δ [ppm] = 1.34 (s, 6 H), 2.76 (s, 3 H), 2.83 - 2.95 (m, 2 H), 4.69 - 4.75 (m, 2 H), 5.39 (s, 2 H), 7.00 - 7.05 (m, 1 H), 7.07 - 7.11 (m, 1 H), 7.25 - 7.34 (m, 1 H), 7.62 - 7.72 (m, 1 H), 9.52 (dd, 1 H), 11.36 (s, 1 H).

### B. Bewertung der pharmakologischen Wirksamkeit

Es werden die folgenden Abkürzungen verwendet:

| | |
|---|---|
| ATP | Adenosintriphosphat |
| Brij35 | Polyoxyethylen(23)laurylether |
| BSA | Rinderserumalbumin |
| DTT | Dithiothreitol |
| TEA | Triethanolamin |

Die pharmakologische Wirkung der erfindungsgemäßen Verbindungen kann in folgenden Assays gezeigt werden:

### B-1. Gefäßrelaxierende Wirkung in vitro

Die Bestimmung der relaxierenden Wirkung der erfindungsgemäßen Verbindungen an isolierten Gefäßen wurde, wie in JP Stasch et al., Br J Pharmacol. 2002; 135, 333-343 beschrieben, durchgeführt. Kaninchen werden durch Nackenschlag betäubt und entblutet. Die Aorta wird entnommen, von anhaftendem Gewebe befreit, in 1.5 mm breite Ringe geteilt und einzeln unter einer Vorspannung in 5 ml-Organbäder mit 37°C warmer, Carbogen-begaster Krebs-Henseleit-Lösung folgender Zusammensetzung gebracht (jeweils mM): Natriumchlorid: 119; Kaliumchlorid: 4.8; Calciumchlorid-Dihydrat: 1; Magnesiumsulfat-Heptahydrat: 1.4; Kaliumdihydrogenphosphat: 1.2; Natriumhydrogencarbonat: 25; Glucose: 10. Die Kontraktionskraft wird mit Statham UC2-Zellen erfasst, verstärkt und über A/D-Wandler (DAS-1802 HC, Keithley Instruments München) digitalisiert sowie parallel auf Linienschreiber registriert.

Zur Erzeugung einer Kontraktion wird Phenylephrin dem Bad kumulativ in ansteigender Konzentration zugesetzt. Nach mehreren Kontrollzyklen wird die zu untersuchende Substanz in jedem weiteren Durchgang in jeweils steigender Dosierung zugesetzt und die Höhe der Kontraktion mit der Höhe der im letzten Vordurchgang erreichten Kontraktion verglichen. Daraus wird die Konzentration errechnet, die erforderlich ist, um die Höhe des Kontrollwertes um 50% zu reduzieren (IC₅₀-Wert). Das Standardapplikationsvolumen beträgt 5 µl, der DMSO-Anteil in der Badlösung entspricht 0.1%.

### B-2. Wirkung an rekombinanter Guanylatcyclase-Reporterzelllinie

Die zelluläre Wirkung der erfindungsgemäßen Verbindungen wird an einer rekombinanten Guanylatcyclase-Reporterzelllinie, wie in F. Wunder et al., Anal. Biochem. 339, 104-112 (2005) beschrieben, bestimmt.

Repräsentative MEC-Werte (MEC = minimal effektive Konzentration) für die erfindungsgemäßen Verbindungen sind in der nachstehenden Tabelle 1 wiedergegeben (zum Teil als Mittelwerte aus Einzelbestimmungen):

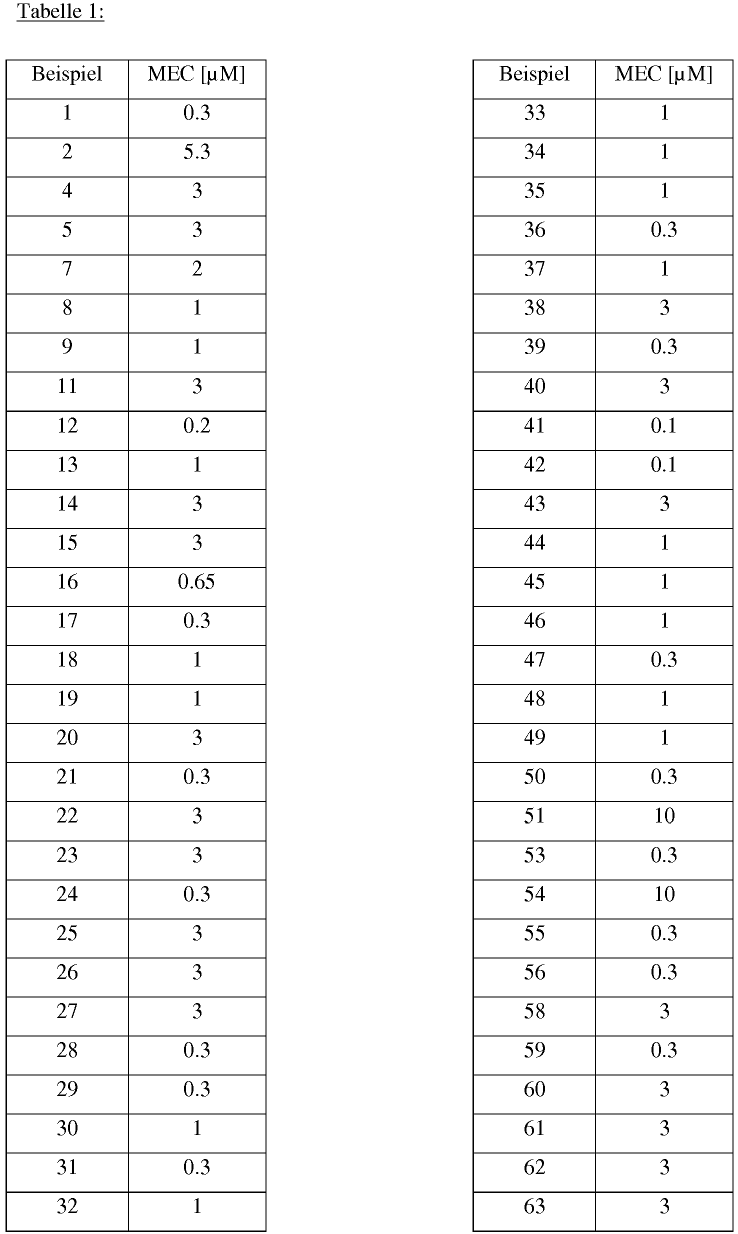

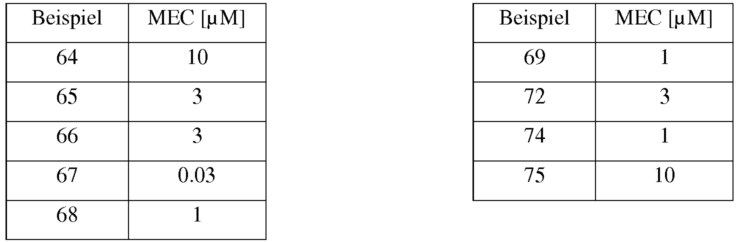

### B-3. Inhibition der humanen Phosphodiesterase 5 (PDE 5)

PDE 5-Präparationen werden aus humanen Plättchen durch Aufschluss (Microfluidizer®, 800 bar, 3 Passagen), gefolgt von Zentrifugation (75000 g, 60 min, 4°C) und Ionenaustauscher-Chromatographie des Überstandes auf einer Mono Q 10/10 Säule (linearer Natriumchlorid-Gradient, Elution mit einer 0.2-0.3M Lösung von Natriumchlorid in Puffer (20 mM Hepes pH 7.2, 2 mM Magnesiumchlorid) gewonnen. Fraktionen, die PDE 5 Aktivität aufweisen, werden vereinigt (PDE 5-Präparat) und bei -80°C gelagert.

Die Testsubstanzen werden zur Bestimmung ihrer in vitro Wirkung an humaner PDE 5 in 100% DMSO aufgelöst und seriell verdünnt. Typischerweise werden Verdünnungsreihen (1:3) von 200 µM bis 0.091 µM hergestellt (resultierende Endkonzentrationen im Test: 4 µM bis 0.0018 µM). Jeweils 2 µL der verdünnten Substanzlösungen werden in die Vertiefungen von Mikrotiterplatten (Isoplate-96 /200W; Perkin Elmer) vorgelegt. Anschließend werden 50 µL einer Verdünnung des oben beschriebenen PDE 5-Präparats hinzugefügt. Die Verdünnung des PDE 5 Präparats wird so gewählt, dass während der späteren Inkubation weniger als 70% des Substrates umgesetzt wird (typische Verdünnung: 1: 100; Verdünnungspuffer: 50 mM Tris/Salzsäure pH 7.5, 8.3 mM Magnesiumchlorid, 1.7 mM EDTA, 0.2% BSA). Das Substrat [8-³H] cyclisches Guanosin-3',5'-monophosphat (1 µCi/µL; Perin Elmer) wird 1:2000 mit Assaypuffer (50 mM Tris/Salzsäure pH 7.5, 8.3 mM Magnesiumchlorid, 1.7 mM EDTA) auf eine Konzentration von 0.0005µCi/µL verdünnt. Durch Zugabe von 50 µL (0.025 µCi) des verdünnten Substrates wird die Enzymreaktion schließlich gestartet. Die Testansätze werden für 60 min bei Raumtemperatur inkubiert und die Reaktion durch Zugabe von 25 µL einer Suspension von 18 mg/mL Yttrium Scintillation Proximity Beads in Wasser (Phosphodiesterase beads für SPA Assays, RPNQ 0150, Perkin Elmer) gestoppt. Die Mikrotiterplatten werden mit einer Folie versiegelt und für 60 min bei Raumtemperatur stehengelassen. Anschließend werden die Platten für 30 s pro Vertiefung in einem Microbeta Szintillationszähler (Perkin Elmer) vermessen. IC₅₀-Werte werden anhand der graphischen Auftragung der Substanzkonzentration gegen die prozentuale PDE 5-Inhibition bestimmt.

Repräsentative Werte IC₅₀-Werte für die erfindungsgemäßen Verbindungen sind in der nachstehenden Tabelle (Tabelle 2; zum Teil als Mittelwerte aus Einzelbestimmungen)) wiedergegeben:

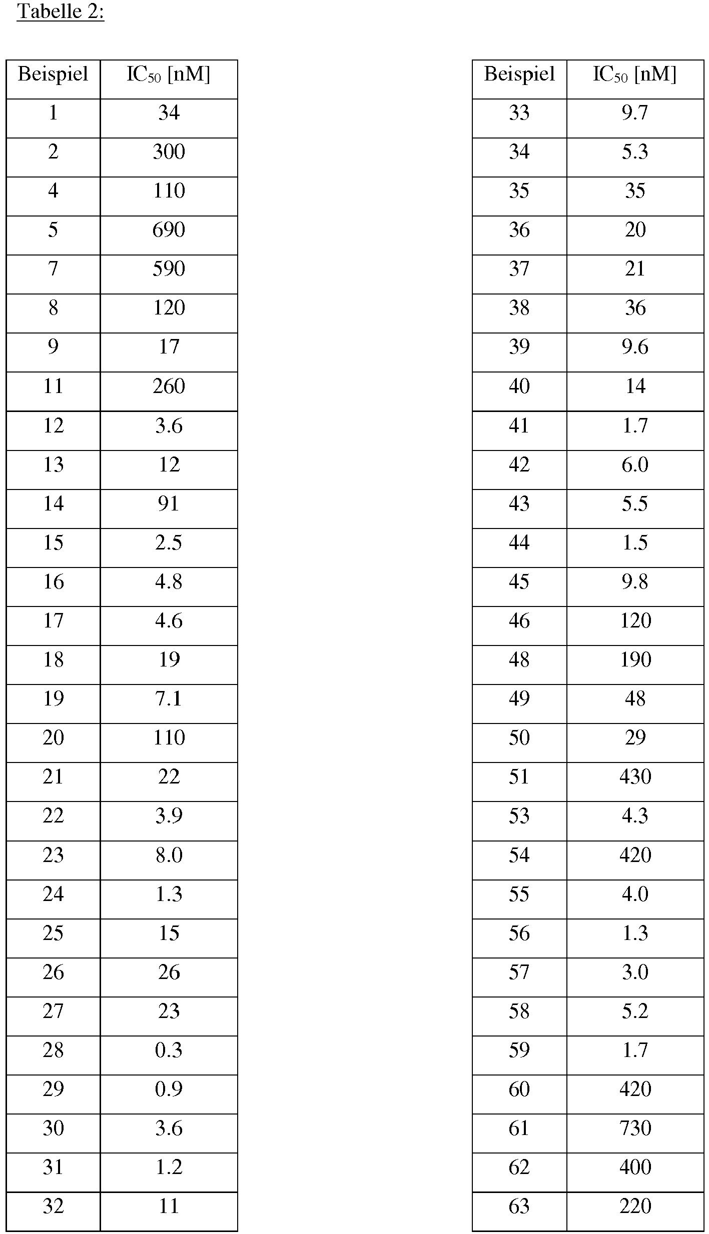

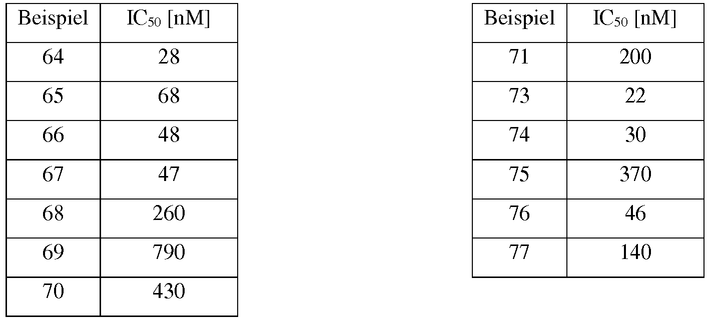

### B-4. Blutdruckmessung an narkotisierten Ratten

Männliche Wistar-Ratten mit einem Körpergewicht von 300 - 350 g werden mit Thiopental (100 mg/kg i.p.) anästhesiert. Nach der Tracheotomie wird in die Femoralarterie ein Katheter zur Blutdruckmessung eingeführt. Die zu prüfenden Substanzen werden als Lösungen entweder oral mittels Schlundsonde oder über die Femoralvene intravenös verabreicht (Stasch et al. Br. J. Pharmacol. 2002; 135: 344-355).

### B-5. Radiotelemetrische Blutdruckmessung an wachen, spontan hypertensiven Ratten

Für die im Folgenden beschriebene Blutdruckmessung an wachen Ratten wird ein im Handel erhältliches Telemetriesystem der Firma DATA SCIENCES INTERNATIONAL DSI, USA eingesetzt.

Das System besteht aus 3 Hauptkomponenten:
Implantierbare Sender (Physiotel® Telemetrietransmitter)
Empfänger (Physiotel® Receiver), die über einen Multiplexer (DSI Data Exchange Matrix) mit einem
Datenakquisitionscomputer verbunden sind.

Die Telemetrieanlage ermöglicht eine kontinuierliche Erfassung von Blutdruck Herzfrequenz und Körperbewegung an wachen Tieren in ihrem gewohnten Lebensraum.

### Tiermaterial

Die Untersuchungen werden an ausgewachsenen weiblichen spontan hypertensiven Ratten (SHR Okamoto) mit einem Körpergewicht von >200 g durchgeführt. SHR/NCrl von Okamoto Kyoto School of Medicine, 1963 wurden aus männlichen Wistar Kyoto Ratten mit stark erhöhtem Blutdruck und weiblichen mit leicht erhöhtem Blutdruck gekreuzt und in der F13 an die U.S. National Institutes of Health abgegeben.

Die Versuchstiere werden nach Senderimplantation einzeln in Makrolon - Käfigen Typ 3 gehalten. Sie haben freien Zugang zu Standardfutter und Wasser.

Der Tag - Nacht - Rhythmus im Versuchslabor wird per Raumbeleuchtung um 6:00 Uhr morgens und um 19:00 Uhr abends gewechselt.

### Senderimplantation

Die eingesetzten Telemetriesender TA11 PA - C40 werden den Versuchstieren mindestens 14 Tage vor dem ersten Versuchseinsatz unter aseptischen Bedingungen chirurgisch implantiert. Die so instrumentierten Tiere sind nach Abheilen der Wunde und Einwachsen des Implantats wiederholt einsetzbar.

Zur Implantation werden die nüchternen Tiere mit Pentobabital (Nembutal, Sanofi: 50mg/kg i.p.) narkotisiert und an der Bauchseite weiträumig rasiert und desinfiziert. Nach Eröffnung des Bauchraumes entlang der Linea alba wird der flüssigkeitsgefüllte Meßkatheter des Systems oberhalb der Bifurcation nach cranial in die Aorta descendens eingesetzt und mit Gewebekleber (VetBonD TM, 3M) befestigt. Das Sendergehäuse wird intraperitoneal an der Bauchwandmuskulatur fixiert und die Wunde wird schichtweise verschlossen.

Postoperativ wird zur Infektionsprophylaxe ein Antibiotikum verabreicht (Tardomyocel COMP Bayer 1ml/kg s.c.)

### Substanzen und Lösungen

Wenn nicht anders beschrieben werden die zu untersuchenden Substanzen jeweils einer Gruppe von Tieren (n = 6) per Schlundsonde oral verabreicht. Entsprechend einem Applikationsvolumen von 5 ml/kg Körpergewicht werden die Testsubstanzen in geeigneten Lösungsmittelgemischen gelöst oder in 0.5% iger Tylose suspendiert.

Eine Lösungsmittel- behandelte Gruppe von Tieren wird als Kontrolle eingesetzt.

### Versuchsablauf

Die vorhandene Telemetrie - Meßeinrichtung ist für 24 Tiere konfiguriert. Jeder Versuch wird unter einer Versuchsnummer registiert (VJahr Monat Tag).

Den in der Anlage lebenden instrumentierten Ratten ist jeweils eine eigene Empfangsantenne zugeordnet (1010 Receiver, DSI).

Die implantierten Sender sind über einen eingebauten Magnetschalter von außen aktivierbar. Sie werden bei Versuchsvorlauf auf Sendung geschaltet. Die ausgestrahlten Signale können durch ein Datenakquisitionssystem (Dataquest TM A.R.T. for WINDOWS, DSI) online erfasst und entsprechend aufgearbeitet werden. Die Ablage der Daten erfolgt jeweils in einem hierfür eröffneten Ordner der die Versuchsnummer trägt.

### Im Standardablauf werden über je 10 Sekunden Dauer gemessen

- Systolischer Blutdruck (SBP)
- Diastolischer Blutdruck (DBP)
- Arterieller Mitteldruck (MAP)
- Herzfrequenz (HR)
- Aktivität (ACT)

Die Messwerterfassung wird rechnergesteuert in 5 Minuten Abständen wiederholt. Die als Absolutwert erhobenen Quelldaten werden im Diagramm mit dem aktuell gemessenen Barometerdruck (Ambient Pressure Reference Monitor; APR-1) korrigiert und in Einzeldaten abgelegt. Weitere technische Details sind der umfangreichen Dokumentation der Herstellerfirma (DSI) zu entnehmen.

Wenn nicht anders beschrieben erfolgt die Verabreichung der Prüfsubstanzen am Versuchstag um 9.00 Uhr. Im Anschluss an die Applikation werden die oben beschriebenen Parameter 24 Stunden gemessen.

### Auswertung

Nach Versuchsende werden die erhobenen Einzeldaten mit der Analysis-Software (DATAQUEST TM A. R.T. TM ANALYSIS) sortiert. Als Leerwert werden hier 2 Stunden vor Applikation angenommen, so dass der selektierte Datensatz den Zeitraum von 7:00 Uhr am Versuchstag bis 9:00 Uhr am Folgetag umfasst.

Die Daten werden über eine voreinstellbare Zeit durch Mittelwertbestimmung geglättet (15 Minuten Average) und als Textdatei auf einen Datenträger übertragen. Die so vorsortierten und komprimierten Messwerte werden in Excel-Vorlagen übertragen und tabellarisch dargestellt. Die Ablage der erhobenen Daten erfolgt pro Versuchstag in einem eigenen Ordner, der die Versuchsnummer trägt. Ergebnisse und Versuchsprotokolle werden in Papierform nach Nummern sortiert in Ordnern abgelegt.

### Literatur:

Klaus Witte, Kai Hu, Johanna Swiatek, Claudia Müssig, Georg Ertl and Björn Lemmer: Experimental heart failure in rats: effects on cardiovascular circadian rhythms and on myocardial β-adrenergic signaling. Cardiovasc Res 47 (2): 203-405, 2000; Kozo Okamoto: Spontaneous hypertension in rats. Int Rev Exp Pathol 7: 227- 270, 1969; Maarten van den Buuse: Circadian Rhythms of Blood Pressure, Heart Rate, and Locomotor Activity in Spontaneously Hypertensive Rats as Measured With Radio-Telemetry. Physiology & Behavior 55(4): 783-787, 1994.

### B-6. Bestimmung der Organ-protektiven Wirkungen im Langzeitversuch an Ratten.

Die Organ-protektiven Wirkungen der erfindungsgemäßen Verbindungen werden in einem therapeutisch relevanten "low nitric oxide (NO) / high renin" Hypertoniemodell an der Ratten gezeigt. Die Studie wurde in Anlehnung an die kürzlich erschienene Publikation durchgeführt (Sharkovska Y, et al. J Hypertension 2010; 28: 1666-1675). Dabei werden Renin-transgene Ratten (TGR(mRen2)27), denen der NO-Synthase-Inhibitor L-NAME über das Trinkwasser verabreicht wurde, gleichzeitig mit der erfindungsgemäßen Verbindung oder Vehikel über mehrere Wochen behandelt. Haemodynamische und renale Parameter werden während des Behanlungszeitraums bestimmt. Am Ende der Langzeitstudie wird die Organprotektion (Niere, Lunge, Herz, Aorta) durch histopathologische Untersuchungen, Biomarker, Expressionsanalysen und kardiovaskuläre Plasmaparameter gezeigt.

### B-7. Messungen des pulmonal-arteriellen Drucks (PAP) in wachen Hunden unter Hypoxiebedingungen

Für die im Folgenden beschriebene Blutdruckmessung an wachen Hunden wird zum Beispiel ein Telemetriesystem der Firma DATA SCIENCES INTERNATIONAL DSI, USA eingesetzt. Das System besteht aus implantierbaren Drucksendern, Empfänger und einem Datenakquisitionscomputer. Die Telemetrieanlage ermöglicht eine kontinuierliche Erfassung von Blutdrücken und der Herzfrequenz an wachen Tieren. Die eingesetzten Telemetriesender werden den Versuchstieren vor dem ersten Versuchseinsatz unter aseptischen Bedingungen chirurgisch implantiert. Die so instrumentierten Tiere sind nach Abheilen der Wunde und Einwachsen des Implantats wiederholt einsetzbar. Die Untersuchungen werden an erwachsenen, männlichen Beagle Hunden durchgeführt. Technische Details können der Dokumentation der Herstellerfirma (DSI) entnommen werden.

### Substanzen und Lösungen

Die zu untersuchenden Substanzen werden jeweils einer Gruppe von Hunden (n = 3-6) oral mittels einer Gelatine-Kapsel oder intravenös in geeigneten Lösungsmittelgemischen verabreicht. Eine Vehikel- behandelte Gruppe von Tieren wird als Kontrolle eingesetzt.

### Versuchsablauf

Für die Messungen unter Hypoxiebedingungen werden die Tiere in eine Kammer überführt, in der eine hypoxische Atmosphäre (ca. 10% Sauerstoffgehalt) herrscht. Diese wird mit kommerziell erhältlichen Hypoxiegeneratoren (Firma Hoehenbalance, Cologne, Germany) erzeugt. Im Standardablauf werden z.B. ein und fünf Stunden nach Substanzgabe die Hunde für 30 min in die Hypoxiekammer überführt. Dabei erfolgt die Messung von Drücken und Herzfrequenz mittels Telemetrie ca. 10 min vor und nach Eintritt in die Hypoxiekammer, als auch während des Aufenthaltes in der Hypoxiekammer.

### Auswertung

In gesunden Hunden kommt es unter Hypoxie zu einem schnellen Anstieg des PAP. Durch die Gabe von Substanzen kann dieser Anstieg reduziert werden. Für die Quantifizierung des PAP Anstieges und der Herzfrequenz- bzw. systemischen Blutdruck-Unterschiede werden die durch Mittelwertbestimmung geglätteten Daten vor und während der Hypoxieperiode verglichen. Die graphische Darstellung der Verläufe der gemessenen Parameter erfolgt mit der Prism Software (GraphPad, USA).

### B-8. Bestimmung pharmakokinetischer Kenngrößen nach intravenöser und oraler Gabe

Die pharmakokinetischen Parameter der erfindungsgemäßen Verbindungen werden in männlichen CD-1-Mäusen, männlichen Wistar-Ratten und weiblichen Beagle-Hunden bestimmt. Die intravenöse Gabe erfolgt bei Mäusen und Ratten mittels einer speziesspezifischen Plasma/DMSO-Formulierung und bei Hunden mittels einer Wasser/PEG400/Ethanol-Formulierung. Die orale Gabe der gelösten Substanz mittels Schlundsonde wird in allen Spezies basierend auf einer Wasser/PEG400/Ethanol-Formulierung durchgeführt. Den Ratten wird zur vereinfachten Blutabnahme vor der Substanzgabe ein Silikonkatheter in die rechte *Vena jugularis externa* gelegt. Die Operation erfolgt mindestens einen Tag vor dem Versuch unter Isofluran-Narkose und unter Gabe eines Analgetikums (Atropin/Rimadyl (3/1) 0.1 mL s.c.). Die Blutabnahme (in der Regel mehr als 10 Zeitpunkte) erfolgt in einem Zeitfenster, welches terminale Zeitpunkte von mindestens 24 bis maximal 72 Stunden nach Substanzgabe beinhaltet. Das Blut wird bei der Entnahme in heparinisierte Röhrchen geleitet. So dann wird mittels Zentrifugation das Blutplasma gewonnen und gegebenenfalls bis zur weiteren Bearbeitung bei -20°C gelagert.

Den Proben der erfindungsgemäßen Verbindungen, Kalibrierproben und Qualifier wird ein interner Standard zugesetzt (dies kann auch eine chemisch nicht verwandte Substanz sein) und es folgt eine Proteinfällung mittels Acetonitril im Überschuss. Nach Zugabe einer Puffer-Lösung, die an die LC-Bedingungen angepasst ist, und folgendem Vortexen wird bei 1000 g zentrifugiert. Der Überstand wird mittels LC-MS/MS unter Verwendung von C18-reversed-phase-Säulen und variablen Eluenten-Gemischen vermessen. Die Quantifizierung der Substanzen erfolgt anhand der Peakhöhen oder -flächen aus extrahierten Ionenchromatogrammen spezifischer selected ion monitoring-Experimente.

Aus den ermittelten Plasmakonzentration-Zeit-Verläufen werden die pharmakokinetischen Kenngrößen wie AUC, Cₘₐₓ, t_{1/2} (terminale Halbwertszeit), F (Bioverfügbarkeit), MRT (Mean Residence Time) und CL (Clearance) mittels eines validierten pharmakokinetischen Rechenprogramms berechnet.

Da die Substanzquantifizierung in Plasma durchgeführt wird, muss die Blut/Plasma-Verteilung der Substanz bestimmt werden, um die pharmakokinetischen Parameter entsprechend anpassen zu können. Dazu wird eine definierte Menge Substanz in heparinisiertem Vollblut der entsprechenden Spezies für 20 min im Taumelrollenmischer inkubiert. Nach Zentrifugation bei 1000g wird die Konzentration im Plasma gemessen (mittels LC-MS/MS; s.o.) und durch Quotientenbildung der C_{Blut}/C_{Plasma}-Wert ermittelt.

### B-9. Metabolismus-Untersuchung

Zur Bestimmung des Metabolismus-Profils der erfindungsgemäßen Verbindungen werden diese mit rekombinanten humanen Cytochrom P450 (CYP) Enzymen, Lebermikrosomen oder mit primären frischen Hepatozyten verschiedener Tierspezies (z.B. Ratte, Hund) als auch humanen Ursprungs inkubiert, um Informationen über einen möglichst kompletten hepatischen Phase I- und Phase II-Metabolismus sowie über die am Metabolismus beteiligten Enzyme zu erhalten und zu vergleichen.

Die erfindungsgemäßen Verbindungen wurden mit einer Konzentration von etwa 0.1-10 µM inkubiert. Dazu wurden Stammlösungen der erfindungsgemäßen Verbindungen mit einer Konzentration von 0.01-1 mM in Acetonitril hergestellt, und dann mit einer 1:100 Verdünnung in den Inkubationsansatz pipettiert. Die Lebermikrosomen und rekombinanten Enzyme wurden in 50 mM Kaliumphosphatpuffer pH 7.4 mit und ohne NADPH-generierendem System, bestehend aus 1 mM NADP⁺, 10 mM Glucose-6-phosphat und 1 Unit Glucose-6-phosphat Dehydrogenase, bei 37°C inkubiert. Primäre Hepatozyten wurden in Suspension in Williams E Medium ebenfalls bei 37°C inkubiert. Nach einer Inkubationszeit von 0 - 4h wurden die Inkubationsansätze mit Acetonitril abgestoppt (Endkonzentration ca. 30%) und das Protein bei ca. 15000 x g abzentrifugiert. Die so abgestoppten Proben wurden entweder direkt analysiert oder bis zur Analyse bei -20°C gelagert.

Die Analyse erfolgt mittels Hochleistungsflüssigkeits-Chromatographie mit Ultraviolett- und massenspektrometrischer Detektion (HPLC-UV-MS/MS). Dazu werden die Überstände der Inkubationsproben mit geeigneten C18-reversed-phase-Säulen und variablen Eluenten-Gemischen aus Acetonitril und 10 mM wässriger Ammoniumformiat-Lösung oder 0.05 % Ameisensäure chromatographiert. Die UV-Chromatogramme in Verbindung mit massenspektrometrischen Daten dienen zur Identifizierung, Strukturaufklärung und quantitativen Abschätzung der Metabolite, und der quantitativen metabolischen Abnahme der erfindungsgemäßen Verbindung in den Inkubationsansätzen.

### B-10. Caco-2 Permeabilitäts-Test

Die Permeabilität einer Testsubstanz wurde mit Hilfe der Caco-2 Zelllinie, einem etablierten *in vitro* Modell für Permeabilitätsvorhersagen an der gastrointestinalen Barriere, bestimmt (Artursson, P. and Karlsson, J. (1991). Correlation between oral drug absorption in humans and apparent drug permeability coefficients in human intestinal epithelial (Caco-2) cells. Biochem. Biophys. 175 (3), 880-885). Die Caco-2 Zellen (ACC No. 169, DSMZ, Deutsche Sammlung von Mikroorganismen und Zellkulturen, Braunschweig, Deutschland) wurden in 24-Well Platen mit Einsatz ausgesät und 14 bis 16 Tage kultiviert. Für die Permeabilitätsstudien wurde die Testsubstanz in DMSO gelöst und mit Transportpuffer (Hanks Buffered Salt Solution, Gibco/Invitrogen, mit 19.9 mM Glukose und 9.8 mM HEPES) auf die finale Testkonzentration verdünnt. Um die Permeabilität von apikal nach basolateral (PₐₚₚA-B) der Testsubstanz zu bestimmen, wurde die Lösung mit der Testsubstanz auf die apikale Seite des Caco-2 Zellmonolayers gegeben und Transportpuffer auf die basolaterale Seite. Um die Permeabilität von basolateral nach apikal (PₐₚₚB-A) der Testsubstanz zu bestimmen, wurde die Lösung mit der Testsubstanz auf die basolaterale Seite des Caco-2 Zellmonolayers gegeben und Transportpuffer auf die apikale Seite. Zu Beginn des Experiments wurden Proben aus dem jeweiligen Donor-Kompartiment genommen, um die Massenbilanz sicher zu stellen. Nach einer Inkubation von zwei Stunden bei 37° C wurden Proben aus beiden Kompartimenten genommen. Die Proben wurden mittels LC-MS/MS analysiert und die apparenten Permeabilitätskoeffizienten (Pₐₚₚ) berechnet. Die Permeabilität von Lucifer Yellow wurde für jeden Zellmonolayer bestimmt, um die Integrität der Zellschicht sicher zu stellen. Die Permeabilität von Atenolol (Marker für niedrige Permeabilität) und Sulfasalazin (Marker für aktive Exkretion) wurde in jedem Testlauf als Qualitätskontrolle mitbestimmt.

### B-11. hERG Kaliumstrom Assay

Der sogenannte hERG (human ether-a-go-go related gene) Kaliumstrom trägt wesentlich zur Repolarisierung des humanen kardialen Aktionspotentials bei (Scheel et al., 2011). Eine Inhibition dieses Stroms durch Pharmaka kann in seltenen Fällen potentiell letale Herzrhythmusstörungen zur Folge haben, und wird deshalb frühzeitig während der Arzneimittelentwicklung untersucht.

Der hier verwendete funktionelle hERG Assay basiert auf einer recombinanten HEK293 ZellLinie, die das KCNH2(HERG)-Gen stabil exprimiert (Zhou et al., 1998). Diese Zellen werden mittels der "whole-cell voltage-clamp" Technik (Hamill et al., 1981) in einem automatisierten System (Patchliner™; Nanion, München, D) untersucht, welches die Membranspannung kontrolliert und den hERG Kalium-Strom bei Zimmertemperatur misst. Die PatchControlHT™ Software (Nanion) steuert Patchliner System, Datenerfassung und Datenanalyse. Die Spannungskontrolle erfolgt durch 2 EPC-10 quadro Verstärker unter Kontrolle der PatchMasterPro™ Software (beide: HEKA Elektronik, Lambrecht, D). NPC-16 Chips mit mittlerem Widerstand (∼2 MΩ; Nanion) dienen als planares Substrat für die Voltage-Clamp Experimente.

NPC-16 Chips werden mit intra- und extrazellulärer Lösung (vgl. Himmel, 2007) sowie mit Zellsuspension befüllt. Nach Bildung eines Giga-Ohm-Seals und Herstellen des Ganzzell-Modus (einschliesslich mehrerer automatisierter Qualitätskontrollschritte) wird die Zellmembran auf das Haltepotential -80 mV geklemmt. Das nachfolgende Spannungsklemm-Protokoll ändert die Kommandospannung auf +20 mV (Dauer 1000 ms), -120 mV (Dauer 500 ms), und zurück zum Haltepotential -80 mV; dies wird alle 12 s wiederholt. Nach einer initialen Stabilisierungsphase (ca 5-6 Minuten) wird Testsubstanzlösung in aufsteigenden Konzentrationen (z.B. 0.1, 1, und 10 µmol/L) zupipettiert (Exposition ca 5-6 Minuten pro Konzentration), gefolgt von mehreren Auswaschschritten.

Die Amplitude des einwärtsgerichteten "Tail"-Stroms, der durch eine Potentialänderung von +20 mV auf -120 mV erzeugt wird, dient zur Quantifizierung des hERG Kaliumstroms, und wird als Funktion der Zeit dargestellt (IgorPro™ Software). Die Stromamplitude am Ende verschiedener Zeitabschnitte (z.B. Stabilisierungsphase vor Testsubstanz, erste/zweite/dritte Konzentration Testsubstanz) dient zur Erstellung einer Konzentrations-Wirkungs-Kurve, aus der die halbmaximale Hemmkonzentration IC₅₀ der Testsubstanz errechnet wird.
Hamill OP, Marty A, Neher E, Sakmann B, Sigworth FJ. Improved patch-clamp techniques for high-resolution current recording from cells and cell-free membrane patches. Pfluegers Arch 1981; 391:85-100.
Himmel HM. Suitability of commonly used excipients for electrophysiological in-vitro safety pharmacology assessment of effects on hERG potassium current and on rabbit Purkinje fiber action potential. J Pharmacol Toxicol Methods 2007;56:145-158.
Scheel O, Himmel H, Rascher-Eggstein G, Knott T. Introduction of a modular automated voltage-clamp platform and its correlation with manual human ether-a-go-go related gene voltage-clamp data. Assay Drug Dev Technol 2011;9:600-607.
Zhou ZF, Gong Q, Ye B, Fan Z, Makielski JC, Robertson GA, January CT. Properties of hERG channels stably expressed in HEK293 cells studied at physiological temperature. Biophys J 1998;74:230-241.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindung der Formel (I) 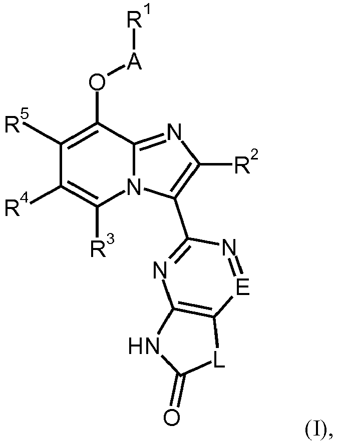 in welcher
A für CH₂, CD₂ oder CH(CH₃) steht,
R¹ für (C₄-C₆)-Alkyl, (C₃-C₇)-Cycloalkyl oder Phenyl steht,
wobei (C₄-C₆)-Alkyl mit 1 oder 2 Substituenten unabhängig voneinander ausgewählt aus der Gruppe Fluor und Trifluormethyl substituiert sein kann,
wobei (C₃-C₇)-Cycloalkyl mit 1 bis 4 Substituenten unabhängig voneinander ausgewählt aus der Gruppe Fluor, Trifluormethyl und (C₁-C₄)-Alkyl substituiert sein kann,
und
wobei Phenyl mit 1 bis 4 Substituenten unabhängig voneinander ausgewählt aus der Gruppe Halogen, Cyano, Monofluormethyl, Difluormethyl, Trifluormethyl, (C₁-C₄)-Alkyl, (C₃-C₆)-Cycloalkyl, (C₁-C₄)-Alkoxy, Difluormethoxy und Trifluormethoxy substituiert sein kann,
R² für Wasserstoff, (C₁-C₄)-Alkyl, (C₁-C₄)-Alkoxy-(C₁-C₄)-Alkyl, Cyclopropyl, Monofluormethyl, Difluormethyl oder Trifluormethyl steht,
R³ für Wasserstoff steht,
R⁴ für Wasserstoff, Halogen, Cyano, Monofluormethyl, Difluormethyl, Trifluormethyl, (C₁-C₄)-Alkyl, Ethinyl, (C₃-C₇)-Cycloalkyl oder (C₁-C₄)-Alkoxy steht,
R⁵ für Wasserstoff steht,
E für Stickstoff oder CR⁶ steht,
wobei
R⁶ für Wasserstoff, Deuterium, Halogen, Cyano, Difluormethyl, Trifluormethyl, (C₁-C₆)-Alkyl, (C₂-C₆)-Alkinyl, Cyclopropyl, Cyclobutyl, Hydroxy, -OR⁷, -NR⁸R⁹, Hydroxycarbonyl, (C₁-C₄)-Alkoxycarbonyl,-C(=O)-NR¹⁰R¹¹, 5- oder 6-gliedriges Heteroaryl steht,
worin (C₁-C₆)-Alkyl mit 1 bis 3 Substituenten unabhängig voneinander ausgewählt aus der Gruppe Fluor, Difluormethyl, Trifluormethyl, (C₁-C₄)-Alkoxy, Hydroxy, Amino, -N(C=O)R¹², (C₁-C₄)-Alkylsulfonylamino, (C₃-C₆)-Cycloalkylsulfonyl-amino, Cyclopropyl und Cyclobutyl substituiert sein kann,
worin R¹² für (C₃-C₇)-Cycloalkyl oder (C₁-C₄)-Alkyl steht,
worin (C₁-C₄)-Alkyl mit Trifluormethyl oder Difluormethyl substituiert sein kann,
worin (C₂-C₆)-Alkinyl mit 1 oder 2 Substituenten unabhängig voneinander ausgewählt aus der Gruppe Difluormethyl, Trifluormethyl, Hydroxy, Amino, Cyclopropyl und Cyclobutyl substituiert sein kann,
worin 5- oder 6-gliedriges Heteroaryl mit 1 bis 3 Substituenten unabhängig voneinander ausgewählt aus der Gruppe Fluor, Chlor, (C₁-C₄)-Alkyl, Hydroxy, Amino und Cyclopropyl substituiert sein kann,
worin R⁷ für (C₁-C₆)-Alkyl oder 5-gliedriges Heteroaryl steht,
worin (C₁-C₆)-Alkyl mit Trifluormethyl, (C₁-C₄)-Alkoxy, Hydroxy, Cyclopropyl oder Cyclobutyl substituiert sein kann,
worin R⁸ für Wasserstoff, (C₁-C₆)-Alkyl oder (C₃-C₇)-Cycloalkyl steht,
worin (C₃-C₇)-Cycloalkyl mit 1 bis 3 Substituenten unabhängig voneinander ausgewählt aus der Gruppe (C₁-C₄)-Alkyl, Hydroxy, Amino, Fluor, Trifluormethyl und Difluormethyl substituiert sein kann,
und
worin (C₁-C₆)-Alkyl mit 1 bis 4 Substituenten unabhängig voneinander ausgewählt aus der Gruppe Fluor, (C₁-C₄)-Alkyl, (C₃-C₇)-Cycloalkyl, (C₁-C₄)-Alkoxy, Hydroxy, Amino, Trifluormethyl, Difluormethyl, Monofluormethyl, 5- bis 7-gliedriges Aza-Heterocyclyl und Phenyl substituiert sein kann,
worin Phenyl mit 1 bis 3 Substituenten unabhängig voneinander ausgewählt aus der Gruppe Halogen, Cyano, (C₁-C₄)-Alkyl und (C₁-C₄)-Alkoxy substituiert sein kann,
worin 5- bis 7-gliedriges Aza-Heterocyclyl mit 1 bis 4 Substituenten Fluor substituiert sein kann,
und
worin (C₃-C₇)-Cycloalkyl mit 1 bis 3 Substituenten unabhängig voneinander ausgewählt aus der Gruppe Halogen, (C₁-C₄)-Alkyl und Hydroxy substituiert sein kann,
worin R⁹ für Wasserstoff oder (C₁-C₆)-Alkyl steht,
oder
R⁸ und R⁹ zusammen mit dem Stickstoffatom, an das sie gebunden sind, einen 3- bis 8-gliedrigen Heterocyclus bilden,
worin der 3- bis 8-gliedrige Heterocyclus mit 1 oder 2 Substituenten unabhängig voneinander ausgewählt aus der Gruppe Fluor, (C₁-C₄)-Alkyl, Hydroxycarbonyl, (C₁-C₄)-Alkoxycarbonyl, Hydroxy und Amino substituiert sein kann,
worin (C₁-C₄)-Alkyl mit Hydroxycarbonyl, (C₁-C₄)-Alkoxycarbonyl, Hydroxy oder Amino substituiert sein kann,
worin R¹⁰ für Wasserstoff, (C₁-C₆)-Alkyl oder (C₃-C₇)-Cycloalkyl steht,
worin (C₃-C₇)-Cycloalkyl mit 1 bis 3 Substituenten unabhängig voneinander ausgewählt aus der Gruppe (C₁-C₆)-Alkyl, Hydroxy, Trifluormethyl und Difluormethyl substituiert sein kann,
und
worin (C₁-C₆)-Alkyl mit mit 1 bis 4 Substituenten unabhängig voneinander ausgewählt aus der Gruppe Fluor, (C₃-C₇)-Cycloalkyl (C₁-C₄)-Alkoxy, Hydroxy, Amino, Trifluormethyl und Difluormethyl substituiert sein kann,
worin R¹¹ für Wasserstoff oder (C₁-C₄)-Alkyl steht,
oder
R¹⁰ und R¹¹ zusammen mit dem Stickstoffatom, an das sie gebunden sind, einen 3- bis 7-gliedrigen Heterocyclus bilden,
worin der 3- bis 7-gliedrige Heterocyclus mit 1 bis 3 Substituenten unabhängig voneinander ausgewählt aus der Gruppe Fluor, (C₁-C₄)-Alkyl, Hydroxy und Amino substituiert sein kann,
worin (C₁-C₄)-Alkyl mit Hydroxy substituiert sein kann,
L für eine Gruppe #¹-CR^{13A}R^{13B}-(CR^{14A}R^{14B})ₘ-#² steht,
wobei
#¹ für die Anknüpfstelle an die Carbonylgruppe steht,
#² für die Anknüpfstelle an den Pyrimidin- bzw. Triazinring steht,
m für eine Zahl 0, 1 oder 2 steht,,
R^{13A} für Wasserstoff, Trifluormethyl oder (C₁-C₄)-Alkyl steht,
R^{13B} für Wasserstoff, Difluormethyl, Trifluormethyl, (C₁-C₄)-Alkyl oder (C₃-C₇)-Cycloalkyl steht,
worin (C₁-C₆)-Alkyl mit 1 bis 3 Substituenten unabhängig voneinander ausgewählt aus der Gruppe Fluor, Cyano, Trifluormethyl, (C₃-C₇)-Cycloalkyl, Difluormethoxy und Trifluormethoxy substituiert sein kann,
oder
R^{13A} und R^{13B} zusammen mit dem Kohlenstoffatom, an das sie gebunden sind, einen 3- bis 6-gliedrigen Carbocyclus bilden,
R^{14A} für Wasserstoff, Fluor, (C₁-C₄)-Alkyl oder Hydroxy steht,
R^{14B} für Wasserstoff, Fluor, (C₁-C₄)-Alkyl oder Trifluormethyl steht,
sowie ihre *N-*Oxide, Salze, Solvate, Salze der *N-*Oxide und Solvate der *N-*Oxide und Salze.

2. Verbindung der Formel (I) nach Anspruch 1, in welcher
A für CH₂ steht,
R¹ für Phenyl steht,
wobei Phenyl mit 1 bis 4 Substituenten unabhängig voneinander ausgewählt aus der Gruppe Fluor, Chlor, Cyano und Methyl substituiert sein kann,
R² für Wasserstoff, Methyl, Ethyl oder Cyclopropyl steht,
R³ für Wasserstoff steht,
R⁴ für Wasserstoff, Fluor, Chlor, Methyl oder Ethyl steht,
R⁵ für Wasserstoff steht,
E für Stickstoff oder CR⁶ steht,
wobei
R⁶ für Wasserstoff, Chlor, Iod, Cyano, (C₁-C₆)-Alkyl, (C₂-C₆)-Alkinyl, Cyclopropyl, Hydroxy, -OR⁷, -NR⁸R⁹, Hydroxycarbonyl, (C₁-C₄)-Alkoxycarbonyl, -C(=O)-NR¹⁰R¹¹ oder 5-gliedriges Heteroaryl steht,
worin (C₁-C₆)-Alkyl mit 1 bis 3 Substituenten unabhängig voneinander ausgewählt aus der Gruppe Fluor, Difluormethyl, Trifluormethyl, Methoxy, Ethoxy, Hydroxy, Amino, -N(C=O)R¹², Methylsulfonylamino, Cyclopropyl und Cyclobutyl substituiert sein kann,
worin R¹² für Cyclopropyl, Cyclobutyl, Methyl oder Ethyl steht,
worin (C₂-C₆)-Alkinyl mit Cyclopropyl oder Cyclobutyl substituiert sein kann,
worin 5-gliedriges Heteroaryl mit Chlor, Methyl, Ethyl oder Hydroxy substituiert sein kann,
worin R⁷ für (C₁-C₄)-Alkyl oder Pyrazolyl steht,
worin (C₁-C₄)-Alkyl mit Trifluormethyl, Methoxy, Hydroxy oder Cyclopropyl substituiert sein kann,
worin R⁸ für Wasserstoff, (C₁-C₄)-Alkyl oder (C₃-C₆)-Cycloalkyl steht,
worin (C₃-C₆)-Cycloalkyl mit 1 bis 4 Substituenten unabhängig voneinander ausgewählt aus der Gruppe Methyl, Ethyl oder Hydroxy substituiert sein kann,
und
worin (C₁-C₄)-Alkyl mit 1 bis 4 Substituenten unabhängig voneinander ausgewählt aus der Gruppe Fluor, (C₁-C₄)-Alkyl, (C₃-C₅)-Cycloalkyl, Pyrrolidinyl, Piperidinyl, Methoxy, Ethoxy, Hydroxy, Amino, Trifluormethyl, Difluormethyl, Monofluormethyl und Phenyl substituiert sein kann,
worin Phenyl mit 1 bis 3 Substituenten unabhängig voneinander ausgewählt aus der Gruppe Fluor, Chlor, Cyano und Methoxy substituiert sein kann,
worin Pyrrolidinyl und Piperidinyl zweimal mit Fluor, substituiert sein kann,
und
worin (C₃-C₇)-Cycloalkyl mit Hydroxy substituiert sein kann,
worin R⁹ für Wasserstoff oder Methyl steht,
oder
R⁸ und R⁹ zusammen mit dem Stickstoffatom, an das sie gebunden sind, einen 4- bis 7-gliedrigen Heterocyclus bilden,
worin der 4- bis 7-gliedrige Heterocyclus mit 1 oder 2 Substituenten unabhängig voneinander ausgewählt aus der Gruppe (C₁-C₄)-Alkyl, Hydroxycarbonyl, Hydroxy und Amino substituiert sein kann,
worin (C₁-C₄)-Alkyl mit Hydroxycarbonyl, Hydroxy oder Amino substituiert sein kann,
worin R¹⁰ für Wasserstoff, (C₁-C₄)-Alkyl oder (C₃-C₆)-Cycloalkyl steht,
worin (C₃-C₆)-Cycloalkyl mit 1 bis 3 Substituenten unabhängig voneinander ausgewählt aus der Gruppe Methyl, Ethyl oder Hydroxy substituiert sein kann,
und
worin (C₁-C₄)-Alkyl mit mit 1 oder 2 Substituenten unabhängig voneinander ausgewählt aus der Gruppe Fluor, Hydroxy, Amino, Trifluormethyl und Difluormethyl substituiert sein kann,
worin R¹¹ für Wasserstoff oder Methyl steht,
oder
R¹⁰ und R¹¹ zusammen mit dem Stickstoffatom, an das sie gebunden sind, einen 4- bis 6-gliedrigen Heterocyclus bilden,
worin der 4- bis 6-gliedrige Heterocyclus mit 1 bis 3 Substituenten unabhängig voneinander ausgewählt aus der Gruppe Fluor, Methyl, Ethyl, Hydroxy und Amino substituiert sein kann,
worin Methyl und Ethyl mit Hydroxy substituiert sein können,
L für eine Gruppe #¹-CR^{13A}R^{13B}-(CR^{14A}R^{14B})ₘ-#² steht,
wobei
#¹ für die Anknüpfstelle an die Carbonylgruppe steht,
#² für die Anknüpfstelle an den Pyrimidin- bzw. Triazinring steht,
m für eine Zahl 0 steht,
R^{13A} für Wasserstoff oder Methyl steht,
R^{13B} für Wasserstoff, Difluormethyl, Trifluormethyl oder Methyl steht,
oder
R^{13A} und R^{13B} zusammen mit dem Kohlenstoffatom, an das sie gebunden sind, einen Cyclopropylring bilden,
sowie ihre *N-*Oxide, Salze, Solvate, Salze der *N-*Oxide und Solvate der *N-*Oxide und Salze.

3. Verbindung der Formel (I) nach Anspruch 1 oder 2, in welcher
A für CH₂ steht,
R¹ für eine Phenyl-Gruppe der Formel 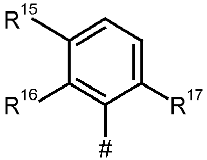 steht, wobei
# für die Anknüpfstelle an A steht,
und
R¹⁵ für Wasserstoff oder Fluor steht,
R¹⁶ und R¹⁷ für Fluor stehen,
R² für Methyl steht,
R³ für Wasserstoff steht,
R⁴ für Wasserstoff, Chlor oder Methyl steht,
R⁵ für Wasserstoff steht,
E für Stickstoff oder CR⁶ steht,
wobei
R⁶ für Wasserstoff, Chlor, Ethinyl, Hydroxy, -OR⁷, -NR⁸R⁹, -C(=O)-NR¹⁰R¹¹, 1H-pyrazol-1-yl oder 1,3-thiazol-5-yl steht,
worin Ethinyl mit Cyclopropyl substituiert ist,
worin 1H-pyrazol-1-yl und 1,3-thiazol-5-yl mit Methyl, Ethyl oder Hydroxy substituiert sein können,
worin R⁷ für Methyl, Ethyl oder 1H-pyrazol-4-yl steht,
worin Methyl mit Cyclopropyl substituiert sein kann,
worin Ethyl mit Trifluormethyl, Methoxy oder Hydroxy substituiert sein kann,
worin R⁸ für Wasserstoff, Ethyl, Propyl oder (C₄-C₆)-Cycloalkyl steht,
worin (C₄-C₆)-Cycloalkyl mit 1 oder 2 Substituenten Methyl oder Hydroxy substituiert sein kann,
und
worin Ethyl und Propyl mit 1 bis 3 Substituenten unabhängig voneinander ausgewählt aus der Gruppe Fluor, Methyl, Ethyl, Propyl, Cyclopropyl, Methoxy, Hydroxy, Amino, Trifluormethyl, Difluormethyl, Monofluormethyl und Phenyl substituiert sein können,
worin Phenyl mit 1 oder 2 Substituenten unabhängig voneinander ausgewählt aus der Gruppe Fluor, Chlor und Methoxy substituiert sein kann,
und
worin (C₄-C₇)-Cycloalkyl mit Hydroxy substituiert sein kann,
worin R⁹ für Wasserstoff steht,
oder
R⁸ und R⁹ zusammen mit dem Stickstoffatom, an das sie gebunden sind, einen Piperidinyl-, Pyrrolidinyl- oder 3-Azabicyclo[3.1.0]hex-3-yl-Ring bilden,
worin der Piperidinyl- und Pyrrolidinyl-Ring mit Methyl substituiert sein kann,
worin Methyl mit Hydroxycarbonyl oder Hydroxy substituiert sein kann,
und
worin der 3-Azabicyclo[3.1.0]hex-3-yl-Ring mit Amino substituiert sein kann,
worin R¹⁰ für Wasserstoff, Methyl, Ethyl, n-Propyl oder Cyclopropyl steht,
worin Methyl, Ethyl, n-Propyl mit 1 oder 2 Substituenten unabhängig voneinander ausgewählt aus der Gruppe Fluor, Amino und Trifluormethyl substituiert sein können,
worin R¹¹ für Wasserstoff steht,
oder
R¹⁰ und R¹¹ zusammen mit dem Stickstoffatom, an das sie gebunden sind, einen Pyrrolidinyl-, Piperidinyl- oder Piperazinyl-Ring bilden,
worin der Pyrrolidinyl-Ring mit 1 bis 3 Substituenten unabhängig voneinander ausgewählt aus der Gruppe Fluor, Methyl, Hydroxy und Amino substituiert sein kann,
worin Methyl mit Hydroxy substituiert sein kann,
worin der Piperazinyl-Ring am Stickstoffatom mit Methyl substituiert sein kann,
L für eine Gruppe #¹-CR^{13A}R^{13B}-#² steht,
wobei
#¹ für die Anknüpfstelle an die Carbonylgruppe steht,
#² für die Anknüpfstelle an den Pyrimidin- bzw. Triazinring steht,
R^{13A} für Methyl steht,
R^{13B} für Trifluormethyl oder Methyl steht,
sowie ihre *N-*Oxide, Salze, Solvate, Salze der *N-*Oxide und Solvate der *N-*Oxide und Salze.

4. Verfahren zur Herstellung von Verbindungen der Formel (I), wie in den Ansprüchen 1 bis 3 definiert, **dadurch gekennzeichnet, dass** man
eine Verbindung der Formel (II) 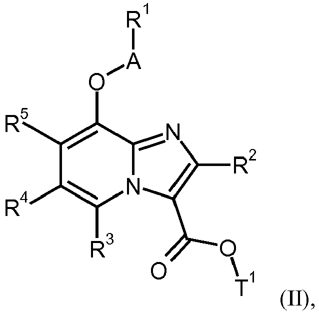 in welcher A, R¹, R², R³, R⁴, R⁵ jeweils die oben angegebenen Bedeutungen haben und
T¹ für (C₁-C₄)-Alkyl oder Benzyl steht,
in einem inerten Lösungsmittel in Gegenwart einer geeigneten Base oder Säure zu einer Carbonsäure der Formel (III) 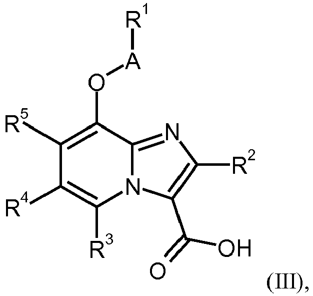 in welcher A, R¹, R², R³, R⁴ und R⁵ jeweils die oben angegebenen Bedeutungen haben,
umsetzt, und diese in der Folge in einem inerten Lösungsmittel unter Amidkupplungsbedingungen mit einem Ammoniumsalz in eine Verbindung der Formel (IV) 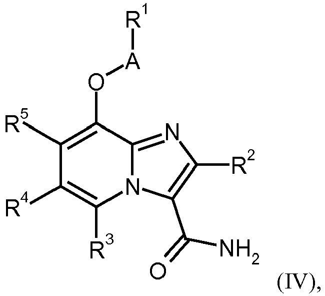 in welcher A, R¹, R², R³, R⁴ und R⁵ jeweils die oben angegebenen Bedeutungen haben, überführt, und diese anschließend in einem inerten Lösungsmittel mit Trifluoressigsäureanhydrid zu einer Verbindung der Formel (V) 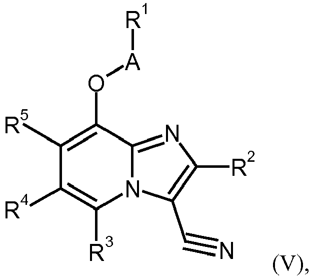 in welcher A, R¹, R², R³, R⁴ und R⁵ jeweils die oben angegebenen Bedeutungen haben, umsetzt, diese in Anwesenheit eines Alkylaluminium-Reagenzes in einem inerten Lösungsmittel in ein Amidin der Formel (VI) 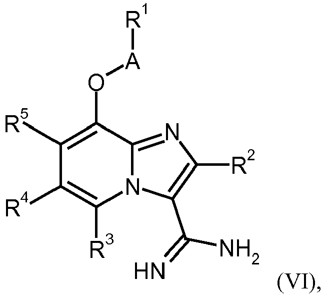 in welcher A, R¹, R², R³, R⁴ und R⁵ jeweils die oben angegebenen Bedeutungen haben, überführt,
oder
eine Verbindung der Formel (V) in einem geeignten Lösungsmittel in Gegenwart einer geeigneten Base mit Hydroxylamin Hydrochlorid zunächst in eine Verbindung der Formel (VIa) 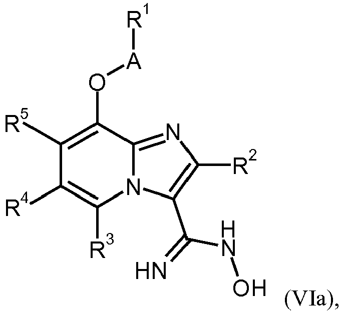 in welcher A, R¹, R², R³, R⁴ und R⁵ jeweils die oben angegebenen Bedeutungen haben, überführt, diese dann durch Hydrogenolyse in Gegenwart eines Palladiumkatalysators, wie beispielsweise Palladium auf Aktivkohle, in einem inerten Lösungsmittel, wie beispielsweise Ethanol oder Essigsäureethylester, in ein Amidin der Formel (VI) überführt,
diese in einem inerten Lösungsmittel in Gegenwart einer geeigneten Base mit einer Verbindung der Formel (VII) 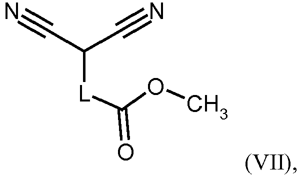 zu einer Verbindung der Formel (VIII) 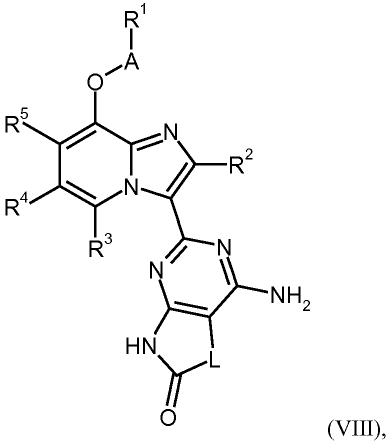 in welcher A, R¹, R², R³, R⁴, R⁵ und L jeweils die oben angegebenen Bedeutungen haben, umsetzt, die Aminogruppe in einem inerten Lösungsmittel mit Isopentylnitrit und einem Halogen-Äquivalent in eine Verbindung der Formel (IX) 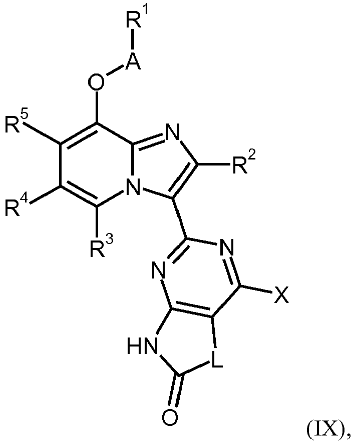 in welcher A, R¹, R², R³, R⁴, R⁵ und L jeweils die oben angegebenen Bedeutungen haben und
X für Chlor, Brom oder Iod steht
überführt, und diese
[A] im Anschluss in einem inerten Lösungsmittel gegebenenfalls in Gegenwart einer geeigneten Base mit einer Verbindung der Formel (X) 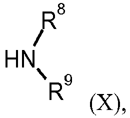 in welcher R⁸ und R⁹ die oben angegebene Bedeutungen haben,
zu einer Verbindung der Formel (I-A) 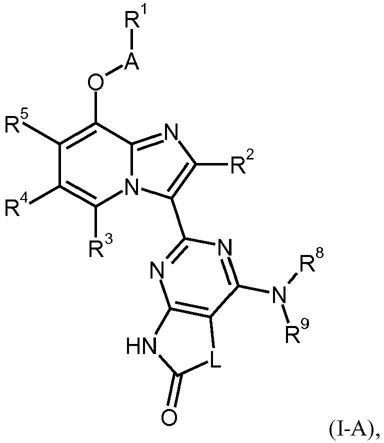 in welcher A, R¹, R², R³, R⁴, R⁵, R⁸, R⁹ und L jeweils die oben angegebenen Bedeutungen haben umsetzt,
oder
[B] das Iodid der Formel (IX) in einem inerten Lösungsmittel gegebenenfalls in Gegenwart einer geeigneten Base und Kupfer(I)iodid mit einer Verbindung der Formel (XI)
HO-R⁷ (XI),
in welcher R⁷ die oben angegebene Bedeutung hat,
zu einer Verbindung der Formel (I-B) 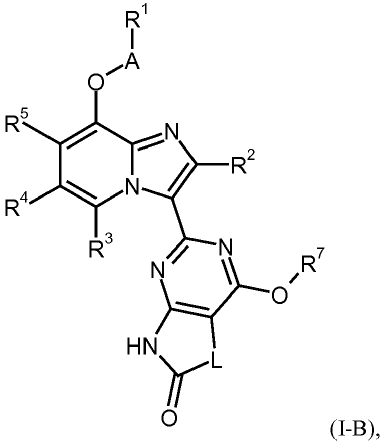 in welcher A, R¹, R², R³, R⁴, R⁵, R⁷ und L jeweils die oben angegebenen Bedeutungen haben umsetzt,
oder
[C] das Iodid der Formel (IX) in einem inerten Lösungsmittel gegebenenfalls in Gegenwart einer geeigneten Base mit Kupfer(I)cyanid zu einer Verbindung der Formel (I-C) 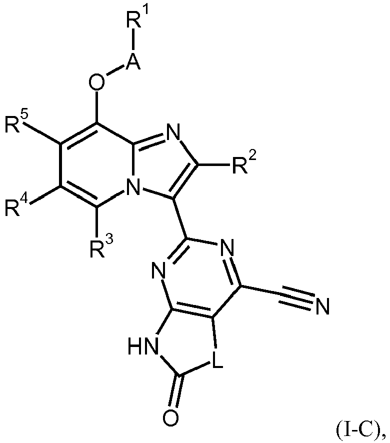 in welcher A, R¹, R², R³, R⁴, R⁵ und L jeweils die oben angegebenen Bedeutungen haben umsetzt, und diese in einem inerten Lösungsmittel mit einer geeigneten wässrigen Base in eine Verbindung der Formel (I-D) 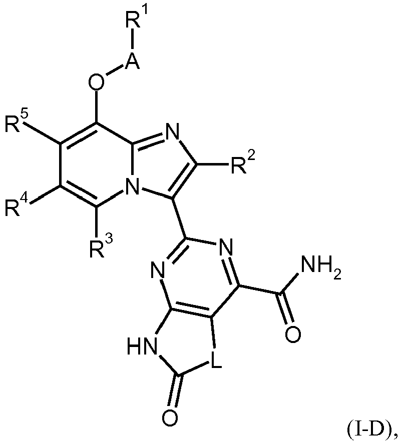 in welcher A, R¹, R², R³, R⁴, R⁵ und L jeweils die oben angegebenen Bedeutungen haben überführt, diese im Anschluß in einem inerten Lösungsmittel mit einer geeigneten wässrigen Säure in eine Säure der Formel (I-E) 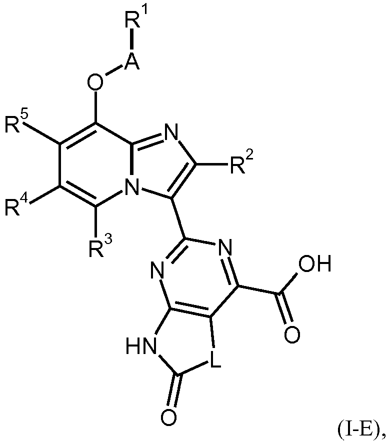 in welcher A, R¹, R², R³, R⁴, R⁵ und L jeweils die oben angegebenen Bedeutungen haben überführt, und diese in der Folge in einem inerten Lösungsmittel unter Amidkupplungsbedingungen mit einem Amin der Formel (XII) 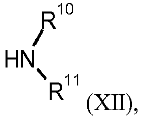 in welcher R¹⁰ und R¹¹ jeweils die oben angegebenen Bedeutungen haben, in eine Verbindung der Formel (I-F) 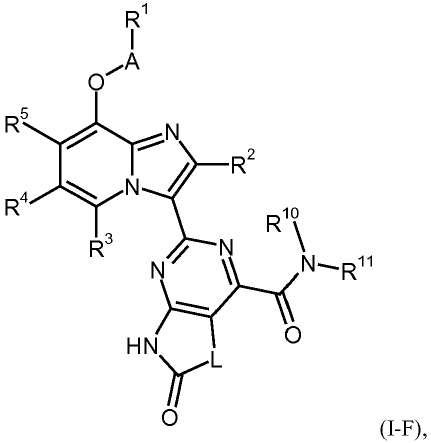 in welcher A, R¹, R², R³, R⁴, R⁵, R¹⁰, R¹¹ und L jeweils die oben angegebenen Bedeutungen haben,
überführt,
und gegebenenfalls die resultierenden Verbindungen der Formel (I) gegebenenfalls mit den entsprechenden (i) Lösungsmitteln und/oder (ii) Säuren oder Basen in ihre Solvate, Salze und/oder Solvate der Salze überführt.

5. Verbindung der Formel (I), wie in einem der Ansprüche 1 bis 3 definiert, zur Verwendung bei der Behandlung und/oder Prophylaxe von Krankheiten.

6. Verwendung einer Verbindung der Formel (I), wie in einem der Ansprüche 1 bis 3 definiert, zur Herstellung eines Arzneimittels zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen, Arteriosklerose, Demenzerkrankungen und erektiler Dysfunktion.

7. Arzneimittel enthaltend eine Verbindung der Formel (I), wie in einem der Ansprüche 1 bis 3 definiert, in Kombination mit einem inerten, nicht-toxischen, pharmazeutisch geeigneten Hilfsstoff.

8. Arzneimittel enthaltend eine Verbindung der Formel (I), wie in einem der Ansprüche 1 bis 3 definiert, in Kombination mit einem weiteren Wirkstoff ausgewählt aus der Gruppe bestehend aus organischen Nitraten, NO-Donatoren, cGMP-PDE-Inhibitoren, antithrombotisch wirkenden Mitteln, den Blutdruck senkenden Mitteln sowie den Fettstoffwechsel verändernden Mitteln.

9. Arzneimittel nach Anspruch 7 oder 8 zur Verwendung bei der Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen, Arteriosklerose, Demenzerkrankungen und erektiler Dysfunktion.

## Claims

1. Compound of the formula (I) 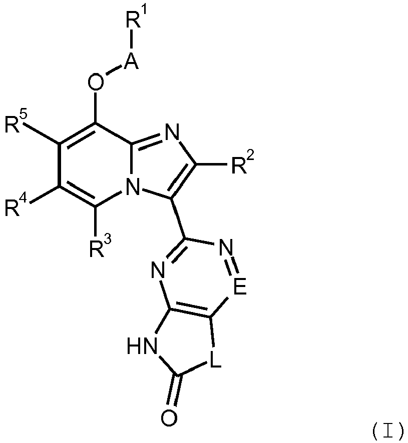 in which
A represents CH₂, CD₂ or CH(CH₃),
R¹ represents (C₄-C₆)-alkyl, (C₃-C₇)-cycloalkyl or phenyl,
where (C₄-C₆)-alkyl may be substituted by 1 or 2 substituents independently of one another selected from the group consisting of fluorine and trifluoromethyl,
where (C₃-C₇)-cycloalkyl may be substituted by 1 to 4 substituents independently of one another selected from the group consisting of fluorine, trifluoromethyl and (C₁-C₄)-alkyl,
and
where phenyl may be substituted by 1 to 4 substituents independently of one another selected from the group consisting of halogen, cyano, monofluoromethyl, difluoromethyl, trifluoromethyl, (C₁-C₄)-alkyl, (C₃-C₆)-cycloalkyl, (C₁-C₄)-alkoxy, difluoromethoxy and trifluoromethoxy,
R² represents hydrogen, (C₁-C₄)-alkyl, (C₁-C₄)-alkoxy-(C₁-C₄)-alkyl, cyclopropyl, monofluoromethyl, difluoromethyl or trifluoromethyl,
R³ represents hydrogen,
R⁴ represents hydrogen, halogen, cyano, monofluoromethyl, difluoromethyl, trifluoromethyl, (C₁-C₄)-alkyl, ethynyl, (C₃-C₇)-cycloalkyl or (C₁-C₄)-alkoxy,
R⁵ represents hydrogen,
E represents nitrogen or CR⁶,
where
R⁶ represents hydrogen, deuterium, halogen, cyano, difluoromethyl, trifluoromethyl, (C₁-C₆)-alkyl, (C₂-C₆)-alkynyl, cyclopropyl, cyclobutyl, hydroxy, -OR⁷,-NR⁸R⁹, hydroxycarbonyl, (C₁-C₄)-alkoxycarbonyl, -C(=O)-NR¹⁰R¹¹, 5- or 6-membered heteroaryl,
in which (C₁-C₆)-alkyl may be substituted by 1 to 3 substituents independently of one another selected from the group consisting of fluorine, difluoromethyl, trifluoromethyl, (C₁-C₄)-alkoxy, hydroxy, amino, -N(C=O)R¹², (C₁-C₄)-alkylsulphonylamino, (C₃-C₆)-cycloalkylsulphonylamino, cyclopropyl and cyclobutyl,
in which R¹² represents (C₃-C₇)-cycloalkyl or (C₁-C₄)-alkyl,
in which (C₁-C₄)-alkyl may be substituted by trifluoromethyl or difluoromethyl,
in which (C₂-C₆)-alkynyl may be substituted by 1 or 2 substituents independently of one another selected from the group consisting of difluoromethyl, trifluoromethyl, hydroxy, amino, cyclopropyl and cyclobutyl,
in which 5- or 6-membered heteroaryl may be substituted by 1 to 3 substituents independently of one another selected from the group consisting of fluorine, chlorine, (C₁-C₄)-alkyl, hydroxy, amino and cyclopropyl,
in which R⁷ represents (C₁-C₆)-alkyl or 5-membered heteroaryl,
in which (C₁-C₆)-alkyl may be substituted by trifluoromethyl, (C₁-C₄)-alkoxy, hydroxy, cyclopropyl or cyclobutyl,
in which R⁸ represents hydrogen, (C₁-C₆)-alkyl or (C₃-C₇)-cycloalkyl,
in which (C₃-C₇)-cycloalkyl may be substituted by 1 to 3 substituents independently of one another selected from the group consisting of (C₁-C₄)-alkyl, hydroxy, amino, fluorine, trifluoromethyl and difluoromethyl,
and
in which (C₁-C₆)-alkyl may be substituted by 1 to 4 substituents independently of one another selected from the group consisting of fluorine, (C₁-C₄)-alkyl, (C₃-C₇)-cycloalkyl, (C₁-C₄)-alkoxy, hydroxy, amino, trifluoromethyl, difluoromethyl, monofluoromethyl, 5- to 7-membered azaheterocyclyl and phenyl,
in which phenyl may be substituted by 1 to 3 substituents independently of one another selected from the group consisting of halogen, cyano, (C₁-C₄)-alkyl and (C₁-C₄)-alkoxy,
in which 5- to 7-membered azaheterocyclyl may be substituted by 1 to 4 fluorine substituents,
and
in which (C₃-C₇)-cycloalkyl may be substituted by 1 to 3 substituents independently of one another selected from the group consisting of halogen, (C₁-C₄)-alkyl and hydroxy,
in which R⁹ represents hydrogen or (C₁-C₆)-alkyl,
or
R⁸ and R⁹ together with the nitrogen atom to which they are attached form a 3- to 8-membered heterocycle,
in which the 3- to 8-membered heterocycle may be substituted by 1 or 2 substituents independently of one another selected from the group consisting of fluorine, (C₁-C₄)-alkyl, hydroxycarbonyl, (C₁-C₄)-alkoxycarbonyl, hydroxy and amino,
in which (C₁-C₄)-alkyl may be substituted by hydroxycarbonyl, (C₁-C₄)-alkoxycarbonyl, hydroxy or amino,
in which R¹⁰ represents hydrogen, (C₁-C₆)-alkyl or (C₃-C₇)-cycloalkyl,
in which (C₃-C₇)-cycloalkyl may be substituted by 1 to 3 substituents independently of one another selected from the group consisting of (C₁-C₆)-alkyl, hydroxy, trifluoromethyl and difluoromethyl,
and
in which (C₁-C₆)-alkyl may be substituted by 1 to 4 substituents independently of one another selected from the group consisting of fluorine, (C₃-C₇)-cycloalkyl, (C₁-C₄)-alkoxy, hydroxy, amino, trifluoromethyl and difluoromethyl,
in which R¹¹ represents hydrogen or (C₁-C₄)-alkyl,
or
R¹⁰ and R¹¹ together with the nitrogen atom to which they are attached form a 3- to 7-membered heterocycle,
in which the 3- to 7-membered heterocycle may be substituted by 1 to 3 substituents independently of one another selected from the group consisting of fluorine, (C₁-C₄)-alkyl, hydroxy and amino,
in which (C₁-C₄)-alkyl may be substituted by hydroxy,
L represents a #¹-CR^{13A}R^{13B}-(CR^{14A}R^{14B})ₘ-#² group, where
#¹ represents the point of attachment to the carbonyl group,
#² represents the point of attachment to the pyrimidine or triazine ring,
m represents a number 0, 1 or 2,
R^{13A} represents hydrogen, trifluoromethyl or (C₁-C₄)-alkyl,
R^{13B} represents hydrogen, difluoromethyl, trifluoromethyl, (C₁-C₄)-alkyl or (C₃-C₇)-cycloalkyl,
in which (C₁-C₆)-alkyl may be substituted by 1 to 3 substituents independently of one another selected from the group consisting of fluorine, cyano, trifluoromethyl, (C₃-C₇)-cycloalkyl, difluoromethoxy and trifluoromethoxy,
or
R^{13A} and R^{13B} together with the carbon atom to which they are attached form a 3-to 6-membered carbocycle,
R^{14A} represents hydrogen, fluorine, (C₁-C₄)-alkyl or hydroxy,
R^{14B} represents hydrogen, fluorine, (C₁-C₄)-alkyl or trifluoromethyl,
and the *N*-oxides, salts, solvates, salts of the *N-*oxides and solvates of the *N*-oxides and salts thereof.

2. Compound of the formula (I) according to Claim 1 in which
A represents CH₂,
R¹ represents phenyl,
where phenyl may be substituted by 1 to 4 substituents independently of one another selected from the group consisting of fluorine, chlorine, cyano and methyl,
R² represents hydrogen, methyl, ethyl or cyclopropyl,
R³ represents hydrogen,
R⁴ represents hydrogen, fluorine, chlorine, methyl or ethyl,
R⁵ represents hydrogen,
E represents nitrogen or CR⁶,
where
R⁶ represents hydrogen, chlorine, iodine, cyano, (C₁-C₆)-alkyl, (C₂-C₆)-alkynyl, cyclopropyl, hydroxy, -OR⁷, -NR⁸R⁹, hydroxycarbonyl, (C₁-C₄)-alkoxycarbonyl, -C(=O)-NR¹⁰R¹¹ or 5-membered heteroaryl,
in which (C₁-C₆)-alkyl may be substituted by 1 to 3 substituents independently of one another selected from the group consisting of fluorine, difluoromethyl, trifluoromethyl, methoxy, ethoxy, hydroxy, amino, -N(C=O)R¹², methylsulphonylamino, cyclopropyl and cyclobutyl,
in which R¹² represents cyclopropyl, cyclobutyl, methyl or ethyl,
in which (C₂-C₆)-alkynyl may be substituted by cyclopropyl or cyclobutyl,
in which 5-membered heteroaryl may be substituted by chlorine, methyl, ethyl or hydroxy,
in which R⁷ represents (C₁-C₄)-alkyl or pyrazolyl,
in which (C₁-C₄)-alkyl may be substituted by trifluoromethyl, methoxy, hydroxy or cyclopropyl,
in which R⁸ represents hydrogen, (C₁-C₄)-alkyl or (C₃-C₆)-cycloalkyl,
in which (C₃-C₆)-cycloalkyl may be substituted by 1 to 4 substituents independently of one another selected from the group consisting of methyl, ethyl and hydroxy,
and
in which (C₁-C₄)-alkyl may be substituted by 1 to 4 substituents independently of one another selected from the group consisting of fluorine, (C₁-C₄)-alkyl, (C₃-C₅)-cycloalkyl, pyrrolidinyl, piperidinyl, methoxy, ethoxy, hydroxy, amino, trifluoromethyl, difluoromethyl, monofluoromethyl and phenyl,
in which phenyl may be substituted by 1 to 3 substituents independently of one another selected from the group consisting of fluorine, chlorine, cyano and methoxy,
in which pyrrolidinyl and piperidinyl may be disubstituted by fluorine,
and
in which (C₃-C₇)-cycloalkyl may be substituted by hydroxy,
in which R⁹ represents hydrogen or methyl,
or
R⁸ and R⁹ together with the nitrogen atom to which they are attached form a 4- to 7-membered heterocycle,
in which the 4- to 7-membered heterocycle may be substituted by 1 or 2 substituents independently of one another selected from the group consisting of (C₁-C₄)-alkyl, hydroxycarbonyl, hydroxy and amino,
in which (C₁-C₄)-alkyl may be substituted by hydroxycarbonyl, hydroxy or amino,
in which R¹⁰ represents hydrogen, (C₁-C₄)-alkyl or (C₃-C₆)-cycloalkyl,
in which (C₃-C₆)-cycloalkyl may be substituted by 1 to 3 substituents independently of one another selected from the group consisting of methyl, ethyl and hydroxy,
and
in which (C₁-C₄)-alkyl may be substituted by 1 or 2 substituents independently of one another selected from the group consisting of fluorine, hydroxy, amino, trifluoromethyl and difluoromethyl,
in which R¹¹ represents hydrogen or methyl,
or
R¹⁰ and R¹¹ together with the nitrogen atom to which they are attached form a 4- to 6-membered heterocycle,
in which the 4- to 6-membered heterocycle may be substituted by 1 to 3 substituents independently of one another selected from the group consisting of fluorine, methyl, ethyl, hydroxy and amino,
in which methyl and ethyl may be substituted by hydroxy,
L represents a #¹-CR^{13A}R^{13B}-(CR^{14A}R^{14B})ₘ-#² group,
where
#¹ represents the point of attachment to the carbonyl group,
#² represents the point of attachment to the pyrimidine or triazine ring,
m represents a number 0,
R^{13A} represents hydrogen or methyl,
R^{13B} represents hydrogen, difluoromethyl, trifluoromethyl or methyl,
or
R^{13A} and R^{13B} together with the carbon atom to which they are attached form a cyclopropyl ring,
and the *N*-oxides, salts, solvates, salts of the *N-*oxides and solvates of the *N*-oxides and salts thereof.

3. Compound of the formula (I) according to Claim 1 or 2 in which
A represents CH₂,
R¹ represents a phenyl group of the formula 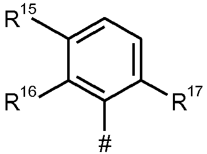 where
# represents the point of attachment to A,
and
R¹⁵ represents hydrogen or fluorine,
R¹⁶ and R¹⁷ represent fluorine,
R² represents methyl,
R³ represents hydrogen,
R⁴ represents hydrogen, chlorine or methyl,
R⁵ represents hydrogen,
E represents nitrogen or CR⁶,
where
R⁶ represents hydrogen, chlorine, ethynyl, hydroxy, -OR⁷, -NR⁸R⁹, -C(=O)-NR¹⁰R¹¹, 1H-pyrazol-1-yl or 1,3-thiazol-5-yl,
in which ethynyl is substituted by cyclopropyl,
in which 1H-pyrazol-1-yl and 1,3-thiazol-5-yl may be substituted by methyl, ethyl or hydroxy,
in which R⁷ represents methyl, ethyl or 1H-pyrazol-4-yl,
in which methyl may be substituted by cyclopropyl,
in which ethyl may be substituted by trifluoromethyl, methoxy or hydroxy,
in which R⁸ represents hydrogen, ethyl, propyl or (C₄-C₆)-cycloalkyl,
in which (C₄-C₆)-cycloalkyl may be substituted by 1 or 2 methyl or hydroxy substituents,
and
in which ethyl and propyl may be substituted by 1 to 3 substituents independently of one another selected from the group consisting of fluorine, methyl, ethyl, propyl, cyclopropyl, methoxy, hydroxy, amino, trifluoromethyl, difluoromethyl, monofluoromethyl and phenyl,
in which phenyl may be substituted by 1 or 2 substituents independently of one another selected from the group consisting of fluorine, chlorine and methoxy,
and
in which (C₄-C₇)-cycloalkyl may be substituted by hydroxy,
in which R⁹ represents hydrogen,
or
R⁸ and R⁹ together with the nitrogen atom to which they are attached form a piperidinyl, pyrrolidinyl or 3-azabicyclo[3.1.0]hex-3-yl ring,
in which the piperidinyl and pyrrolidinyl ring may be substituted by methyl,
in which methyl may be substituted by hydroxycarbonyl or hydroxy,
and
in which the 3-azabicyclo[3.1.0]hex-3-yl ring may be substituted by amino,
in which R¹⁰ represents hydrogen, methyl, ethyl, n-propyl or cyclopropyl,
in which methyl, ethyl and n-propyl may be substituted by 1 or 2 substituents independently of one another selected from the group consisting of fluorine, amino and trifluoromethyl,
in which R¹¹ represents hydrogen,
or
R¹⁰ and R¹¹ together with the nitrogen atom to which they are attached form a pyrrolidinyl, piperidinyl or piperazinyl ring,
in which the pyrrolidinyl ring may be substituted by 1 to 3 substituents independently of one another selected from the group consisting of fluorine, methyl, hydroxy and amino,
in which methyl may be substituted by hydroxy,
in which the piperazinyl ring may be substituted at the nitrogen atom by methyl,
L represents a #¹-CR^{13A}R^{13B}-#² group,
where
#¹ represents the point of attachment to the carbonyl group,
#² represents the point of attachment to the pyrimidine or triazine ring,
R^{13A} represents methyl,
R^{13B} represents trifluoromethyl or methyl,
and the *N*-oxides, salts, solvates, salts of the *N-*oxides and solvates of the *N*-oxides and salts thereof.

4. Process for preparing compounds of the formula (I) as defined in Claims 1 to 3, **characterized in that** a compound of the formula (II) 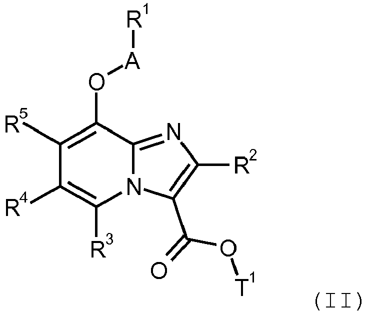 in which A, R¹, R², R³, R⁴ and R⁵ each have the meanings given above and
T¹ represents (C₁-C₄)-alkyl or benzyl,
is reacted in an inert solvent in the presence of a suitable base or acid to give a carboxylic acid of the formula (III) 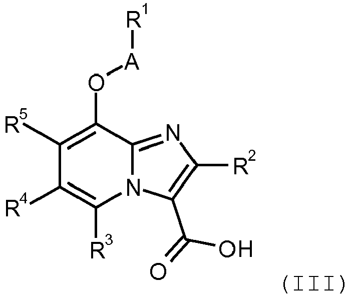 in which A, R¹, R², R³, R⁴ and R⁵ each have the meanings given above,
and this is subsequently converted in an inert solvent under amide coupling conditions with an ammonium salt into a compound of the formula (IV) 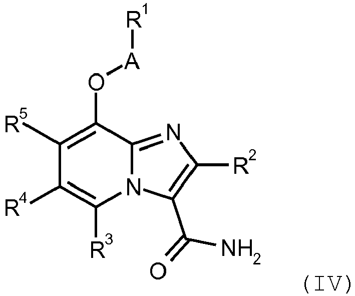 in which A, R¹, R², R³, R⁴ and R⁵ each have the meanings given above, and this is then reacted in an inert solvent with trifluoroacetic anhydride to give a compound of the formula (V) 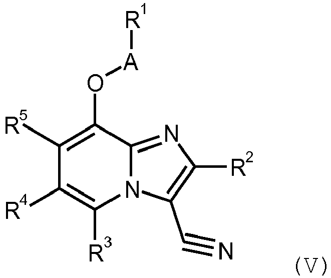 in which A, R¹, R², R³, R⁴ and R⁵ each have the meanings given above, this is converted in the presence of an alkylaluminum reagent in an inert solvent into an amidine of the formula (VI) 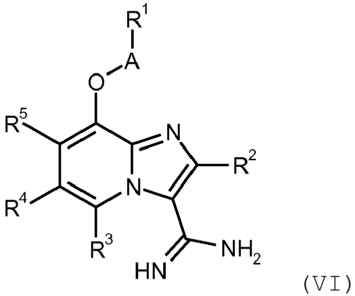 in which A, R¹, R², R³, R⁴ and R⁵ each have the meanings given above, or
a compound of the formula (V) is converted in a suitable solvent in the presence of a suitable base with hydroxylamine hydrochloride initially into a compound of the formula (VIa) 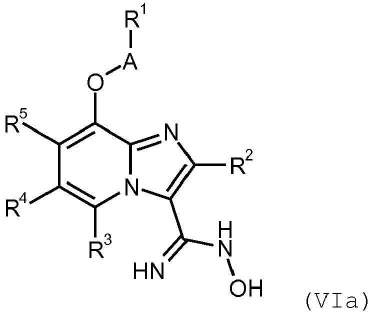 in which A, R¹, R², R³, R⁴ and R⁵ each have the meanings given above, this is then converted by hydrogenolysis in the presence of a palladium catalyst, for example palladium on activated carbon, in an inert solvent, for example ethanol or ethyl acetate, into an amidine of the formula (VI),
this is reacted in an inert solvent in the presence of a suitable base with a compound of the formula (VII) 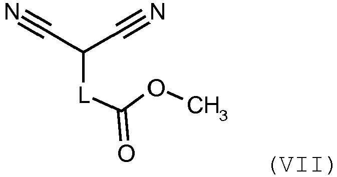 to give a compound of the formula (VIII) 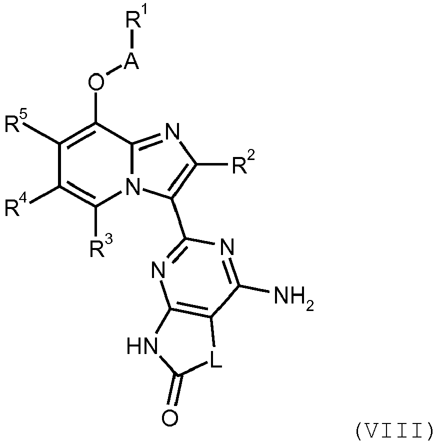 in which A, R¹, R², R³, R⁴, R⁵ and L each have the meanings given above, the amino group is converted in an inert solvent with isopentyl nitrite and a halogen equivalent into a compound of the formula (IX) 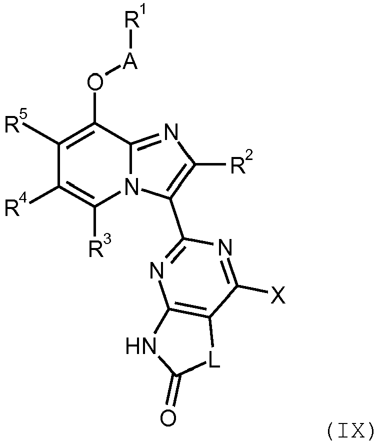 in which A, R¹, R², R³, R⁴, R⁵ and L each have the meanings given above and
X represents chlorine, bromine or iodine,
and this
[A] is subsequently reacted in an inert solvent, optionally in the presence of a suitable base, with a compound of the formula (X) 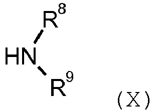 in which R⁸ and R⁹ have the meanings given above, to give a compound of the formula (I-A) 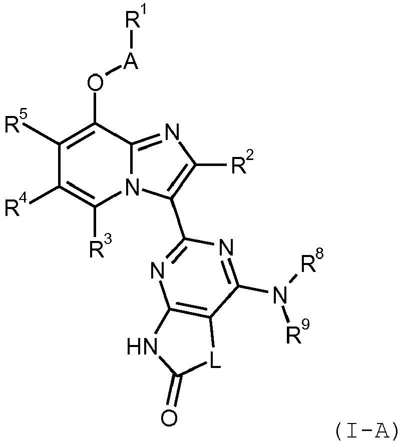 in which A, R¹, R², R³, R⁴, R⁵, R⁸, R⁹ and L each have the meanings given above
or
[B] the iodide of the formula (IX) is reacted in an inert solvent, optionally in the presence of a suitable base and copper(I) iodide, with a compound of the formula (XI)
HO-R⁷ (XI)
in which R⁷ has the meaning given above to give a compound of the formula (I-B) 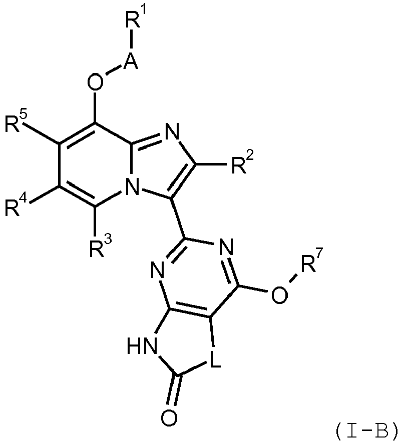 in which A, R¹, R², R³, R⁴, R⁵, R⁷ and L each have the meanings given above
or
[C] the iodide of the formula (IX) is reacted in an inert solvent, optionally in the presence of a suitable base, with copper(I) cyanide to give a compound of the formula (I-C) 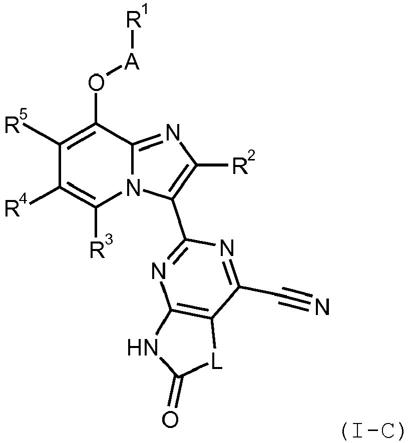 in which A, R¹, R², R³, R⁴, R⁵ and L each have the meanings given above, and this is converted in an inert solvent with a suitable aqueous base into a compound of the formula (I-D) 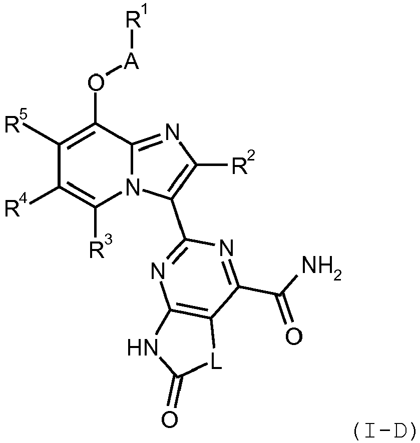 in which A, R¹, R², R³, R⁴, R⁵ and L each have the meanings given above, and this is subsequently converted in an inert solvent with a suitable aqueous acid into an acid of the formula (I-E) 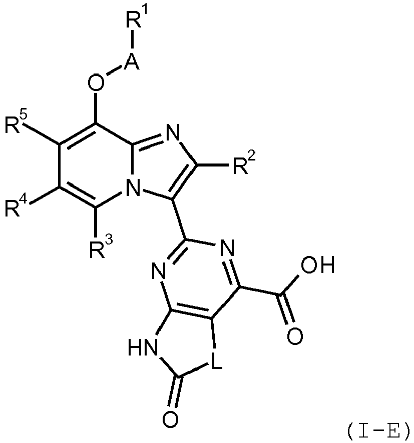 in which A, R¹, R², R³, R⁴, R⁵ and L each have the meanings given above, and this is subsequently converted in an inert solvent under amide coupling conditions with an amine of the formula (XII) 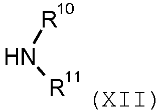 in which R¹⁰ and R¹¹ each have the meanings given above, into a compound of the formula (I-F) 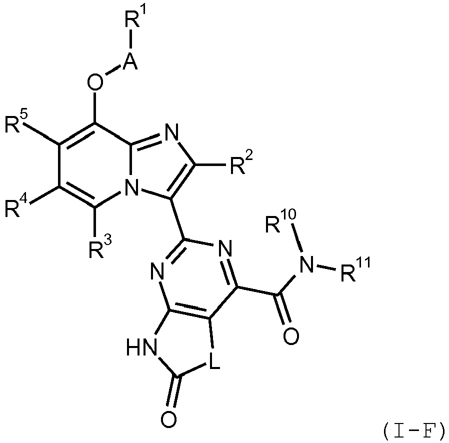 in which A, R¹, R², R³, R⁴, R⁵, R¹⁰, R¹¹ and L each have the meanings given above,
and the resulting compounds of the formula (I) are optionally, optionally with the appropriate (i) solvents and/or (ii) acids or bases, converted into their solvates, salts and/or solvates of the salts.

5. Compound of the formula (I) as defined in any of Claims 1 to 3 for use in the treatment and/or prophylaxis of diseases.

6. Use of a compound of the formula (I) as defined in any of Claims 1 to 3 for producing a medicament for the treatment and/or prophylaxis of heart failure, angina pectoris, hypertension, pulmonary hypertension, ischaemias, vascular disorders, renal insufficiency, thromboembolic disorders, fibrotic disorders, arteriosclerosis, dementia disorders and erectile dysfunction.

7. Medicament comprising a compound of the formula (I) as defined in any of Claims 1 to 3 in combination with an inert, non-toxic, pharmaceutically suitable auxiliary.

8. Medicament comprising a compound of the formula (I) as defined in any of Claims 1 to 3 in combination with a further active compound selected from the group consisting of organic nitrates, NO donors, cGMP-PDE inhibitors, antithrombotic agents, hypotensive agents and lipid metabolism modifiers.

9. Medicament according to Claim 7 or 8 for use in the treatment and/or prophylaxis of heart failure, angina pectoris, hypertension, pulmonary hypertension, ischaemias, vascular disorders, renal insufficiency, thromboembolic disorders, fibrotic disorders, arteriosclerosis, dementia disorders and erectile dysfunction.

## Revendications

1. Composé de formule (I) 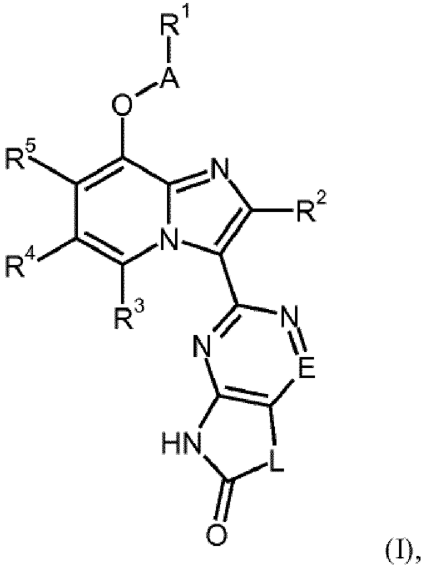 dans lequel
A représente CH₂, CD₂ ou CH(CH₃),
R¹ représente alkyle en (C₄-C₆), cycloalkyle en (C₃-C₇) ou phényle,
l'alkyle en (C₄-C₆) pouvant être substitué avec 1 ou 2 substituants choisis indépendamment l'un de l'autre dans le groupe constitué par fluor et trifluorométhyle,
le cycloalkyle en (C₃-C₇) pouvant être substitué avec 1 à 4 substituants choisis indépendamment les uns des autres dans le groupe constitué par fluor, trifluorométhyle et alkyle en (C₁-C₄),
et
le phényle pouvant être substitué avec 1 à 4 substituants choisis indépendamment les uns des autres dans le groupe constitué par halogène, cyano, monofluorométhyle, difluorométhyle, trifluorométhyle, alkyle en (C₁-C₄), cycloalkyle en (C₃-C₆), alcoxy en (C₁-C₄), difluorométhoxy et trifluorométhoxy,
R² représente hydrogène, alkyle en (C₁-C₄), alcoxy en (C₁-C₄)-alkyle en (C₁-C₄), cyclopropyle, monofluorométhyle, difluorométhyle ou trifluorométhyle,
R³ représente hydrogène,
R⁴ représente hydrogène, halogène, cyano, monofluorométhyle, difluorométhyle, trifluorométhyle, alkyle en (C₁-C₄), éthynyle, cycloalkyle en (C₃-C₇) ou alcoxy en (C₁-C₄),
R⁵ représente hydrogène,
E représente azote ou CR⁶,
R⁶ représentant hydrogène, deutérium, halogène, cyano, difluorométhyle, trifluorométhyle, alkyle en (C₁-C₆), alcynyle en (C₂-C₆), cyclopropyle, cyclobutyle, hydroxy, -OR⁷, -NR⁸R⁹, hydroxycarbonyle, alcoxycarbonyle en (C₁-C₄), -C(=O)-NR¹⁰R¹¹, hétéroaryle à 5 ou 6 chaînons,
l'alkyle en (C₁-C₆) pouvant être substitué avec 1 à 3 substituants choisis indépendamment les uns des autres dans le groupe constitué par fluor, difluorométhyle, trifluorométhyle, alcoxy en (C₁-C₄), hydroxy, amino, -N(C=O)R¹², alkylsulfonylamino en (C₁-C₄), cycloalkylsulfonylamino en (C₃-C₆), cyclopropyle et cyclobutyle,
R¹² représentant cycloalkyle en (C₃-C₇) ou alkyle en (C₁-C₄),
l'alkyle en (C₁-C₄) pouvant être substitué avec trifluorométhyle ou difluorométhyle,
l'alcynyle en (C₂-C₆) pouvant être substitué avec 1 ou 2 substituants choisis indépendamment l'un de l'autre dans le groupe constitué par difluorométhyle, trifluorométhyle, hydroxy, amino, cyclopropyle et cyclobutyle,
l'hétéroaryle à 5 ou 6 chaînons pouvant être substitué avec 1 à 3 substituants choisis indépendamment les uns des autres dans le groupe constitué par fluor, chlore, alkyle en (C₁-C₄), hydroxy, amino et cyclopropyle,
R⁷ représentant alkyle en (C₁-C₆) ou hétéroaryle à 5 chaînons,
l'alkyle en (C₁-C₆) pouvant être substitué avec trifluorométhyle, alcoxy en (C₁-C₄), hydroxy, cyclopropyle ou cyclobutyle,
R⁸ représentant hydrogène, alkyle en (C₁-C₆) ou cycloalkyle en (C₃-C₇),
le cycloalkyle en (C₃-C₇) pouvant être substitué avec 1 à 3 substituants choisis indépendamment les uns des autres dans le groupe constitué par alkyle en (C₁-C₄), hydroxy, amino, fluor, trifluorométhyle et difluorométhyle,
et
l'alkyle en (C₁-C₆) pouvant être substitué avec 1 à 4 substituants choisis indépendamment les uns des autres dans le groupe constitué par fluor, alkyle en (C₁-C₄), cycloalkyle en (C₃-C₇), alcoxy en (C₁-C₄), hydroxy, amino, trifluorométhyle, difluorométhyle, monofluorométhyle, aza-hétérocyclyle de 5 à 7 chaînons et phényle,
le phényle pouvant être substitué avec 1 à 3 substituants choisis indépendamment les uns des autres dans le groupe constitué par halogène, cyano, alkyle en (C₁-C₄) et alcoxy en (C₁-C₄),
l'aza-hétérocyclyle de 5 à 7 chaînons pouvant être substitué avec 1 à 4 substituants fluor,
et
le cycloalkyle en (C₃-C₇) pouvant être substitué avec 1 à 3 substituants choisis indépendamment les uns des autres dans le groupe constitué par halogène, alkyle en (C₁-C₄) et hydroxy,
R⁹ représentant hydrogène ou alkyle en (C₁-C₆), ou
R⁸ et R⁹ formant ensemble avec l'atome d'azote auquel ils sont reliés un hétérocycle de 3 à 8 chaînons,
l'hétérocycle de 3 à 8 chaînons pouvant être substitué avec 1 ou 2 substituants choisis indépendamment l'un de l'autre dans le groupe constitué par fluor, alkyle en (C₁-C₄), hydroxycarbonyle, alcoxycarbonyle en (C₁-C₄), hydroxy et amino,
l'alkyle en (C₁-C₄) pouvant être substitué avec hydroxycarbonyle, alcoxycarbonyle en (C₁-C₄), hydroxy ou amino,
R¹⁰ représentant hydrogène, alkyle en (C₁-C₆) ou cycloalkyle en (C₃-C₇),
le cycloalkyle en (C₃-C₇) pouvant être substitué avec 1 à 3 substituants choisis indépendamment les uns des autres dans le groupe constitué par alkyle en (C₁-C₆), hydroxy, trifluorométhyle et difluorométhyle,
et
l'alkyle en (C₁-C₆) pouvant être substitué avec 1 à 4 substituants choisis indépendamment les uns des autres dans le groupe constitué par fluor, cycloalkyle en (C₃-C₇), alcoxy en (C₁-C₄), hydroxy, amino, trifluorométhyle et difluorométhyle,
R¹¹ représentant hydrogène ou alkyle en (C₁-C₄),
ou
R¹⁰ et R¹¹ formant ensemble avec l'atome d'azote auquel ils sont reliés à hétérocycle de 3 à 7 chaînons,
l'hétérocycle de 3 à 7 chaînons pouvant être substitué avec 1 à 3 substituants choisis indépendamment les uns des autres dans le groupe constitué par fluor, alkyle en (C₁-C₄), hydroxy et amino,
l'alkyle en (C₁-C₄) pouvant être substitué avec hydroxy,
L représente un groupe #¹-CR^{13A}R^{13B}-(CR^{14A}R^{14B})ₘ-#²,
#¹ représentant l'emplacement de liaison au groupe carbonyle,
#² représentant l'emplacement de liaison au cycle pyrimidine ou triazine,
m représentant un nombre 0, 1 ou 2,
R^{13A} représentant hydrogène, trifluorométhyle ou alkyle en (C₁-C₄),
R^{13B} représentant hydrogène, difluorométhyle, trifluorométhyle, alkyle en (C₁-C₄) ou cycloalkyle en (C₃-C₇),
l'alkyle en (C₁-C₆) pouvant être substitué avec 1 à 3 substituants choisis indépendamment les uns des autres dans le groupe constitué par fluor, cyano, trifluorométhyle, cycloalkyle en (C₃-C₇), difluorométhoxy et trifluorométhoxy,
ou
R^{13A} et R^{13B} formant ensemble avec l'atome de carbone auquel ils sont reliés un carbocycle de 3 à 6 chaînons,
R^{14A} représentant hydrogène, fluor, alkyle en (C₁-C₄) ou hydroxy,
R^{14B} représentant hydrogène, fluor, alkyle en (C₁-C₄) ou trifluorométhyle,
ainsi que ses *N*-oxydes, sels, solvates, sels des *N-*oxydes et solvates des *N*-oxydes et des sels.

2. Composé de formule (I) selon la revendication 1, dans lequel
A représente CH₂,
R¹ représente phényle,
le phényle pouvant être substitué avec 1 à 4 substituants choisis indépendamment les uns des autres dans le groupe constitué par fluor, chlore, cyano et méthyle,
R² représente hydrogène, méthyle, éthyle ou cyclopropyle,
R³ représente hydrogène,
R⁴ représente hydrogène, fluor, chlore, méthyle ou éthyle,
R⁵ représente hydrogène,
E représente azote ou CR⁶,
R⁶ représentant hydrogène, chlore, iode, cyano, alkyle en (C₁-C₆), alcynyle en (C₂-C₆), cyclopropyle, hydroxy, -OR⁷, -NR⁸R⁹, hydroxycarbonyle, alcoxycarbonyle en (C₁-C₄), -C(=O)-NR¹⁰R¹¹ ou hétéroaryle à 5 chaînons,
l'alkyle en (C₁-C₆) pouvant être substitué avec 1 à 3 substituants choisis indépendamment les uns des autres dans le groupe constitué par fluor, difluorométhyle, trifluorométhyle, méthoxy, éthoxy, hydroxy, amino, -N(C=O)R¹², méthylsulfonylamino, cyclopropyle et cyclobutyle,
R¹² représentant cyclopropyle, cyclobutyle, méthyle ou éthyle,
l'alcynyle en (C₂-C₆) pouvant être substitué avec cyclopropyle ou cyclobutyle,
l'hétéroaryle à 5 chaînons pouvant être substitué avec chlore, méthyle, éthyle ou hydroxy,
R⁷ représentant alkyle en (C₁-C₄) ou pyrazolyle,
l'alkyle en (C₁-C₄) pouvant être substitué avec trifluorométhyle, méthoxy, hydroxy ou cyclopropyle,
R⁸ représentant hydrogène, alkyle en (C₁-C₄) ou cycloalkyle en (C₃-C₆),
le cycloalkyle en (C₃-C₆) pouvant être substitué avec 1 à 4 substituants choisis indépendamment les uns des autres dans le groupe constitué par méthyle, éthyle ou hydroxy,
et
l'alkyle en (C₁-C₄) pouvant être substitué avec 1 à 4 substituants choisis indépendamment les uns des autres dans le groupe constitué par fluor, alkyle en (C₁-C₄), cycloalkyle en (C₃-C₅), pyrrolidinyle, pipéridinyle, méthoxy, éthoxy, hydroxy, amino, trifluorométhyle, difluorométhyle, monofluorométhyle et phényle,
le phényle pouvant être substitué avec 1 à 3 substituants choisis indépendamment les uns des autres dans le groupe constitué par fluor, chlore, cyano et méthoxy,
le pyrrolidinyle et le pipéridinyle pouvant être substitués deux fois avec fluor,
et
le cycloalkyle en (C₃-C₇) pouvant être substitué avec hydroxy,
R⁹ représentant hydrogène ou méthyle,
ou
R⁸ et R⁹ formant ensemble avec l'atome d'azote auquel ils sont reliés un hétérocycle de 4 à 7 chaînons,
l'hétérocycle de 4 à 7 chaînons pouvant être substitué avec 1 ou 2 substituants choisis indépendamment l'un de l'autre dans le groupe constitué par alkyle en (C₁-C₄), hydroxycarbonyle, hydroxy et amino,
l'alkyle en (C₁-C₄) pouvant être substitué avec hydroxycarbonyle, hydroxy ou amino,
R¹⁰ représentant hydrogène, alkyle en (C₁-C₄) ou cycloalkyle en (C₃-C₆),
le cycloalkyle en (C₃-C₆) pouvant être substitué avec 1 à 3 substituants choisis indépendamment les uns des autres dans le groupe constitué par méthyle, éthyle ou hydroxy,
et
l'alkyle en (C₁-C₄) pouvant être substitué avec 1 ou 2 substituants choisis indépendamment l'un de l'autre dans le groupe constitué par fluor, hydroxy, amino, trifluorométhyle et difluorométhyle,
R¹¹ représentant hydrogène ou méthyle,
ou
R¹⁰ et R¹¹ formant ensemble avec l'atome d'azote auquel ils sont reliés à hétérocycle de 4 à 6 chaînons,
l'hétérocycle de 4 à 6 chaînons pouvant être substitué avec 1 à 3 substituants choisis indépendamment les uns des autres dans le groupe constitué par fluor, méthyle, éthyle, hydroxy et amino,
le méthyle et l'éthyle pouvant être substitués avec hydroxy,
L représente un groupe #¹-CR^{13A}R^{13B}-(CR^{14A}R^{14B})ₘ-#²,
#¹ représentant l'emplacement de liaison au groupe carbonyle,
#² représentant l'emplacement de liaison au cycle pyrimidine ou triazine,
m représentant un nombre 0,
R^{13A} représentant hydrogène ou méthyle,
R^{13B} représentant hydrogène, difluorométhyle, trifluorométhyle ou méthyle,
ou
R^{13A} et R^{13B} formant ensemble avec l'atome de carbone auquel ils sont reliés un cycle cyclopropyle, ainsi que ses *N*-oxydes, sels, solvates, sels des *N-*oxydes et solvates des *N*-oxydes et des sels.

3. Composé de formule (I) selon la revendication 1 ou 2, dans lequel
A représente CH₂,
R¹ représente un groupe phényle de formule 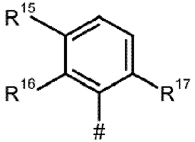 dans laquelle
# représente l'emplacement de liaison à A,
et
R¹⁵ représente hydrogène ou fluor,
R¹⁶ et R¹⁷ représentent fluor,
R² représente méthyle,
R³ représente hydrogène,
R⁴ représente hydrogène, chlore ou méthyle,
R⁵ représente hydrogène,
E représente azote ou CR⁶,
R⁶ représentant hydrogène, chlore, éthynyle, hydroxy, -OR⁷, -NR⁸R⁹, -C(=O)-NR¹⁰R¹¹, 1H-pyrazol-1-yle ou 1,3-thiazol-5-yle,
l'éthynyle étant substitué avec cyclopropyle,
le 1H-pyrazol-1-yle et le 1,3-thiazol-5-yle pouvant être substitués avec méthyle, éthyle ou hydroxy,
R⁷ représentant méthyle, éthyle ou 1H-pyrazol-4-yle,
le méthyle pouvant être substitué avec cyclopropyle,
l'éthyle pouvant être substitué avec trifluorométhyle, méthoxy ou hydroxy,
R⁸ représentant hydrogène, éthyle, propyle ou cycloalkyle en (C₄-C₆),
le cycloalkyle en (C₄-C₆) pouvant être substitué avec 1 ou 2 substituants méthyle ou hydroxy,
et
l'éthyle et le propyle pouvant être substitués avec 1 à 3 substituants choisis indépendamment les uns des autres dans le groupe constitué par fluor, méthyle, éthyle, propyle, cyclopropyle, méthoxy, hydroxy, amino, trifluorométhyle, difluorométhyle, monofluorométhyle et phényle,
le phényle pouvant être substitué avec 1 ou 2 substituants choisis indépendamment l'un de l'autre dans le groupe constitué par fluor, chlore et méthoxy,
et
le cycloalkyle en (C₄-C₇) pouvant être substitué avec hydroxy,
R⁹ représentant hydrogène,
ou
R⁸ et R⁹ formant ensemble avec l'atome d'azote auquel ils sont reliés un cycle pipéridinyle, pyrrolidinyle ou 3-azabicyclo[3.1.0]hex-3-yle,
les cycles pipéridinyle et pyrrolidinyle pouvant être substitués avec méthyle,
le méthyle pouvant être substitué avec hydroxycarbonyle ou hydroxy,
et
le cycle 3-azabicyclo[3.1.0]hex-3-yle pouvant être substitué avec amino,
R¹⁰ représentant hydrogène, méthyle, éthyle, n-propyle ou cyclopropyle,
le méthyle, l'éthyle, le n-propyle pouvant être substitués avec 1 ou 2 substituants choisis indépendamment l'un de l'autre dans le groupe constitué par fluor, amino et trifluorométhyle,
R¹¹ représentant hydrogène,
ou
R¹⁰ et R¹¹ formant ensemble avec l'atome d'azote auquel ils sont reliés un cycle pyrrolidinyle, pipéridinyle ou pipérazinyle,
le cycle pyrrolidinyle pouvant être substitué avec 1 à 3 substituants choisis indépendamment les uns des autres dans le groupe constitué par fluor, méthyle, éthyle, hydroxy et amino,
le méthyle pouvant être substitué avec hydroxy,
le cycle pipérazinyle pouvant être substitué avec méthyle sur l'atome d'azote,
L représente un groupe #¹-CR^{13A}R^{13B}-#²,
#¹ représentant l'emplacement de liaison au groupe carbonyle,
#² représentant l'emplacement de liaison au cycle pyrimidine ou triazine,
R^{13A} représentant méthyle,
R^{13B} représentant trifluorométhyle ou méthyle,
ainsi que ses *N*-oxydes, sels, solvates, sels des *N-*oxydes et solvates des *N*-oxydes et des sels.

4. Procédé de fabrication de composés de formule (I), tels que définis dans les revendications 1 à 3,
**caractérisé en ce que**
un composé de formule (II) 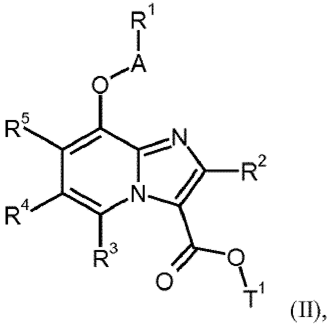 dans laquelle A, R¹, R², R³, R⁴, R⁵ ont chacun les significations indiquées précédemment et
T¹ représente alkyle en (C₁-C₄) ou benzyle,
est mis en réaction dans un solvant inerte en présence d'une base ou d'un acide approprié pour former un acide carboxylique de formule (III) 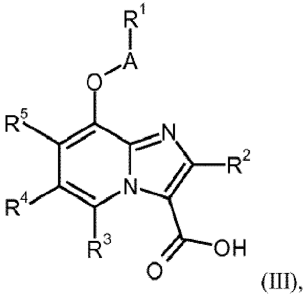 dans laquelle A, R¹, R², R³, R⁴ et R⁵ ont chacun les significations indiquées précédemment,
et celui-ci est ensuite transformé dans un solvant inerte en conditions de couplage d'amide avec un sel d'ammonium en un composé de formule (IV) 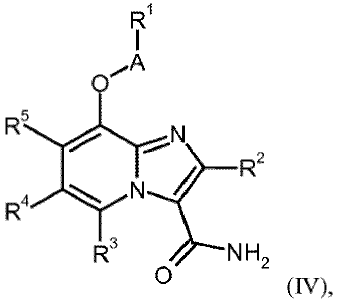 dans laquelle A, R¹, R², R³, R⁴ et R⁵ ont chacun les significations indiquées précédemment, puis celui-ci est mis en réaction dans un solvant inerte avec de l'anhydride d'acide trifluoroacétique pour former un composé de formule (V) 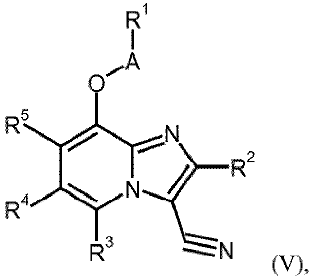 dans laquelle A, R¹, R², R³, R⁴ et R⁵ ont chacun les significations indiquées précédemment, celui-ci est transformé en présence d'un réactif d'alkylaluminium dans un solvant inerte en une amidine de formule (VI) 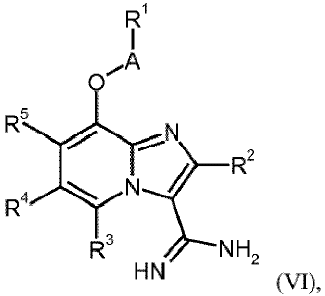 dans laquelle A, R¹, R², R³, R⁴ et R⁵ ont chacun les significations indiquées précédemment,
ou
un composé de formule (V) est transformé dans un solvant approprié en présence d'une base appropriée avec du chlorhydrate d'hydroxylamine tout d'abord en un composé de formule (VIa) 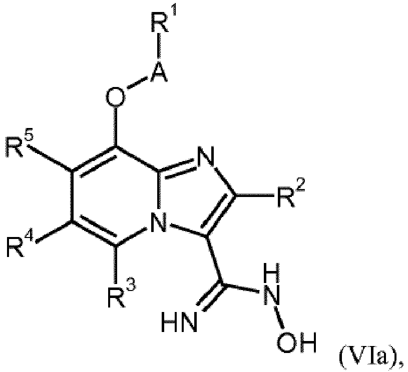 dans laquelle A, R¹, R², R³, R⁴ et R⁵ ont chacun les significations indiquées précédemment, puis celui-ci est transformé par hydrogénolyse en présence d'un catalyseur de palladium, tel que par exemple du palladium sur du charbon actif, dans un solvant inerte, tel que par exemple de l'éthanol ou de l'ester éthylique de l'acide acétique, en une amidine de formule (VI),
celle-ci est mise en réaction dans un solvant inerte en présence d'une base appropriée avec un composé de formule (VII) 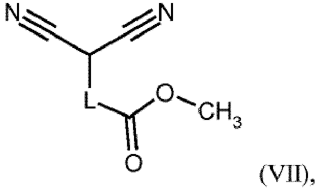 pour former un composé de formule (VIII) 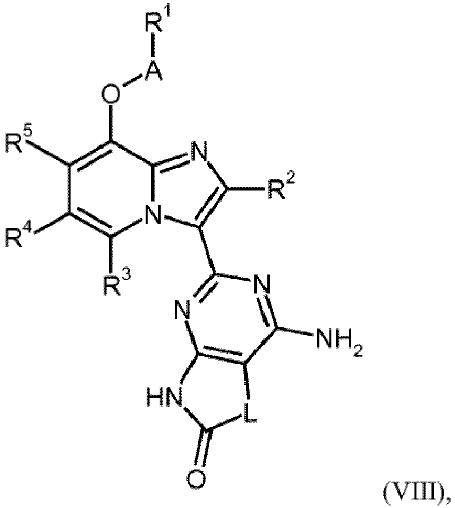 dans laquelle A, R¹, R², R³, R⁴, R⁵ et L ont chacun les significations indiquées précédemment, le groupe amino est transformé dans un solvant inerte avec de l'isopentylnitrite et un équivalent d'halogène en un composé de formule (IX) 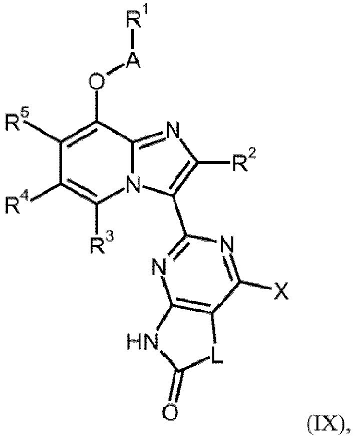 dans laquelle A, R¹, R², R³, R⁴, R⁵ et L ont chacun les significations indiquées précédemment,
et
X représente chlore, brome ou iode,
et celui-ci
[A] est ensuite mis en réaction dans un solvant inerte éventuellement en présence d'une base appropriée avec un composé de formule (X) 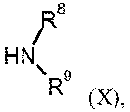 dans laquelle R⁸ et R⁹ ont les significations indiquées précédemment,
pour former un composé de formule (I-A) 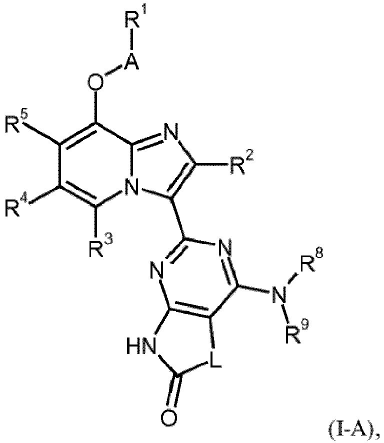 dans laquelle A, R¹, R², R³, R⁴, R⁵, R⁸, R⁹ et L ont chacun les significations indiquées précédemment,
ou
[B] l'iodure de formule (IX) est mis en réaction dans un solvant inerte éventuellement en présence d'une base appropriée et d'iodure de cuivre (I) avec un composé de formule (XI)
HO-R⁷ (XI)
dans laquelle R⁷ a la signification indiquée précédemment,
pour former un composé de formule (I-B) 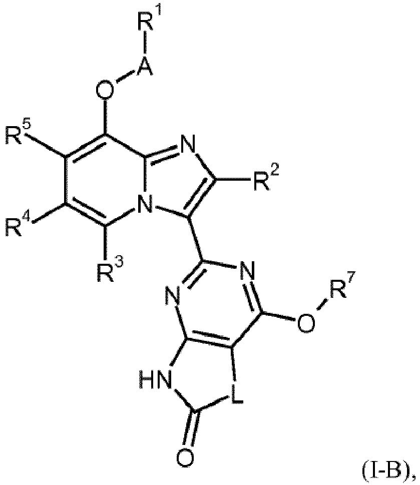 dans laquelle A, R¹, R², R³, R⁴, R⁵, R⁷ et L ont chacun les significations indiquées précédemment,
ou
[C] l'iodure de formule (IX) est mis en réaction dans un solvant inerte éventuellement en présence d'une base appropriée avec du cyanure de cuivre (I) pour former un composé de formule (I-C) 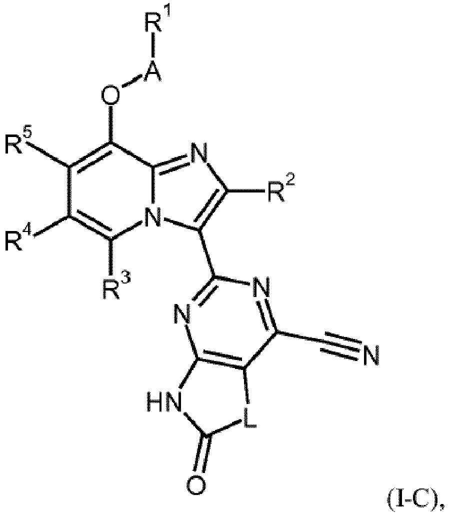 dans laquelle A, R¹, R², R³, R⁴, R⁵ et L ont chacun les significations indiquées précédemment, et celui-ci est transformé dans un solvant inerte avec une base aqueuse appropriée en un composé de formule (I-D) 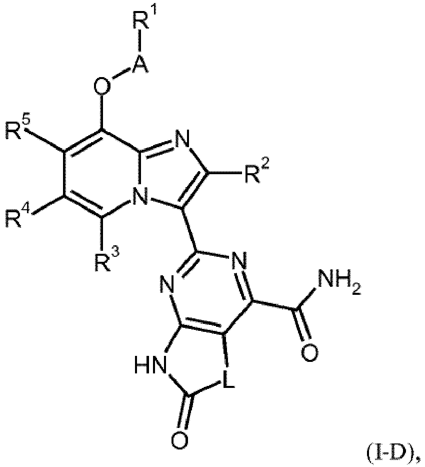 dans laquelle A, R¹, R², R³, R⁴, R⁵ et L ont chacun les significations indiquées précédemment, puis celui-ci est transformé dans un solvant inerte avec un acide aqueux approprié en un acide de formule (I-E) 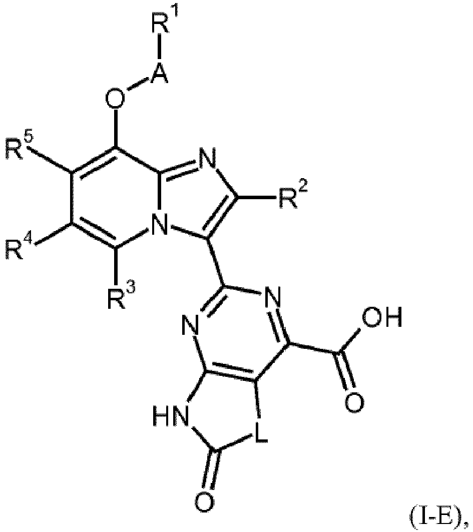 dans laquelle A, R¹, R², R³, R⁴, R⁵ et L ont chacun les significations indiquées précédemment, puis celui-ci est transformé dans un solvant inerte en conditions de couplage d'amide avec une amine de formule (XII) 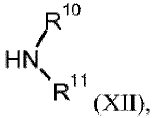 dans laquelle R¹⁰ et R¹¹ ont chacun les significations indiquées précédemment, en un composé de formule (I-F) 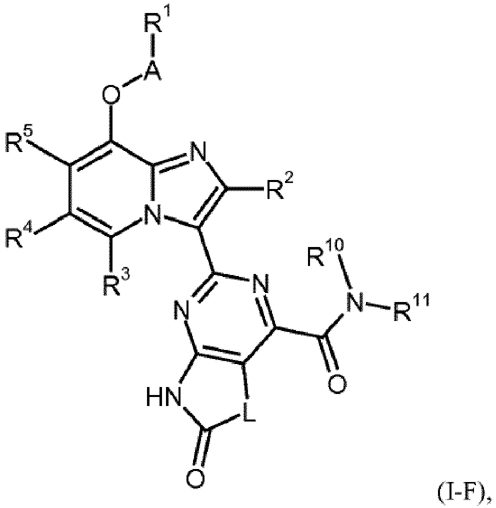 dans laquelle A, R¹, R², R³, R⁴, R⁵, R¹⁰, R¹¹ et L ont chacun les significations indiquées précédemment,
et les composés de formule (I) résultants sont éventuellement transformés avec (i) les solvants et/ou (ii) les acides ou les bases appropriés en leurs solvates, sels et/ou solvates des sels.

5. Composé de formule (I), tel que défini dans l'une quelconque des revendications 1 à 3, destiné à une utilisation lors du traitement et/ou de la prophylaxie de maladies.

6. Utilisation d'un composé de formule (I), tel que défini dans l'une quelconque des revendications 1 à 3, pour la fabrication d'un médicament pour le traitement et/ou la prophylaxie de l'insuffisance cardiaque, de l'angine de poitrine, de l'hypertension, de l'hypertension pulmonaire, des ischémies, des maladies vasculaires, de l'insuffisance rénale, des maladies thromboemboliques, des maladies fibrotiques, de l'artériosclérose, des démences et du dysfonctionnement érectile.

7. Médicament, contenant un composé de formule (I), tel que défini dans l'une quelconque des revendications 1 à 3, en combinaison avec un adjuvant inerte, non toxique, pharmaceutiquement approprié.

8. Médicament, contenant un composé de formule (I), tel que défini dans l'une quelconque des revendications 1 à 3, en combinaison avec un autre agent actif choisi dans le groupe constitué par les nitrates organiques, les donneurs de NO, les inhibiteurs de cGMP-PDE, les agents à effet antithrombotique, les agents abaissant la tension artérielle, ainsi que les agents modifiant le métabolisme lipidique.

9. Médicament selon la revendication 7 ou 8, destiné à une utilisation lors du traitement et/ou de la prophylaxie de l'insuffisance cardiaque, de l'angine de poitrine, de l'hypertension, de l'hypertension pulmonaire, des ischémies, des maladies vasculaires, de l'insuffisance rénale, des maladies thromboemboliques, des maladies fibrotiques, de l'artériosclérose, des démences et du dysfonctionnement érectile.
